# EUROPEAN PATENT SPECIFICATION

(11) **EP 1 691 789 B1**
(45) Date of publication and mention of the grant of the patent: **20.12.2017**
(21) Application number: 04812185.9
(22) Date of filing: 24.11.2004
(51) Int. Cl.: A61K 9/20, A61K 9/16, A61K 9/50, A61K 31/403

(54) **CARVEDILOL FREE BASE, SALTS, ANHYDROUS FORMS OR SOLVATE THEREOF, CORRESPONDING PHARMACEUTICAL COMPOSITIONS, CONTROLLED RELEASE FORMULATIONS, AND TREATMENT OR DELIVERY METHODS**
CARVEDILOL-FREIE BASE, SALZE, WASSERFREIE FORMEN ODER SOLVATE DAVON, ENTSPRECHENDE PHARMAZEUTISCHE ZUSAMMENSETZUNGEN, FORMULIERUNGEN MIT KONTROLLIERTER FREISETZUNG UND BEHANDLUNGS- ODER ABGABEVERFAHREN
BASE LIBRE DE CARVEDILOL, SES SELS, FORMES ANHYDRES OU SOLVATES, COMPOSITIONS PHARMACEUTIQUES CORRESPONDANTES, FORMULATIONS A LIBERATION CONTROLEE, ET PROCEDES DE TRAITEMENT OU D'ADMINISTRATION

(30) Priority: 25.11.2003 US 524991 P; 30.08.2004 US 605680 P
(43) Date of publication of application: 23.08.2006
(73) Proprietor: SmithKline Beecham (Cork) Limited, Carrigaline Cork (IE); FLAMEL TECHNOLOGIES, 69693 Venissieux Cédex (FR)
(72) Inventor: CASTAN, Catherine, F-69693 Venissieux Cedex (FR); CROWLEY, Patrick, J., Collegeville, PA 19426 (US); GUIMBERTEAU, Florence, F-69693 Venissieux Cedex (FR); MEYRUEIX, Remi, F-69693 Venissieux Cedex (FR); OH, Choon, Collegeville, PA 19426 (US); SOULA, Gérard, F-69693 Venissieux Cedex (FR)
(74) Representative: Shore, Andrew David
(86) International application number: PCT/US2004/039614
(87) International publication number: WO 2005/051322

(56) References cited:
- WO-A1-99/24017
- WO-A1-03/097018
- WO-A2-02/065834
- US-A- 5 286 497
- US-A- 6 056 968
- US-A1- 2003 035 836
- US-B1- 6 515 010
- US-B2- 6 491 949

## Description

### Field of the Invention

The present invention relates to carvedilol phosphate salts, anhydrous forms, or solvates thereof, corresponding pharmaceutical compositions or controlled release formulations, delivery methods of carvedilol forms to the gastrointestingal tract or methods to treat cardiovascular diseases, which may include, but are not limited to hypertension, congestive heart failure, atherosclerosis, and angina.

The present invention relates to controlled release formulations, which comprise various cavedilol forms, which may include, but are not limited to carvedilol phosphate salts, anhydrous forms or solvates thereof.

### Background of the Invention

### Carvedilol

The compound, 1-(carbazol-4-yloxy-3-[[2-(o-methoxyphenoxy) ethyl]-amino]-2-propanol is known as Carvedilol. Carvedilol is depicted by the following chemical structure:

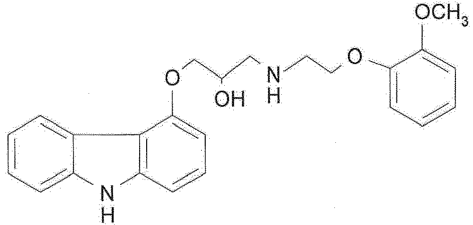

Carvedilol is disclosed in U.S. Patent No. 4,503,067 to Wiedemann et al. (i.e., assigned to Boehringer Mannheim, GmbH, Mannheim-Waldhof, Fed. Rep. of Germany), which was issued on March 5, 1985.

Currently, carvedilol is synthesized as free base for incorporation in medication that is available commercially. The aforementioned free base form of carvedilol is a racemic mixture of R(+) and S(-) enantiomers, where non-selective β-adrenoreceptor blocking activity is exhibited by the S(-) enantiomer and α-adrenergic blocking activity is exhibited by both R(+) and S(-) enantiomers. Those unique features or characteristics associated with such a racemic carvedilol mixture contributes to two complementary pharmacologic actions: i.e., mixed venous and arterial vasodilation and non-cardioselective, beta-adrenergic blockade.

Carvedilol is used for treatment of hypertension, congestive heart failure, and angina.

The currently commercially available carvedilol product is a conventional, tablet prescribed as a twice-a-day (BID) medication in the United States. The commercially available carvedilol formulation is in an immediate release or rapidly releasing carvedilol in its free base form, where the nature or the chemical and physical formulation properties are such that by the time the carvedilol leaves the stomach, it is either in solution or it is in the form of a suspension of fine particles, i.e. a form from which carvedilol can be readily absorbed.

Carvedilol contains an α-hydroxyl secondary amine functional group, which has a pKa of 7.8. Carvedilol exhibits predictable solubility behaviour in neutral or alkaline media, i.e. above a pH of 9.0, the solubility of carvedilol is relatively low (< 1 µg/mL). The solubility of carvedilol increases with decreasing pH and reaches a plateau near pH = 5, i.e. where saturation solubility is about 23 µg/mL at pH = 7 and about 100 µg/mL at pH = 5 at room temperature. At lower pH values (i.e., at a pH of 1 to 4 in various buffer systems), solubility of carvedilol is limited by the solubility of its protonated form or its corresponding salt formed *in-situ.* For example, a hydrochloride salt of carvedilol generated *in situ* an acidic medium, which simulates gastric fluid, is less soluble in such medium.

In addition, the presence of the α-hydroxyl secondary amine group in the carvedilol chemical structure confers a propensity upon the compound to chemically react with excipients normally included in a dosage form to aid manufacture, maintain quality, or enhances dissolution rate. For example, the α-hydroxyl secondary amine group of carvedilol can react with aldehydes or ester functional groups through nucleophilic reactions. Common chemical functional group residues associated with conventionally used excipients, include ester, aldehyde or other chemical residue functional groups. This often results in marginal or unacceptable chemical stability upon storage.

### Pharmaceutical Compositions/Formulations and Controlled-Release Technology

In the medical treatment of mammals, a desire to maintain a constant concentration of a pharmaceutical composition within the blood stream of human or animal patient, is dependent upon regular administration of such a composition, such as in an oral tablet or capsule form. Regularity of oral administration of various drug dosage forms is important as a typical pharmaceutical composition form is released immediately upon dissolution in a recipients stomach. Thus, any interruption in a patient's tablet supply regimen causes a consequent drug or pharmaceutical concentration reduction in the patient's blood.

Therefore, for ease of patient use, it is often desirable to maintain a controlled concentration of an pharmaceutical active drug agent or composition at a predetermined site for an extended period of time.

The use of controlled release technology allows for release of a pharmaceutical composition at a constant rate at a desired concentration into a patient's system over many hours. For example, if a controlled release tablet contains a sufficient drug or composition amount to maintain a desired concentration for twelve or more hours, there would be no need for a patient to take tablets frequently and would reduce interrupting a patient's drug regime.

As conventionally known in the art, many different examples have been developed to accomplish such results.

For example, U.S. Pat. No. 3,845,770 to Theeuwes et al. teaches a device that provides a controlled release via a core tablet including an active agent coated with a semipermeable membrane permeable only to a fluid present in the environment of use (i.e., water), where the active agent or another component of the core tablet exhibits osmotic activity and the rate of release is dependent upon the permeability of the semipermeable membrane.

U.S. Pat. No. 4,624,847 to Ayer et al. describes an osmotic dispensing device, where a drug mixed with an osmopolymer and/or osmagent is in a compartment surrounded by a semipermeable wall with an osmotic passageway to the compartment. Other patents describing various osmotic dispensing devices include: U.S. Pat. No. 4,519,801 to Edgren; U.S. Pat. No. 4,111,203 to Theeuwes; U.S. Pat. No. 4,777,049 to Magruder et al.; U.S. Pat. No. 4,612,008 to Wong et al.; U.S. Pat. No. 4,610,686 to Ayer et al.; U.S. Pat. No. 4,036,227 to Zaffaroni et al.; U.S. Pat. No. 4,553,973 to Edgren; U.S. Pat. No. 4,077,407 to Theeuwes et al.; and U.S. Pat. No. 4,609,374 to Ayer.

U.S. Pat. No. 4,218,433 to Kooichi et al. describes a tablet with a water insoluble coating and a water soluble component that releases an active component at a constant rate due to an indentation formed on its surface.

U.S. Pat. No. 4,687,660 to Baker et al. describes an osmotic dispensing device without a preformed single passageway to release water-soluble drugs, where based upon an osmotic gradient formed from water insoluble film coated core containing a drug is combined with excipient and an osmotic enhancing agent.

U.S. Pat. No. 4,816,262 to McMullen relates to a controlled release disc-like configured tablet with a centrally extending cylindrical hole that allows for zero order or constant release of the active agent.

Devices with an impermeable coating covering various portions of the device include: U.S. Pat. No. 4,814,183 to Zentner relates to a controlled release device with a charged resin core encased in a water insoluble semipermeable material that is impermeable to core components, but permeable to the passage of an external fluid in the environment of use. U.S. Pat. No. 4,814,182 to Graham et al. describes a controlled release device which comprises an active ingredient/hydrogel mixture with at least one surface of the device having a coating impermeable to aqueous media. U.S. Pat. No. 4,792,448 to Ranade relates to a cylindrical tablet or bolus with a impermeable coated core having an active ingredient blended with inert excipients and formed into a cylindrical tablet preferably having a flat cylindrical side and a convex top and bottom.

Moreover, numerous prior art references also describe producing alternate sustained-release systems, with the aim of providing medicinal forms, which may be taken once a day, to prolong the action of a medicinal product. (see, for example, Formes Pharmaceutiques Nouvelles, Buri, Puisieux, Doelker et Benoit, Lavoisier 1985, pages I75-227).

These include monolithic systems, where dose to be administered is in the form of a solid object, such as a tablet. DE Pat. Appn. No. 39 43 242 (FR No. 2 670 112) discloses "matrix" type granules, which comprise active particles ("AP") and inert excipent(s), useful for making tablets. Such granules, which are distinct from microcapsules, consist of a multitude of particles included in a roughly spherical matrix comprising a cellulosic polymer, a vinylic or acrylic polymer, a plasticizer and a lubricating agent.

U.S. Pat. No. 4,461,759 to Dunn describes a oral solid dosage coated tablet, which includes active particles ("AP") protected from harmful effects of stomach acidity that are released at a constant rate in the gastrointestinal tract.

U.S. Pat. No. 5,028,434 to Barclay et al. and Inter.I' Appln. No. WO 91/16885 describes a monolithic tablet form using a microporous film coating that allows controlled release of active particles via osmotic pressure.

Other literature examples of microparticulate pharmaceutical systems giving a sustained release of active particles ("AP" or "AP's") include: U.S. Pat. No. 5,286,497 to Hendrickson et al., which relates to a once a day controlled release diltiazem formulation, which contains a blend of rapid release bead and delayed release coated diltiazem beads with different dissolution rates.

Consequently, the short residence time in the small intestine poses a considerable problem to those skilled in the art interested in developing sustained-absorption medicinal products intended for oral administration. The medicinal product administered orally is, in effect, subject to the natural transit of the gastrointestinal tract, thereby limiting its residence time. Now, the small intestine is the preferred location for systemic absorption and it represents the ideal site for making APs available. Thus, it is easy to appreciate the value of a pharmaceutical form having an increased residence time in the small intestine, in view of the sustained in vivo absorption of an AP, beyond normal transit time in the small intestine.

Many studies have been performed regarding the time for gastrointestinal transit. These studies show that the duration of gastric transit is very variable, in particular as a function of feeding, and that it is between a few minutes and a few hours. On the other hand, the duration of transit in the small intestine is particularly constant and, more precisely, is 3 hours plus or minus one hour (see for example S. S. Davis: Assessment of gastrointestinal transit and drug absorption, in Noval drug delivery and its therapeutic application, Ed L. F. Prescott-W.S. Nimmo, 1989, J. Wiley & Son, page 89-101).

In light of the foregoing, novel carvedilol salt, solvate, or anhydrous forms thereof, corresponding pharmaceutical compositions or controlled release formulations containing carvedilol free base or novel carvedilol salt, solvate, or anhydrous forms thereof, with greater aqueous solubility, chemical stability, prolonged residence time, absorption in the gastrointestingal tract, especially such as in the small intestine, etc. would offer many potential benefits for provision of medicinal products containing the drug carvedilol.

Examples of such benefits would include products with the ability to achieve desired or prolonged drug levels in a systemic system by sustaining absorption along the gastro-intestinal tract of mammals (i.e., such as humans), particularly in regions of neutral pH, where a drug, such as carvedilol, has minimal solubility.

Surprisingly, it has now been shown that novel forms of carvedilol salts, anhydrous forms or solvates thereof, which may isolated as, but not limited to crystalline or other solid forms, exhibit much higher aqueous solubility than the corresponding free base or other prepared carvedilol salts, which may include, but are not limited to crystalline forms or other solid forms.

Such carvedilol salts, anhydrous forms or solvates thereof, which may include, but are not limited to crystalline forms or other solid forms, also have potential to improve the stability of carvedilol in pharmaceutical compositions or controlled-release formulations due to the fact that the secondary amine functional group attached to the carvedilol core structure, a moiety pivotal to degradation processes, is protonated as a salt.

Such carvedilol salts, anhydrous forms or solvates thereof, which may include, but are not limited to crystalline forms or other solid forms, in pharmaceutical compositions or controlled-release formulations also have potential to lead to prolonged residence, absorption time, and/or good tolerance levels in the gastrointestinal tract, such as the small intestine, colon, etc.

In light of the above, a need exists to develop carvedilol free base or different carvedilol salts, anhydrous forms or solvates forms thereof, different corresponding compositions or controlled release formulations, respectively, which have greater aqueous solubility, chemical stability, good tolerance levels, sustained or prolonged drug or absorption properties or transit levels (i.e., such as in neutral gastrointestinal tract pH regions, etc.).

There also exists a need to develop methods of delivery of carvedilol (such as carvedilol free base or as a carvedilol salt, solvate or anhydrous form thereof) to the gastrointestinal tract or methods of treatment for cardiovascular diseases or associated disorders, which may include, but are not limited to hypertension, congestive heart failure, atherosclerosis or angina, etc., which comprises administration of the such carvedilol free base, carvedilol salt, anhydrous forms or solvate forms thereof, corresponding pharmaceutical compositions, or controlled release dosage formulations.

The present invention is directed to overcoming these and other problems encountered in the art.

### Summary of the Invention

The present invention relates to carvedilol phosphate salts, anhydrous forms, or solvates thereof, corresponding pharmaceutical compositions or controlled release formulations, and delivery methods of carvedilol forms to the gastrointestingal tract or methods to treat cardiovascular diseases, which may include, but are not limited to hypertension, congestive heart failure, atherosclerosis, and angina.

The present invention generally relates to control release formulations, which comprise carvedilol phosphate salts, anhydrous forms or solvates thereof.

In particular, the present invention relates to a controlled release formulation, which comprises a carvedilol phosphate salt, solvate or anhydrous form; where the aforementioned controlled release formulation following oral dosage exhibits a substantially biphasic plasma profile with a first plasma concentration peak level and a first Tₘₐₓ pulse occurring within 1-4 hours of ingestion and a second a plasma concentration peak level and second Tₘₐₓ pulse occurring within 5-8 hours after ingestion.

### Brief Description of the Figures

### Carvedilol Phosphate Salts

Figure 1 is an x-ray powder diffractogram for carvedilol dihydrogen phosphate hemihydrate (Form I).
Figure 2 shows the thermal analysis results for carvedilol dihydrogen phosphate hemihydrate (Form I).
Figure 3 is an FT-Raman spectrum for carvedilol dihydrogen phosphate hemihydrate (Form I).
Figure 4 is an FT-Raman spectrum for carvedilol dihydrogen phosphate hemihydrate in the 4000-2000 cm⁻¹ region of the spectrum (Form I).
Figure 5 is an FT-Raman spectrum for carvedilol dihydrogen phosphate hemihydrate in the 2000-400 cm⁻¹ region of the spectrum (Form I).
Figure 6 is an FT-IR spectrum for carvedilol dihydrogen phosphate hemihydrate (Form I).
Figure 7 is an FT-IR spectrum for carvedilol dihydrogen phosphate hemihydrate in the 4000-2000 cm⁻¹ region of the spectrum (Form I).
Figure 8 is an FT-IR spectrum for carvedilol dihydrogen phosphate hemihydrate in the 2000-500 cm⁻¹ region of the spectrum (Form I).
Figure 9 is an x-ray powder diffractogram for carvedilol dihydrogen phosphate dihydrate (Form II).
Figure 10 shows the thermal analysis results for carvedilol dihydrogen phosphate dihydrate (Form II).
Figure 11 is an FT-Raman spectrum for carvedilol dihydrogen phosphate dihydrate (Form II).
Figure 12 is an FT-Raman spectrum for carvedilol dihydrogen phosphate dihydrate in the 4000-2000 cm⁻¹ region of the spectrum (Form II).
Figure 13 is an FT-Raman spectrum for carvedilol dihydrogen phosphate dihydrate in the 2000-400 cm⁻¹ region of the spectrum (Form II).
Figure 14 is an FT-IR spectrum for carvedilol dihydrogen phosphate dihydrate (Form II).
Figure 15 is an FT-IR spectrum for carvedilol dihydrogen phosphate dihydrate in the 4000-2000 cm⁻¹ region of the spectrum (Form II).
Figure 16 is an FT-IR spectrum for carvedilol dihydrogen phosphate dihydrate in the 2000-500 cm⁻¹ region of the spectrum (Form II).
Figure 17 shows the thermal analysis results for carvedilol dihydrogen phosphate methanol solvate (Form III).
Figure 18 is an FT-Raman spectrum for carvedilol dihydrogen phosphate methanol solvate (Form III).
Figure 19 is an FT-Raman spectrum for carvedilol dihydrogen phosphate methanol solvate in the 4000-2000 cm⁻¹ region of the spectrum (Form III).
Figure 20 is an FT-Raman spectrum for carvedilol dihydrogen phosphate methanol solvate in the 2000-400 cm⁻¹ region of the spectrum (Form III).
Figure 21 is an FT-IR spectrum for carvedilol dihydrogen phosphate methanol solvate (Form III).
Figure 22 is an FT-IR spectrum for carvedilol dihydrogen phosphate methanol solvate in the 4000-2000 cm⁻¹ region of the spectrum (Form III).
Figure 23 is an FT-IR spectrum for carvedilol dihydrogen phosphate methanol solvate in the 2000-500 cm⁻¹ region of the spectrum (Form III).
Figure 24 is an x-ray powder diffractogram for carvedilol dihydrogen phosphate methanol solvate (Form III).
Figure 25 is an x-ray powder diffractogram for carvedilol dihydrogen phosphate dihydrate (Form IV).
Figure 26 is a solid state ¹³C NMR for carvedilol dihydrogen phosphate dihydrate (Form I).
Figure 27 is a solid state ³¹P NMR for carvedilol dihydrogen phosphate dihydrate (Form I).
Figure 28 is an x-ray powder diffractogram for carvedilol dihydrogen phosphate (Form V).
Figure 29 is an x-ray powder diffractogram for carvedilol hydrogen phosphate (Form VI).

### Carvedilol HBr Salts

Figure 30 is an x-ray powder diffractogram for carvedilol hydrobromide monohydrate.
Figure 31 is a differential scanning calorimetry thermogram for carvedilol hydrobromide monohydrate.
Figure 32 is an FT-Raman spectrum for carvedilol hydrobromide monohydrate.
Figure 33 is an FT-Raman spectrum for carvedilol hydrobromide monohydrate in the 4000-2000 cm⁻¹ region of the spectrum.
Figure 34 is an FT-Raman spectrum for carvedilol hydrobromide monohydrate in the 2000-400 cm⁻¹ region of the spectrum.
Figure 35 is an FT-IR spectrum for carvedilol hydrobromide monohydrate.
Figure 36 is an FT-IR spectrum for carvedilol hydrobromide monohydrate in the 4000-2000 cm⁻¹ region of the spectrum.
Figure 37 is an FT-IR spectrum for carvedilol hydrobromide monohydrate in the 2000-500 cm⁻¹ region of the spectrum.
Figure 38 is a view of a single molecule of carvedilol hydrobromide monohydrate. The hydroxyl group and the water molecule are disordered.
Figure 39 are views of molecules of carvedilol hydrobromide monohydrate showing the N-H···Br···H-N interactions. The top view focuses on Br1 and the bottom view focuses on Br2. The interaction between the carvedilol cation and the bromine anion is unusual. Each carvedilol molecule makes two chemically different contacts to the bromine anions. Each bromine anion sits on a crystallographic special position (that is, on a crystallographic two-fold axis) which means that there are two half bromine anions interacting with each carvedilol cation.
Figure 40 is a differential scanning calorimetry thermogram for carvedilol hydrobromide dioxane solvate.
Figure 41 is an FT-Raman spectrum for carvedilol hydrobromide dioxane solvate.
Figure 42 is an FT-Raman spectrum for carvedilol hydrobromide dioxane solvate in the 4000-2000 cm⁻¹ region of the spectrum.
Figure 43 is an FT-Raman spectrum for carvedilol hydrobromide dioxane solvate in the 2000-400 cm⁻¹ region of the spectrum.
Figure 44 is an FT-IR spectrum for carvedilol hydrobromide dioxane solvate.
Figure 45 is an FT-IR spectrum for carvedilol hydrobromide dioxane solvate in the 4000-2000 cm⁻¹ region of the spectrum.
Figure 46 is an FT-IR spectrum for carvedilol hydrobromide dioxane solvate in the 2000-500 cm⁻¹ region of the spectrum.
Figure 47 is a differential scanning calorimetry thermogram for carvedilol hydrobromide 1-pentanol solvate.
Figure 48 is an FT-Raman spectrum for carvedilol hydrobromide 1-pentanol solvate.
Figure 49 is an FT-Raman spectrum for carvedilol hydrobromide 1-pentanol solvate in the 4000-2000 cm⁻¹ region of the spectrum.
Figure 50 is an FT-Raman spectrum for carvedilol hydrobromide 1-pentanol solvate in the 2000-400 cm⁻¹ region of the spectrum.
Figure 51 is an FT-IR spectrum for carvedilol hydrobromide 1-pentanol solvate.
Figure 52 is an FT-IR spectrum for carvedilol hydrobromide 1-pentanol solvate in the 4000-2000 cm⁻¹ region of the spectrum.
Figure 53 is an FT-IR spectrum for carvedilol hydrobromide 1-pentanol solvate in the 2000-500 cm⁻¹ region of the spectrum.
Figure 54 is a differential scanning calorimetry thermogram for carvedilol hydrobromide 2-methyl-1-propanol solvate.
Figure 55 is an FT-Raman spectrum for carvedilol hydrobromide 2-methyl-1-propanol solvate.
Figure 56 is an FT-Raman spectrum for carvedilol hydrobromide 2-methyl-1-propanol solvate in the 4000-2000 cm⁻¹ region of the spectrum.
Figure 57 is an FT-Raman spectrum for carvedilol hydrobromide 2-methyl-1-propanol solvate in the 2000-400 cm⁻¹ region of the spectrum.
Figure 58 is an FT-IR spectrum for carvedilol hydrobromide 2-methyl-1-propanol solvate.
Figure 59 is an FT-IR spectrum for carvedilol hydrobromide 2-methyl-1-propanol solvate in the 4000-2000 cm⁻¹ region of the spectrum.
Figure 60 is an FT-IR spectrum for carvedilol hydrobromide 2-methyl-1-propanol solvate in the 2000-500 cm⁻¹ region of the spectrum.
Figure 61 is a differential scanning calorimetry thermogram for carvedilol hydrobromide trifluoroethanol solvate.
Figure 62 is an FT-Raman spectrum for carvedilol hydrobromide trifluoroethanol solvate.
Figure 63 is an FT-Raman spectrum for carvedilol hydrobromide trifluoroethanol solvate in the 4000-2000 cm⁻¹ region of the spectrum.
Figure 64 is an FT-Raman spectrum for carvedilol hydrobromide trifluoroethanol solvate in the 2000-400 cm⁻¹ region of the spectrum.
Figure 65 is an FT-IR spectrum for carvedilol hydrobromide trifluoroethanol solvate.
Figure 66 is an FT-IR spectrum for carvedilol hydrobromide trifluoroethanol solvate in the 4000-2000 cm⁻¹ region of the spectrum.
Figure 67 is an FT-IR spectrum for carvedilol hydrobromide trifluoroethanol solvate in the 2000-500 cm⁻¹ region of the spectrum.
Figure 68 is a differential scanning calorimetry thermogram for carvedilol hydrobromide 2-propanol solvate.
Figure 69 is an FT-Raman spectrum for carvedilol hydrobromide 2-propanol solvate.
Figure 70 is an FT-Raman spectrum for carvedilol hydrobromide 2-propanol solvate in the 4000-2000 cm⁻¹ region of the spectrum.
Figure 71 is an FT-Raman spectrum for carvedilol hydrobromide 2-propanol solvate in the 2000-400 cm⁻¹ region of the spectrum.
Figure 72 is an FT-IR spectrum for carvedilol hydrobromide 2-propanol solvate.
Figure 73 is an FT-IR spectrum for carvedilol hydrobromide 2-propanol solvate in the 4000-2000 cm⁻¹ region of the spectrum.
Figure 74 is an FT-IR spectrum for carvedilol hydrobromide 2-propanol solvate in the 2000-500 cm⁻¹ region of the spectrum.
Figure 75 is an x-ray powder diffractogram for carvedilol hydrobromide n-propanol solvate #1.
Figure 76 shows the thermal analysis results for carvedilol hydrobromide n-propanol solvate #1.
Figure 77 is an FT-Raman spectrum for carvedilol hydrobromide n-propanol solvate #1.
Figure 78 is an FT-Raman spectrum for carvedilol hydrobromide n-propanol solvate #1 in the 4000-2000 cm⁻¹ region of the spectrum.
Figure 79 is an FT-Raman spectrum for carvedilol hydrobromide n-propanol solvate #1 in the 2000-400 cm⁻¹ region of the spectrum.
Figure 80 is an FT-IR spectrum for carvedilol hydrobromide n-propanol solvate #1.
Figure 81 is an FT-IR spectrum for carvedilol hydrobromide n-propanol solvate #1 in the 4000-2000 cm⁻¹ region of the spectrum.
Figure 82 is an FT-IR spectrum for carvedilol hydrobromide n-propanol solvate #1 in the 2000-500 cm⁻¹ region of the spectrum.
Figure 83 is an x-ray powder diffractogram for carvedilol hydrobromide n-propanol solvate #2.
Figure 84 shows the thermal analysis results for carvedilol hydrobromide n-propanol solvate #2.
Figure 85 is an FT-Raman spectrum for carvedilol hydrobromide n-propanol solvate #2.
Figure 86 is an FT-Raman spectrum for carvedilol hydrobromide n-propanol solvate #2 in the 4000-2000 cm⁻¹ region of the spectrum.
Figure 87 is an FT-Raman spectrum for carvedilol hydrobromide n-propanol solvate #2 in the 2000-400 cm⁻¹ region of the spectrum.
Figure 88 is an FT-IR spectrum for carvedilol hydrobromide n-propanol solvate #2.
Figure 89 is an FT-IR spectrum for carvedilol hydrobromide n-propanol solvate #2 in the 4000-2000 cm⁻¹ region of the spectrum.
Figure 90 is an FT-IR spectrum for carvedilol hydrobromide n-propanol solvate #2 in the 2000-500 cm⁻¹ region of the spectrum.
Figure 91 is an x-ray powder diffractogram for carvedilol hydrobromide anhydrous forms.
Figure 92 shows the thermal analysis results for carvedilol hydrobromide anhydrous forms.
Figure 93 is an FT-Raman spectrum for carvedilol hydrobromide anhydrous forms.
Figure 94 is an FT-Raman spectrum for carvedilol hydrobromide anhydrous forms in the 4000-2000 cm⁻¹ region of the spectrum.
Figure 95 is an FT-Raman spectrum for carvedilol hydrobromide anhydrous forms in the 2000-400 cm⁻¹ region of the spectrum.
Figure 96 is an FT-IR spectrum for carvedilol hydrobromide anhydrous forms.
Figure 97 is an FT-IR spectrum for carvedilol hydrobromide anhydrous forms in the 4000-2000 cm⁻¹ region of the spectrum.
Figure 98 is an FT-IR spectrum for carvedilol hydrobromide anhydrous forms in the 2000-500 cm⁻¹ region of the spectrum.
Figure 99 is an x-ray powder diffractogram for carvedilol hydrobromide ethanol solvate.
Figure 100 shows the thermal analysis results for carvedilol hydrobromide ethanol solvate.
Figure 101 is an FT-Raman spectrum for carvedilol hydrobromide ethanol solvate.
Figure 102 is an FT-Raman spectrum for carvedilol hydrobromide ethanol solvate in the 4000-2000 cm⁻¹ region of the spectrum.
Figure 103 is an FT-Raman spectrum for carvedilol hydrobromide ethanol solvate in the 2000-400 cm⁻¹ region of the spectrum.
Figure 104 is an FT-IR spectrum for carvedilol hydrobromide ethanol solvate.
Figure 105 is an FT-IR spectrum for carvedilol hydrobromide ethanol solvate in the 4000-2000 cm⁻¹ region of the spectrum.
Figure 106 is an FT-IR spectrum for carvedilol hydrobromide ethanol solvate in the 2000-500 cm⁻¹ region of the spectrum.
Figure 107 is an x-ray powder diffractogram for carvedilol hydrobromide dioxane solvate.
Figure 108 is an x-ray powder diffractogram for carvedilol hydrobromide 1-pentanol solvate.
Figure 109 is an x-ray powder diffractogram for carvedilol hydrobromide 2-methyl-1-propanol solvate.
Figure 110 is an x-ray powder diffractogram for carvedilol hydrobromide trifluoroethanol solvate.
Figure 111 is an x-ray powder diffractogram for carvedilol hydrobromide 2-propanol solvate.

### Carvedilol Citrate Salts

Figure 112 is a FT-IR spectrum of carvedilol monocitrate salt.
Figure 113 depicts XRPD patterns of two different batches of Carvedilol monocitrate salt.

### Carvedilol Mandelate Salts

Figure 114 is a FT-IR spectrum of carvedilol mandelate salt.
Figure 115 is a FT-Raman spectrum of carvedilol mandelate salt.

### Carvedilol Lactate Salts

Figure 116 is a FT-IR spectrum of carvedilol lactate salt.
Figure 117 is a FT-Raman spectrum of carvedilol lacatate salt.

### Carvedilol Maleate Salts

Figure 118 is a FT-IR spectrum of carvedilol maleate salt.
Figure 119 is a FT-Raman spectrum of carvedilol maleate salt.

### Carvedilol Sulfate Salts

Figure 120 is a FT-IR spectrum of carvedilol sulfate salt.
Figure 121 is a FT-Raman spectrum of carvedilol sulfate salt.

### Carvedilol Glutarate Salts

Figure 122 is a FT-IR spectrum of carvedilol glutarate salt.
Figure 123 is a FT-Raman spectrum of carvedilol glutarate salt.

### Carvedilol Benzoate Salts

Figure 124 is a FT-IR spectrum of carvedilol benzoate salt.
Figure 125 is a FT-Raman spectrum of carvedilol benzoate salt.

### Drug Solubility Enhancement in GI tract

Figure 126 depicts a pH-solubility profile for carvedilol.

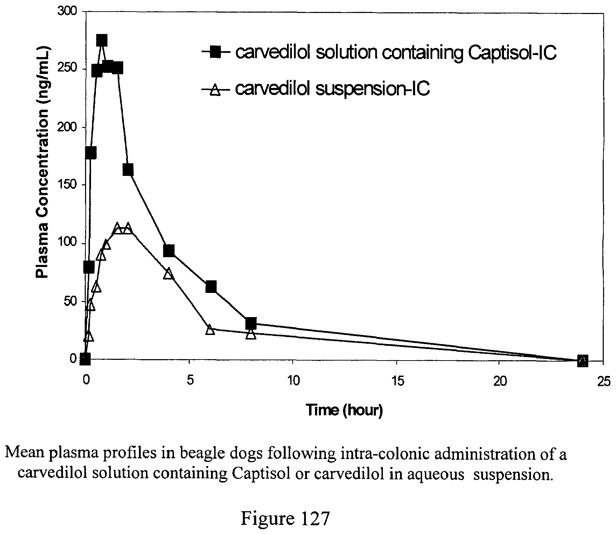

Figure 127 depicts mean plasma profiles in beagle dogs following intra-colonic administration of a carvedilol solution containing captisol or carvedilol in aqueous suspension.
Figure 128 depicts dissolution/solubility profile of carvedilol phosphate in pH = 7.1 tris buffer.

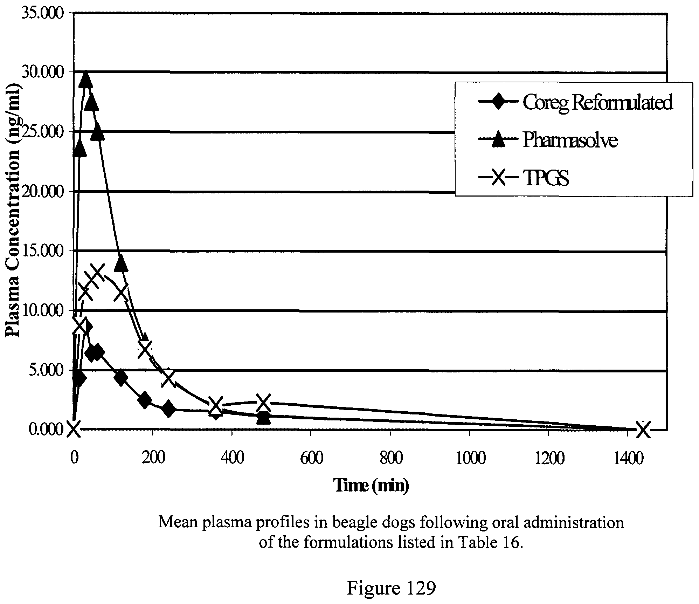

Figure 129 depicts mean plasma profiles in beagle dogs following oral administration of the formulations listed in Table 4.
Figure 130 depicts mean plasma profiles following oral administration of companion capsules filled with four formulations at 10 mg strength to beagle dogs.

### Pharmacodynamic Profiles

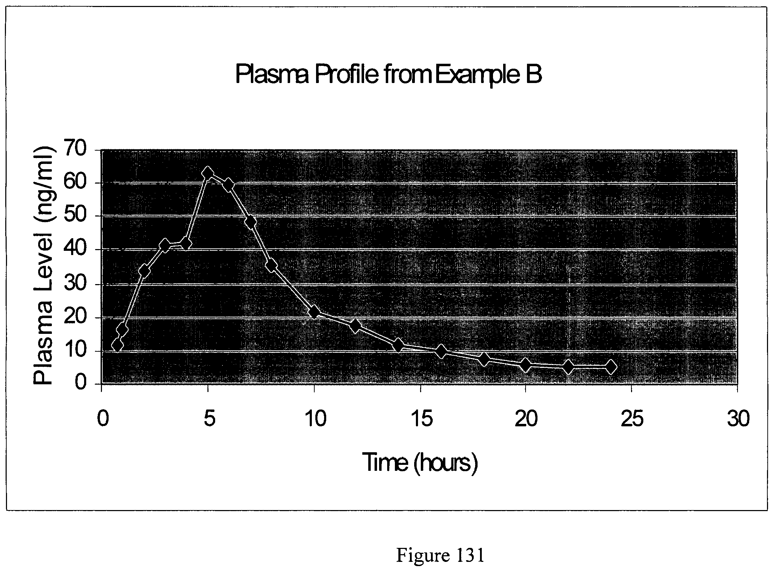

Figure 131 depicts a plasma profile from capsules formulated according to Example 29 (B).
Figure 132 depicts depicts a mean plasma profiles of subjects) for the formulation described in Example 33, Table 23.
Figure 133 depicts a representative individual plasma profile.
Figure 134 depicts a comparison between a test product profile (mean values as in Figure 133) vs. a profile for a conventional (immediate release) product dosed twice daily.

### Detailed Description of the Invention

In general, the present invention relates to a carvedilol phosphate salt, anhydrous form, or solvate thereof, corresponding pharmaceutical compositions or controlled release dosage forms or formulations, and delivery methods of carvedilol forms to the gastrointestingal tract or methods to treat cardiovascular diseases, which may include, but are not limited to hypertension, congestive heart failure, atherosclerosis, and angina.

The present invention relates to control release formulations, which comprise carvedilol phosphate salts, anhydrous forms or solvates thereof.

The present invention generally relates to a controlled release formulation, which comprises a carvedilol phosphate salt, solvate or anhydrous forms; where the aforementioned controlled release formulation following oral dosage exhibits a substantially biphasic plasma profile which exhibits a first plasma concentration peak level and a first Tₘₐₓ pulse within 1-4 hours of ingestion and a second a plasma concentration peak level and second Tₘₐₓ pulse within 5-8 hours after ingestion.

Disclosed is a controlled release formulation, which comprises:
a solubility enhanced carvedilol salt, solvate or anhydrous forms;
where the controlled release formulation following oral dosage exhibits a substantially biphasic plasma profile with a first plasma concentration peak level and a first Tₘₐₓ pulse within 1-4 hours of ingestion and a second plasma concentration peak level and a second Tₘₐₓ pulse within 5-10 hours after ingestion. In an embodiment of the present invention a first Tₘₐₓ pulse may occur within 1-3 hours of ingestion and the second Tₘₐₓ pulse may occur within 5-10 hours after ingestion.

Disclosed is a controlled release formulation, comprising at least one of these components:
[a] carvedilol free base; or
[b] a solubility enhanced carvedilol salt, solvate or anhydrous forms;
where the controlled release formulation is a capsule dosage form is comprised of a pellet or microparticle mixture of immediate release coated pellet populations or controlled release coated pellet populations of differing sizes; or
where the controlled release formulation is a capsule dosage form is comprised of a pellet or microparticle mixture of immediate release coated pellet populations and controlled release coated pellet populations; or
where the controlled release formulation following oral dosage at night exhibits a substantially biphasic plasma peak concentration profile which exhibits a first pharmacokinetic plasma concentration peak level and a first Tₘₐₓ pulse within 1-4 hours of ingestion and a second plasma concentration peak level and a second Tₘₐₓ pulse within 5-8 hours after ingestion.

### Carvedilol Salts, Anhydrous forms, or Solvates Thereof

In general, the present invention relates to carvedilol phosphate salts, anhydrous forms or solvates thereof.

In particular, the present invention relates a novel crystalline salt, anhydrous forms, or solvate form thereof, which may include, but are not limited to crystalline or other solid forms, such as a salt form of 1-(carbazol-4-yloxy-3-[[2-(o-methoxyphenoxy)ethyl] amino]-2-propanol).

Carvedilol free base or all carvedilol salt, anhydrous or solvate compound forms thereof suitable for use in the present invention, which include starting materials (i.e., such as carvedilol), intermediates or products, etc., are prepared as described herein, or by the application or adaptation of known methods, which may be methods used heretofore or described in the literature.

Carvedilol is disclosed and claimed in U.S. Patent No. 4,503,067 to Wiedemann et al. ("U.S. '067 Patent"). Reference should be made to U.S. '067 Patent for its full disclosure, which include methods of preparing or using the carvedilol compound. The entire disclosure of the U.S. '067 Patent is incorporated hereby by reference in its entirety.

U.S. Pat. No. 6,515,010 to Franchini et al. discloses a novel salt form of carvedilol, namely carvedilol methanesulfonate salt form, pharmaceutical compositions containing carvedilol methanesulfonate and the use of the aforementioned compound in the treatment of hypertension, congestive heart failure, and angina, which is hereby incorporated by reference in its entirety.

The present invention relates to a carvedilol compound, which is carvedilol free base or a novel salt, solvate or anhydrous form of carvedilol, which may include, but is not limited to a crystalline salt or other solid form.

In accordance with the present invention, it has been unexpectedly found that carvedilol compounds may be isolated readily, but are not limited to crystalline forms or other solid forms, which display much higher solubility when compared to the free base form of carvedilol.

The present invention is related to pharmaceutically acceptable acid addition salts of carvedilol free base or corresponding forms.

Such pharmaceutically acceptable acid addition salts of carvedilol free base or corresponding forms thereof are formed by reaction with appropriate organic acids or mineral acids, which may include, but are not limited to formation by such methods described herein or conventionally known in the chemical arts.

For example, such acid addition salts may be formed via the following conventional chemical reactions or methods:
reaction of carvedilol free base with a suitable organic acid or mineral acid in an aqueous miscible organic solvent with isolation of the formed acid addition salt by removing the organic solvent by conventional art known techniques; or
reaction of carvedilol free base with a suitable organic acid or mineral acid in an aqueous immiscible organic solvent where the formed acid addition salt is separated directly or isolated by removing the solvent by conventional art known techniques, such as by filtration.

For example, an acid addition salt of carvedilol free base or carvedilol salt, solvate or anhydrous form thereof is an acid addition salt formed from mineral acids or organic acids.

Representative examples of such suitable organic or mineral acids may include, but are not limited to maleic acid, fumaric acid, benzoic acid, ascorbic acid, pamoic acid, succinic acid, bismethylenesalicyclic acid, methane sulphonic or sulfonic acid, acetic acid, propionic acid, tartaric acid, salicyclic acid, citric acid, gluconic acid, aspartic acid, stearic acid, palmitic acid, itaconic acid, glycolic acid, p-aminobenzoic acid, glutamic acid, benzene sulfonic acid or sulphonic acid, hydrochloric acid, hydrobromic acid, sulfuric acid or sulphuric acid, cyclohexylsulfamic acid, phosphoric acid, nitric acid and the like.

In accordance with the present invention, mineral acids may be selected from, but are not limited to hydrobromic acid, hydrochloric acid, phosphoric acid, sulfuric acid or sulphuric acid, and the like; and organic acids may be selected from, but not limited to methansulphuric acid, tartaric acid, maleic acid, acetic acid, citric acid, benzoic acid and the like.

As indicated above, the present invention further relates to carvedilol salt forms, which may include, but are not limited to novel crystalline salt or other solid forms of carvedilol mandelate, carvedilol lactate, carvedilol maleate, carvedilol sulfate, carvedilol glutarate, carvedilol mesylate, carvedilol phosphate, carvedilol citrate, carvedilol hydrogen bromide, carvedilol oxalate, carvedilol hydrogen chloride, carvedilol hydrogen bromide, carvedilol benzoate, or corresponding solvates thereof.

More particularly, the present invention relates to carvedilol salt forms, which may include, but are not limited to carvedilol hydrogen phosphate, carvedilol dihydrogen phosphate, carvedilol dihydrogen phosphate hemihydrate, carvedilol dihydrogen phosphate dihydrate, carvedilol dihydrogen phosphate methanol solvate, carvedilol hydrobromide monohydrate, carvedilol hydrobromide dioxane solvate, carvedilol hydrobromide 1-pentanol solvate, carvedilol hydrobromide 2-methyl-1-propanol solvate, carvedilol hydrobromide trifluoroethanol solvate, carvedilol hydrobromide 2-propanol solvate, carvedilol hydrobromide n-propanol solvate #1, carvedilol hydrobromide n-propanol solvate #2, carvedilol hydrobromide anhydrous forms or anhydrous forms, carvedilol hydrobromide ethanol solvate, carvedilol hydrobromide dioxane solvate, carvedilol monocitrate monohydrate, carvedilol mandelate, carvedilol lactate, carvedilol hydrochloride, carvedilol maleate, carvedilol sulfate, carvedilol glutarate, or corresponding anhydrous forms, solvates thereof.

In accordance with the present invention, other salts or solvates of carvedilol of the present invention may be isolated, but not limited to different solid or crystalline forms. Moreover, a specific identified species of such carvedilol salts (or a specific identified corresponding solvate species) also may be isolated in various different crystalline or solid forms, which may include anhydrous forms or solvate forms. For example, suitable solvates of carvedilol phosphate as defined in the present invention, include, but are not limited to carvedilol dihydrogen phosphate hemihydrate, carvedilol dihydrogen phosphate dihydrate (i.e., which include Forms II and IV, respectively), carvedilol dihydrogen phosphate methanol solvate, and carvedilol hydrogen phosphate.

In light of this, carvedilol salt forms of the present invention (i.e., which may include different polymorphs, ahydrous forms, solvates, or hydrates thereof) may exhibit characteristic polymorphism. As conventionally understood in the art, polymorphism is defined as an ability of a compound to crystallize as more than one distinct crystalline or "polymorphic" species. A polymorph is defined as a solid crystalline phase of a compound with at least two different arrangements or polymorphic forms of that compound molecule in the solid state.

Polymorphic forms of any given compound, including those of the present invention, are defined by the same chemical formula or composition and are as distinct in chemical structure as crystalline structures of two different chemical compounds. Such compounds may differ in packing, geometrical arrangement of respective crystalline lattices, etc.

In light of the foregoing, chemical and/or physical properties or characteristics vary with each distinct polymorphic form, which may include variations in solubility, melting point, density, hardness, crystal shape, optical and electrical properties, vapor pressure, stability, etc.

Solvates and/or hydrates of crystalline carvedilol salt forms of the present invention also may be formed when solvent molecules are incorporated into the crystalline lattice structure of the compound molecule during the crystallization process. For example, solvate forms of the present invention may incorporate nonaqueous solvents such as methanol and the like as described herein below. Hydrate forms are solvate forms, which incorporate water as a solvent into a crystalline lattice.

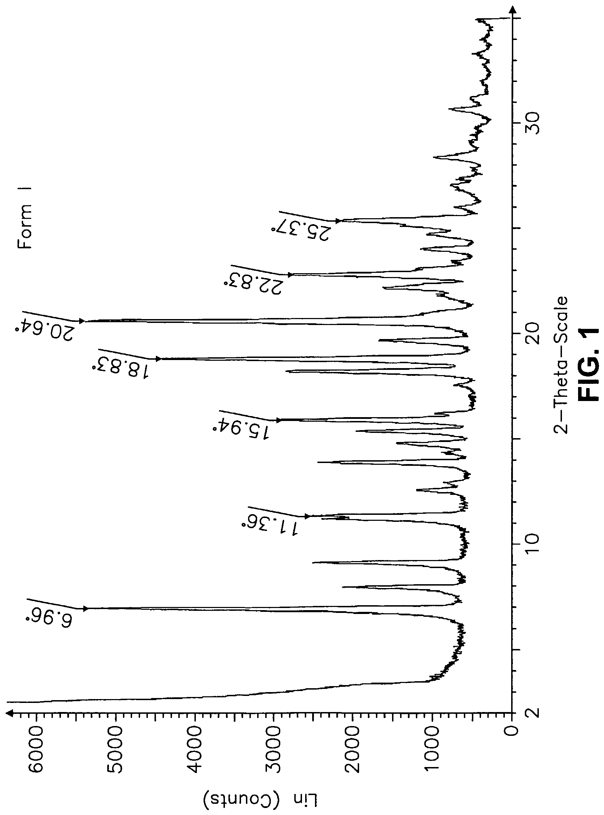

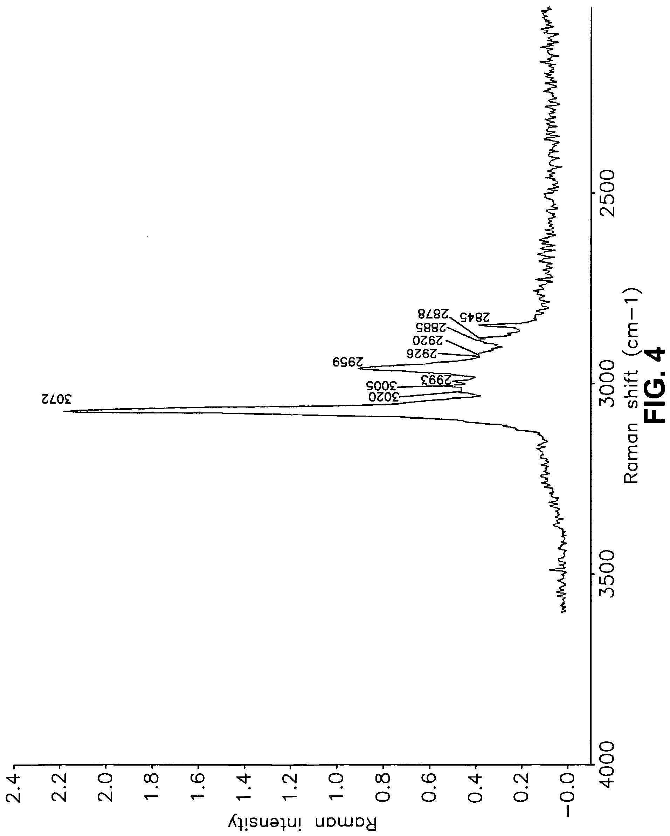

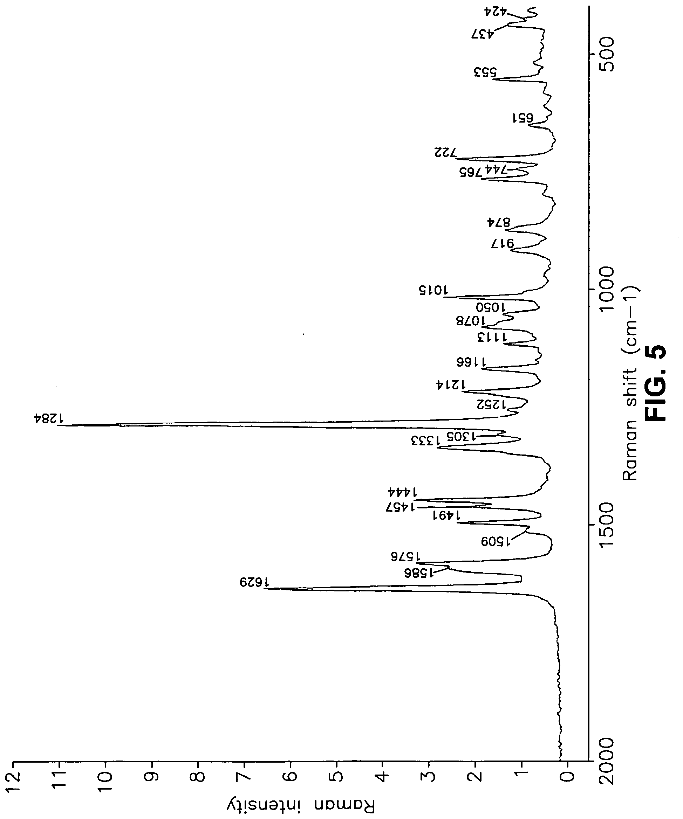

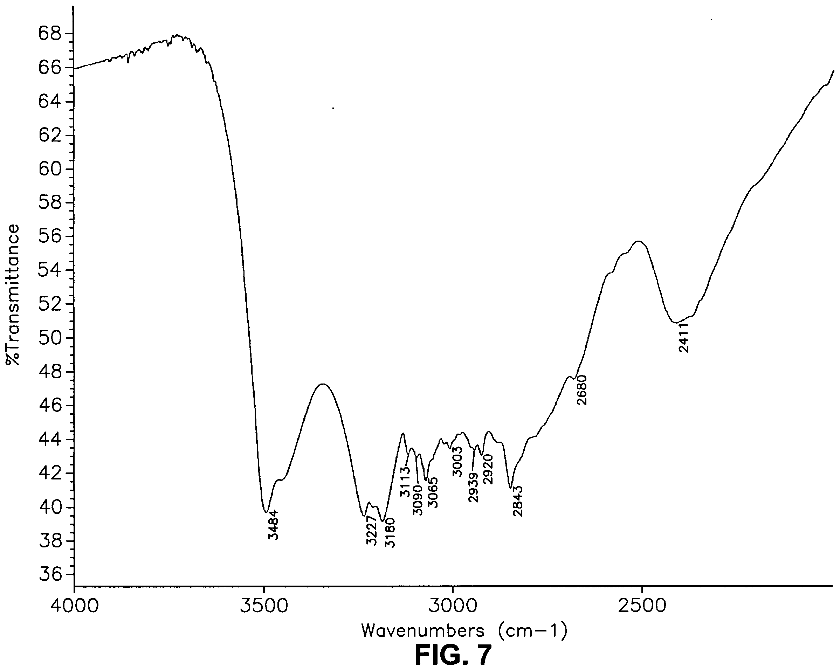

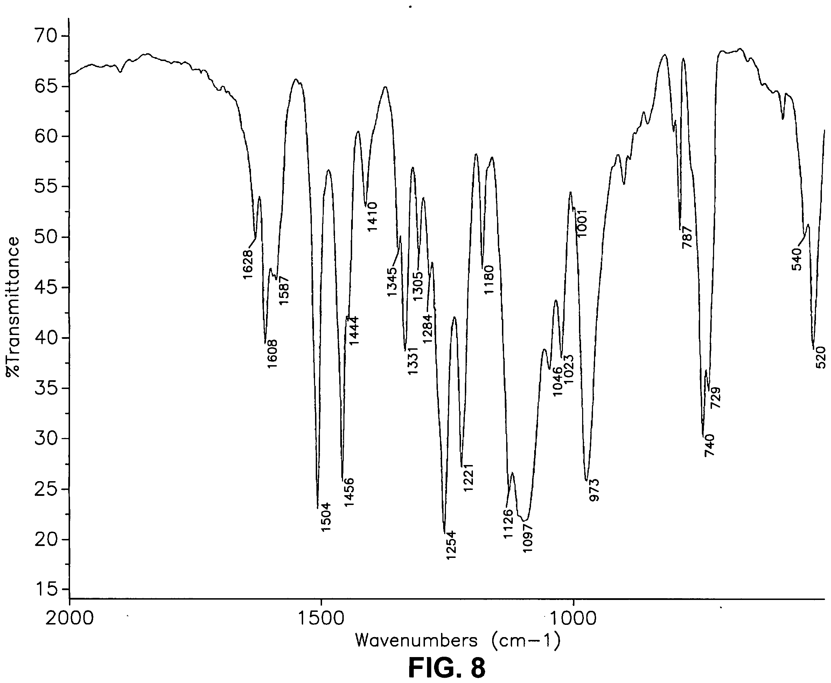

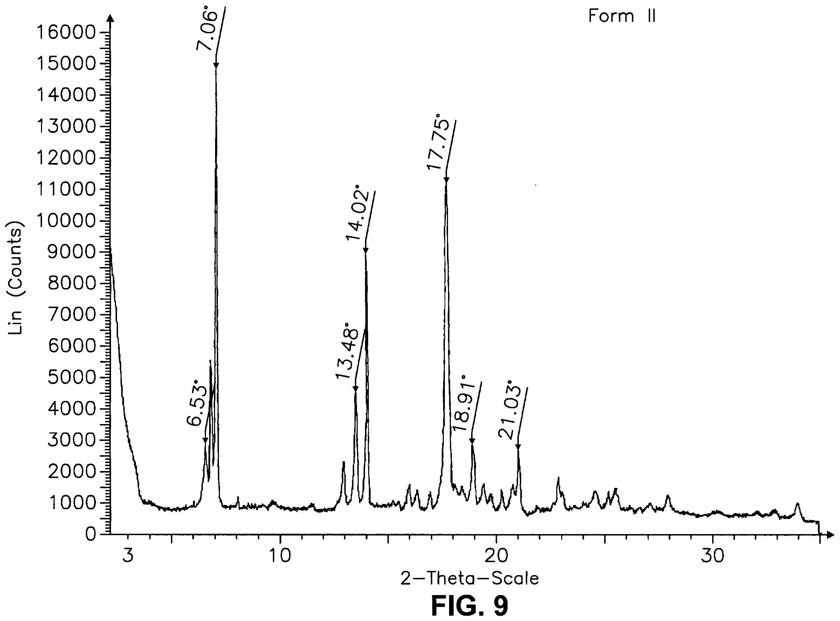

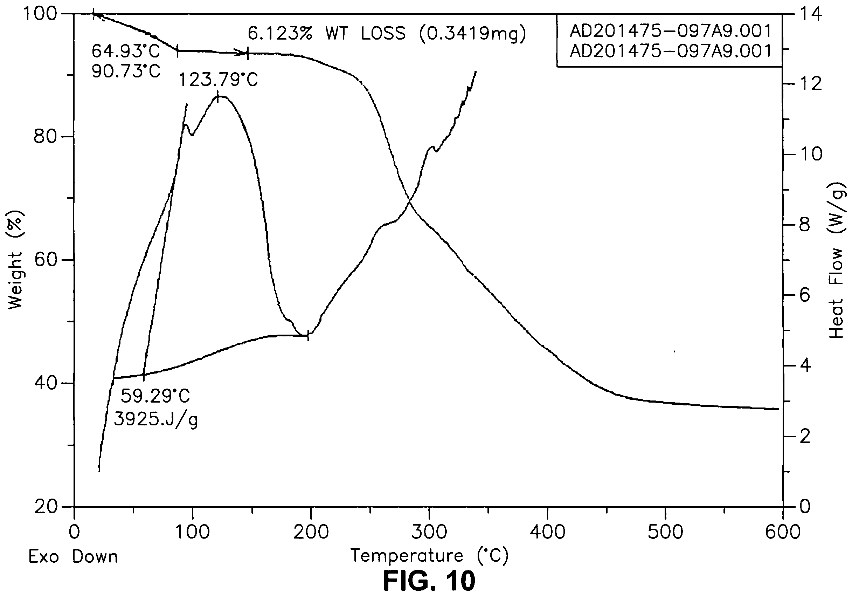

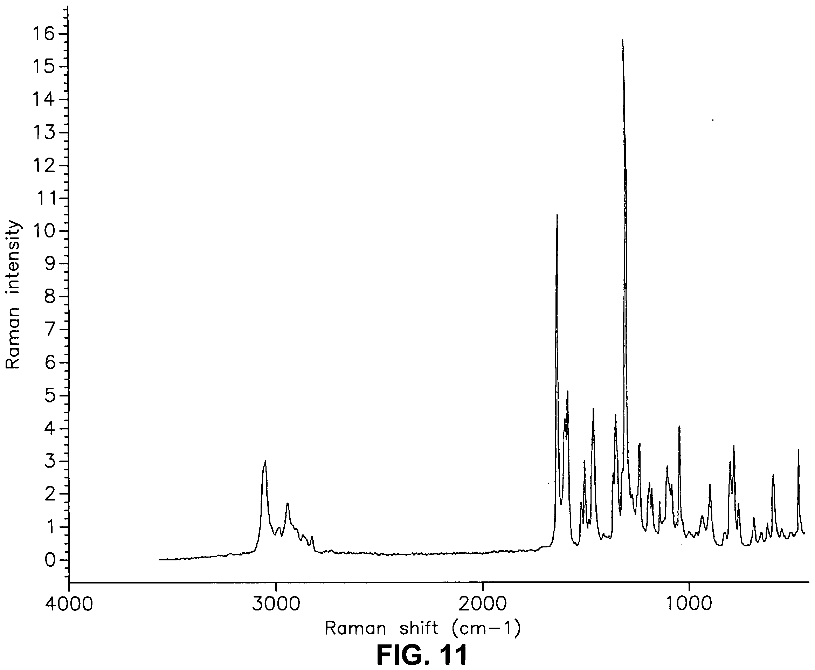

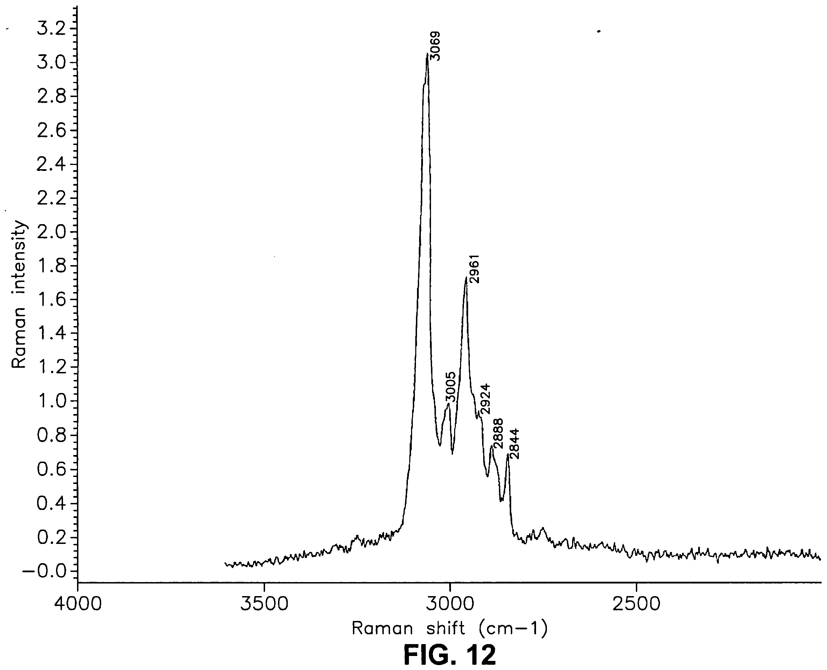

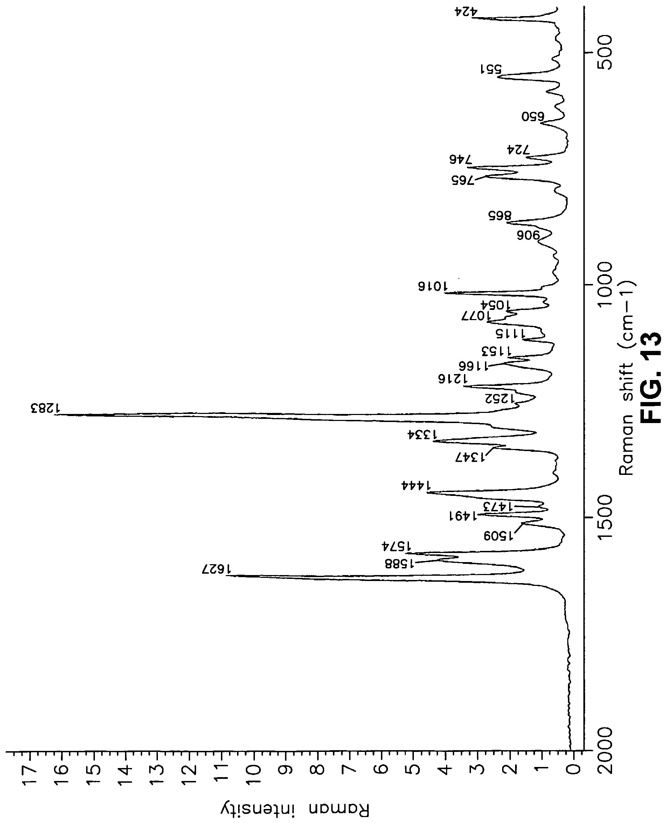

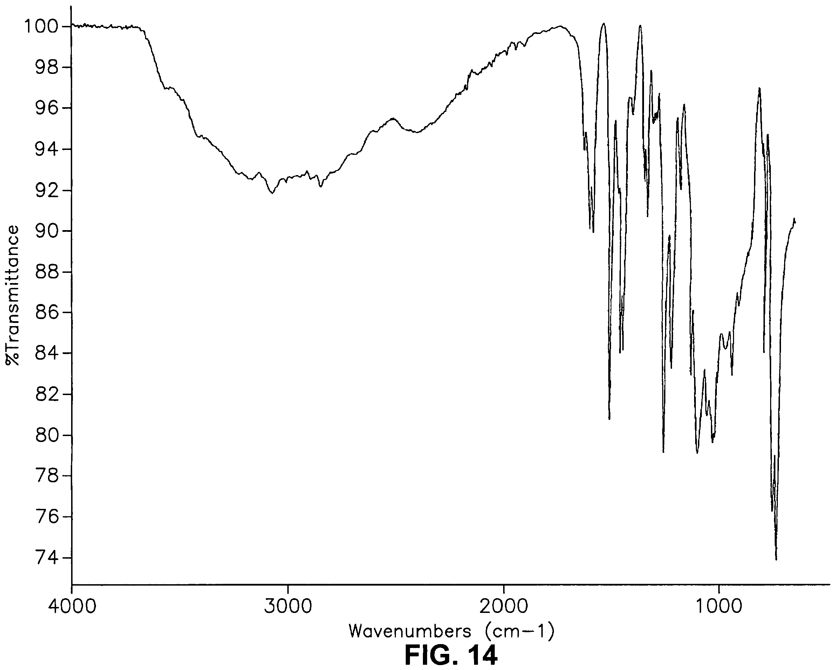

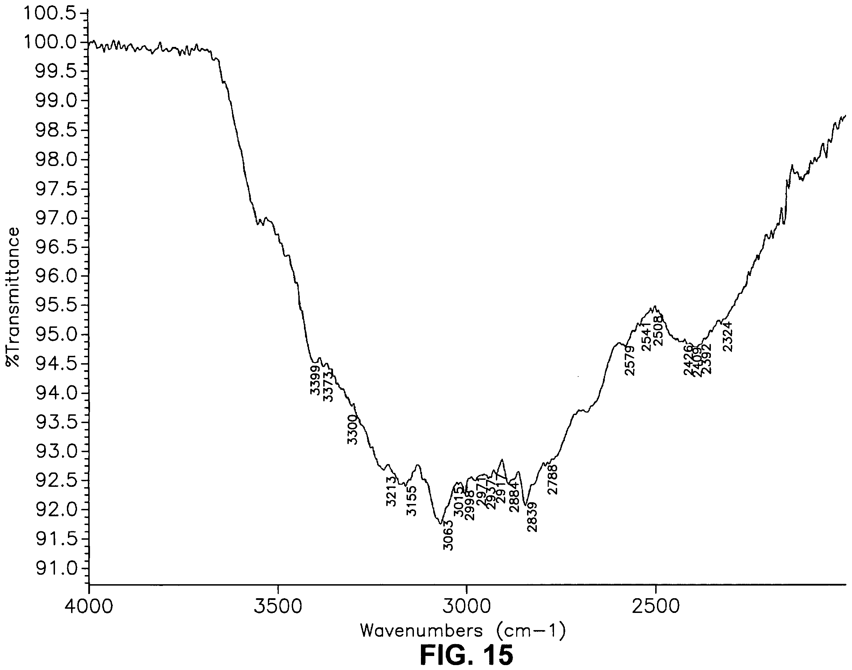

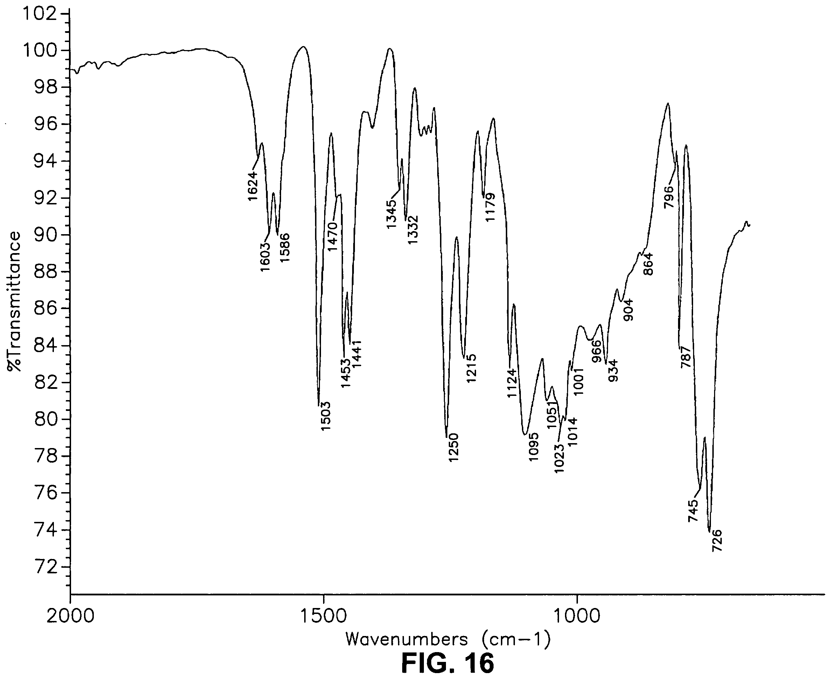

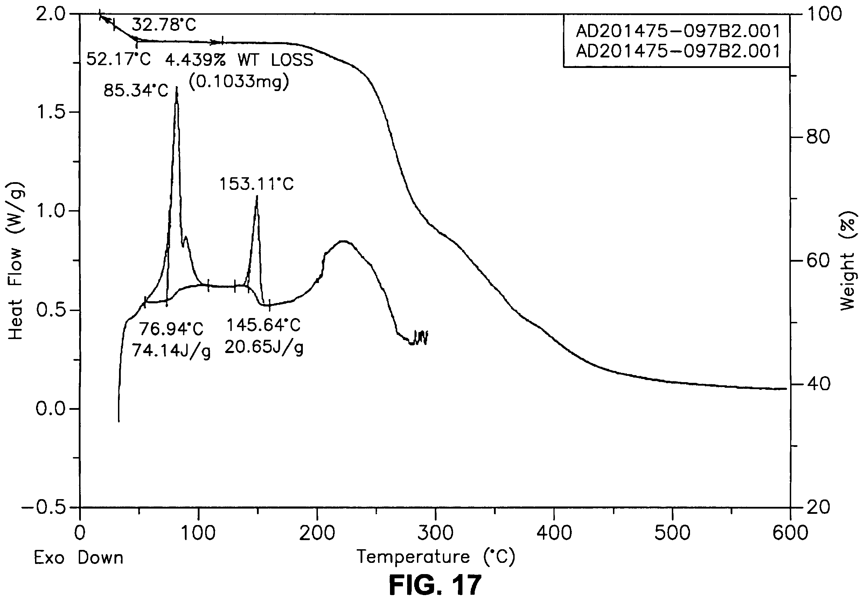

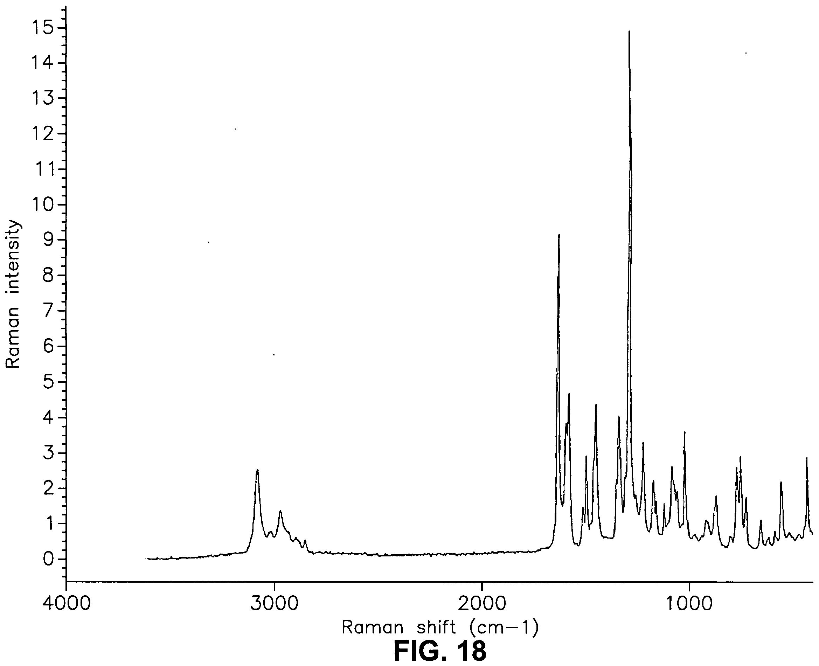

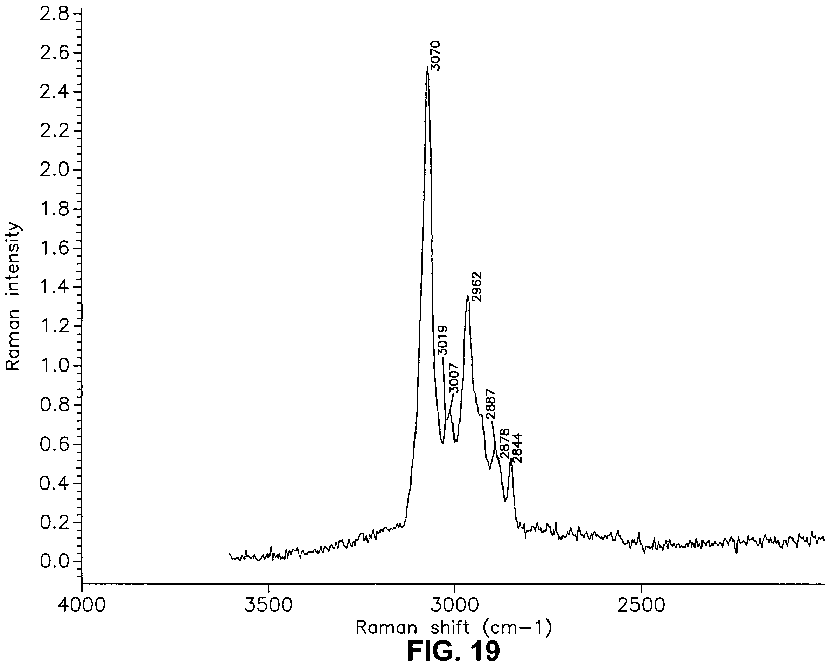

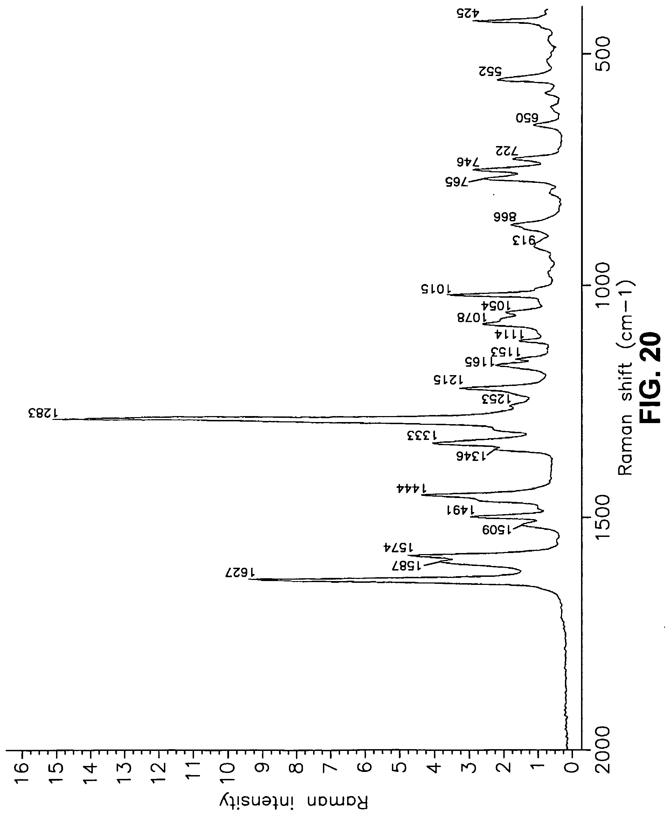

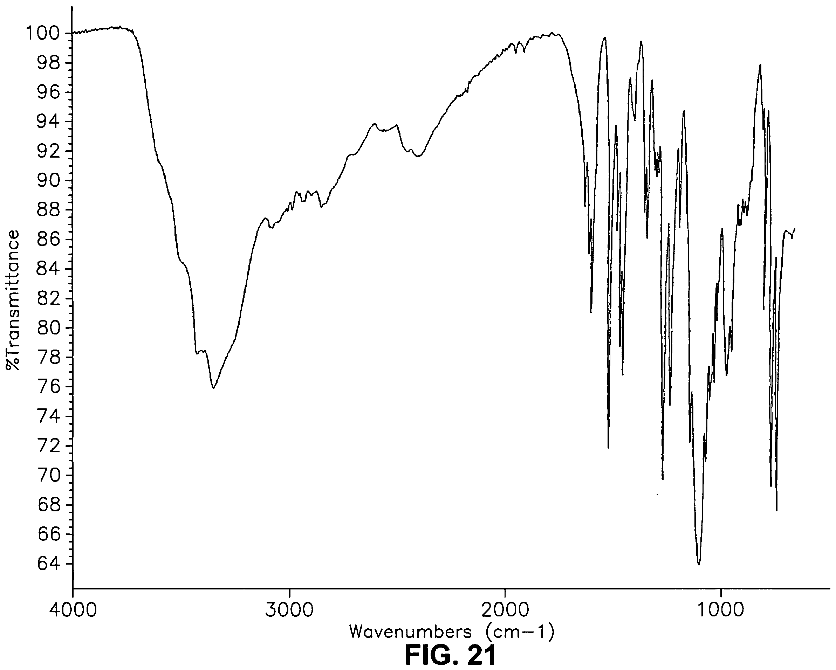

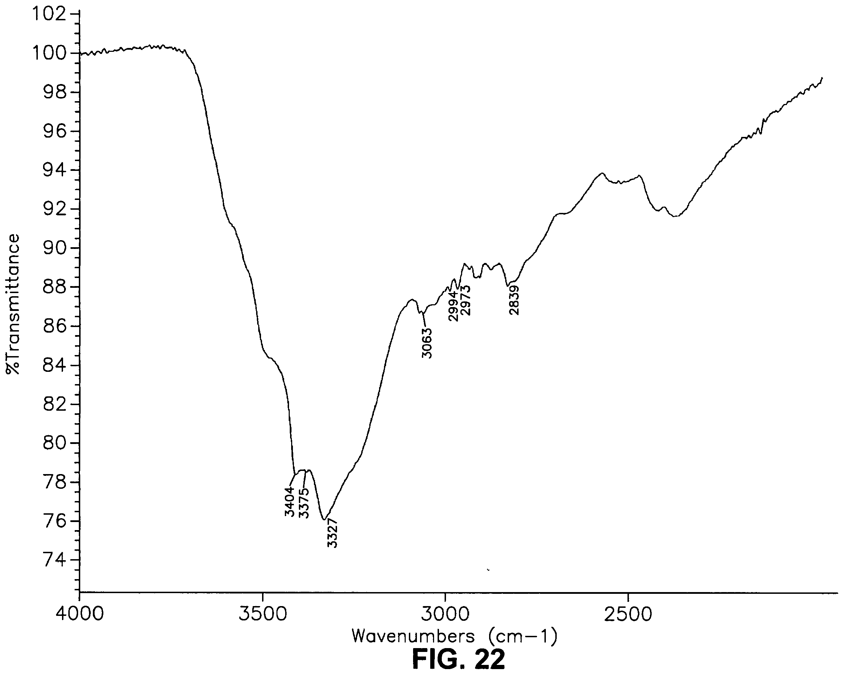

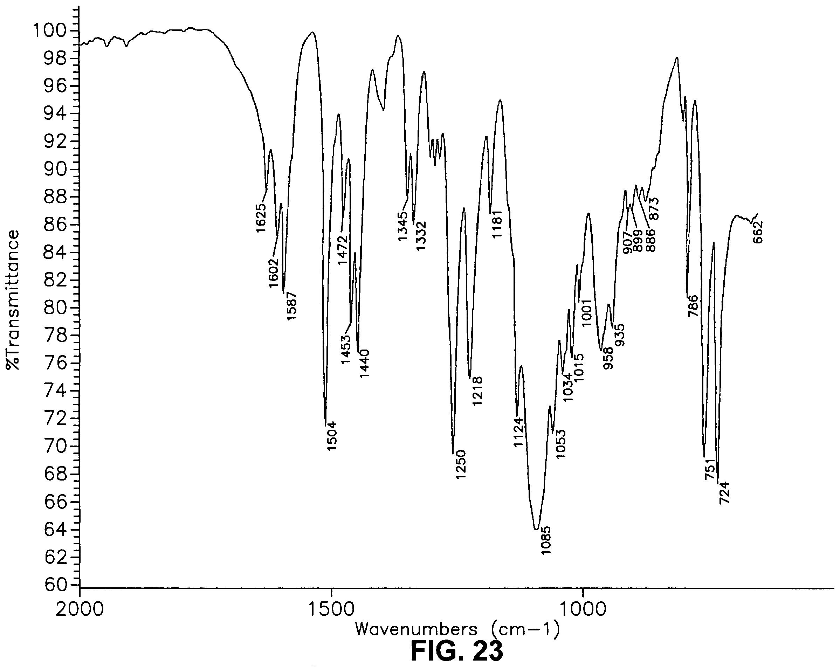

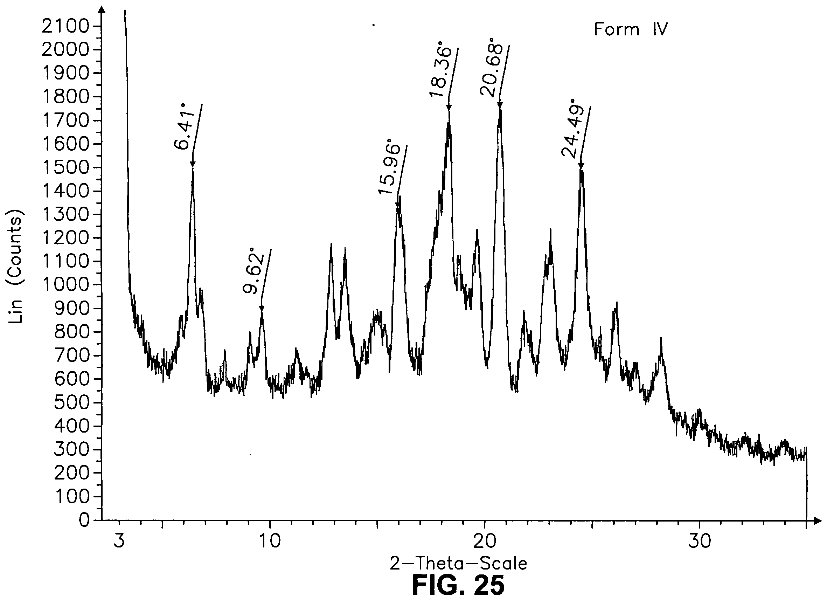

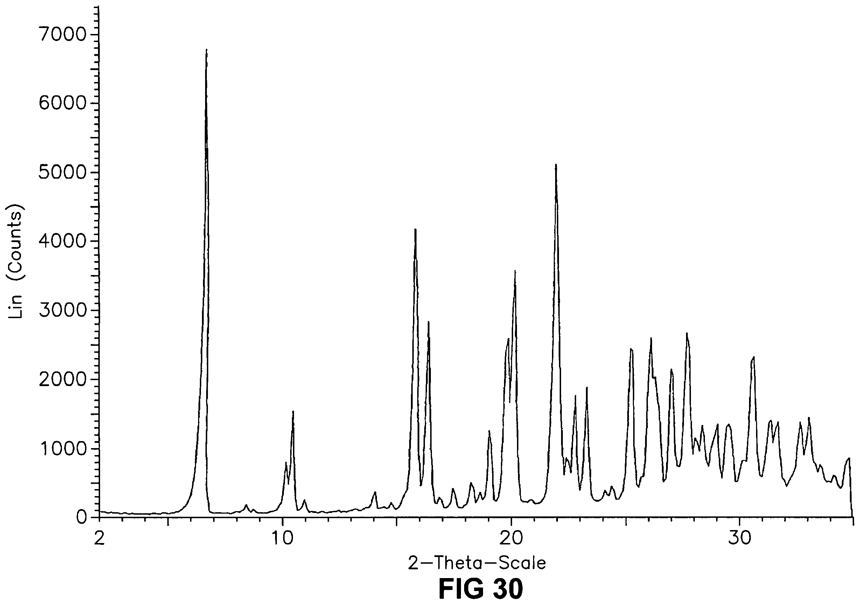

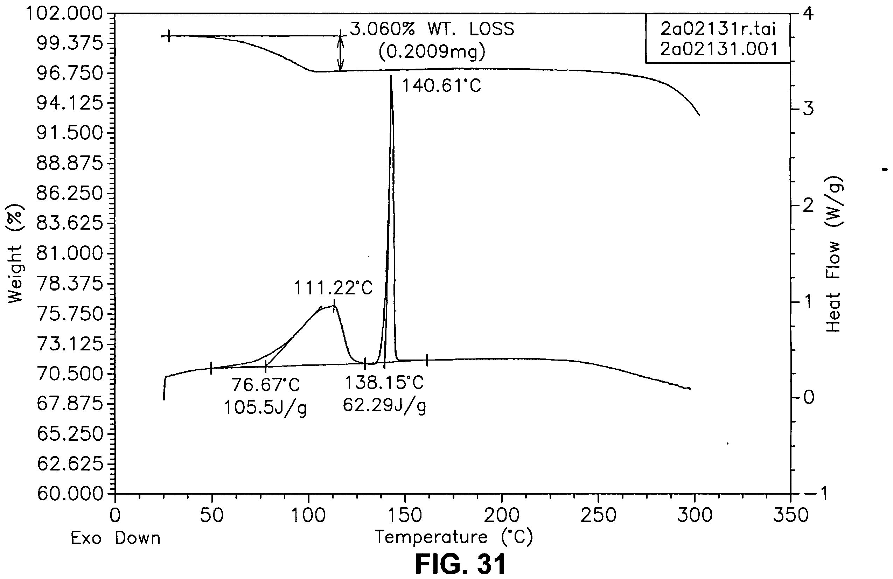

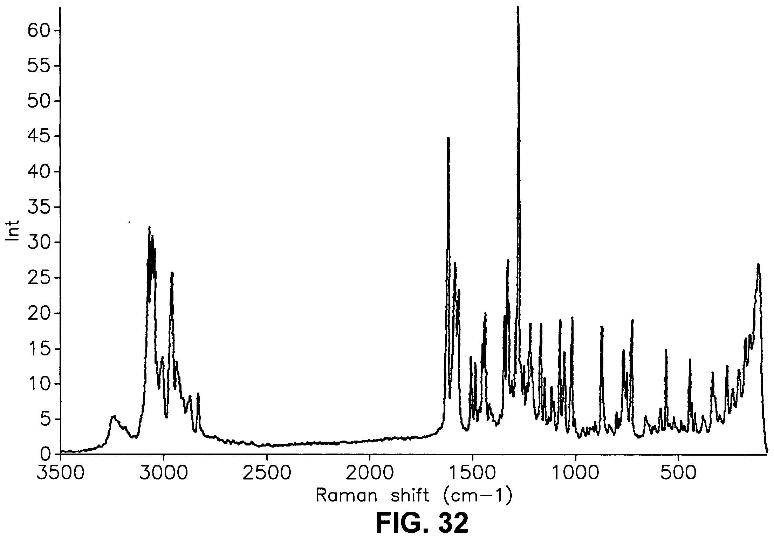

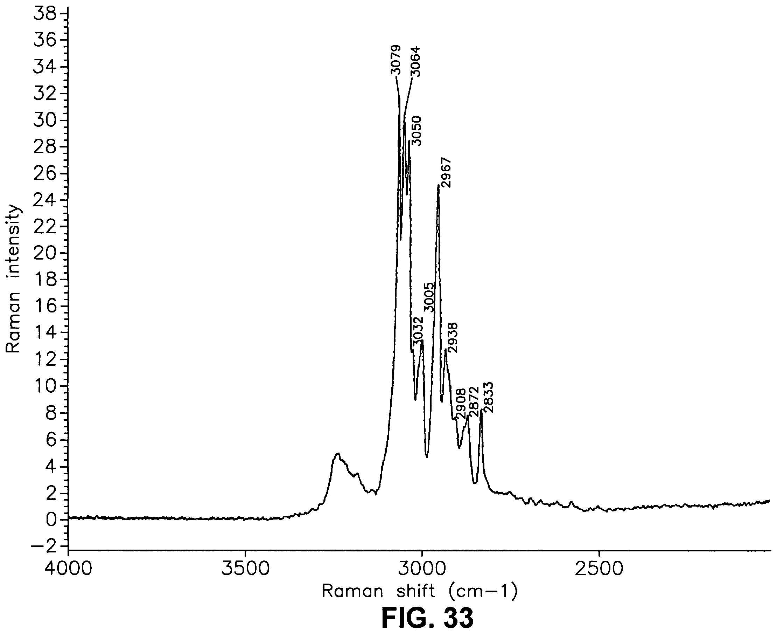

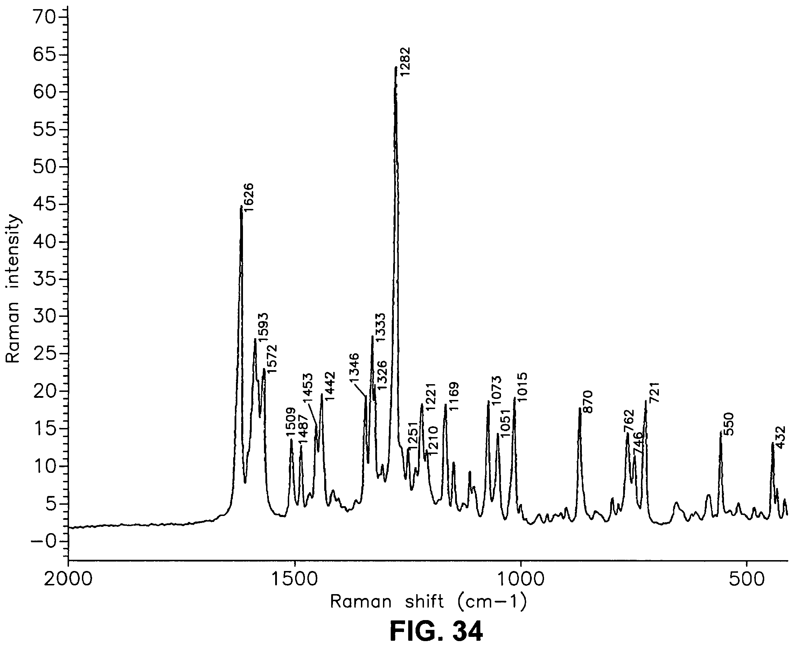

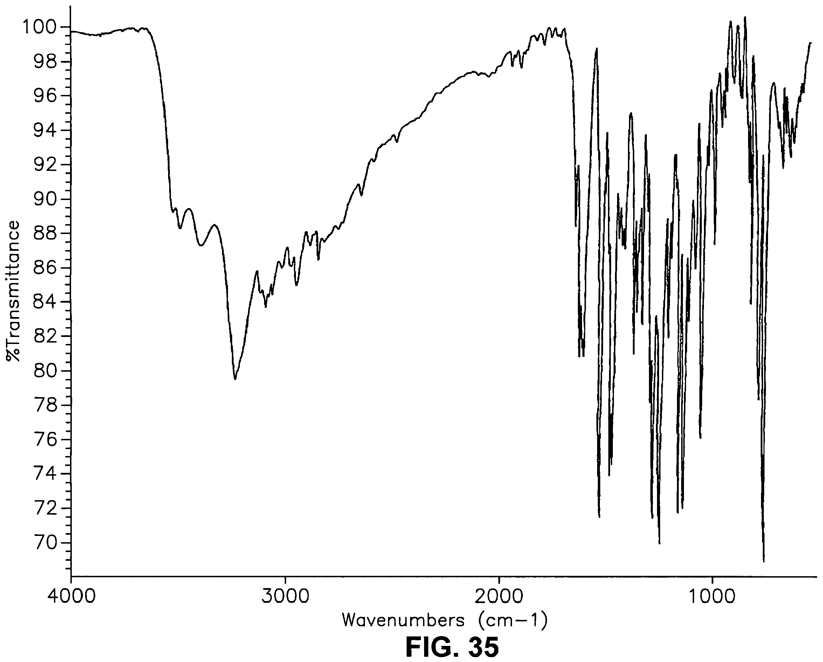

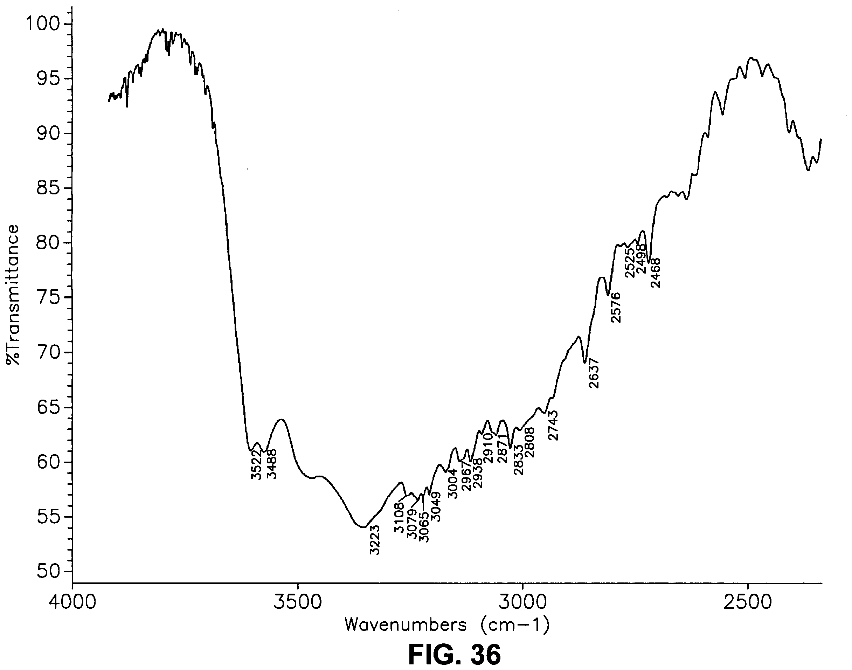

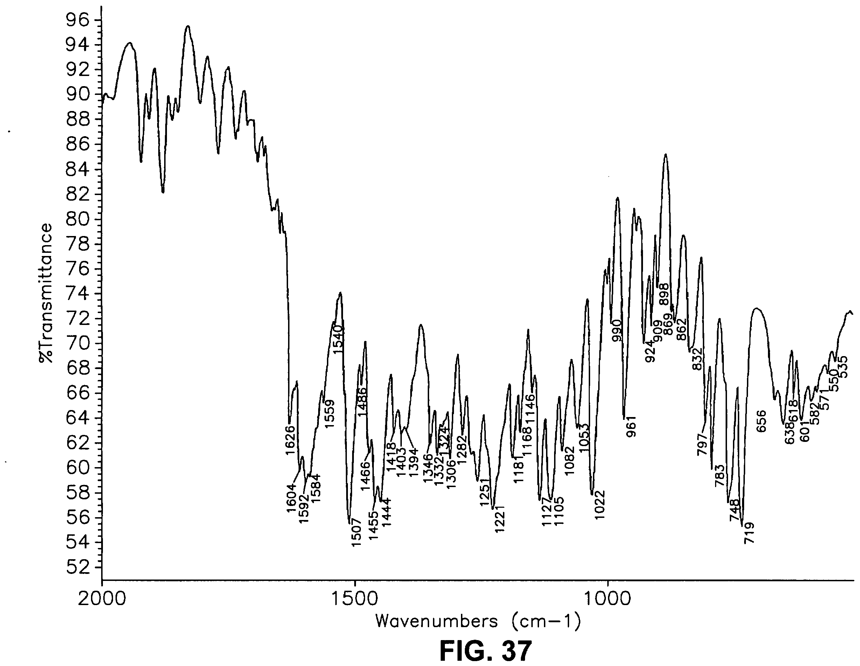

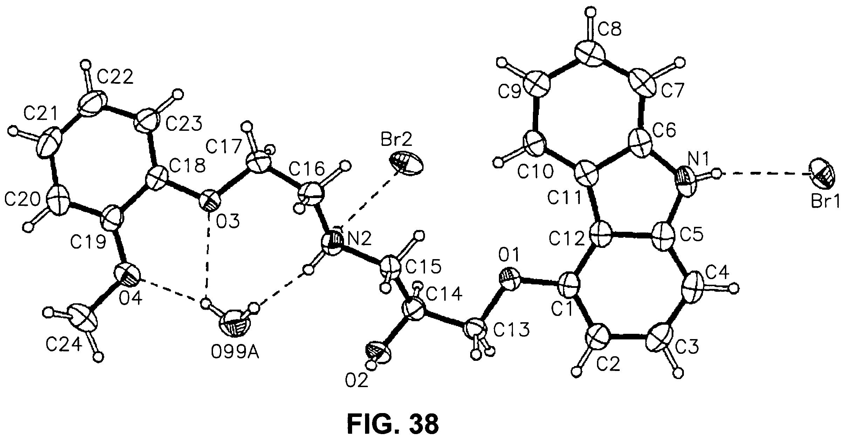

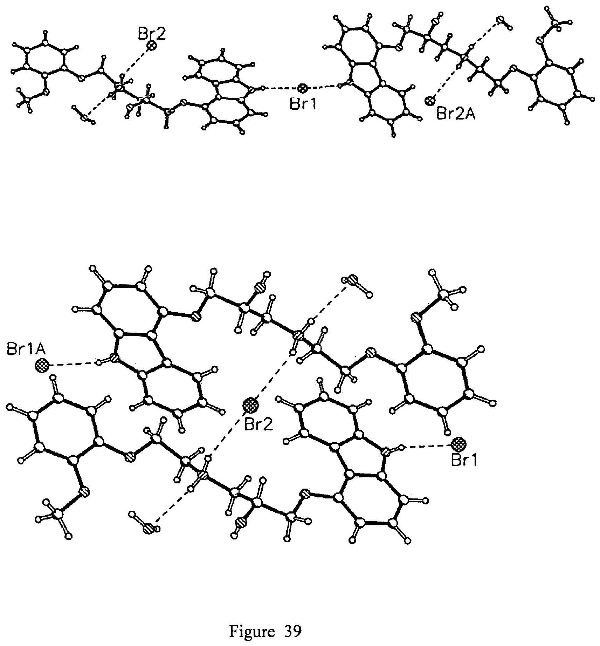

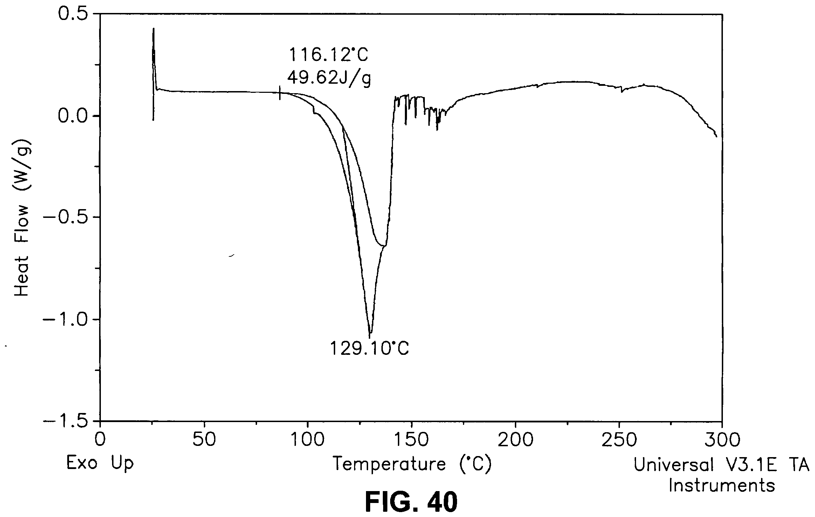

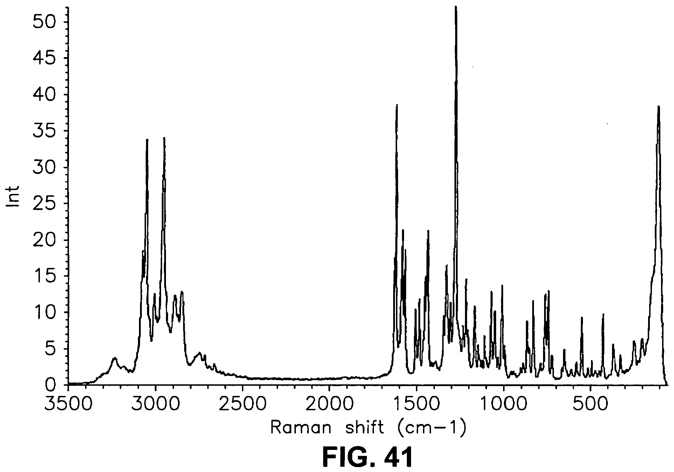

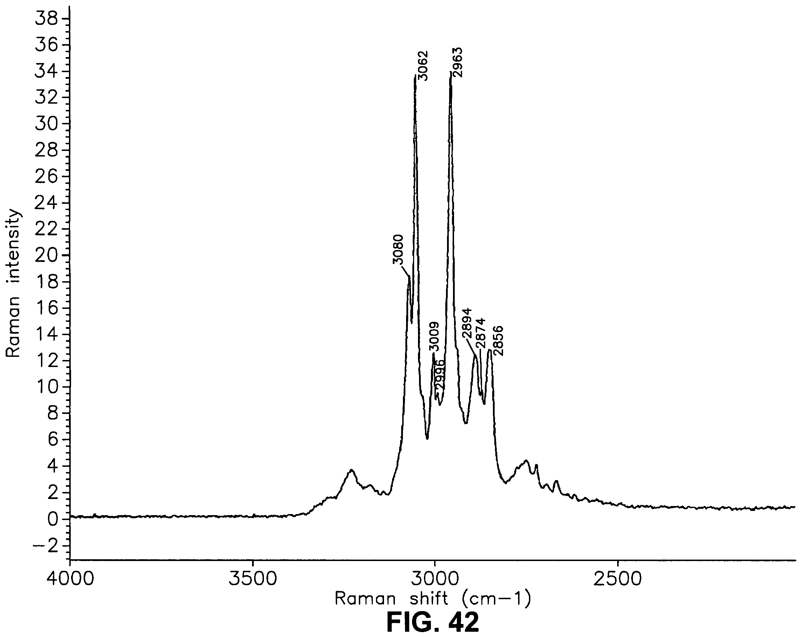

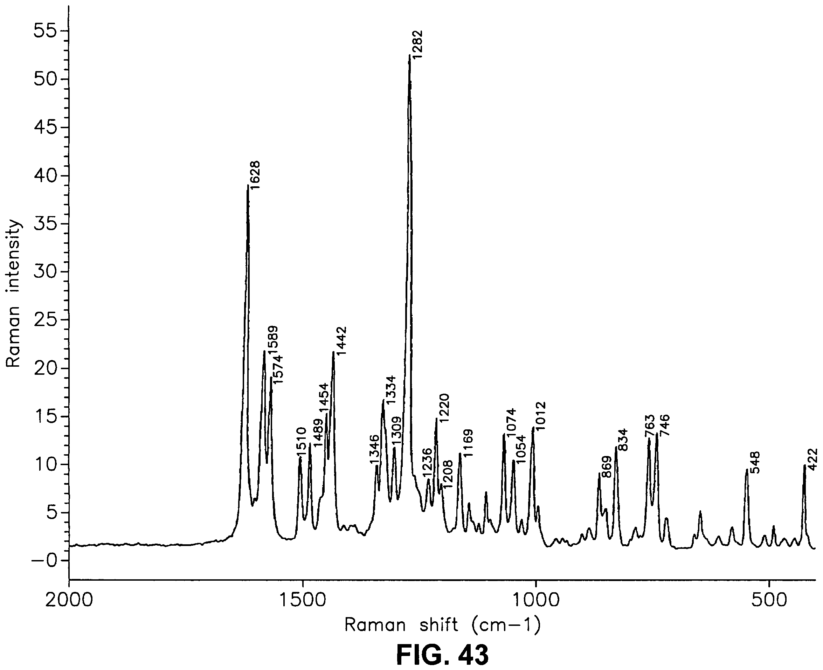

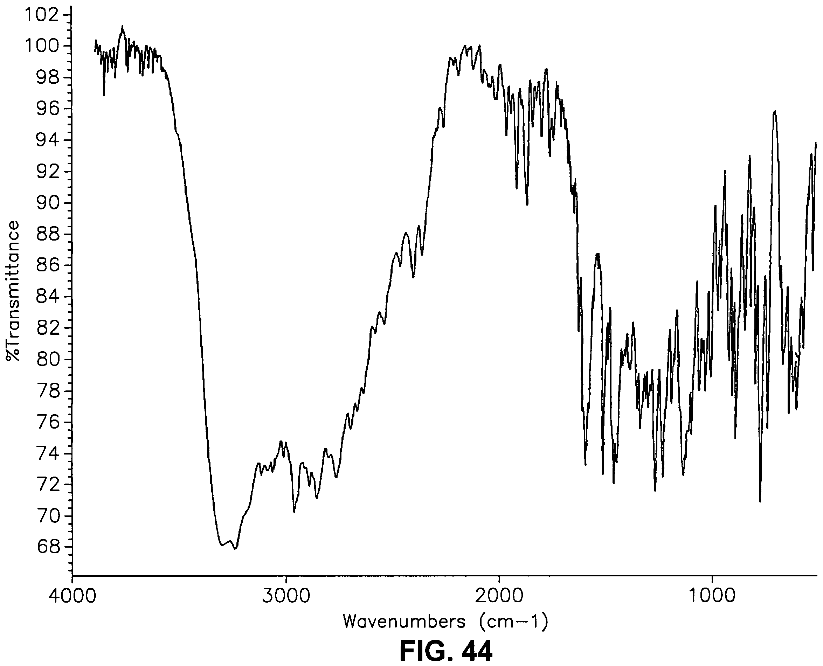

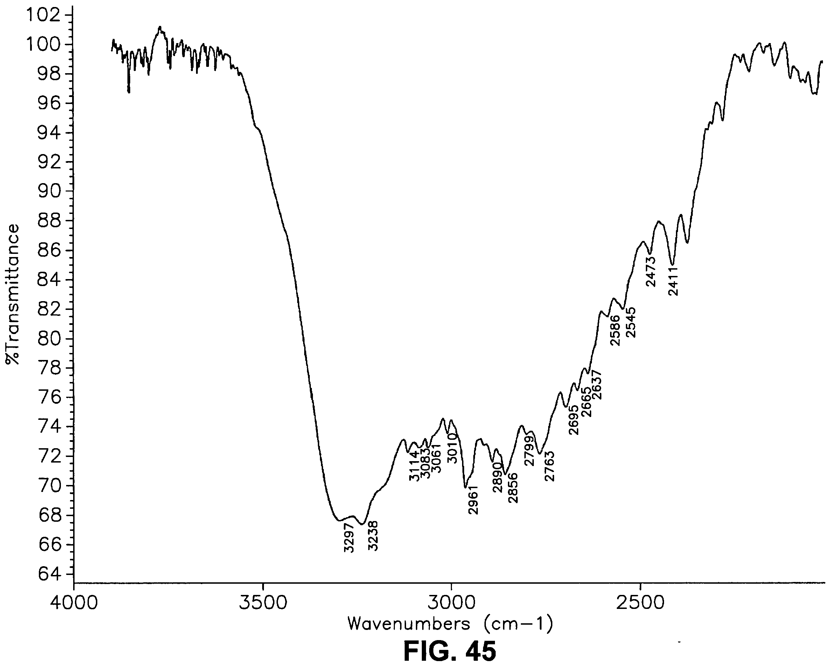

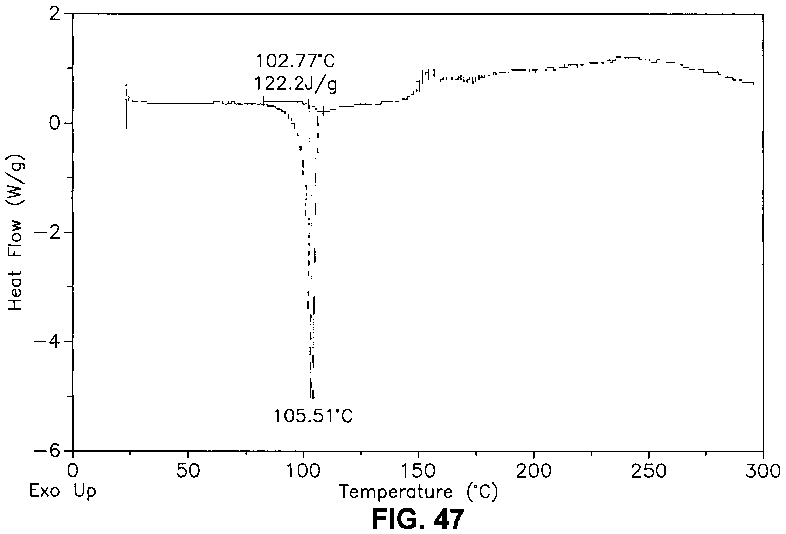

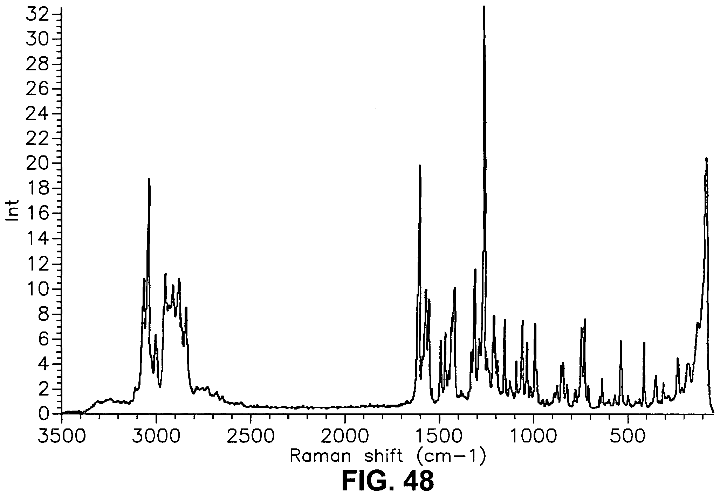

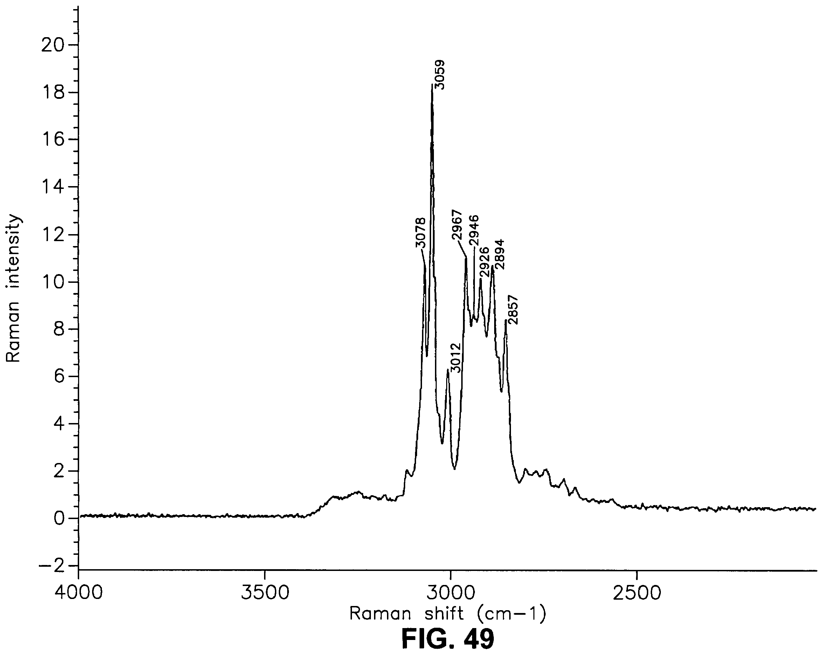

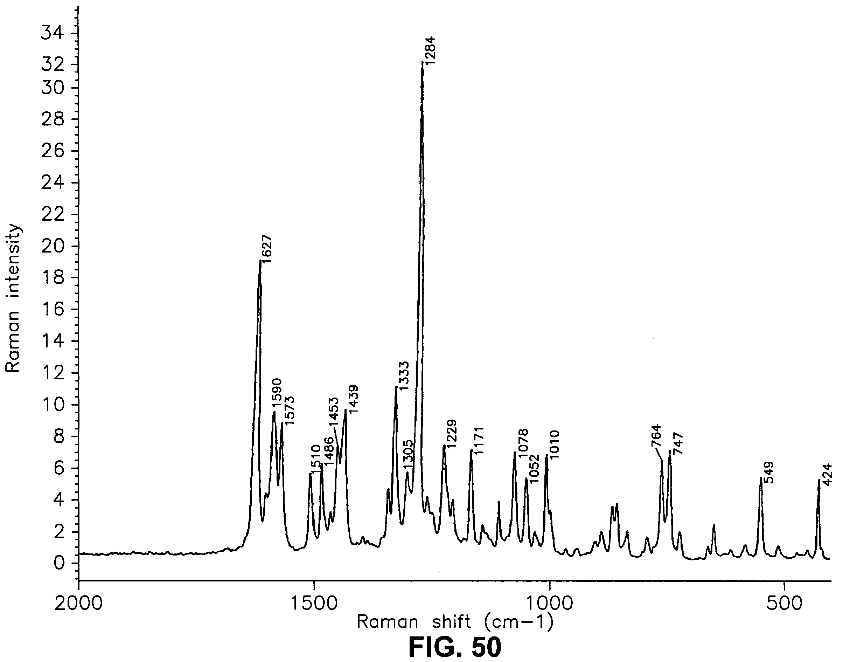

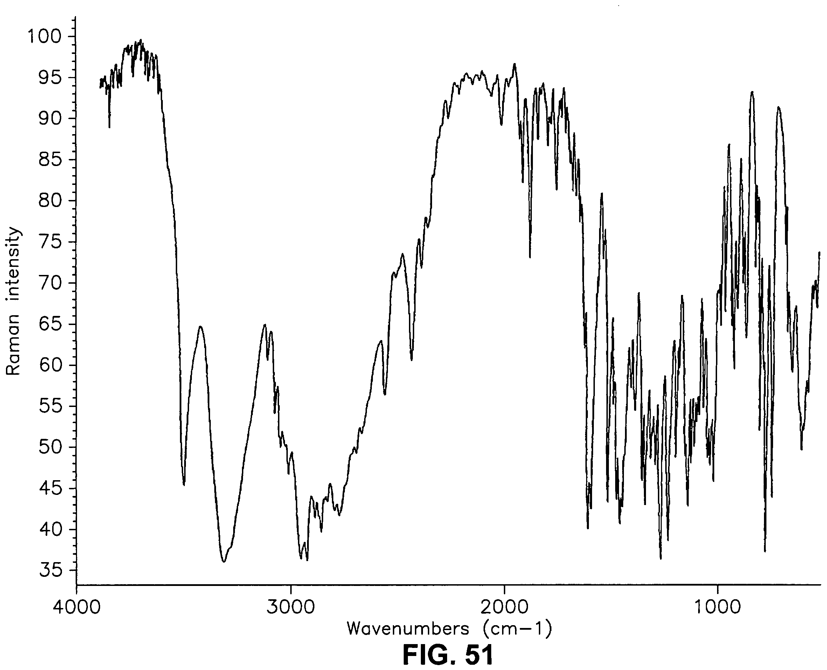

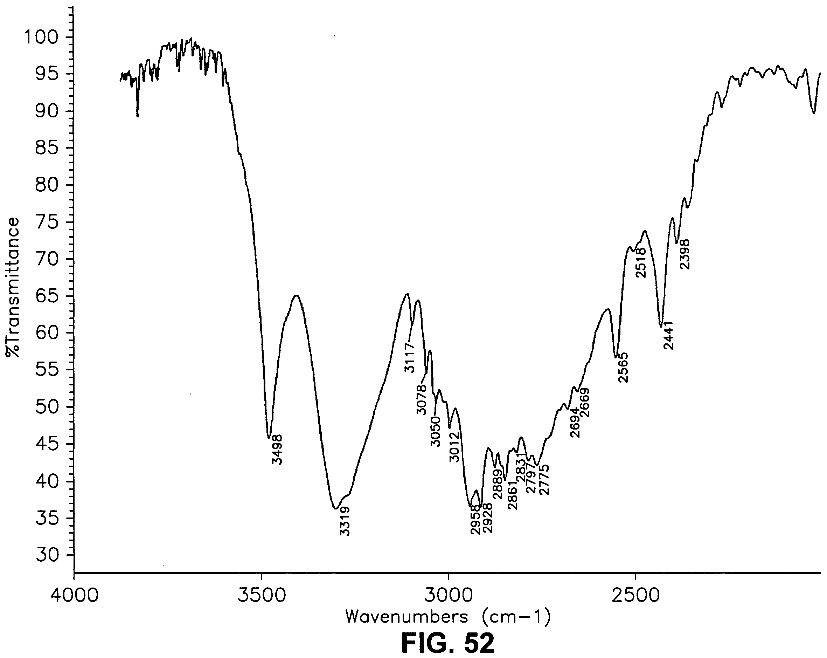

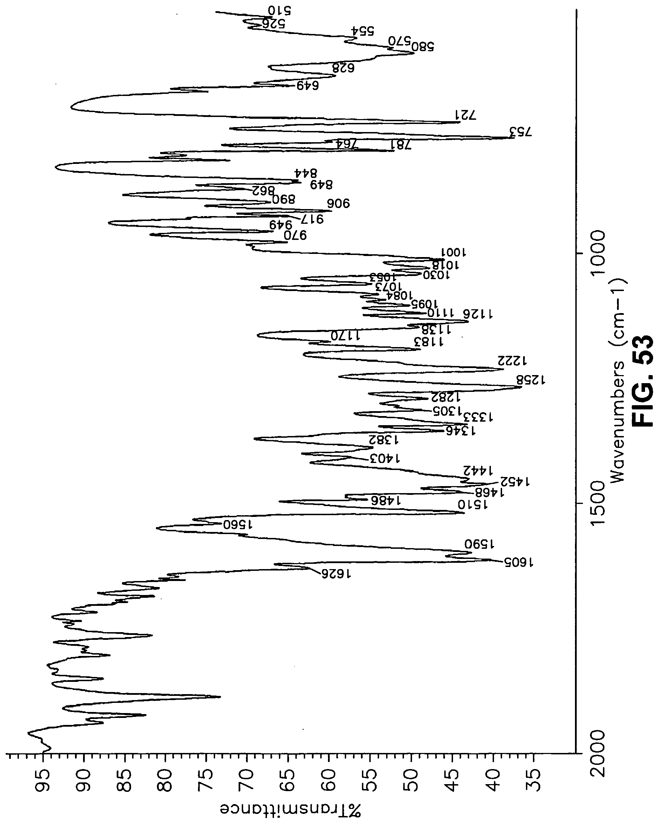

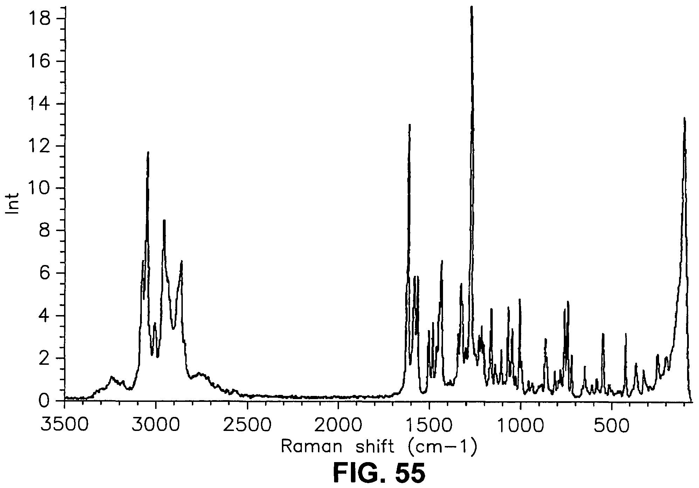

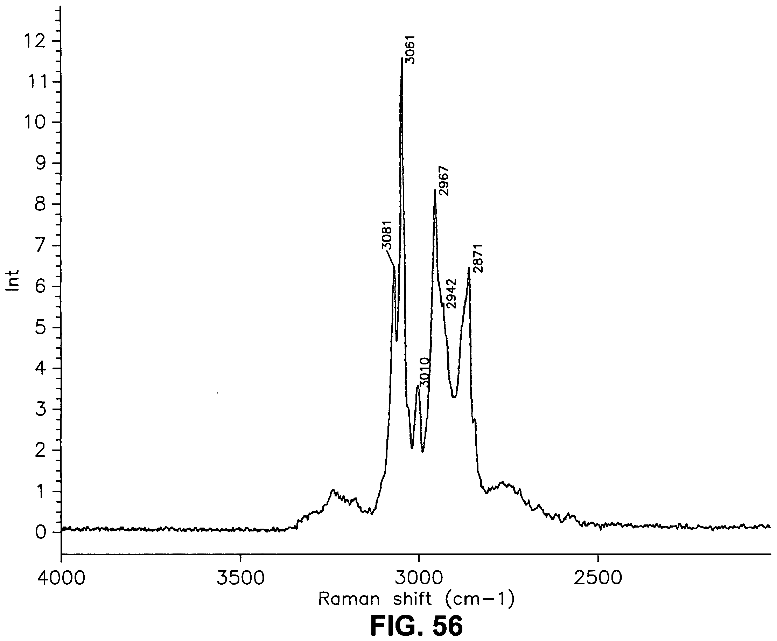

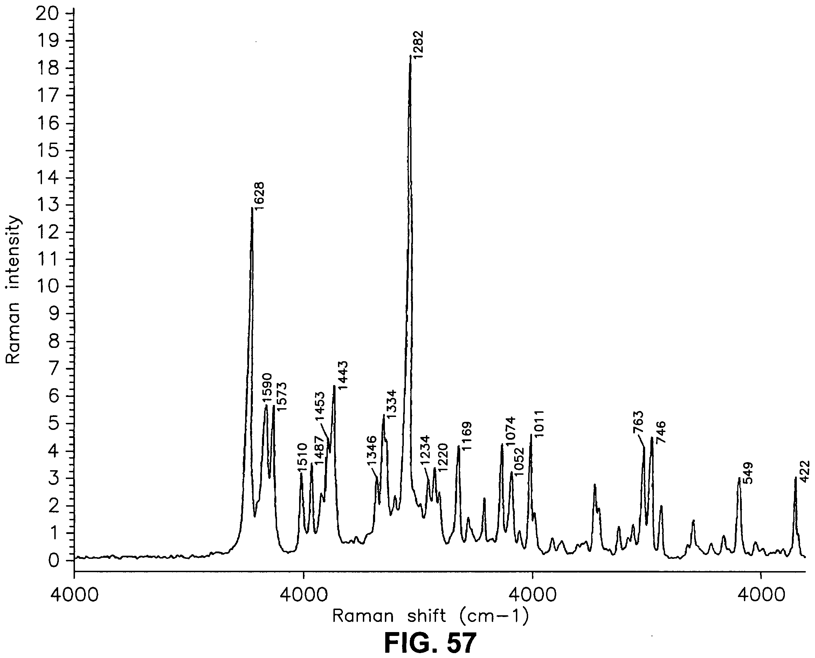

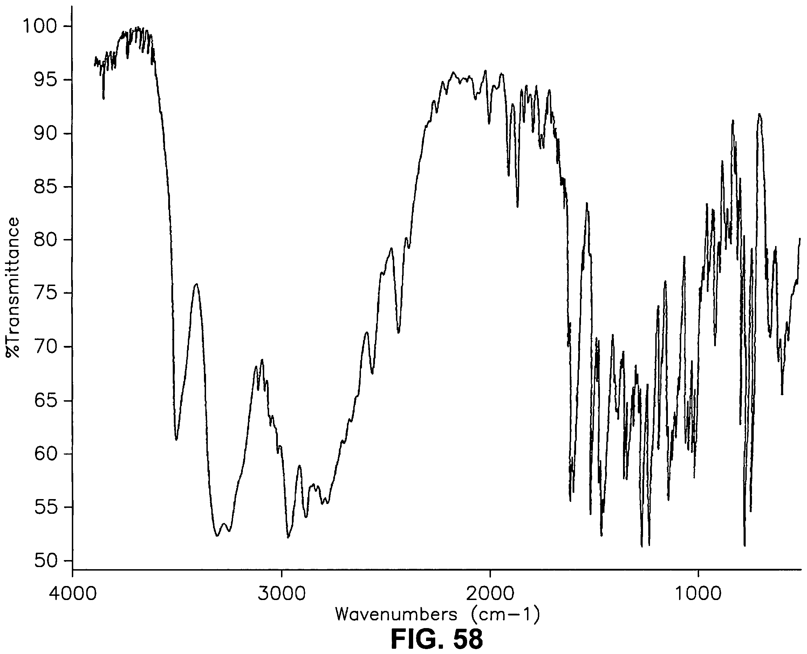

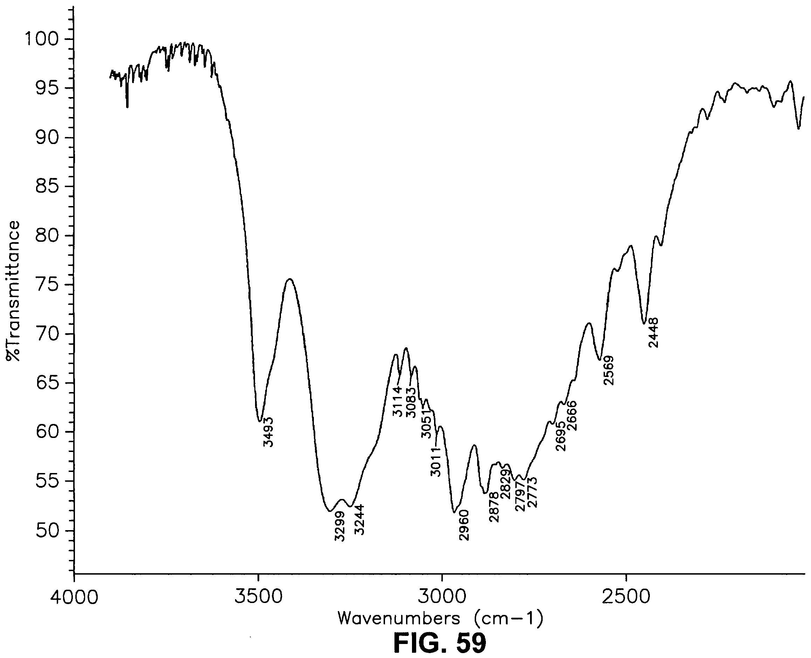

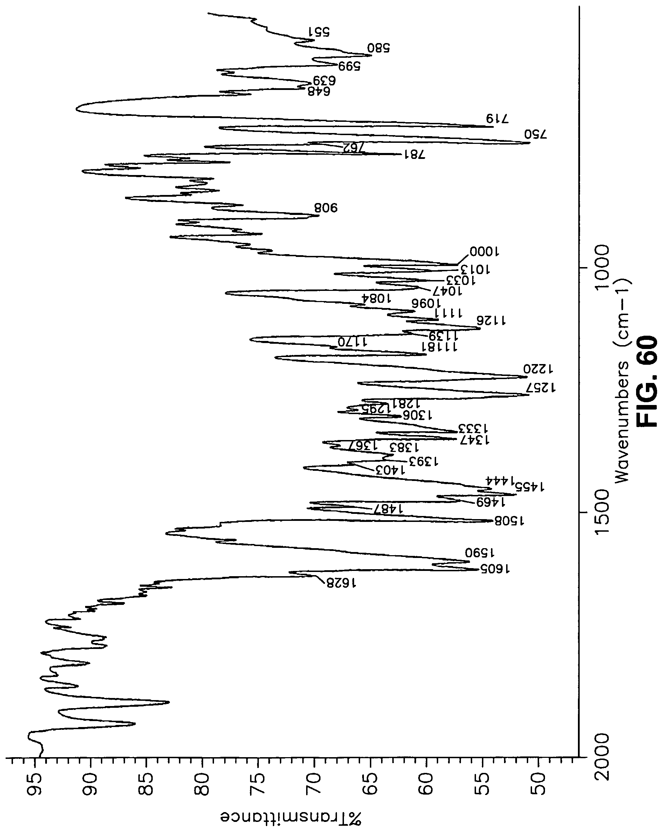

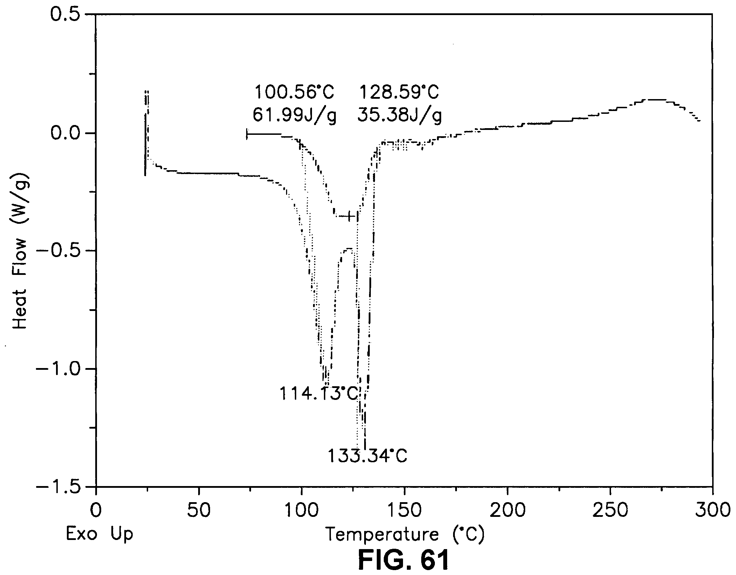

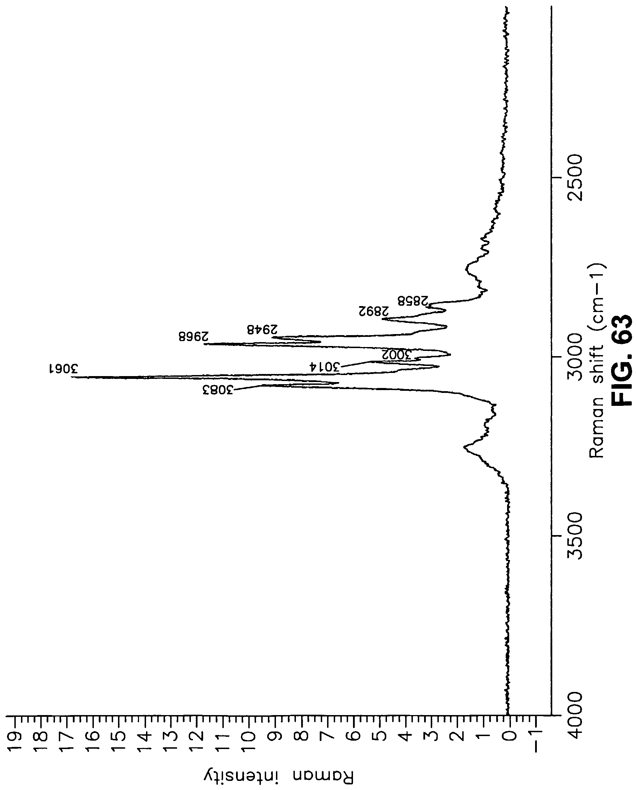

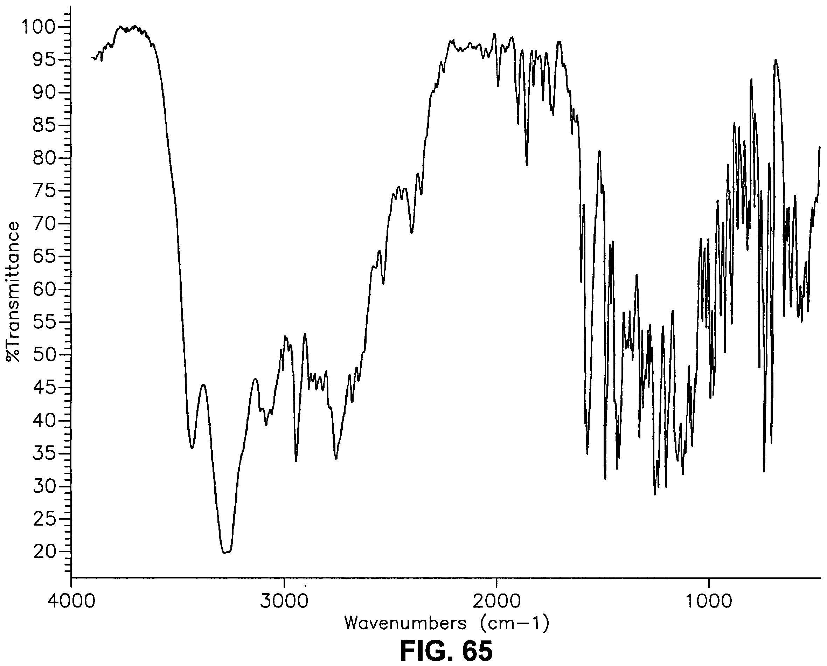

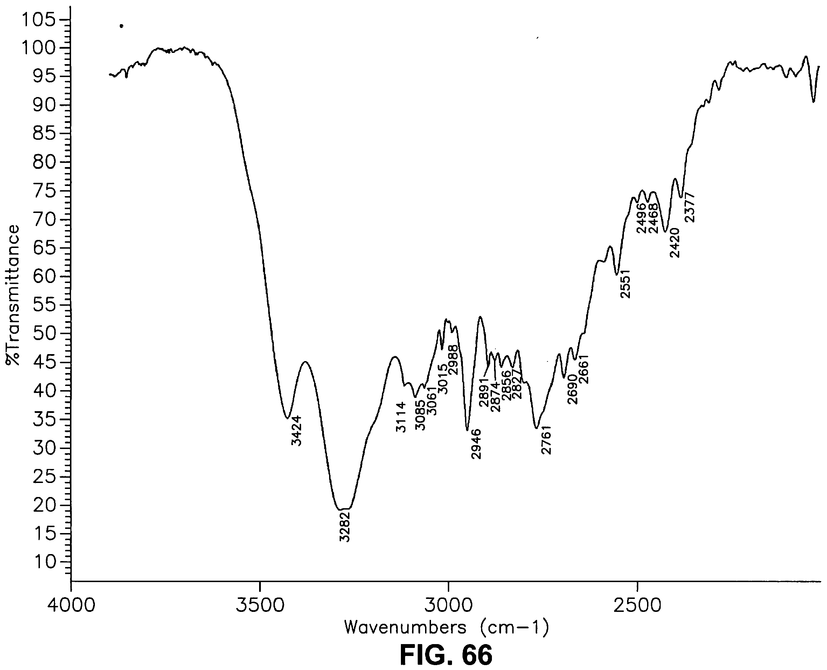

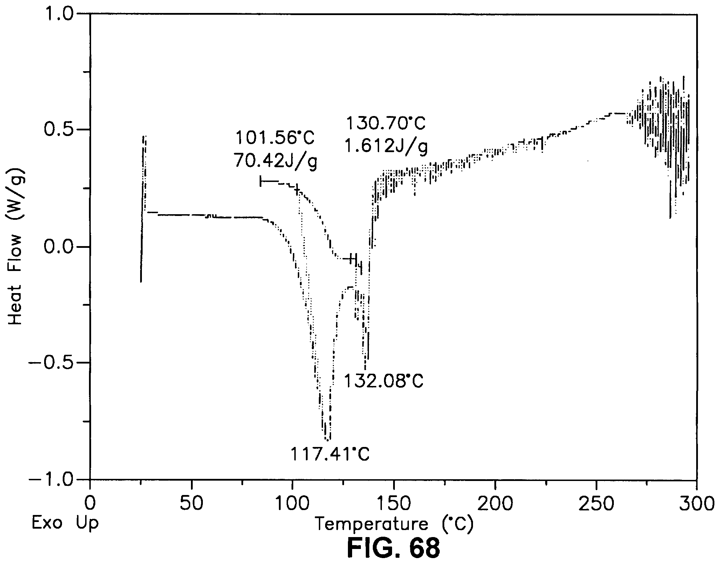

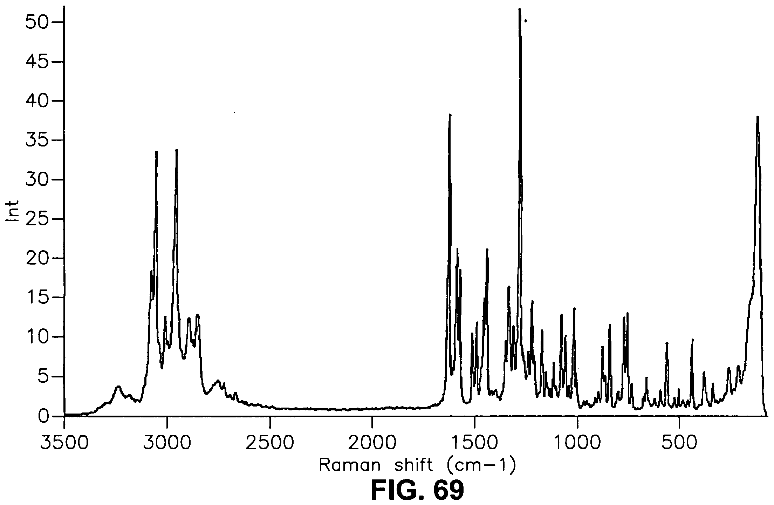

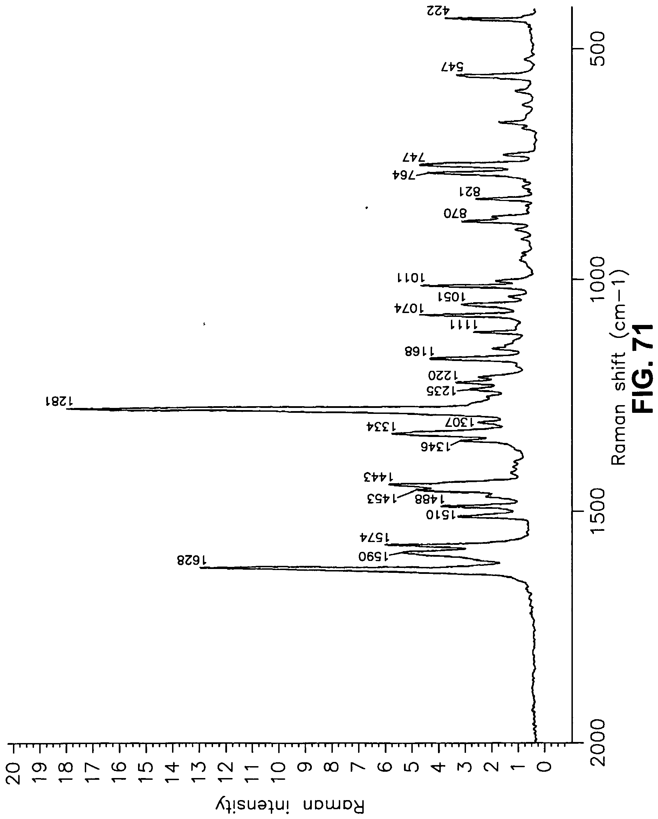

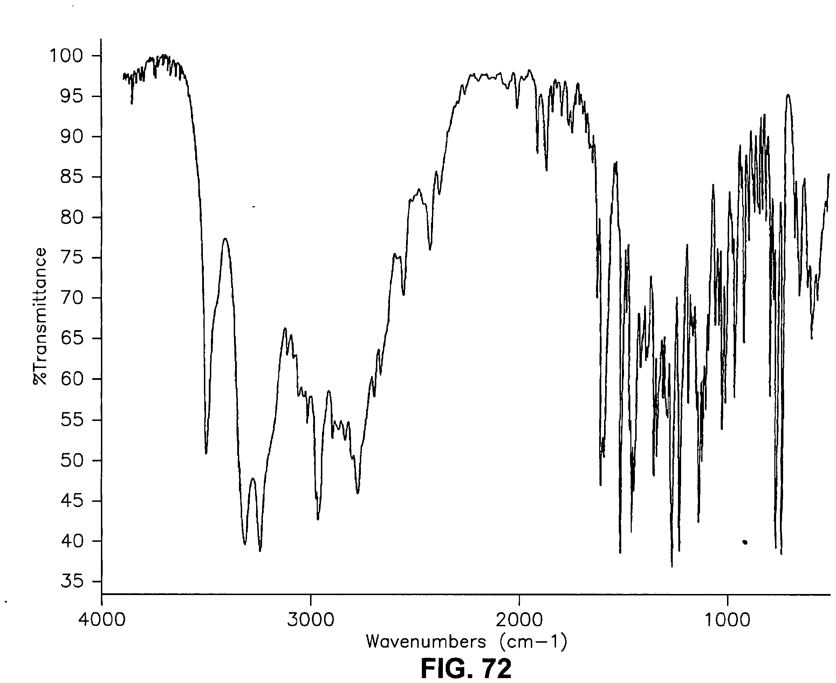

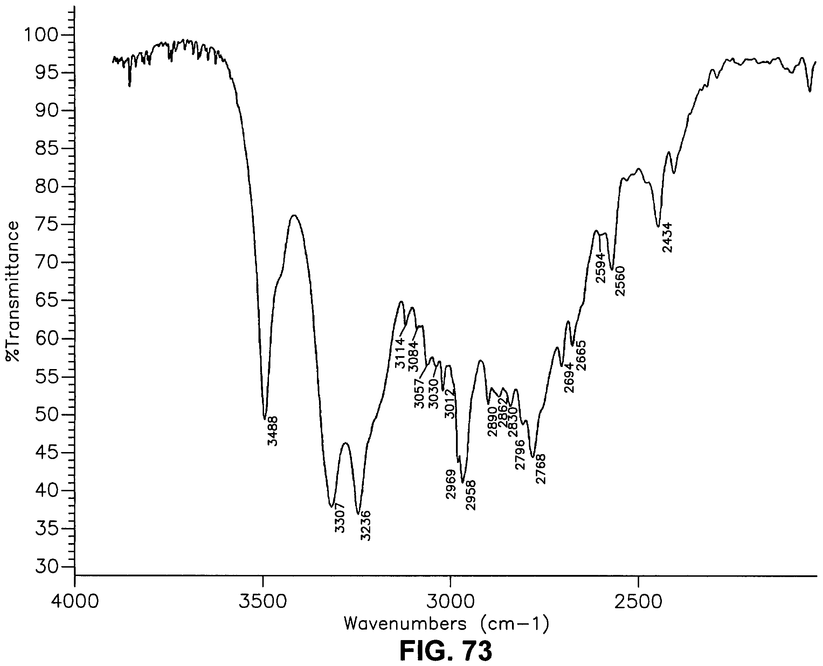

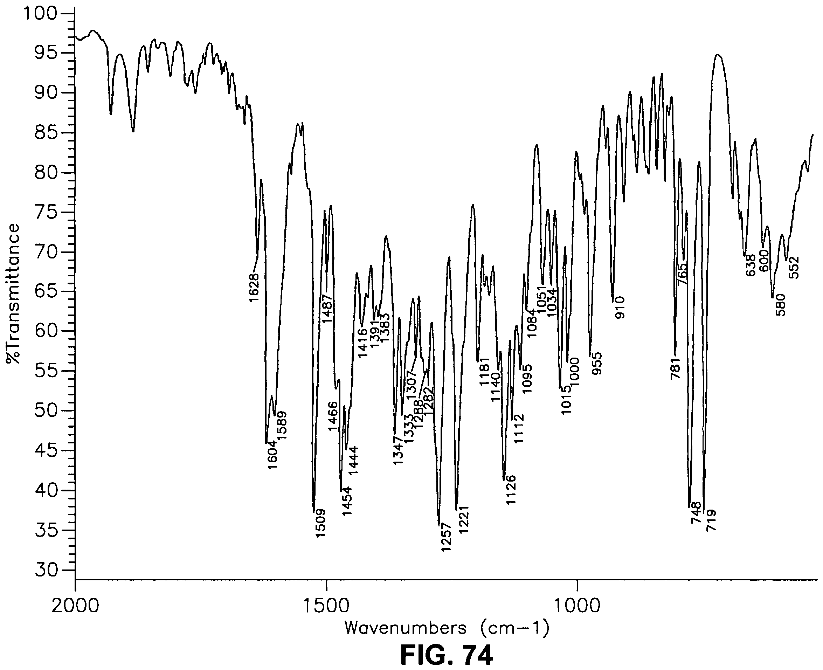

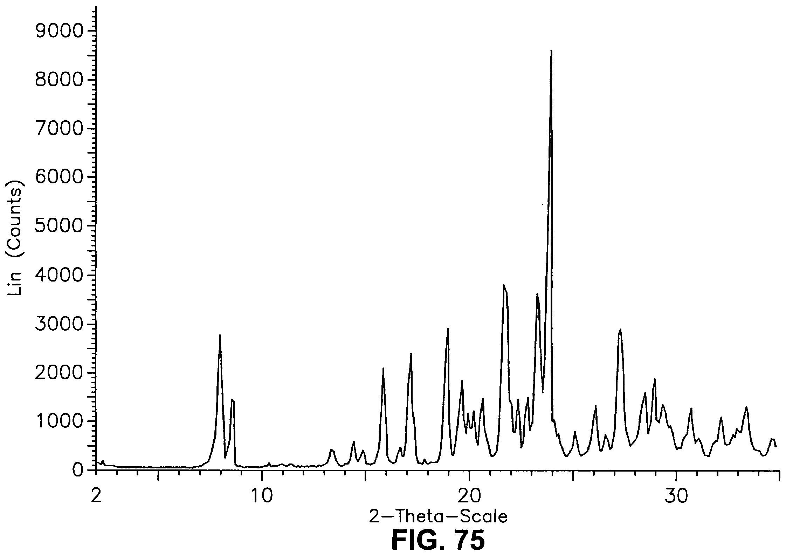

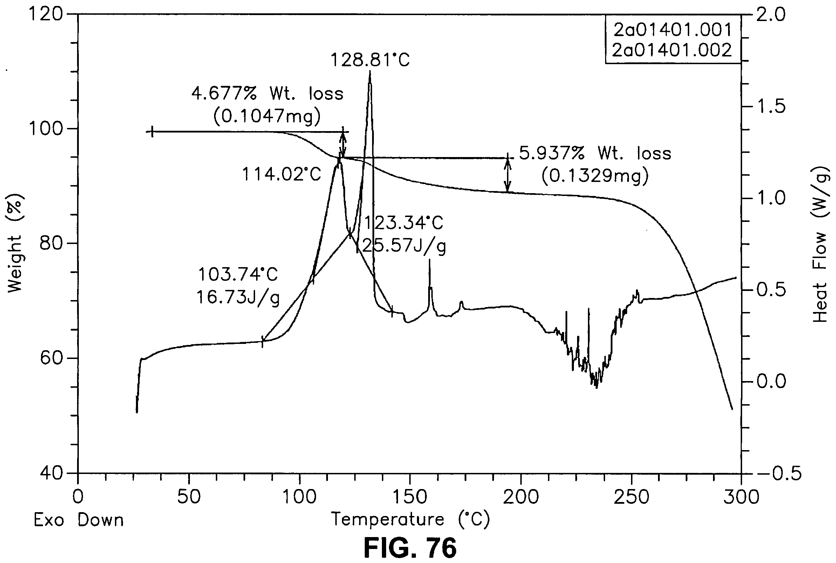

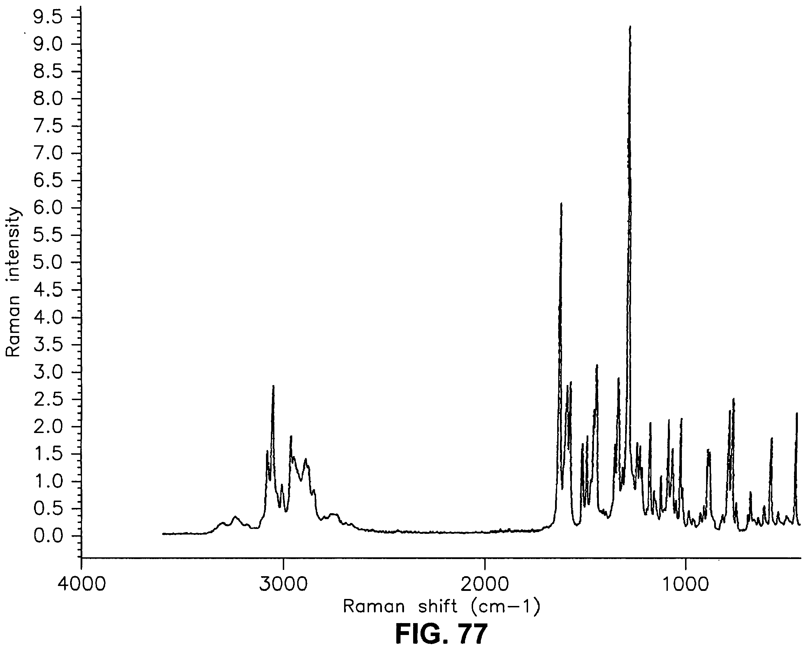

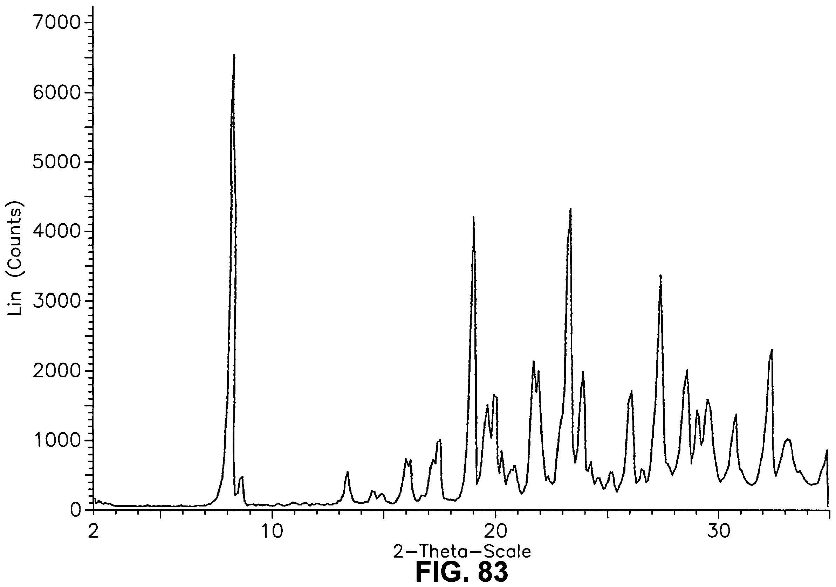

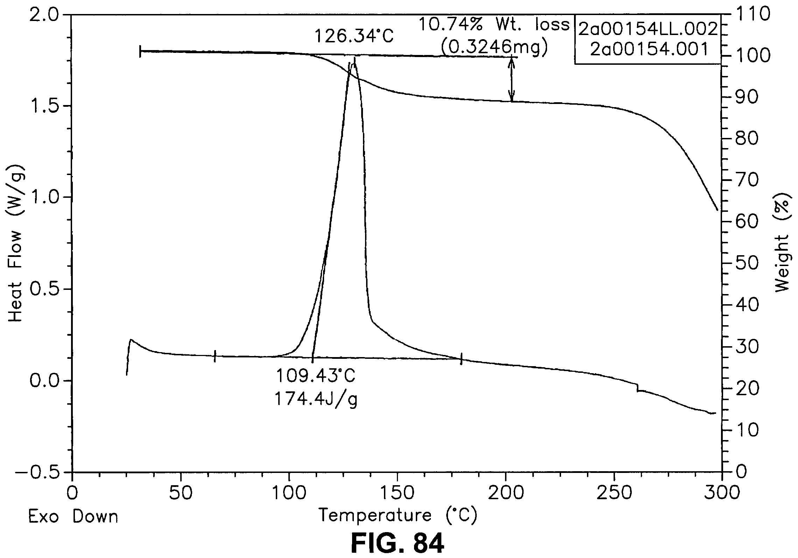

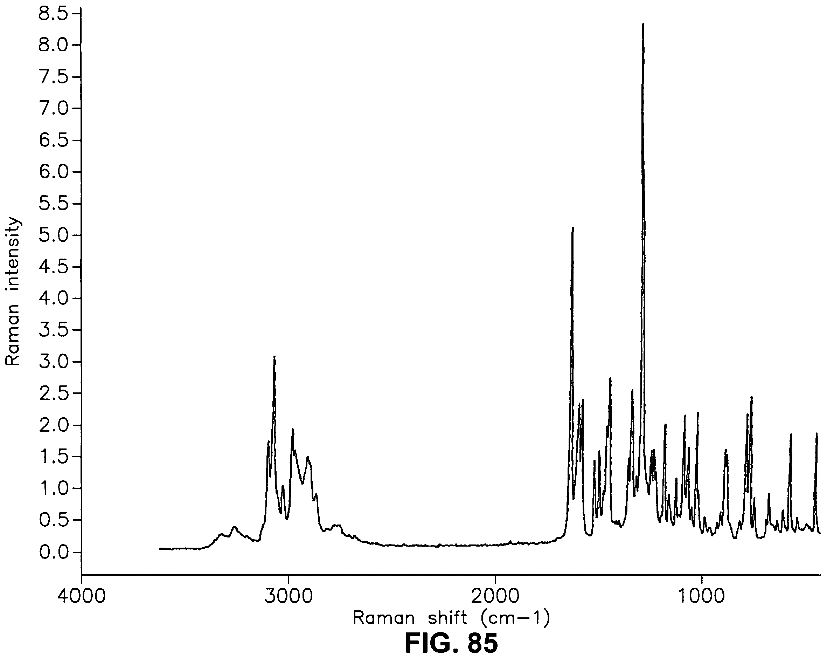

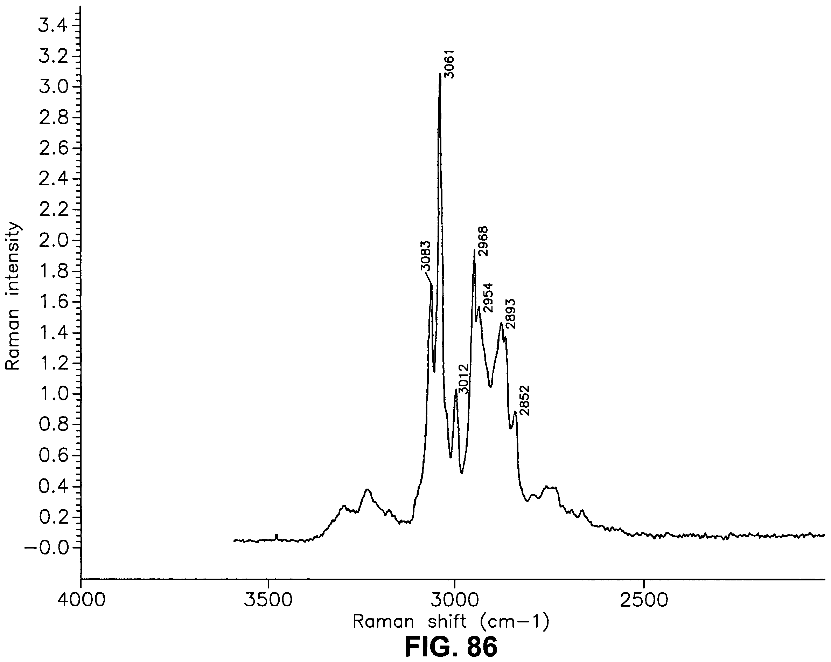

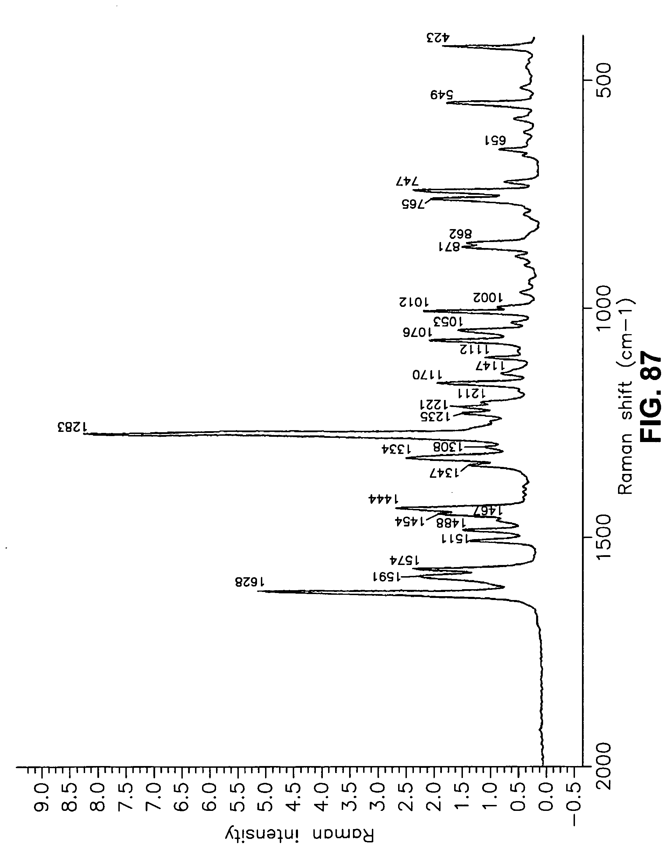

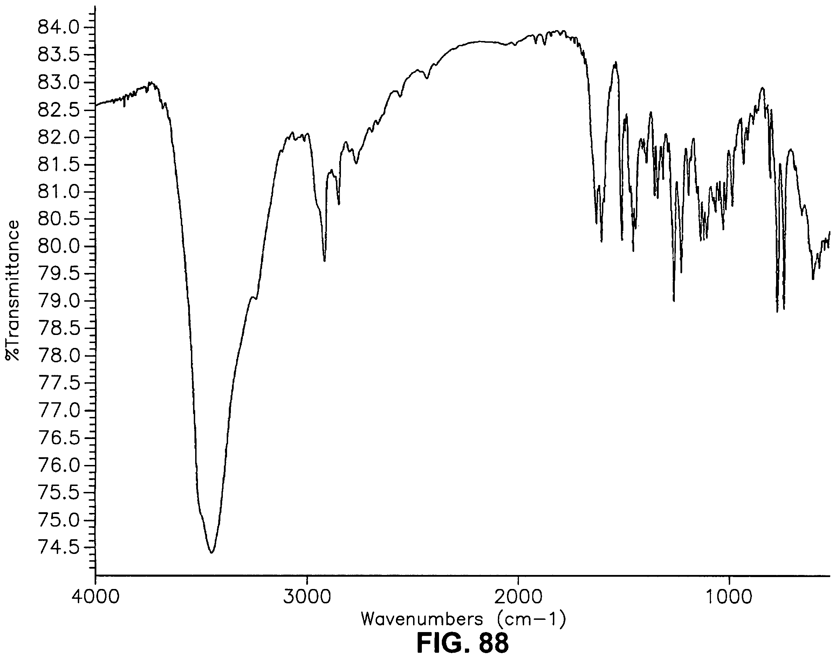

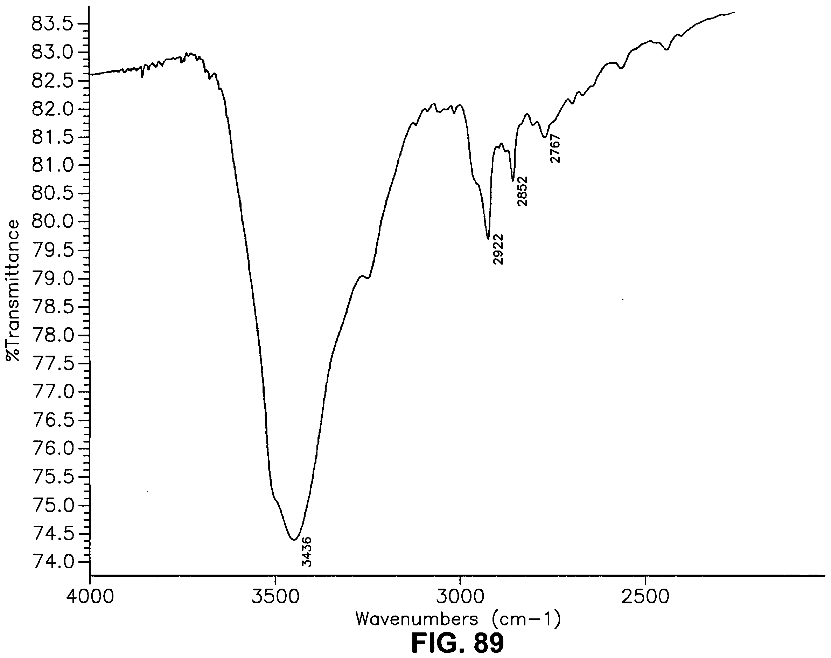

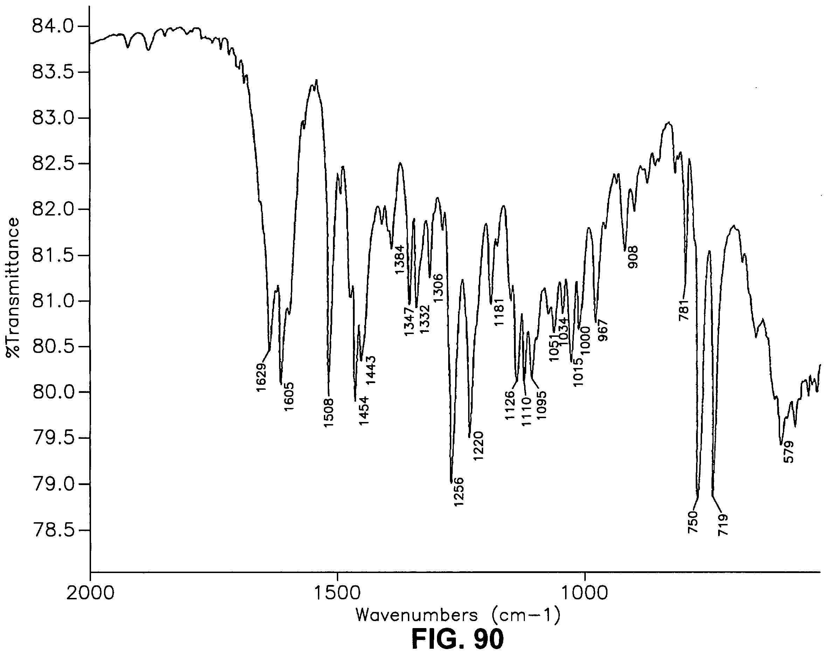

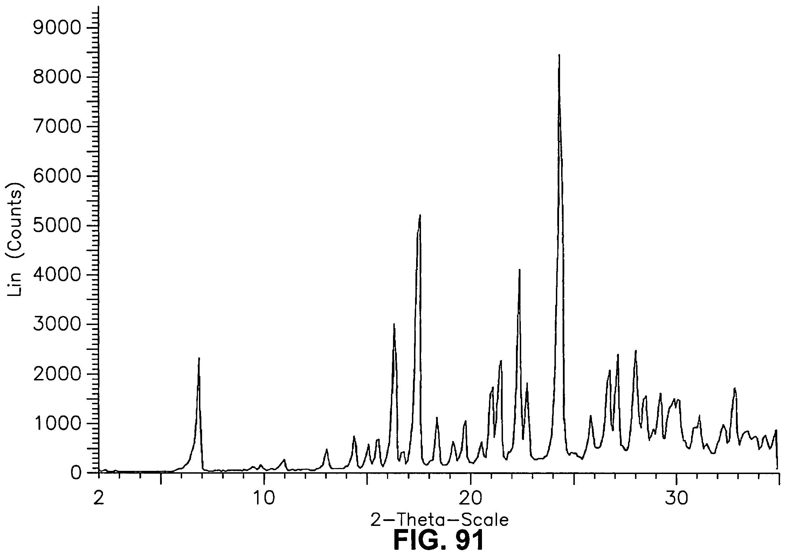

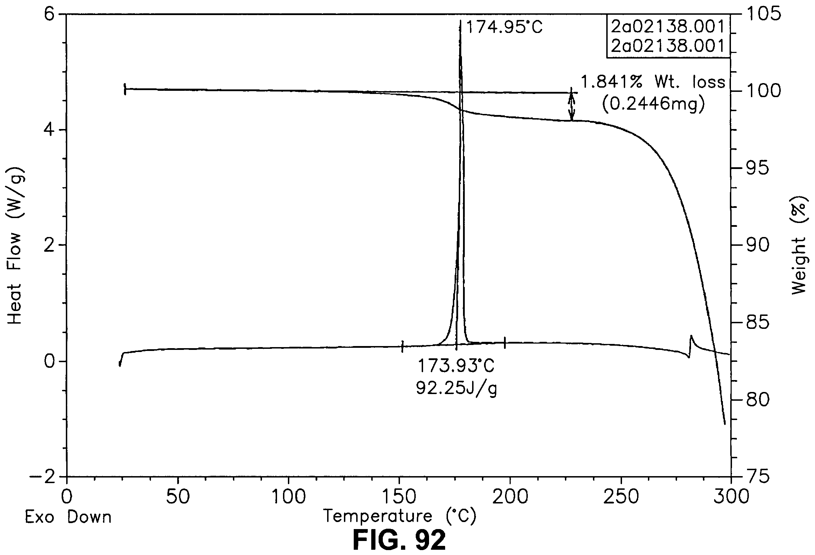

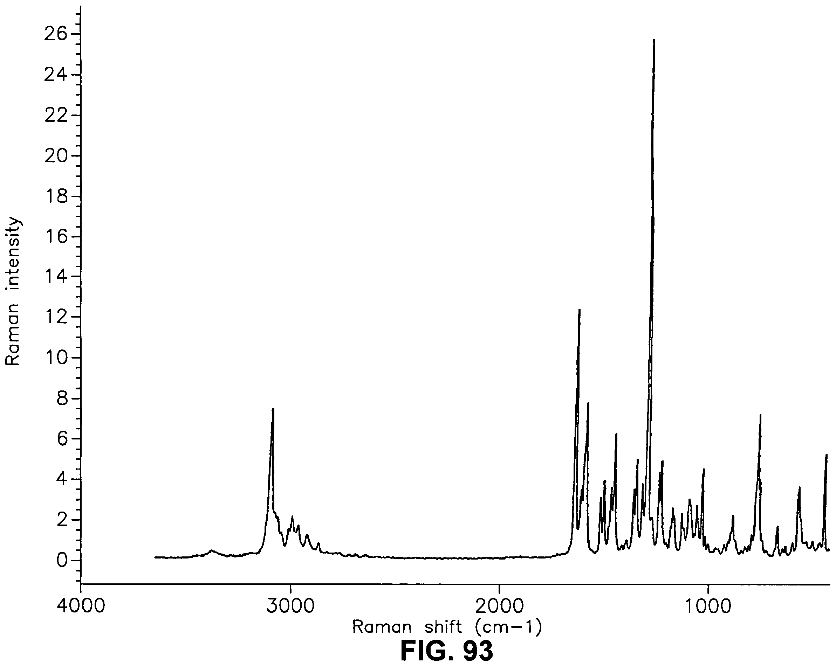

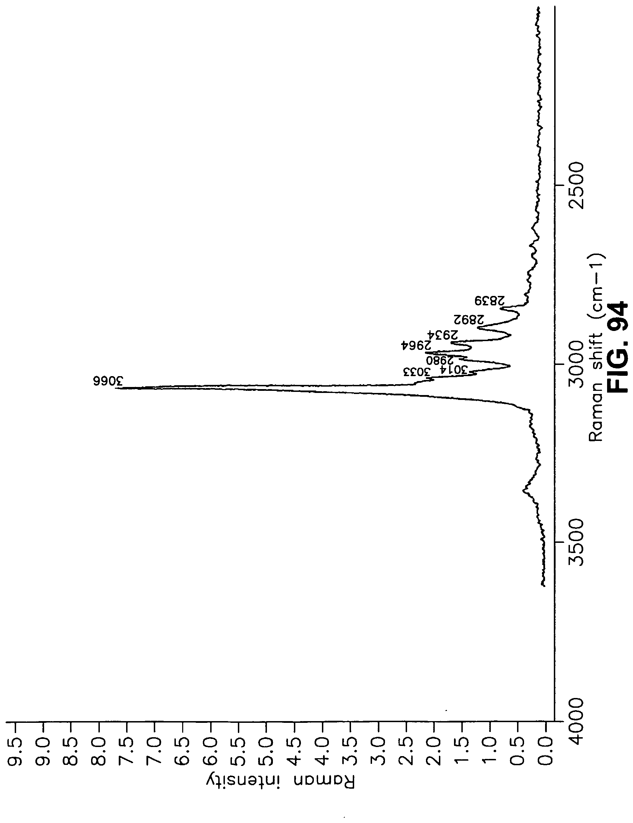

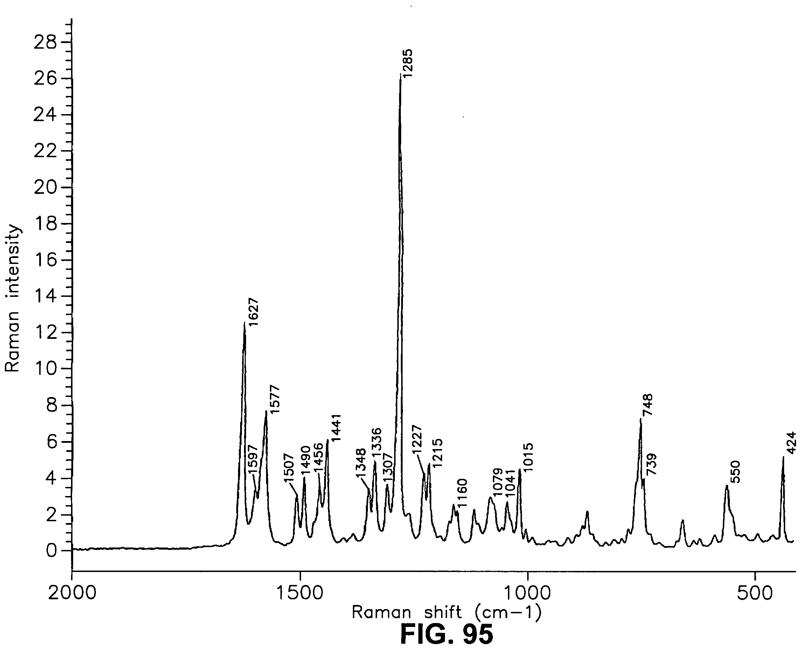

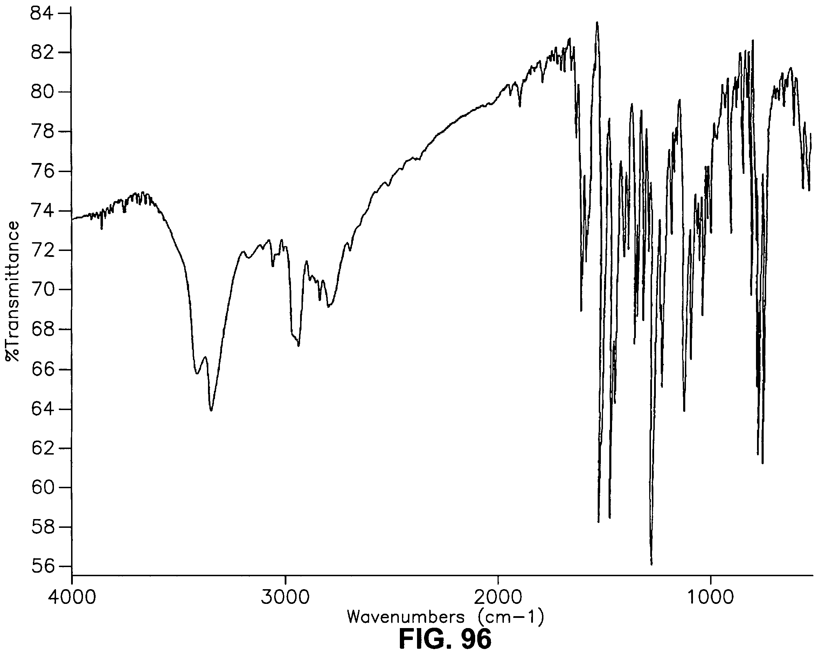

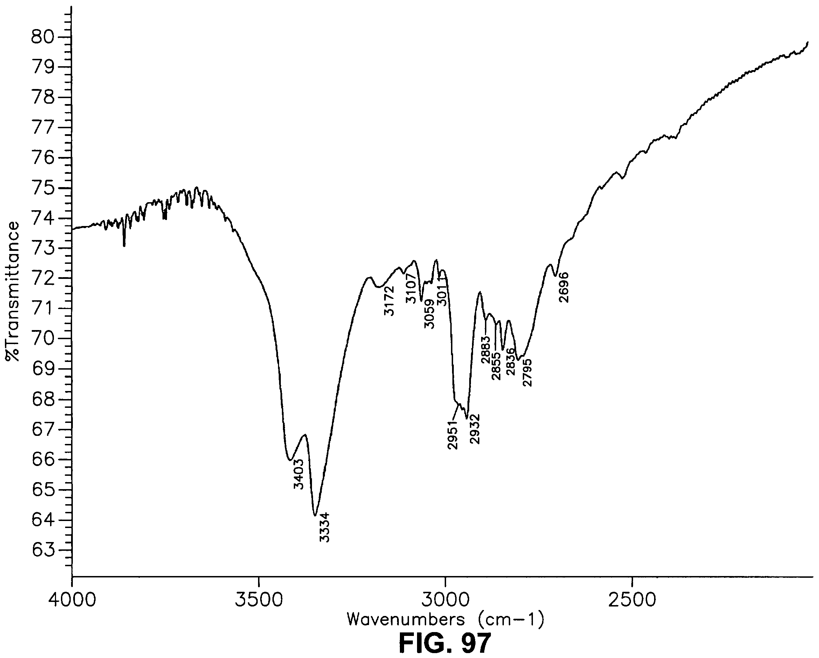

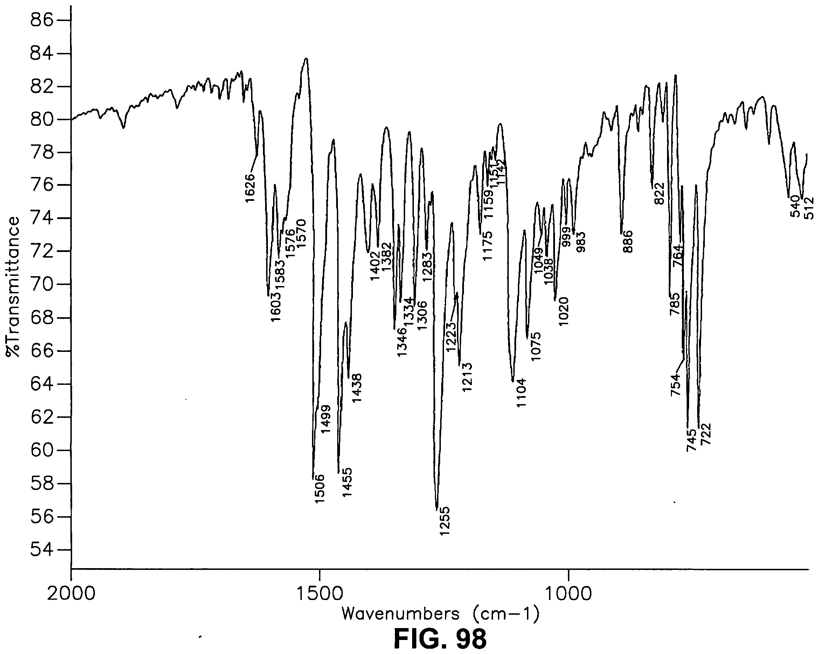

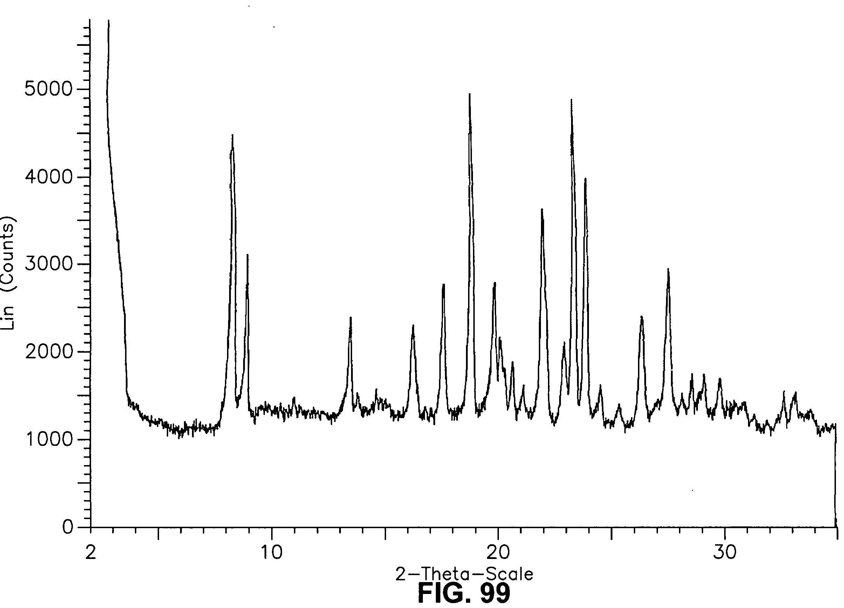

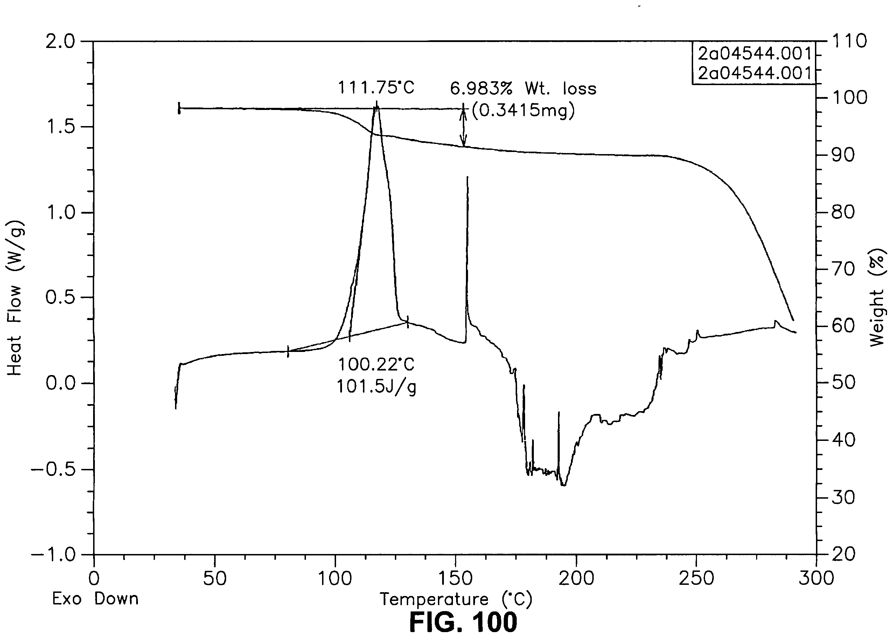

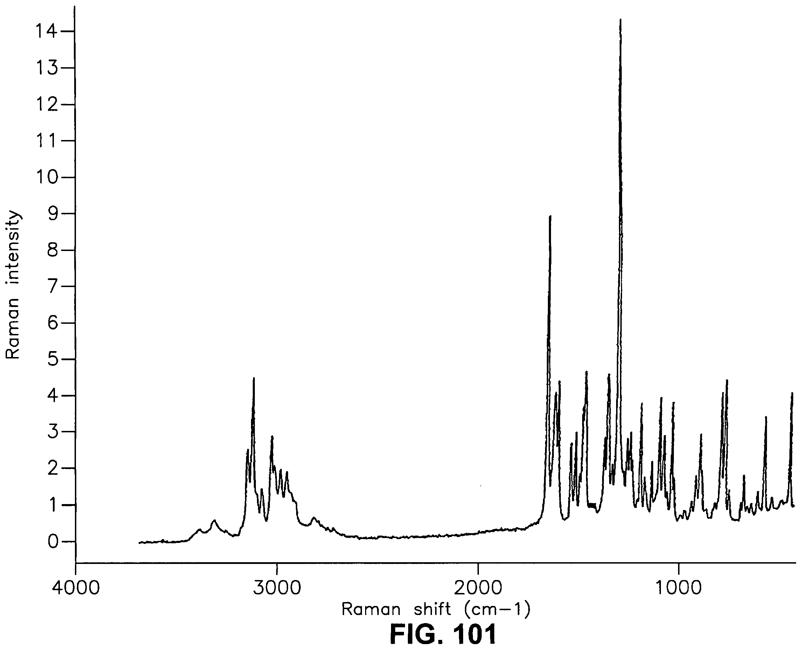

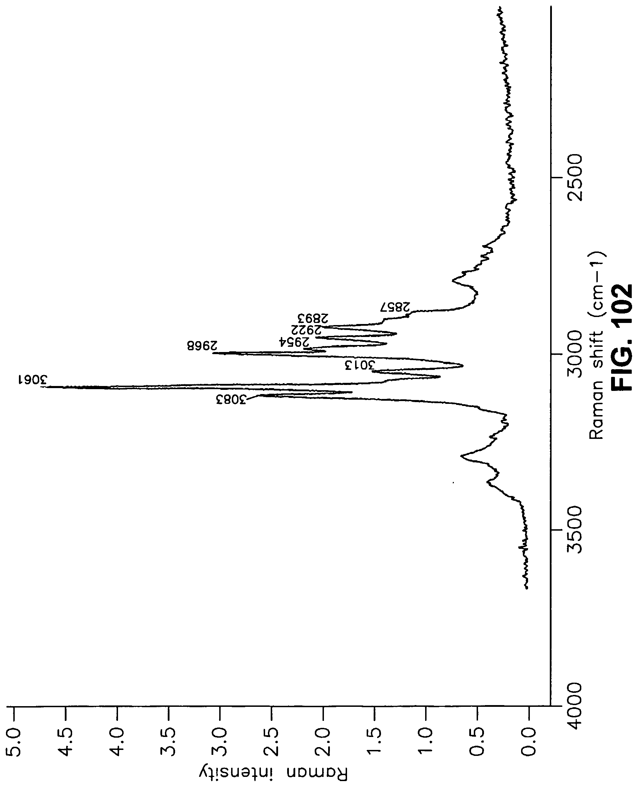

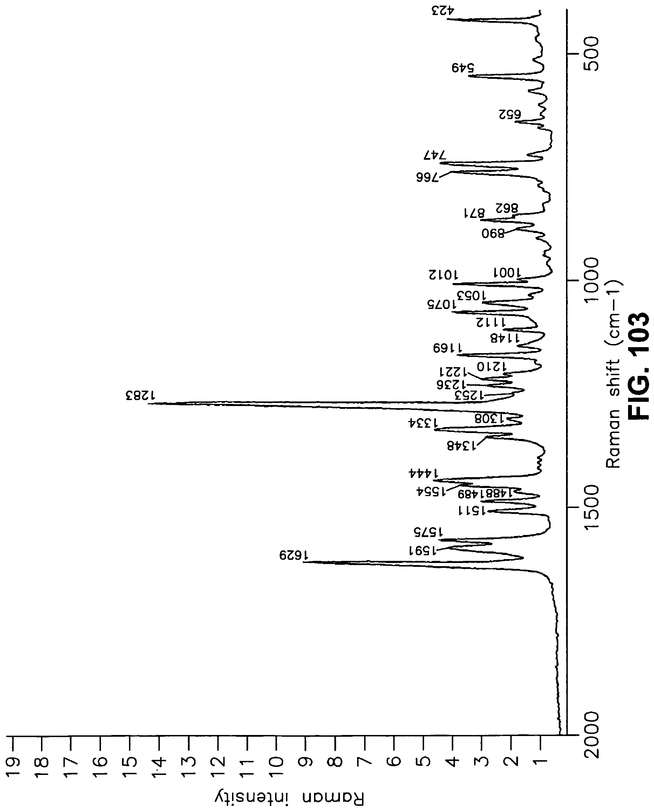

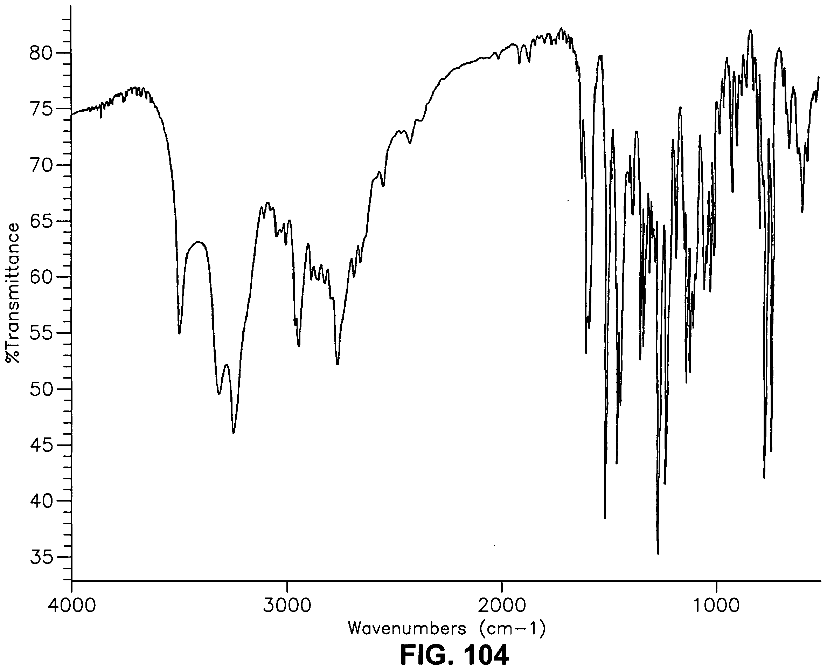

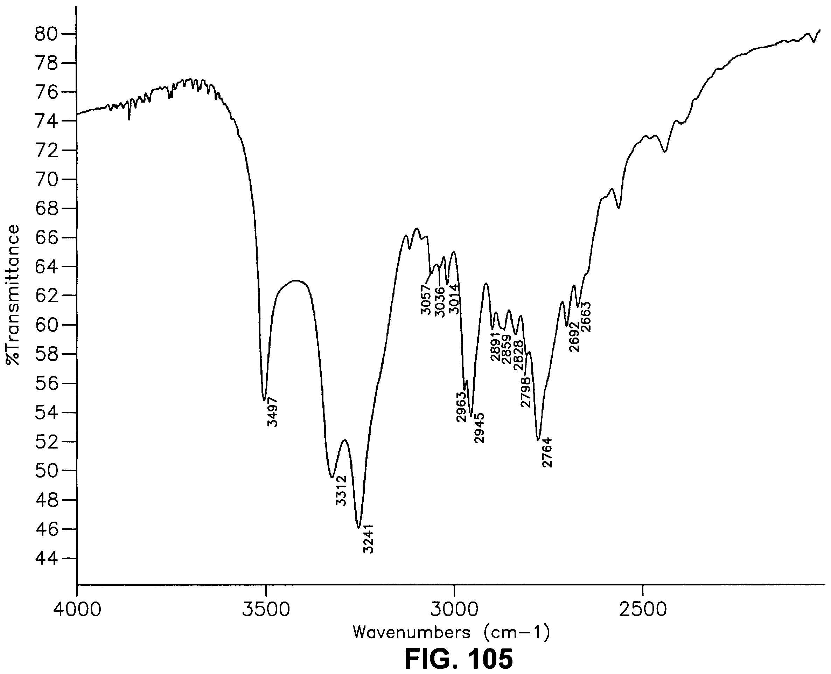

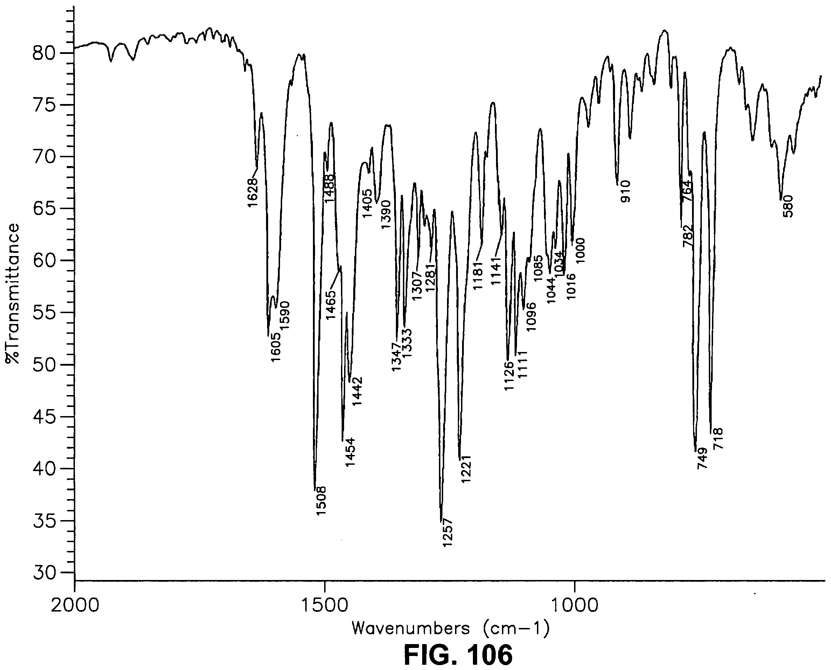

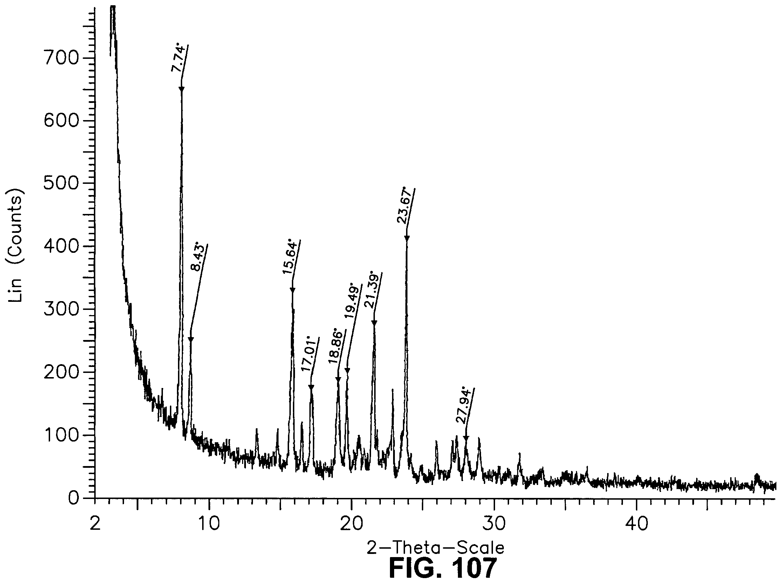

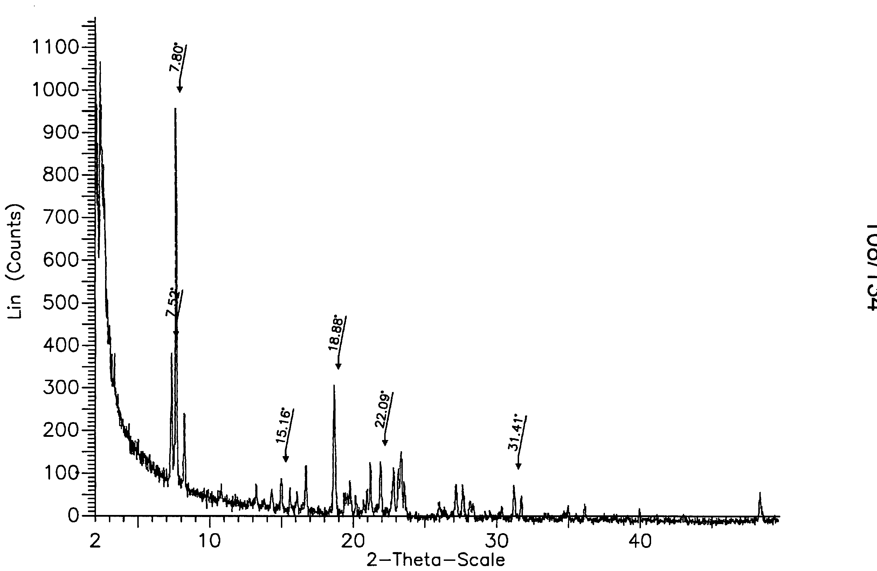

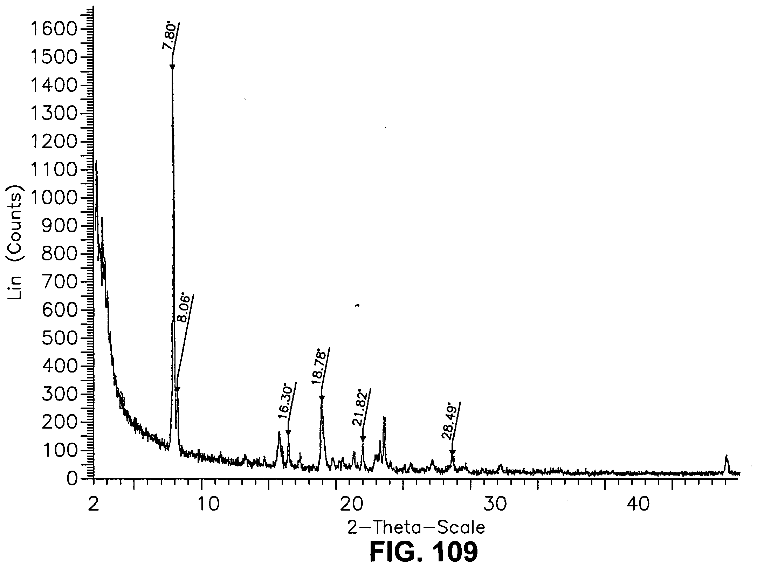

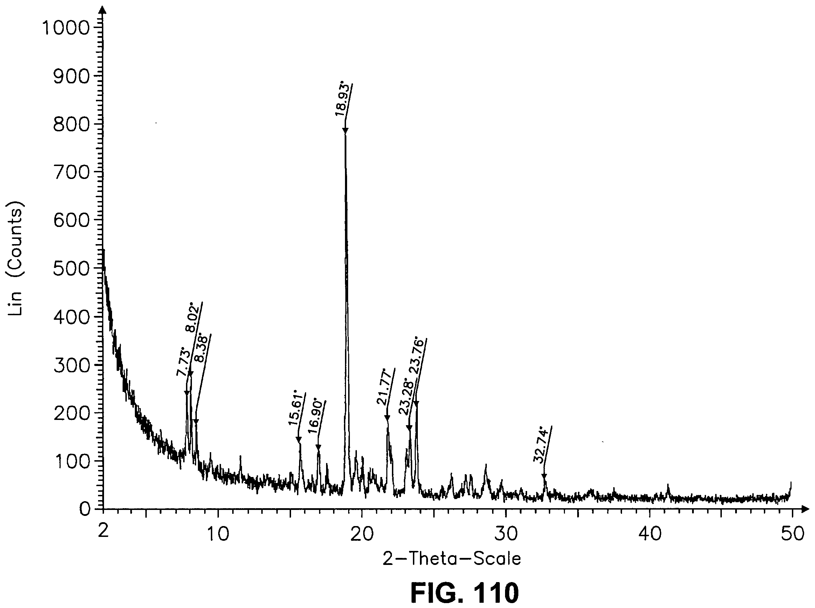

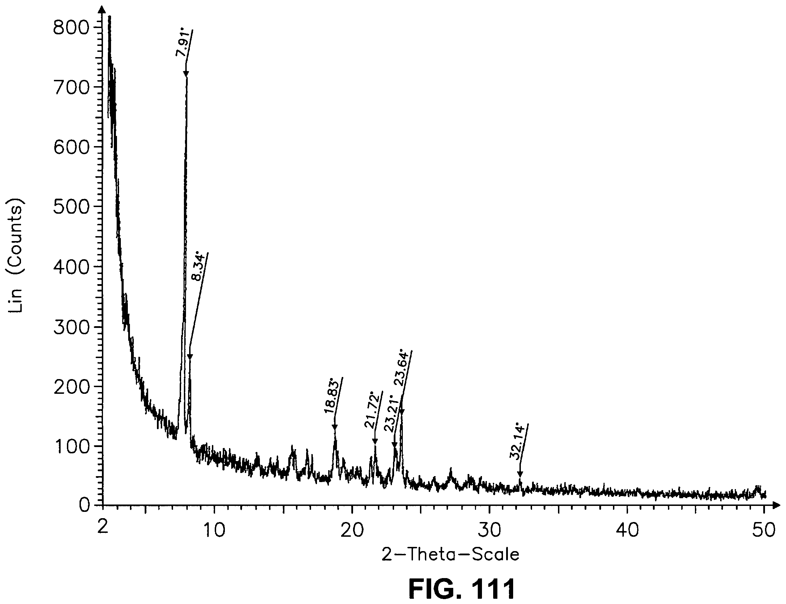

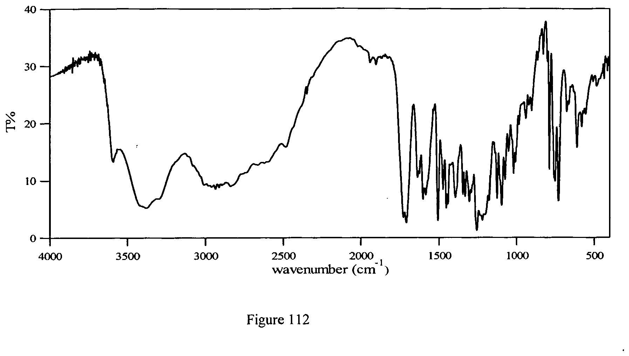

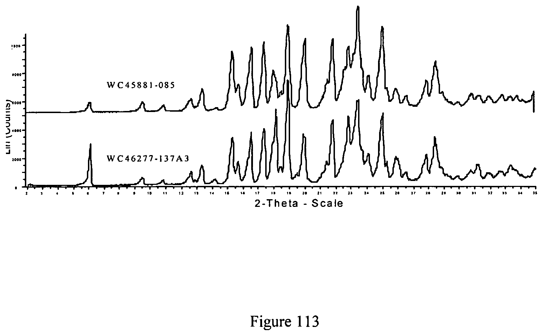

In general, Figures 1-125 depict spectroscopic and other characterizing data for different, specific, and distinct carvedilol salt, anhydrous forms, or solvate forms thereof, which may include, but are not limited to crystalline or other solid forms . For example, carvedilol dihydrogen phosphate, may be isolated as two different and distinct crystalline forms, Forms II and IV, respectively represented and substantially shown Figures 9 to 6 (for Form II) and Figure 25 (for Form IV), which represent spectroscopic and/or other characterizing data.

It is recognized that the compounds of the present invention may exist in forms as stereoisomers, regioisomers, or diastereiomers. These compounds may contain one or more asymmetric carbon atoms and may exist in racemic and optically active forms. For example, carvedilol may exist as racemic mixture of R(+) and S(-) enantiomers, or in separate respectively optical forms, i.e., existing separately as either the R(+) enantiomer form or in the S(+) enantiomer form. All of these individual compounds, isomers, and mixtures thereof are included within the scope of the present invention.

Carvedilol salts of the present invention may be prepared by various techniques, such as those exemplified below.

For example, crystalline carvedilol dihydrogen phosphate hemihydrate of the instant invention can be prepared by crystallization from an acetone-water solvent system containing carvedilol and H₃PO₄. Also suitable solvates of carvedilol phosphate salts of present invention may be prepared by preparing a slurrying a carvedilol phosphate salt, such as a carvedilol dihydrogen salt, in a solvent, such as methanol.

In another example, crystalline carvedilol hydrobromide monohydrate of the present invention can be prepared by crystallization from an acetone-water solvent system containing carvedilol and hydrobromic acid. Also, suitable solvates of carvedilol hydrobromide salts may be made by preparing a slurry of the carvedilol hydrobromide salt in a solvent (i.e., such as dioxane, 1-pentanol, 2-methyl-1-propanol, trifluoroethanol, 2-propanol and n-propanol. In particular, solvates of carvedilol hydrobromide as defined in the present invention, include, but are not limited to carvedilol hydrobromide 1-pentanol solvate, carvedilol hydrobromide 2-methyl-1-pentanol solvate, carvedilol hydrobromide trifluoroethanol solvate, carvedilol hydrobromide 2-propanol solvate, carvedilol hydrobromide n-propanol solvate #1, carvedilol hydrobromide n-propanol solvate #2, carvedilol hydrobromide ethanol solvate, carvedilol hydrobromide anhydrous forms), and/or dissolving the carvedilol hydrobromide salt in the aforementioned solvents and allowing the salt to crystallize out. Carvedilol hydrobromide anhydrous forms can be prepared by dissolving carvedilol in a solvent, such as dichloromethane, acetonitrile or isopropyl acetate, followed by the addition of anhydrous HBr (HBr in acetic acid or gaseous HBr).

In yet another example, the crystalline carvedilol citrate salt of the instant invention can be prepared by making an aqueous citric acid solution saturated with carvedilol, either by lowering the temperature of the solution, or slowly evaporating water from the solution. In addition, it can be prepared by crystallization from an acetone-water solvent system containing carvedilol and citric acid.

A particularly useful and surprising characteristic of the crystalline form of carvedilol citrate salt stems from the fact that citric acid is a prochiral molecule. Consequently, a 1 to 1 ratio of racemic diasteromers are present in the crystalline carvedilol citrate salt lattice. This avoids generation of yet more optically active forms that could potentially complicate stability, dissolution rates, *in vivo* absorption metabolism and possibly pharmacologic effects.

According to the instant invention, the various salt forms of carvedilol and/or corresponding solvates thereof are distinguished from each other using different characterization or identification techniques. Such techniques, include solid state ¹³C Nuclear Magnetic Resonance (NMR), ³¹P Nuclear Magnetic Resonance (NMR), Infrared (IR), Raman, X-ray powder diffraction, etc. and/or other techniques, such as Differential Scanning Calorimetry (DSC) (i.e., which measures the amount of energy (heat) absorbed or released by a sample as it is heated, cooled or held at constant temperature).

In general, the aforementioned solid state NMR techniques are nondestructive techniques to yield spectra, which depict an NMR peak for each magnetically non-equivalent carbon site the solid-state

For example, in identification of compounds of the present invention, ¹³C NMR spectrum of a powdered microcrystalline organic molecules reflect that the number of peaks observed for a given sample will depend on the number of chemically unique carbons per molecule and the number of non-equivalent molecules per unit cell. Peak positions (chemical shifts) of carbon atoms reflect the chemical environment of the carbon in much the same manner as in solution-state ¹³C NMR. Although peaks can overlap, each peak is in principle assignable to a single type of carbon. Therefore, an approximate count of the number of carbon sites observed yields useful information about the crystalline phase of a small organic molecule.

Based upon the foregoing, the same principles apply to phosphorus, which has additional advantages due to high sensitivity of the ³¹P nucleus.

Polymorphism also can be studied by comparison of ¹³C and ³¹P spectra. In the case of amorphous material, broadened peak shapes are usually observed, reflecting the range of environments experienced by the ¹³C or ³¹P sites in amorphous material types.

Specifically, carvedilol salts, anhydrous forms or solvates thereof, which may include novel crystalline forms are characterized substantially by spectroscopic data as described below and depicted in Figures 1-125.

Examples of spectroscopic data associated with specific carvedilol salt, anhydrous forms or solvate forms are described below.

For example, crystalline carvedilol dihydrogen phosphate hemihydrate (see, Example 1: Form I) is identified by an x-ray diffraction pattern as shown substantially in Figure 1, which depicts characteristic peaks in degrees two-theta (2θ): i.e., 7.0 ± 0.2 (2θ), 11.4 ± 0.2 (2θ), 15.9 ± 0.2 (2θ), 18.8 ± 0.2 (2θ), 20.6 ± 0.2 (2θ), 22.8 ± 0.2 (2θ), and 25.4 ± 0.2 (2θ).

Crystalline carvedilol dihydrogen phosphate dihydrate (see, Example 2: Form II) is identified by an x-ray diffraction pattern as shown substantially in Figure 9, which depicts characteristic peaks in degrees two-theta (2θ): i.e., 6.5 ± 0.2 (2θ), 7.1 ± 0.2 (2θ), 13.5 ± 0.2 (2θ), 14.0 ± 0.2 (2θ), 17.8 ± 0.2 (2θ), 18.9 ± 0.2 (2θ), and 21.0 ± 0.2 (2θ).

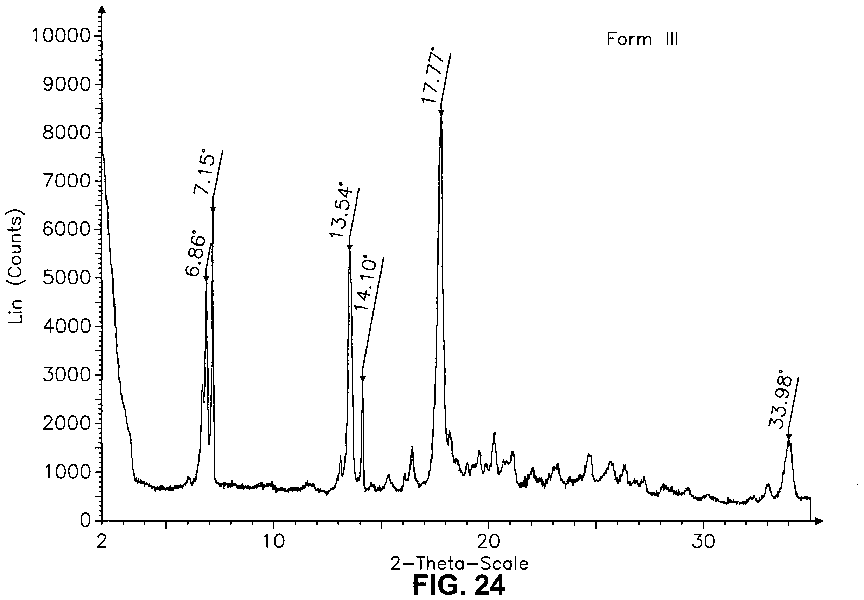

Crystalline carvedilol dihydrogen phosphate methanol solvate (see, Example 3: Form III) is identified by an x-ray diffraction pattern as shown substantially in Figure 24, which depicts characteristic peaks in degrees two-theta (2θ): i.e., 6.9 ± 0.2 (2θ), 7.2 ± 0.2 (2θ), 13.5 ± 0.2 (2θ), 14.1 ± 0.2 (2θ), 17.8 ± 0.2 (2θ), and 34.0 ± 0.2 (2θ).

Crystalline carvedilol dihydrogen phosphate dihydrate (see, Example 4: Form IV) is identified by an x-ray diffraction pattern as shown substantially in Figure 24, which depicts characteristic peaks in degrees two-theta (2θ): i.e., 6.4 ± 0.2 (2θ), 9.6 ± 0.2 (2θ), 16.0 ± 0.2 (2θ), 18.4 ± 0.2 (2θ), 20.7 ± 0.2 (2θ), and 24.5 ± 0.2 (2θ).

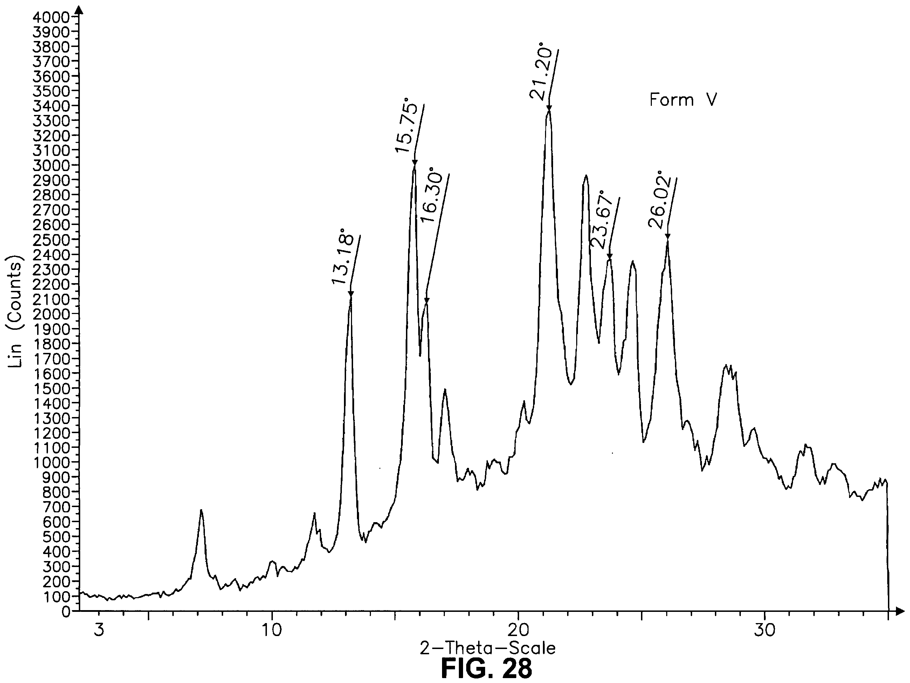

Crystalline carvedilol dihydrogen phosphate preparation (see, Example 5: Form V) is identified by an x-ray diffraction pattern as shown substantially in Figure 28, which depicts characteristic peaks in degrees two-theta (2θ): i.e., 13.2 ± 0.2 (2θ), 15.8 ± 0.2 (2θ), 16.3 ± 0.2 (2θ), 21.2 ± 0.2 (2θ), 23.7 ± 0.2 (2θ), and 26.0 ± 0.2 (2θ).

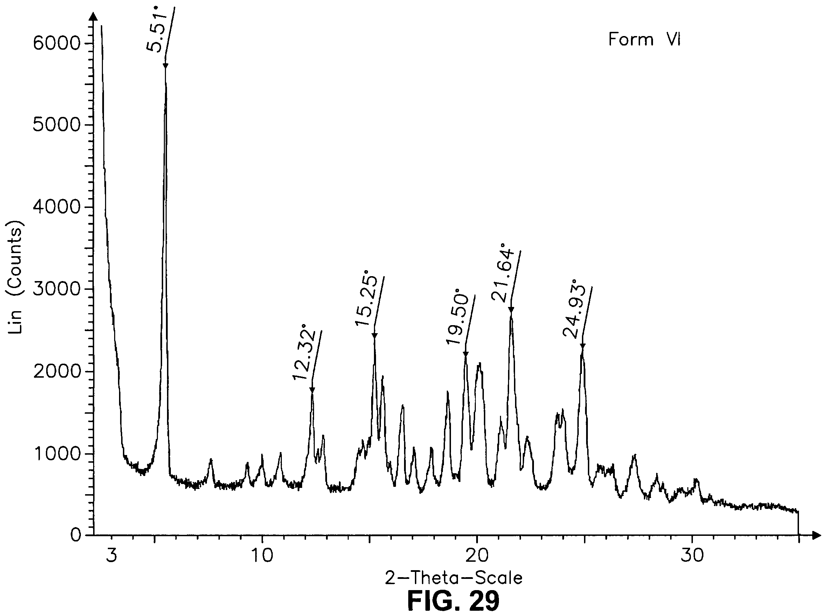

Crystalline carvedilol hydrogen phosphate preparation (see, Example 6: Form VI) is identified by an x-ray diffraction pattern as shown substantially in Figure 29, which depicts characteristic peaks in degrees two-theta (2θ): i.e., 5.5 ± 0.2 (2θ), 12.3 ± 0.2 (2θ), 15.3 ± 0.2 (2θ), 19.5 ± 0.2 (2θ), 21.6 ± 0.2 (2θ), and 24.9 ± 0.2 (2θ).

Crystalline carvedilol hydrobromide monohydrate (see, Example 8: Form 1) is identified by an x-ray diffraction pattern as shown substantially in Figure 1, which depicts characteristic peaks in degrees two-theta (2θ): i.e., 6.5 ± 0.2 (2θ), 10.3 ± 0.2 (2θ), 15.7 ± 0.2 (2θ), 16.3 ± 0.2 (2θ), 19.8 ± 0.2 (2θ), 20.1 ± 0.2 (2θ), 21.9 ± 0.2 (2θ), 25.2 ± 0.2 (2θ), and 30.6± 0.2 (2θ).

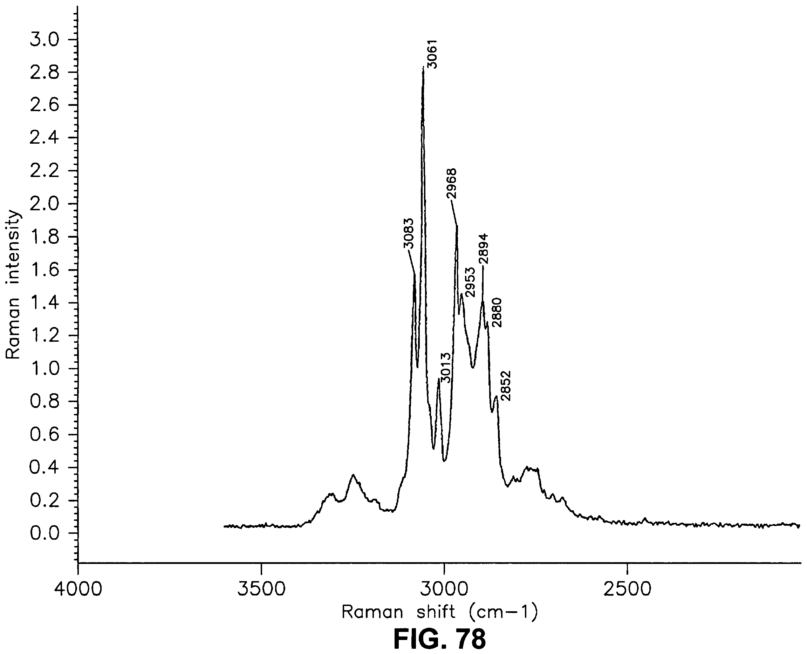

Crystalline carvedilol hydrobromide dioxane solvate (see, Example 9: Form 2) also is identified by an x-ray diffraction pattern as shown substantially in Figure 78, which depicts characteristic peaks in degrees two-theta (2θ): i.e., 7.7 ± 0.2 (2θ), 8.4 ± 0.2 (2θ), 15.6 ± 0.2 (2θ), 17.0 ± 0.2 (2θ), 18.7 ± 0.2 (2θ), 19.5 ± 0.2 (2θ), 21.4 ± 0.2 (2θ), 23.7 ± 0.2 (2θ), and 27.9 ± 0.2 (2θ).

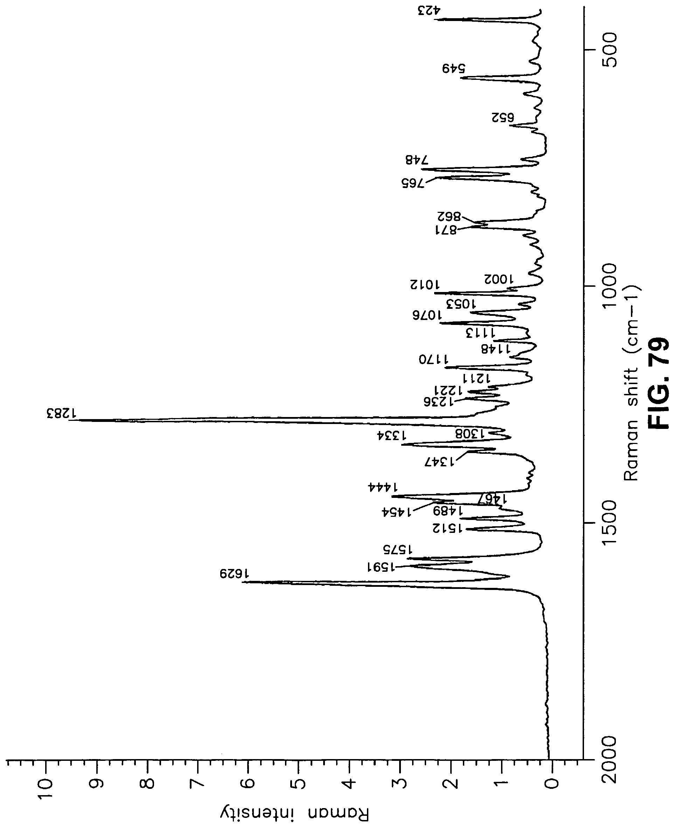

Crystalline carvedilol hydrobromide 1-pentanol solvate (see, Example 10: Form 3) also is identified by an x-ray diffraction pattern as shown substantially in Figure 79, which depicts characteristic peaks in degrees two-theta (2θ): i.e., 77.5 ± 0.2 (2θ), 7.8 ± 0.2 (2θ), 15.2 ± 0.2 (2θ), 18.9 ± 0.2 (2θ), 22.1 ± 0.2 (2θ), and 31.4 ± 0.2 (2θ).

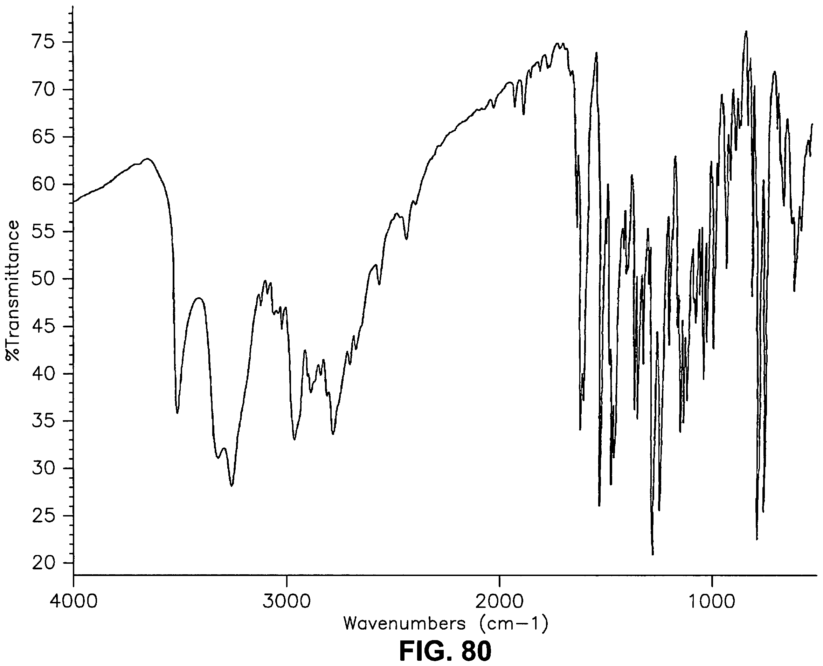

Crystalline carvedilol hydrobromide 2-methyl-1-propanol solvate (see, Example 11: Form 4) also is identified by an x-ray diffraction pattern as shown substantially in Figure 80, which depicts characteristic peaks in degrees two-theta (2θ): i.e., 7.8 ± 0.2 (2θ), 8.1 ± 0.2 (2θ), 16.3 ± 0.2 (2θ), 18.8 ± 0.2 (2θ), 21.8 ± 0.2 (2θ), and 28.5 ± 0.2 (2θ).

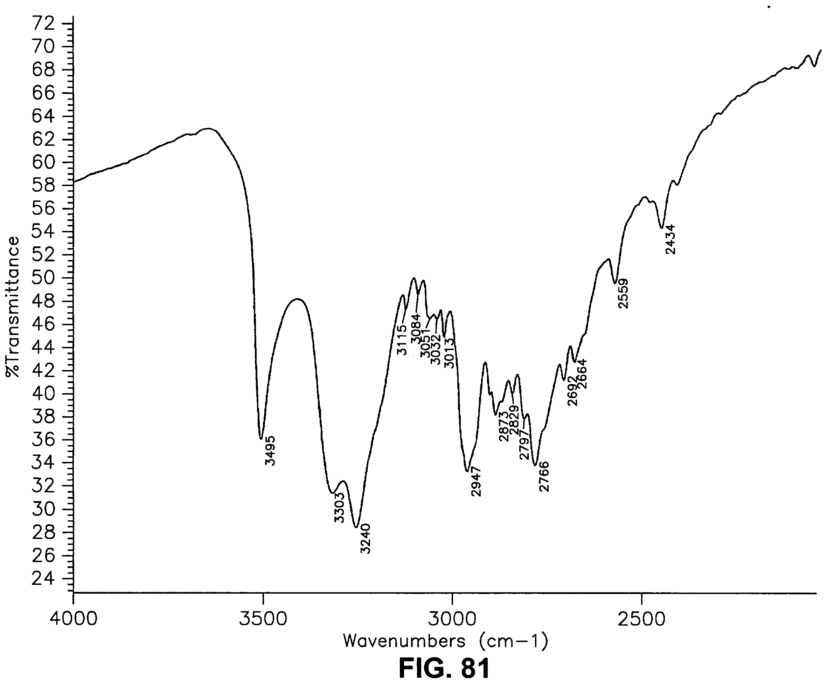

Crystalline carvedilol hydrobromide trifluoroethanol solvate (see, Example 12: Form 5) also is identified by an x-ray diffraction pattern as shown substantially in Figure 81, which depicts characteristic peaks in degrees two-theta (2θ): i.e.,. 7.7 ± 0.2 (2θ), 8.4 ± 0.2 (2θ), 15.6 ± 0.2 (2θ), 16.9 ± 0.2 (2θ), 18.9 ± 0.2 (2θ), 21.8 ± 0.2 (2θ), 23.8 ± 0.2 (2θ), 23.7 ± 0.2 (2θ), and 32.7 ± 0.2 (2θ).

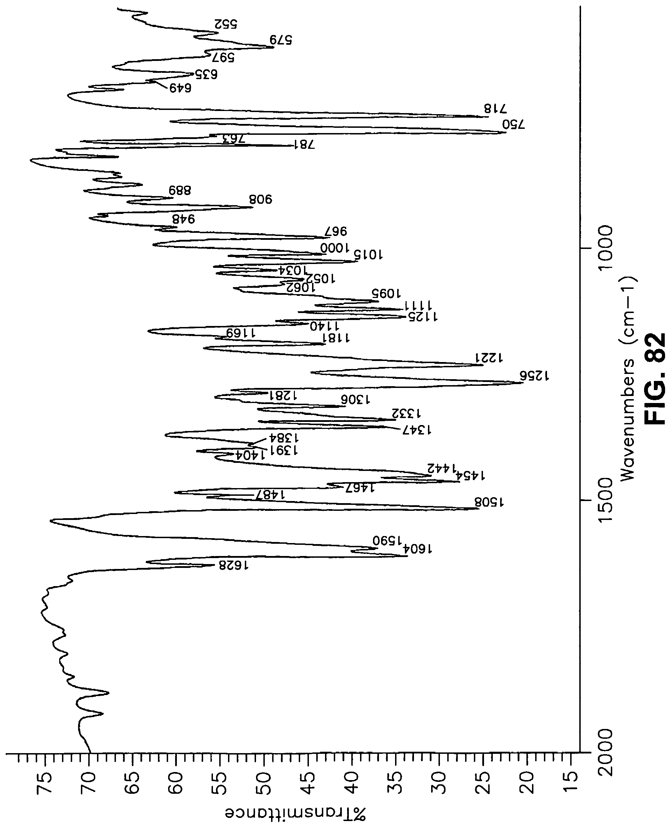

Crystalline carvedilol hydrobromide 2-propanol solvate (see, Example 13: Form 6) also is identified by an x-ray diffraction pattern as shown substantially in Figure 82, which depicts characteristic peaks in degrees two-theta (2θ): i.e.,. 7.9 ± 0.2 (2θ), 8.3 ± 0.2 (2θ), 18.8 ± 0.2 (2θ), 21.7 ± 0.2 (2θ), 23.2 ± 0.2 (2θ), 23.6 ± 0.2 (2θ), and 32.1 ± 0.2 (2θ).

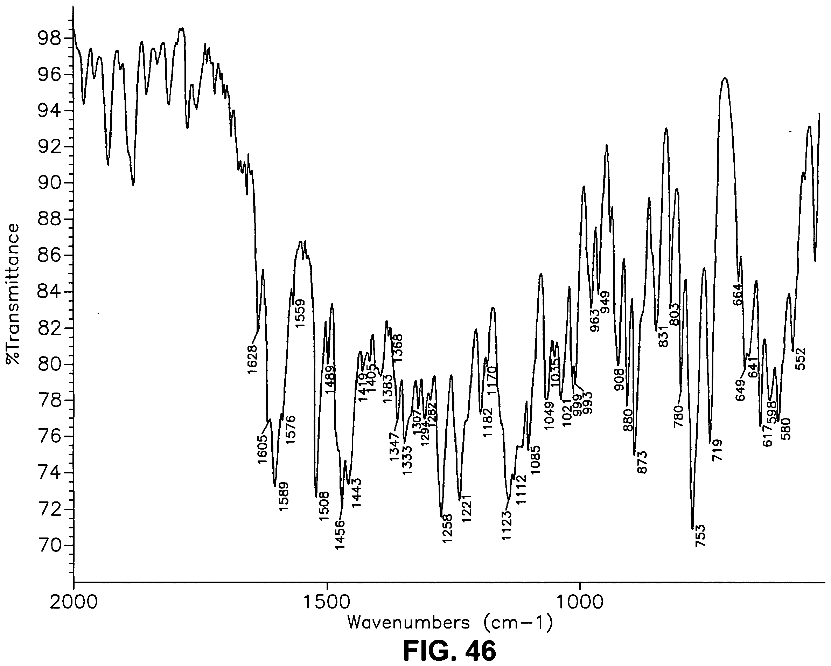

Crystalline carvedilol hydrobromide n-propanol solvate #1 (see, Example 14: Form 7) also is identified by an x-ray diffraction pattern as shown substantially in Figure 46, which depicts characteristic peaks in degrees two-theta (2θ): i.e., 7.9 ± 0.2 (2θ), 8.5 ± 0.2 (2θ), 17.0 ± 0.2 (2θ), 18.8 ± 0.2 (2θ), 21.6 ± 0.2 (2θ), 23.1 ± 0.2 (2θ), 23.6 ± 0.2 (2θ), and 21.2 ± 0.2 (2θ).

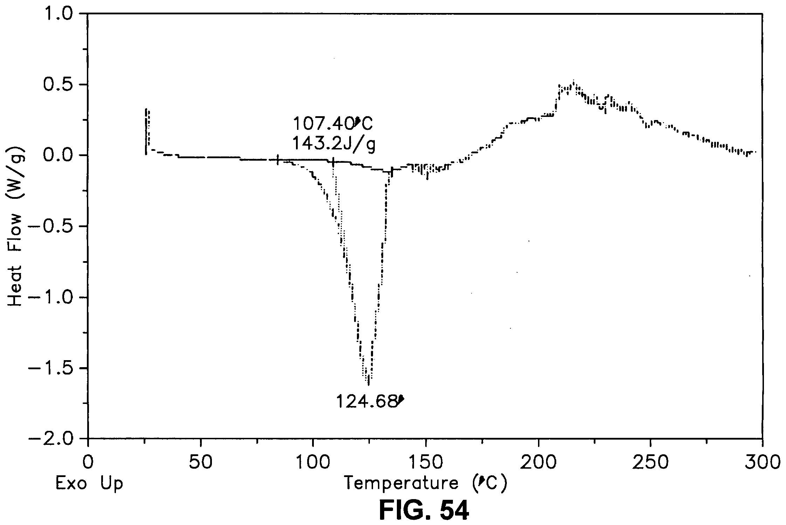

Crystalline carvedilol hydrobromide n-propanol solvate #2 (see, Example 15: Form 8) also is identified by an x-ray diffraction pattern as shown substantially in Figure 54, which depicts characteristic peaks in degrees two-theta (2θ): i.e., 8.0 ± 0.2 (2θ), 18.8 ± 0.2 (2θ), 21.6 ± 0.2 (2θ), 23.1 ± 0.2 (2θ), 25.9 ± 0.2 (2θ), 27.2 ± 0.2 (2θ), 30.6 ± 0.2 (2θ), and 32.2 ± 0.2 (2θ).

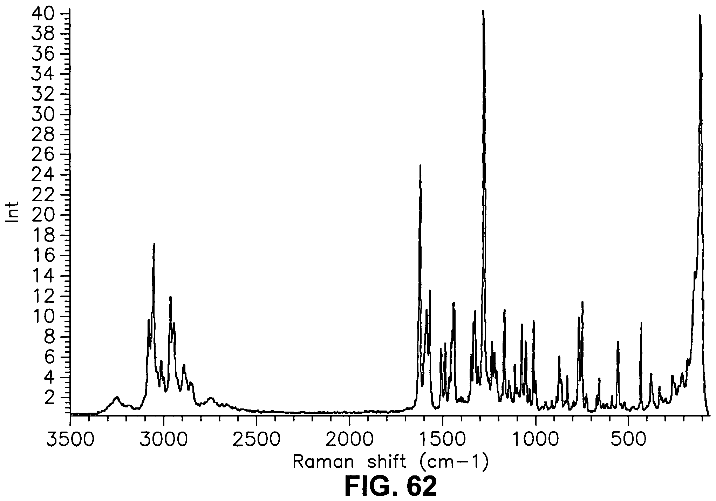

Crystalline carvedilol hydrobromide anhydrous forms (see, Example 16: Form 9) also is identified by an x-ray diffraction pattern as shown substantially in Figure 62, which depicts characteristic peaks in degrees two-theta (2θ): i.e.,. 6.6 ± 0.2 (2θ), 16.1 ± 0.2 (2θ), 17.3 ± 0.2 (2θ), 21.2 ± 0.2 (2θ), 22.1 ± 0.2 (2θ), 24.1 ± 0.2 (2θ), and 27.9 ± 0.2 (2θ).

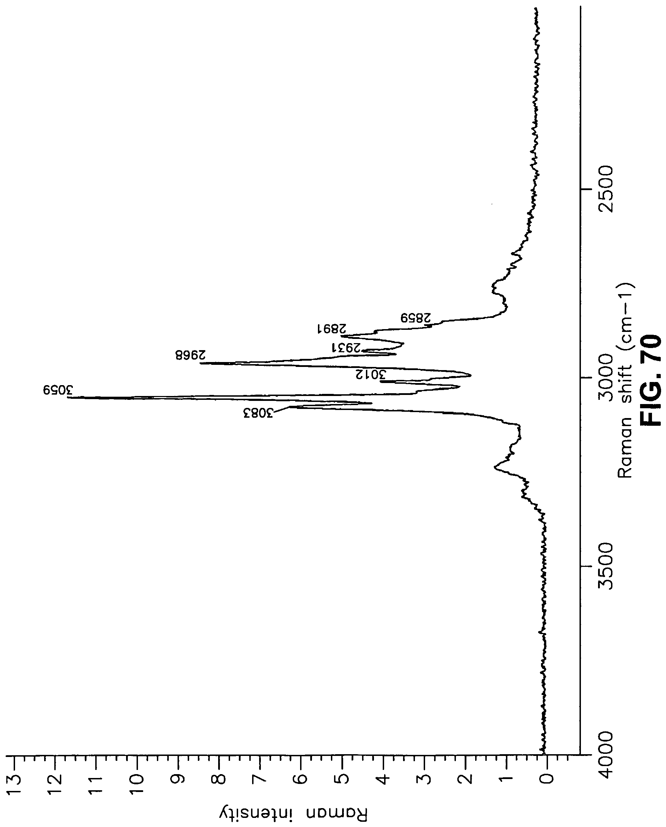

Crystalline carvedilol hydrobromide ethanol solvate (see, Example 17: Form 10) also is identified by an x-ray diffraction pattern as shown substantially in Figure 70, which depicts characteristic peaks in degrees two-theta (2θ): i.e., 8.1 ± 0.2 (2θ), 8.6 ± 0.2 (2θ), 13.2 ± 0.2 (2θ), 17.4 ± 0.2 (2θ), 18.6 ± 0.2 (2θ), 21.8 ± 0.2 (2θ), 23.2 ± 0.2 (2θ), 23.7 ± 0.2 (2θ), and 27.4 ± 0.2 (2θ).

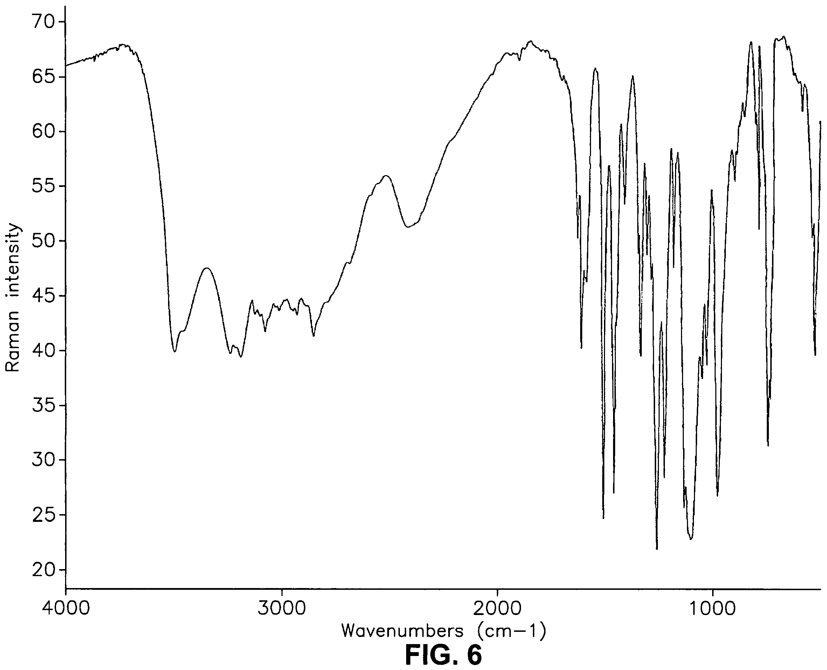

Crystalline carvedilol hydrobromide monohydrate further is identified by an infrared spectrum as shown substantially in Figure 6.

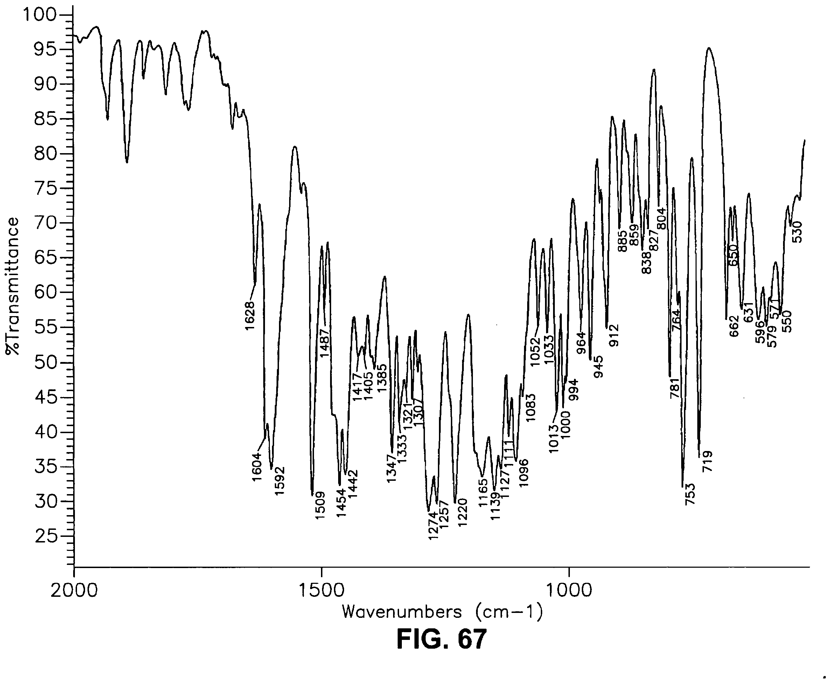

Carvedilol hydrobromide anhydrous forms also an infrared spectrum, which comprises characteristic absorption, bands expressed in wave numbers as shown substantially in Figure 67.

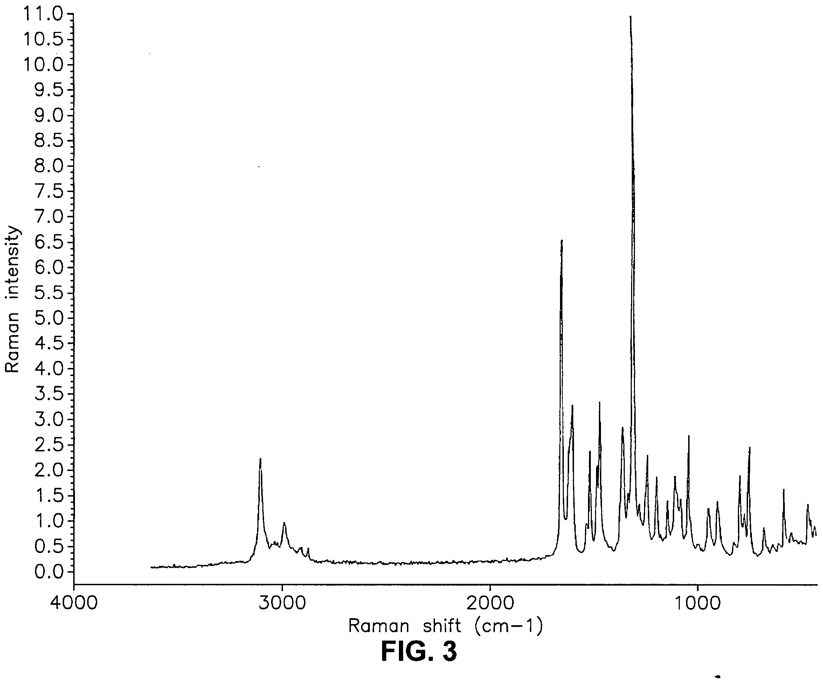

Crystalline carvedilol hydrobromide monohydrate is identified also by a Raman spectrum as shown substantially in Figure 3.

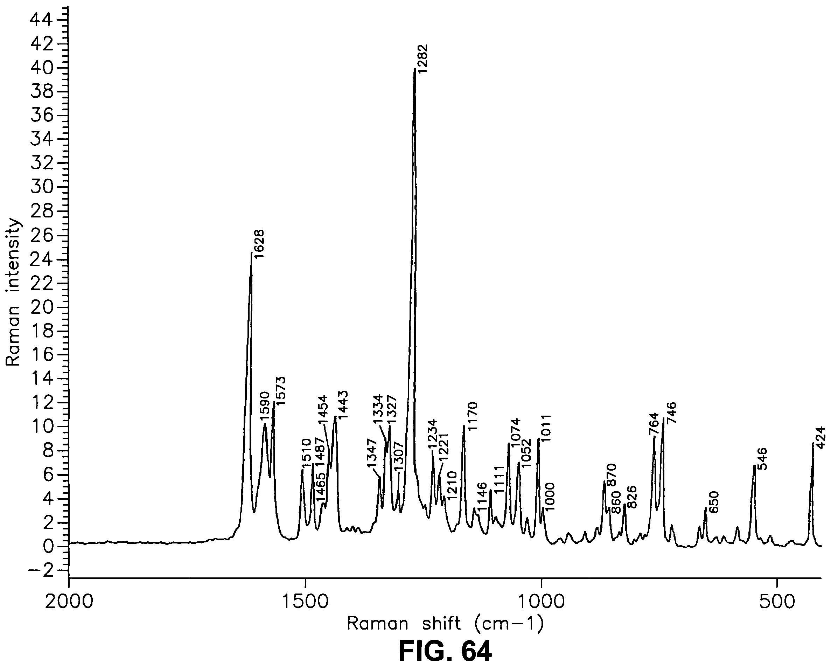

Carvedilol hydrobromide anhydrous forms also a Raman spectrum which comprises characteristic peaks as shown substantially in Figure 64.

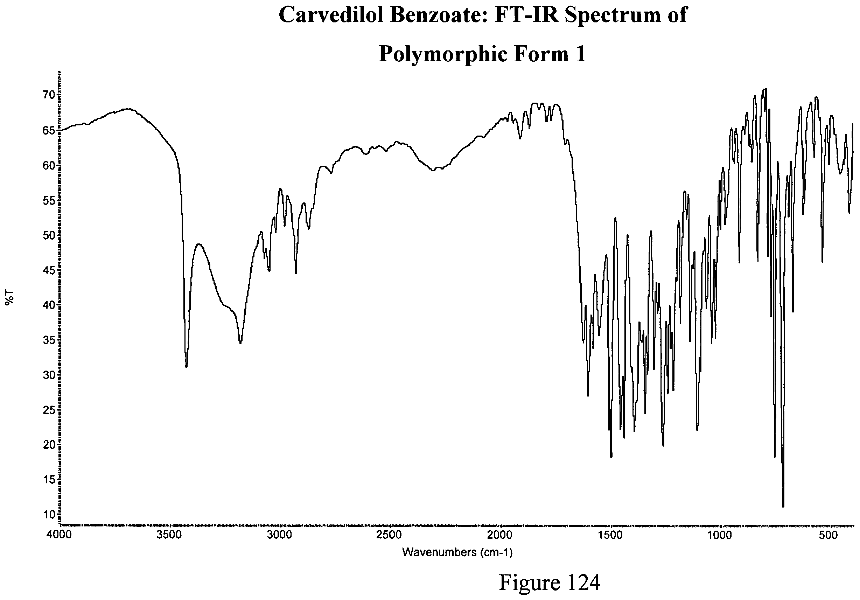

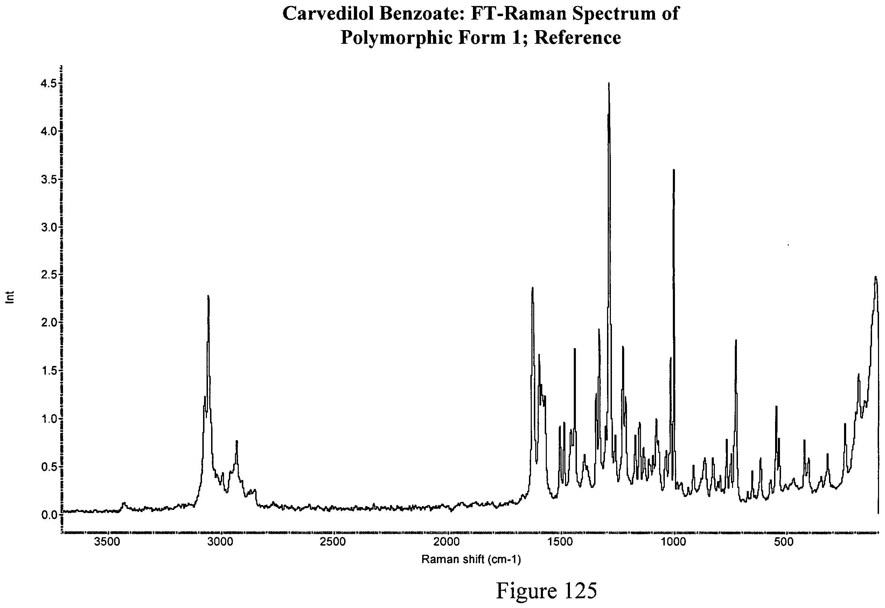

Crystalline carvedilol benzoate (see, Example 22) is identified by an FT-IR spectrum pattern as shown substantially in Figure 124, which depicts characteristic peaks in wavenumbers (cm⁻¹): i.e., 672 cm⁻¹, 718 cm⁻¹, 754 cm⁻¹, 767 cm⁻¹, 1022 cm⁻¹, 1041 cm⁻¹, 1106 cm⁻¹, 1260 cm⁻¹, 1498 cm⁻¹, 1582 cm⁻¹, 1604 cm⁻¹, 1626 cm⁻¹, 2932 cm⁻¹, 3184 cm⁻¹, and 3428 cm⁻¹. Also, crystalline carvedilol benzoate (see, Example 22) is identified by an FT-Raman spectrum pattern as shown substantially in Figure 125, which depicts characteristic peaks in wavenumbers (cm⁻¹): i.e., 108 cm⁻¹, 244 cm⁻¹, 424 cm⁻¹, 538 cm⁻¹, 549 cm⁻¹, 728 cm⁻¹, 1001 cm⁻¹, 1015 cm⁻¹, 1128 cm⁻¹, 1286 cm⁻¹, 1598 cm⁻¹, 1626 cm⁻¹, 2934 cm⁻¹, 3058 cm⁻¹, and 3072 cm⁻¹.

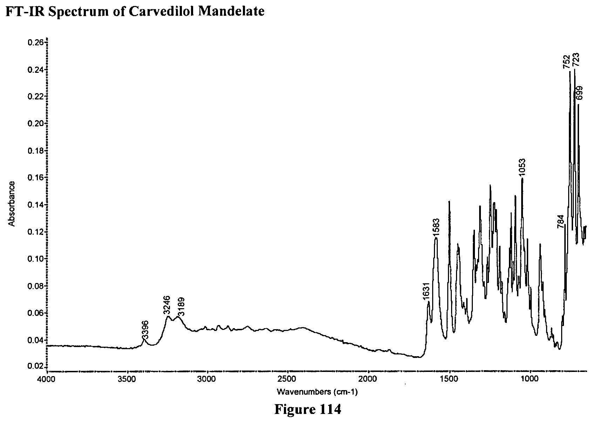

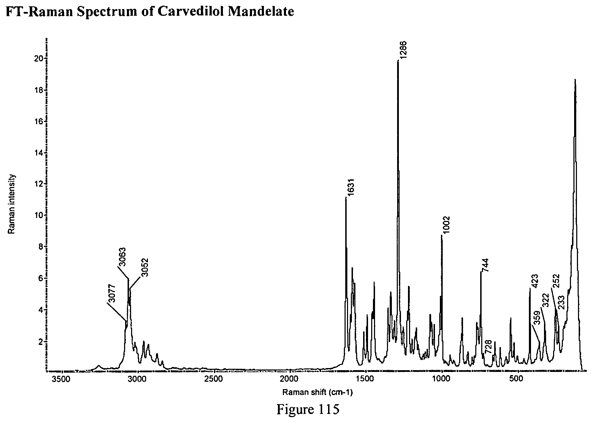

Crystalline carvedilol mandelate (see, Example 23) is identified by an FT-IR spectrum pattern as shown substantially in Figure 114, which depicts characteristic peaks in wavenumbers (cm⁻¹): i.e., 699 cm⁻¹, 723 cm⁻¹, 752 cm⁻¹, 784 cm⁻¹, 1053 cm⁻¹, 1583 cm⁻¹, 1631 cm⁻¹, 3189 cm⁻¹, 3246 cm⁻¹, and 3396 cm⁻¹. Also crystalline carvedilol mandelate (see, Example 23) is identified by an FT-Raman spectrum pattern as shown substantially in Figure 115, which depicts characteristic peaks in wavenumbers (cm⁻¹): i.e., 233 cm⁻¹, 252 cm⁻¹, 322 cm⁻¹, 359 cm⁻¹, 423 cm⁻¹, 744 cm⁻¹, 1002 cm⁻¹, 1286 cm⁻¹, 1631 cm⁻¹, 3052 cm⁻¹, 3063 cm⁻¹, and 3077 cm⁻¹.

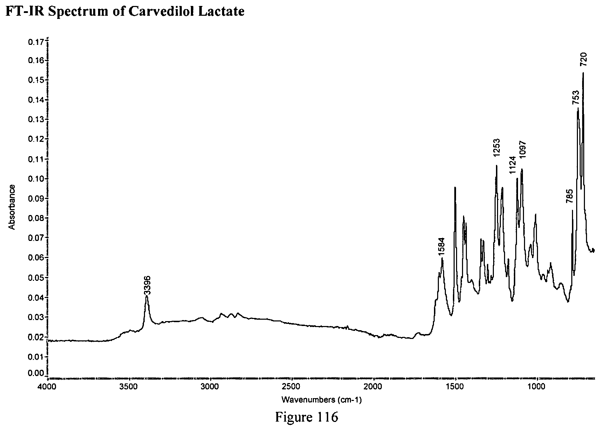

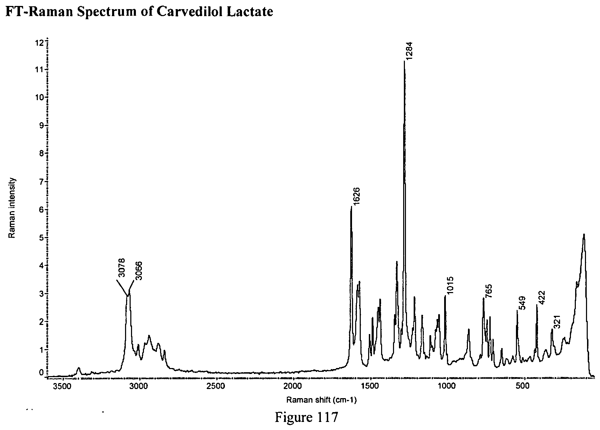

Crystalline carvedilol lactate (see, Example 24) is identified by an FT-IR spectrum pattern as shown substantially in Figure 116, which depicts characteristic peaks in wavenumbers (cm⁻¹): i.e., 720 cm⁻¹, 753 cm⁻¹, 785 cm⁻¹, 1097 cm⁻¹, 1124 cm⁻¹, 1253 cm⁻¹, 1584 cm⁻¹, and 3396 cm⁻¹. Also, crystalline carvedilol lactate (see, Example 24) is identified by an FT-Raman spectrum pattern as shown substantially in Figure 117, which depicts characteristic peaks in wavenumbers (cm⁻¹): i.e., 321 cm⁻¹, 422 cm⁻¹, 549 cm⁻¹, 765 cm⁻¹, 1015 cm⁻¹, 1284 cm⁻¹, 1626 cm⁻¹, 3066 cm⁻¹, and 3078 cm⁻¹.

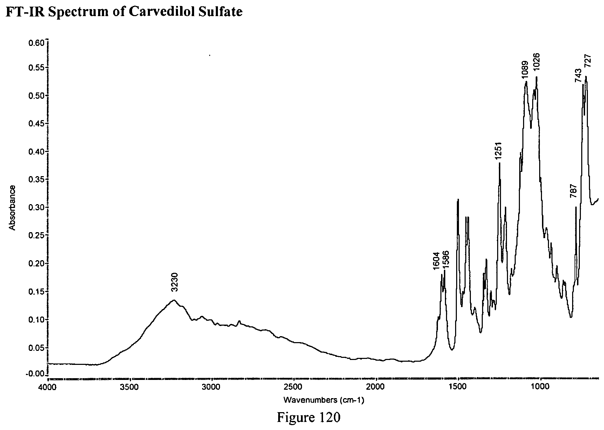

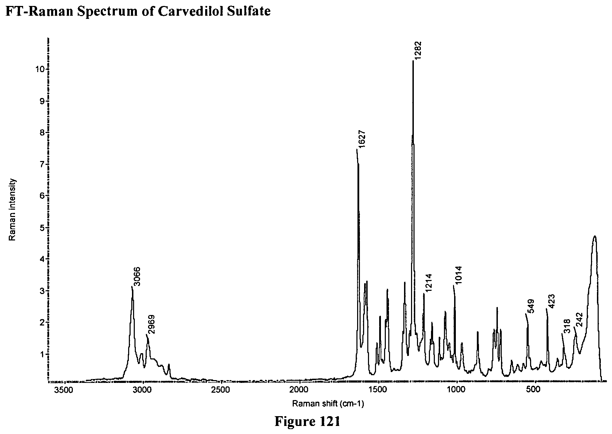

Crystalline carvedilol sulfate (see, Example 25) is identified by an FT-IR spectrum pattern as shown substantially in Figure 120, which depicts characteristic peaks in wavenumbers (cm⁻¹): i.e., 727 cm⁻¹, 743 cm⁻¹, 787 cm⁻¹, 1026 cm⁻¹, 1089 cm⁻¹, 1251 cm⁻¹, 1215 cm⁻¹, 1586 cm⁻¹, 1604 cm⁻¹, and 3230 cm⁻¹. Also, crystalline carvedilol sulfate (see, Example 25) also is identified by an FT-Raman spectrum pattern as shown substantially in Figure 121, which depicts characteristic peaks in wavenumbers (cm⁻¹): i.e., 242 cm⁻¹, 318 cm⁻¹, 423 cm⁻¹, 549 cm⁻¹, 1014 cm⁻¹, 1214 cm⁻¹, 1282 cm⁻¹, 1627 cm⁻¹, 2969 cm⁻¹, and 3066 cm⁻¹.

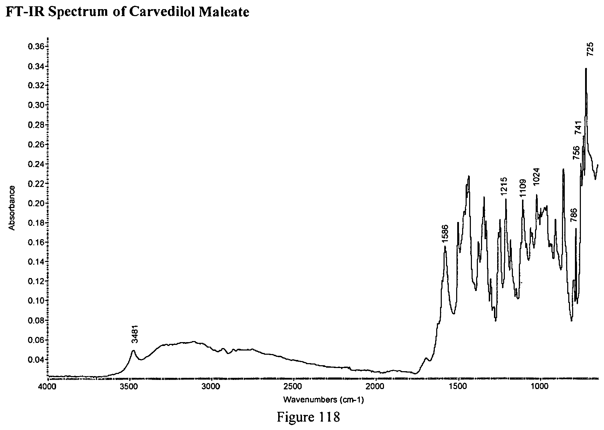

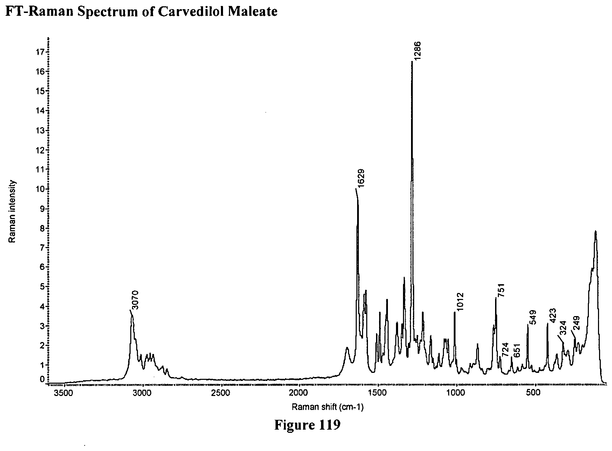

Crystalline carvedilol maleate (see, Example 26) is identified by an FT-IR spectrum pattern as shown substantially in Figure 118, which depicts characteristic peaks in wavenumbers (cm⁻¹): i.e., 725 cm⁻¹, 741 cm⁻¹, 756 cm⁻¹, 786 cm⁻¹, 1024 cm⁻¹, 1109 cm⁻¹, 1215 cm⁻¹, 1586 cm⁻¹, and 3481 cm⁻¹. Also, crystalline carvedilol maleate (see, Example 26) also is identified by an FT-Raman spectrum pattern as shown substantially in Figure 119, which depicts characteristic peaks in wavenumbers (cm⁻¹): i.e., 249 cm⁻¹, 324 cm⁻¹, 423 cm⁻¹, 549 cm⁻¹, 751 cm⁻¹, 1012 cm⁻¹, 1216 cm⁻¹, 1286 cm⁻¹, 1629 cm⁻¹, and 3070 cm⁻¹.

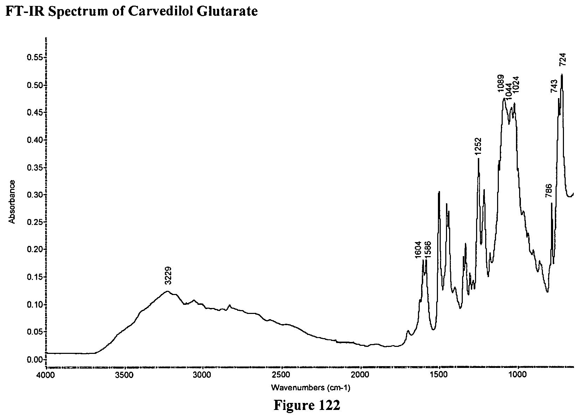

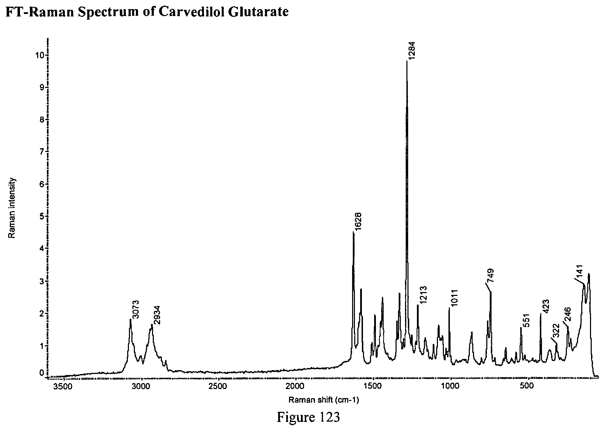

Crystalline carvedilol glutarate (see, Example 27) is identified by an FT-IR spectrum pattern as shown substantially in Figure 122, which depicts characteristic peaks in wavenumbers (cm⁻¹): i.e., 724 cm⁻¹, 743 cm⁻¹, 786 cm⁻¹, 1024 cm⁻¹, 1044 cm⁻¹, 1089 cm⁻¹, 1251 cm⁻¹, 1586 cm⁻¹, 1604 cm⁻¹, and 3229 cm⁻¹. Also, crystalline carvedilol glutarate (see, Example 27) is identified by an FT-Raman spectrum pattern as shown substantially in Figure 123, which depicts characteristic peaks in wavenumbers (cm⁻¹): i.e., 141 cm⁻¹, 246 cm⁻¹, 322 cm⁻¹, 423 cm⁻¹, 551 cm⁻¹, 749 cm⁻¹, 1011 cm⁻¹, 1213 cm⁻¹, 1284 cm⁻¹, 1628 cm⁻¹, 2934 cm⁻¹, and 3073 cm⁻¹.

### Pharmaceutical Compositions, Controlled-Release Formulations, Dosage Regimens and Dosage Forms

In general, the present invention also relates to different dosage forms, pharmaceutical compositions and/or controlled-release formulations, which may contain carvedilol free base or a carvedilol salt, solvate, or anhydrous forms thereof as described herein.

In general, for medications to be optimally effective it is important that administration complies with the stipulated dosage regimen. Poor compliance can compromise safety and efficacy. Compliance is usually a problem with medications for chronic asymptomatic ilnesses and where patients are elderly and/or infirm.

As previously discussed, carvedilol is known as an effective medication for treating hypertension, congestive heart failure, atherosclerosis, and other cardiovascular conditions. Its unique mode of action is a consequence of it being a mixture of R and S isomers with complimentary pharmacological effects. Vasodilation and reduced peripheral resistance are a consequence of the alpha blockade associated with the R isomer. Blood pressure reduction is ascribed to the beta blockade contributed by both R and S isomers.

Currently, carvedilol is administered to treat cardiovascular diseases to a subject in need thereof and is usually administered twice daily. Cardiovascular diseases treatable by methods of the present invention, include, but are not limited to hypertension, congestive heart failure, atherosclerosis, angina, etc.

However, for chronic diseases such as cardiovascular diseases, a once-daily dosage regimen is desirable, to enhance patient compliance and reduce "pill burden". Medication that is dosed once daily facilitates greater compliance with the dosage regimen. This applies especially to chronic asymptomatic illnesses. It follows that medication for a condition like hypertension, atherosclerosis and/or some other cardiac conditions is most effective, from a safety and efficacy perspective if dosed once daily.

In many cases the pharmacokinetics or pharmacodynamics of a drug are such that once a day dosage, using conventional dosage forms provides adequate therapy.

With some drugs it may be necessary to formulate a dosage form that releases the drug over an extended period, to provide sustained plasma levels that evince the desired duration of action. Such modified release dosage forms are invariably designed to provide plasma levels that do not fluctuate significantly over time.

It is well established that there is a strong relationship between frequency of dosage and compliance. Medication that is dosed once daily is considered best from a convenience and compliance perspective than when more frequent doses are necessary. Thus, medication for a chronic and "silent" condition like hypertension, and other cardiac conditions is most effective, from a safety and efficacy perspective if dosed once daily.

The pharmacokinetics or pharmacodynamics of a drug may be such that once-a-day dosage, using conventional dosage forms provides adequate therapy. However, with some drugs it may be necessary to formulate so that the dosage form releases the drug over an extended period, in order to sustain plasma levels to provide the desired duration of action. Such modified release dosage forms are traditionally designed to provide plasma levels of drug that do not fluctuate significantly over time.

However, a medication providing constant plasma levels may not always be optimal for treating hypertension, atherosclerosis or related conditions. Blood pressure is influenced by cirdadian rhythm. It rises in the morning on awakening (so-called "morning surge"), is maximum during daytime activities and falls at night, particularly between around midnight to 3 am (see, Anar.Y.A, White.W.B; Drugs (1998) 55 (5) 631-643*;* Chronotherapeutics for Cardiovascular Disease). "Morning-surge may be a factor in the higher incidence of cardiovascular incidents like stroke, acute myocardial infarction and angina pectoris that occur in the early morning.

Blood pressure also can remain elevated at night in some hypertensives, particularly the elderly. These have been termed "non-dippers' and such a condition is associated with increased cardiovascular morbidity (see, Kario.K, Matsuo.T, Kobayashi.H, Imiya.M, Matsuo.M, Shimida.K; Hypertension (1996) 27 (1) 130-135*.* Nocturnal Fall of Blood Pressure and Silent Cerebrovascular Damage in Elderly Hypertensive Patients).

Drug absorption following oral dosage requires that drug first dissolves in the gastro-intestinal milieu. In most cases such dissolution is primarily a function of drug solubility. If solubility is affected by pH it is likely that absorption will vary in different regions of the gastro intestinal tract, because pH varies from acidic in the stomach to more neutral values in the intestine.

Such pH-dependent solubility can complicate dosage form design when drug absorption needs to be prolonged, delayed or otherwise controlled, to evince a sustained or delayed action effect. Variations in solubility can lead to variable dissolution, absorption and consequent therapeutic effect.

A case can therefore be made that plasma levels ought be optimal at times of high risk, provided that there is an association between plasma level and pharmacodynamic effect. In general, cardiovascular medication has been designed with such requirements in mind (see, White.W.H, Andes.R.J, Maclntyre.J.M, Black.H.R, Sica.D.A; The American Journal of Cardiology (1995) 76, 375-380. Nocturnal Dosing of a Novel Delivery System of Verapamil for Systemic Hypertension). For example, the beta blockade-associated effect on blood pressure is proportional to dose (see, De May.C.D, Breithaupt.K, Schloos.J, Neugebauer.G, Palm.D, Belz.G.G; Clinical Pharmacology & Therapeutics ((1994) 55, (3) 329-337. Dose-Effect and Pharmacokinetic and Pharmacodynamic Relationships of Beta-Adrenergic Receptor Blocking Properties of Various Doses of Carvedilol in Healthy Humans).

It would be beneficial therefore, in cases where there is good association between plasma level and clinical response, that optimal levels of drug be present before and during times of high risk so that the cardiovascular system is stabilized and not vulnerable to dramatic change, or that levels of the pharmacological agent are not sub therapeutic. Some recent cardiovascular medications have been designed to provide for "early morning cover" (White.W.H, Andes.R.J, MacIntyre.J.M, Black.H.R, Sica.D.A; The American Journal of Cardiology (1995) 76 375-380. Nocturnal Dosing of a Novel Delivery System of Verapamil for Systemic Hypertension.) but none appear to be available that provide cover for the "non-dipping" period as well as protecting against morning surge.

The beta blockade-associated effect on blood pressure is proportional to dose (De May.C.D, Breithaupt.K, Schloos.J, Neugebauer.G, Palm.D, Belz.G.G; Clinical Pharmacology & Therapeutics ((1994) 55 (3)329-337*.* Dose-Effect and Pharmacokinetic and Pharmacodynamic Relationships of Beta-Adrenergic Receptor Blocking Properties of Various Doses of Carvedilol in Healthy Humans..

Hence, therapy is likely to be more effective if adequate plasma levels are provided before and during times of greatest risk. Thus, a dosage form taken at night (bedtime), that delivers drug in two phases *viz* during the midnight-3am period and prior to and during "morning surge" activities ought provide optimum pharmacological-based therapy. At the same time it is important that adequate levels are maintained throughout the full dosage period, to provide reliable and stable control.

A further advantage of an optimally designed dosage form concerns rate of release of drug from the unit immediately after ingestion. Alpha blockade evinces a vasodilation effect and associated reduction of peripheral resistance. If drug plasma levels rise too rapidly this can lead to postural hypotension and risk of falling over. More gradual rise in plasma levels would, conceivably make for a safer medication.

With the above considerations, it will be evident that an optimally designed, once daily dosage form of carvedilol, taken at night should have the following features:
release drug at a slower rate following ingestion so that plasma buildup is gradual, thereby avoiding rapid fall in blood pressure and minimizing risk of orthostatic hypotension-related adverse events;
provide adequate plasma levels of drug about 1-3 hours after dosing, with subsequent falloff as time progresses;
provide a "later or second peak", about 5-10 hours after dosing with gradual reduction of plasma levels thereafter; and
provide that plasma levels that do not fall below the minimum level for effectiveness such that plasma levels after 24 hours should be comparable to those obtained when dosing twice daily dosage (as current commercial COREG® medication in the United States).

Moreover, a profile associated with such a once daily dosage of carvedilol, would exhibit a first peak at about 1 hours to about 3 hours, which should be lower than the later or second peak as physiological activity is at a minimum during sleep so control requires less drug.

In contrast, plasma levels in the morning ought to reflect the greater activity and associated cardiovascular stress at this time.

Such a profile is consistent with current thinking that medications for cardiovascular conditions, that provide near constant drug concentrations over time may not be optimally designed. Because of circadian variations in blood pressure it may be more appropriate to provide high concentrations of drug at times of greatest need. Furthermore, blood pressure lowering should not be excessive during night time, so as to reduce potential for night-time hypotension and ischaemic stroke (Smith David.G.H: American Journal of Hypertension: (2001) 14 296S-301S. Pharmacology of Cardiovascular Therapeutic Agents).

The physico chemical properties, and pharmacokinetics of carvedilol make it difficult to design the kind of delivery system described above, for the following reasons:
both R and S isomer carvedilol forms are cleared relatively rapidly from the systemic circulation (alpha elimination phase is about 1.5 hours); and
plasma levels are depleted rapidly and substantially following attainment of peak plasma concentrations.

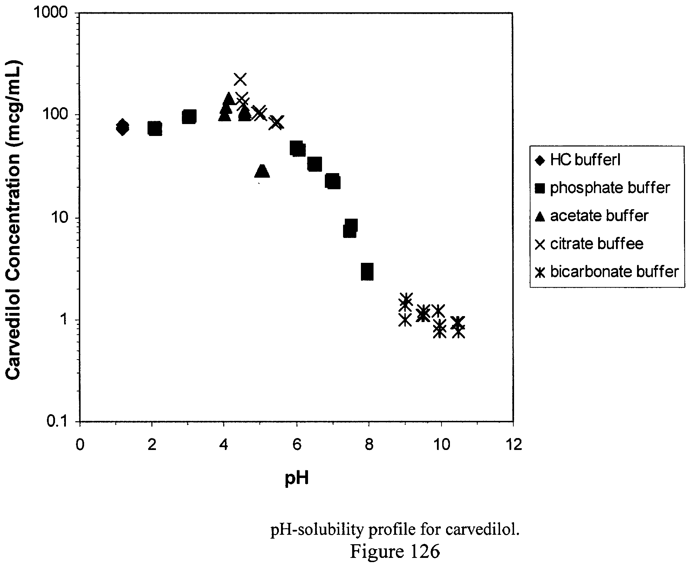

The pH-aqueous solubility of the free base form of carvedilol is such (Figure 126) that absorption is likely to be low, or even non-existent from the neutral regions of the gastro intestinal tract. A drug needs to be in solution if it is to pass from the intestine to systemic circulation and it is generally accepted that, where aqueous solubility is less than about 5mg/ml, absorption following oral dosage can be problematical (Ritschel W.A. Arzneim Forsch (1975), 25, p. 853)). At the pH values encountered in the distal small intestine and colon, solubility of carvedilol free base does not exceed 0.1 mg (100mcg) per ml).

Such a solubility profile makes it difficult to design a dosage form to sustain absorption for long periods by providing slow release of drug from the dosage form as it transits the gastro intestinal tract. At pH values in the middle and lower parts of the small intestine solubility is likely to be insufficient to enable sufficient drug to dissolve to provide adequate absorption flux. This constraint could theoretically be surmounted if it were possible to design a unit that remained in the stomach or upper small intestine, such that drug was released to an environment more conducive to dissolution and absorption. However, the maximum period that a dosage form is retained in the fed stomach is about three hours. This time period possibly might be prolonged if a high fat content meal were consumed at the time of dosage. However, this is probably impractical for "before bedtime" dosage, especially where in any case such a diet is inadvisable for patients with cardiovascular disease.

Thus, it will be obvious to a person skilled in the art that the rapid systemic clearance combined with poor solubility at neutral pH of carvedilol constrain possibilities for designing a unit to provide prolonged absorption and sustained plasma levels. In effect there are formidable, if not insurmountable challenges in the design of a unit incorporating delayed and time-specific release features as well as providing adequate plasma levels over a once-a day dosing period. The absence of any commercially available modified release dosage form of carvedilol, designed for optimal chronotherapeutic effect supports this view.

However, the dose response and time course of carvedilol in the body is such that a conventional dosage form, releasing all the drug immediately on ingestion does not provide once-a-day therapy.

Release from the dosage form needs to be slowed down so that absorption and subsequent systemic residence is prolonged. This however requires that release and dissolution occurs along the GI tract, not just in the stomach.

Hence, it would be expected that therapy would be more effective if peak plasma levels were provided times of greatest risk.

In such a context a carvedilol based dosage form that is taken at night (at bedtime), that delivers drug in two phases to cover the midnight-3 am period, and the early morning surge ought provide optimum therapy, while maintaining a once-daily dosage regimen.

However, the properties of carvedilol drug substance, as well as its pharmacokinetics make it difficult to design such a delivery system, for the following reasons:
[1] absorption from the lower gastro intestinal tract is less efficient than from the stomach. This is probably related to the very low solubility of carvedilol at neutral pH, making it difficult to design a dosage form to sustain absorption for long periods. The maximum period that a dosage form is retained in the fed stomach is around three hours; or
[2] the relatively rapid clearance (alpha elimination phase) means that plasma levels are reduced rapidly and substantially following attainment of the peak plasma concentration.
These considerations, taken together teach that the provision of a dosage form, delivering carvedilol at times of maximum patient risk, and potential optimum benefit is difficult if not impossible.

Nevertheless, it has now, surprisingly been shown that, when a carvedilol salt, solvate or anhydrous forms thereof is utilized, and, when such a carvedilol salt, solvate or anhydrous forms thereof is formulated using appropriate modified release technology, plasma profiles are obtained in human volunteers which are aligned with what knowledge of chronobiology suggests may be optimally beneficial in hypertension, atherosclerosis, congestive heart failure or other cardiovascular disorders. Formulations of the present invention may also include carvedilol free base.

Therefore, solubility of carvedilol free base or various carvedilol salts, or solvates as those described herein may facilitate provision or development of a dosage form, such as a controlled-release formulation, from which the drug substance becomes available for bioabsorption throughout the gastrointestinal tract (i.e., in particular the lower small intestine and colon). See Example 28 herein and corresponding discussion at pages 111-116 of the instant specification.

Parts of the gastrointestinal tract are defined to include generally the stomach (i.e. which includes the antrum and pylorus bowel), small intestine (i.e., which has three parts: the duodenum, jejunum, illeum), large intestine (i.e., which has three parts: the cecum, colon, rectum), liver, gall bladder and pancreas.

In light of the foregoing, the present invention relates to an embodiment where a compound, pharmaceutical composition, or controlled-release formulation or dosage form is presented as a unit dose taken or administered from 1 to 2 times daily, most especially taken or adminstered once daily to achieve the desired effect.

Importantly, the chemical or physical properties of carvedilol forms described herein, which include, but are not limited to the above-identified carvedilol free base or carvedilol salts, anhydrous forms or solvates thereof indicate that those forms may be particularly suitable for inclusion in medicinal agents, pharmaceutical compositions, controlled release formulations or dosage forms, etc.

Treatment regimen for the administration of compounds, pharmaceutical compositions, or controlled-release formulations or dosage forms of the present invention also may be determined readily by those with ordinary skill in art. The quantity of the compound, pharmaceutical composition, or controlled-release formulation or dosage form of the present invention administered may vary over a wide range to provide in a unit dosage in an effective amount based upon the body weight of the patient per day to achieve the desired effect and as based upon the mode of administration.

The scope of the present invention includes all compounds, pharmaceutical compositions, or controlled-release formulations or dosage forms, which is contained in an amount effective to achieve its intended purpose. While individual needs vary, determination of optimal ranges of effective amounts of each component is within the skill of the art.

In accordance with a pharmaceutical composition, dosage form or controlled release formulation of the present invention as described herein (i.e., which include any of the specific embodiment described for various delivery systems or technologies applicable with the present invention), a specific embodiment may include carvedilol free base or carvedilol free base, which may be, but is not limited to, be in a combination with a solubility enhanced carvedilol salt, solvate or anhydrous form(s) thereof.

General definitions suitable to define aspects of the present invention are set forth below.

As used in the present invention, the term controlled release generally is defined as a formulation that achieves slow or controlled release of a drug over an extended period of time.

In the controlled or modified release formulations of the present invention, a portion of the carvedilol free base, salt, solvate or anhydrous form thereof in a formulation is made available as a rapidly releasing, immediate release or or priming dose and where the remainder portion is released in a controlled, delayed or sustained release fashion.

Conventional art known examples of such controlled release systems may include, but are not limited to a matrix tablet or bead formulation, and/or a barrier film coated tablet or bead/pellet formulation.

The term delayed release is defined as any formulation, where release of the drug is delayed for certain time or minimum under acidic conditions but rapid above a certain pH depending on use of pharmaceutically acceptable coating materials or excipients, such the type of polymer used for a barrier film coat. Conventional art known examples of such delayed release systems may include, but are not limited to timed-release tablets and capsules and enteric-coated tablets and beads.

For the purposes of the present application the term "extended release" means contained in a matrix, or combined with excipients, which delay the release of and thereby prolong the duration of action of the active constituent.

Further, "long acting" means having a longer time of elimination (t one-half or t_{1/2}) from the plasma compartment than other drugs of the same class.

The term pulsatile release is meant any multi-unit tablet or capsule formulation where in individual mini-tablets or particulates/pellets/beads are polymer barrier film coated, that utilizes intermittent pulsatile dosings of an active drug from one or more units as a function of time. Such controlled or modified release formulations of the present inventions are formulated in a manner such that release modes such as described above result in release of the active drug form of a carvedilol free base, or a carvedilol salt, solvate or anhydrous form thereof is predominantly affected after administration during passage in the gastrointestinal tract, especially passage through the stomach, small and large intestine and the colon.

General examples of controlled release, pulsatile release and delayed release formulations which are suitable for incorporating compounds of the present invention described in such references as follows, respectively which are hereby incorporated by reference in their entirety: Sustained Release Medications, Chemical Technology, Review No. 177, Ed. J. C. Johnson, Noyes Data Corporation (1980); Controlled Drug Delivery, Fundamentals and Applications, 2nd Edition, Eds. J. R. Robinson, V. H. L. Lee, Mercel Dekkes Inc., New York (1987); Remington's Pharmaceutical Sciences, 16th Edition, Ed. A. Osol, Mack Publishing Company (1980); and/or Solubility Considerations and Design of Controlled Release Dosage Forms, by G. M. Venkatesh, Polymer Preprint, Volume 40, pp 322, 1999 (American Chemical Society).

In accordance with a pharmaceutical composition, dosage form or controlled release formulation of the present invention as described herein (i.e., which include any of the specific embodiment described for various delivery systems or technologies applicable with the present invention), a specific embodiment may include a pharmaceutically acceptable acid addition salts of carvedilol free base or corresponding forms as described herein. Such pharmaceutically acceptable salts of carvedilol free base or corresponding forms are formed with appropriate organic or mineral acids, which may include, but are not limited to formation by methods known in the art.

Representative examples of such suitable organic or mineral acids may include, but are not limited to maleic acid, fumaric acid, benzoic acid, ascorbic acid, pamoic acid, succinic acid, bismethylenesalicyclic acid, methane sulphonic or sulfonic acid, acetic acid, propionic acid, tartaric acid, salicyclic acid, citric acid, gluconic acid, aspartic acid, stearic acid, palmitic acid, itaconic acid, glycolic acid, p-aminobenzoic acid, glutamic acid, benzene sulfonic acid or sulphonic acid, hydrochloric acid, hydrobromic acid, sulfuric acid, cyclohexylsulfamic acid, phosphoric acid, nitric acid and the like.

In accordance with the present invention, mineral acids may be selected from, but are not limited to hydrobromic acid, hydrochloric acid, phosphoric acid, sulfuric acid or sulphuric acid, and the like; and organic acids may be selected from, but not limited to methansulphuric acid, tartaric acid, maleic acid, acetic acid, citric acid, benzoic acid and the like.

Also in accordance with a pharmaceutical composition, dosage form or controlled release formulation of the present invention as described herein (i.e., which include any of the specific embodiment described for various delivery systems or technologies applicable with the present invention), a specific embodiment may include a solubility enhanced carvedilol salt, solvate or anhydrous forms form or forms selected from the group consisting of a novel crystalline salt or other solid forms of carvedilol mandelate, carvedilol lactate, carvedilol maleate, carvedilol sulfate, carvedilol glutarate, carvedilol mesylate, carvedilol phosphate, carvedilol citrate, carvedilol hydrogen bromide, carvedilol oxalate, carvedilol hydrogen chloride, carvedilol hydrogen bromide, carvedilol benzoate, or corresponding solvates thereof with any of the characteristics noted herein, in association with one or more non-toxic pharmaceutically acceptable carriers or diluents thereof, and if desired, other active ingredients.

Further in accordance with a pharmaceutical composition, dosage form or controlled release formulation of the present invention as described herein (i.e., which include any of the specific embodiment described for various delivery systems or technologies applicable with the present invention), a specific embodiment may include, but are not limited to novel crystalline salt or other solid forms of carvedilol hydrogen phosphate, carvedilol dihydrogen phosphate, carvedilol dihydrogen phosphate hemihydrate, carvedilol dihydrogen phosphate dihydrate, carvedilol dihydrogen phosphate methanol solvate, carvedilol hydrobromide monohydrate, carvedilol hydrobromide dioxane solvate, carvedilol hydrobromide 1-pentanol solvate, carvedilol hydrobromide 2-methyl-1-propanol solvate, carvedilol hydrobromide trifluoroethanol solvate, carvedilol hydrobromide 2-propanol solvate, carvedilol hydrobromide n-propanol solvate #1, carvedilol hydrobromide n-propanol solvate #2, carvedilol hydrobromide anhydrous forms or anhydrous forms, carvedilol hydrobromide ethanol solvate, carvedilol hydrobromide dioxane solvate, carvedilol monocitrate monohydrate, carvedilol mandelate, carvedilol lactate, carvedilol hydrochloride, carvedilol maleate, carvedilol sulfate, carvedilol glutarate, or corresponding anhydrous forms, solvates thereof.

Also suitable for use in any of the pharmaceutical compositions, dosage forms or controlled release formulations of the present invention are solubility enhanced carvedilol salt, solvate or anhydrous forms thereof, which may include, but are not limited to novel crystalline salt or other solid forms, selected from the group consisting of carvedilol hydrogen phosphate, carvedilol dihydrogen phosphate, carvedilol dihydrogen phosphate hemihydrate, carvedilol dihydrogen phosphate dihydrate, carvedilol dihydrogen phosphate methanol solvate.

In particular, in accordance with a pharmaceutical composition, dosage form or controlled release formulation of the present invention as described herein (i.e., which include any of the specific embodiment described for various delivery systems or technologies applicable with the present invention), a specific embodiment may include a carvedilol salt, solvate, or anhydrous forms thereof, such as a carvedilol phosphate salt, which may include, but is not limited to or selected from the group consisting of a carvedilol dihydrogen phosphate hemihydrate (Form I), carvedilol dihydrogen phosphate dihydrate (Form II), carvedilol dihydrogen phosphate methanol solvate (Form III), carvedilol dihydrogen phosphate dihydrate (Form IV), carvedilol dihydrogen phosphate (Form V) and carvedilol hydrogen phosphate (Form VI), and the like.

Also, suitable for use in any of the pharmaceutical compositions, dosage forms or controlled release formulations of the present invention is carvedilol dihydrogen phosphate hemihydrate or carvedilol phosphate anhydrous.

Depending upon the treatment being effected, the compounds, or compositions of the present invention can be administered orally, intraperitoneally, or topically, etc. Preferably, the composition is adapted for oral administration.

In general, pharmaceutical compositions of the present invention are prepared using conventional materials and techniques, such as mixing, blending and the like.

In accordance with the present invention, compounds or pharmaceutical composition can also include, but are not limited to, suitable adjuvants, carriers, excipients, or stabilizers, etc. and can be in solid or liquid form such as, tablets, capsules, powders, solutions, suspensions, or emulsions, etc.

Typically, the composition will contain a carvedilol free base or carvedilol salt, solvate or anhydrous form thereof compound of the present invention, such as a salt of carvedilol or active compound(s), together with the adjuvants, carriers or excipients. For example, a pharmaceutical composition of the present invention may comprise, but is not limited to an effective amount of a salt of carvedilol (i.e., such as carvedilol dihydrogen phosphate salts) or corresponding solvates (i.e., as identified herein) thereof, with any of the characteristics noted herein, in association with one or more non-toxic pharmaceutically acceptable carriers or diluents thereof, and if desired, other active ingredients.

These active compounds may also be administered parenterally. Solutions or suspensions of these active compounds for use in such parental administrations can be prepared in water suitably mixed with a surfactant such as hydroxypropylcellulose.

Dispersions can also be prepared in glycerol, liquid polyethylene glycols, and mixtures thereof in oils. Illustrative oils are those of petroleum, animal, vegetable, or synthetic origin, for example, peanut oil, soybean oil, or mineral oil, etc. In general, water, saline, aqueous dextrose and related sugar solution, and glycols such as, propylene glycol or polyethylene glycol, etc., are preferred liquid carriers, particularly for injectable solutions. Under ordinary conditions of storage and use, these preparations contain a preservative to prevent the growth of microorganisms.

In accordance with the present invention, solid unit dosage forms can be conventional types known in the art. The solid form can be a capsule and the like, such as an ordinary gelatin type containing the compounds of the present invention and a carrier, for example, lubricants and inert fillers such as, lactose, sucrose, or cornstarch, etc. In another embodiment, these compounds are tableted with conventional tablet bases such as lactose, sucrose, or cornstarch in combination with binders like acacia, cornstarch, or gelatin, disintegrating agents, such as cornstarch, potato starch, or alginic acid, and a lubricant, like stearic acid or magnesium stearate, etc.

The tablets, capsules, and the like can also contain a binder, such as gum tragacanth, acacia, corn starch, or gelatin; excipients such as dicalcium phosphate; a disintegrating agent such as corn starch, potato starch, alginic acid; a lubricant such as magnesium stearate; and a sweetening agent such as sucrose, lactose, or saccharin, etc. When the dosage unit form is a capsule, it can contain, in addition to materials of the above type, a liquid carrier such as a fatty oil.

Various other materials may be present as coatings or to modify the physical form of the dosage unit. For instance, tablets can be coated with shellac, sugar, or both, etc. A syrup can contain, in addition to active ingredient, sucrose as a sweetening agent, methyl and propylparabens as preservatives, a dye, and flavoring such as cherry or orange flavor, etc.

For oral therapeutic administration, these active compounds can be incorporated with excipients and used in the form of tablets, capsules, elixirs, suspensions, syrups, and the like.

The percentage of the compound in compositions can, of course, be varied as the amount of active carvedilol free base or carvedilol salt, solvate or anhydrous form thereof in such therapeutically useful compositions is such that a suitable dosage will be obtained.

Typically in accordance with the present invention, the oral maintenance dose is between about 25 mg and about 70 mg, preferably given once daily. In accordance with the present invention, the preferred unit dosage forms include tablets or capsules.

It will be appreciated that the actual preferred dosages of the compounds being used in the compositions of this invention will vary according to the particular composition formulated, the mode of administration, the particular site of administration and the host being treated.

In particular, dosing in humans for treatment of diseases according to the present invention should not ordinarily or normally exceed a dosage range of from about 5 mg to about 75 mg of carvedilol free base or an equivalent amount of a carvedilol salt, solvate or anhydrous form thereof. As one of ordinary skill in the art will readily comprehend, the patient should be started on a low dosage regimen of a compound of the present invention and monitored for well-known symptoms of intolerance, e.g., fainting, to such compound. Once the patient is found to tolerate such compound amount, the patient should be brought slowly and incrementally up to the maintenance dose. The preferred course of treatment is to start the patient on a dosage regimen of either approximately or about 8 mg to about 16 mg, given once daily, for approximately two weeks. The choice of initial dosage most appropriate for the particular patient is determined by the practitioner using well-known medical principles, including, but not limited to, body weight. In the event that the patient exhibits medically acceptable tolerance of a compound, corresponding composition or formulation of the present invention for two weeks, the dosage is doubled at the end of the two weeks and the patient is maintained at the new, higher dosage for two more weeks, and observed for signs of intolerance. This course is continued until the patient is brought to a maintenance dose. The preferred maintenance dose for carvedilol free base or an equivalent amount of a carvedilol salt, solvate or anhydrous form thereof is about 32.5 mg to about 65 mg given once daily for patients having a body weight of up to 85 kg. For patients having a body weight of over 85 kg, the maintenance dose is about 65 mg if given once daily.

The active compounds of the present invention may be orally administered, for example, with an inert diluent, or with an assimilable edible carrier, or they can be enclosed in hard or soft shell capsules, or they can be compressed into tablets, or they can be incorporated directly with the food of the diet, etc.

In addition, compounds or pharmaceutical compositions of the present invention may incorporated into controlled or modified release forms, which may incorporate the use of or modification of various controlled release development processes, which may include, but are not limited to technologies such as those conventionally known in the art.

Specific examples of technologies related or used in the formation of pharmaceutical compositions, controlled-release formulations or dosage forms of the present invention are described below.

### General Formulation Technologies

Delivery systems suitable for use in accordance with the present invention, may include, but are not limited to materials as described generally in this section.

The term "active agent" is defined for purposes of the present invention as any chemical substance or composition of the present invention, such as carvedilol free base, or a carvedilol salt, anhydrous form, or solvate thereof, which can be delivered from the device into an environment of use to obtain a desired result. When the active agent is a biologically active drug, such as carvedilol free base, or a carvedilol salt, anhydrous forms, or solvate thereof, or corresponding pharmaceutical composition of the present invention, which is taken orally and the external fluid is gastric fluid, it is preferred that the drug exhibits a between the solubility defined in the United States Pharmacopeia (USP) XXI, page 7 as "freely soluble" (i.e., 1-10 parts solvent per 1 part solute) and "sparingly soluble" (i.e., 30-1000 parts solvent per 1 part solute).

The dosage form, which includes a device or delivery system associated with the present invention can be used in conjunction with a wide range of drugs (i.e., which includes carvedilol free base, or a carvedilol salt, anhydrous forms, or solvate thereof) and is especially well-suited for drugs having a wide therapeutic window, since precise dosing is not very critical for the same. The therapeutic window is commonly defined as the difference between the minimum effective blood concentration and the maximum effective blood concentration and the toxic concentration of the drug.

Depending upon the solubility and the amount of active agent to be included in the core, any generally accepted soluble or insoluble inert pharmaceutical filler (diluent) material may be used to bulk up the core or to solubilize the active agent.

Suitable materials for use in the present invention, include, but are not limited to sucrose, dextrose, lactose, fructose, xylitol, mannitol, sorbitol, dicalcium phosphate, calcium sulfate, calcium carbonate, starches, cellulose, polyethylene glycols, polyvinylpyrollidones, polyvinyl alcohols, sodium or potassium carboxmethylcelluloses, gelatins, mixtures of any of the above, and the like.

In addition, it is possible to directly compress an active agent with a small amount of lubricant when the active agent is soluble in the external fluid and is included in such an amount to provide a suitably sized core.

Lubricant may be mixed with the active agent and excipients prior to compression into a solid core. Any generally accepted pharmaceutical lubricant may be used, which may include, but are not limited to calcium or magnesium soaps and the like.

Active agents can be formulated with a small amount of a binder material such as, for example, gelatin or polyvinylpyrollidone (i.e. 94% - 99.75% of the core comprises the active agent). In such cases, the components of the core may be subjected to wet granulation. For example, highly soluble pharmaceutically active compounds such as potassium chloride may be directly compressed into an acceptable core with the inclusion of 0.25 percent magnesium stearate without being in admixture with an excipient.

The particular excipient chosen is dependent in part upon the solubility of the active agent in the environmental fluid. The ratio of active agent to excipient is based in part upon relative solubility of the active agent in the external fluid and the desired rate of release. If the active agent is relatively soluble, it may be desirable to slow down the eroding of the core by using a relatively insoluble excipient such as dicalcium phosphate.

Representative materials suitable for use in the present invention as a coating include those materials commonly considered to be insoluble in the art, which may include, but are not limited to materials, such as ethyl cellulose, acrylate polymers, polyamides (nylons), polymethacrylates, polyalkenes (polyethylene, polypropylene), bio-degradable polymers (including homo- or hetero-polymers of polyhydroxy butyric or valeric acids and homo or hetero-polymers of polylactic, polyglycolic, polybutyric, polyvaleric, and polycaprolactic acids), waxes, natural oils, other hydrophobic insoluble materials such as polydimethylsiloxane, hydrophilic materials such as cross-linked sodium carboxymethyl cellulose and cross-linked sodium or uncross-linked carboxymethyl starch and the like. Many other polymers considered to be relatively insoluble as conventionally used in the art also would be useful in the present invention.

It is also possible to use relatively thick coatings of materials in the present invention, which are considered in the art to be relatively soluble in, environmental fluid, which may include, but are not limited to materials, such as polyvinylpyrrolidone, cellulose ethers including hydroxypropylmethylcellulose, methylcellulose, hydroxyethylcellulose, hydroxypropylcellulose, sodium carboxymethyl cellulose, sodium carboxymethyl starch, enteric materials (such as cellulose acetate phthallate, polyvinylalcohol phthallate, shellac, zein, hydroxypropylmethyl cellulose phthallate, cellulose acetate trimaleate, etc) and the like.

In certain embodiments of the present invention, it may be advantageous to include one or more release modifying agents which aids in the release of the active agent from a suitable device of the present invention in the environment of use.

For example, the inclusion of a surfactant or an effervescent base may be helpful in certain cases to overcome surface tension effects, etc. Other releasing modifying agents may include osmagents (i.e., which osmotically deliver the active agent from the device by providing an osmotic pressure gradient against the external fluid and are particularly useful when the active agent has limited solubility in the environment of use), swelling agents (i.e., which may include, but are not limited to hydrophilic pharmaceutically acceptable compounds with various swelling rates in water) or other pharmaceutically acceptable agents (i.e., provided in an amount sufficient to facilitate the entry of the environmental fluid without causing the disruption of the impermeable coating).

Alternatively, release modifying agents, such as hydrophobic materials and insoluble polymers, may be used to slow the release of active agent from the device or release modifying agents may be used in conjunction with the present invention to include ion exchange resins.

Surfactants useful as release modifying agents in the present invention can be anionic, cationic, nonionic, or amphoteric. Examples of such surfactants or release modifying agents, may include, but are not limited to sodium lauryl sulfate, sodium dodecyl sulfate, sorbitan esters, polysorbates, pluronics, potassium laurate, and the like.

Effervescent bases useful as release modifying agents in the present invention, may include, but are not limited to sodium glycine carbonate, sodium carbonate, potassium carbonate, sodium bicarbonate, potassium bicarbonate, calcium bicarbonate, and the like.

Osmagents useful as release modifying agents in the present invention may include, but are not limited to sodium chloride, calcium chloride, calcium lactate, sodium sulfate, lactose, glucose, sucrose, mannitol, urea, and many other organic and inorganic compounds known in the art and the like.

Examples of suitable swelling agents for use in the present invention may include, but are not limited to, crosslinked polyvinylpyrrolidones (for example, such as polyplasdone, crospovidone and the like), crosslinked carboxyalkylcelluloses, crosslinked carboxymethylcellulose (for example, such as crosslinked sodium croscarmellose and the like), hydrophilic polymers of high molar mass (i.e., which may be, but are not limited to being greater than or equal to 100,000 Dalton) which may include, but are not limited to: polyvinylpyrrolidone(s), polyalkylene oxides (for example, such as polyethylene oxide or polypropylene oxide and the like), hydroxyalkylcelluloses (for example, such as hydroxypropylcellulose, hydroxypropylmethylcellulose and the like), carboxyalkylcellulose (for example, carboxymethylcellulose and the like), modified starch (for example, sodium glycolate and the like), starch or natural starch (for example, such as corn , wheat, rice, potato and the like), cellulose (for example, which may be, but not limited to being in powder form or microcrystalline form), sodium alginate, potassium polacriline , and corresponding blends or mixtures thereof.

For example, a swelling agent for use in the present invention may be selected from, but not limited to, the following sub-set or group of compounds or materials: crosslinked polyvinylpyrrolidone (for example, polyplasdone, crospovidone and the like), crosslinked carboxyalkylcelluloses, such as crosslinked carboxymethylcellulose (for example, crosslinked sodium croscarmellose and the like), etc.

Examples of other suitable pharmaceutically acceptable agents for use in the present invention, include synthetic gums, which further may include, but are not limited to hydroxypropylmethylcelluloses (HPMC) hydroxypropyl cellulose, carboxymethyl cellulose, and natural gums such as xanthan gum, locust bean gum, acacia, tragacanth, guar gum, carrageenan, and propylene glycol alginate, and the like.

Examples of suitable hydrophobic materials useful as release modifying agents in the present invention, include vegetable oils, which may include, but are not limited to hydrogenated cottonseed oil, hydrogenated castor oil, and the like. An example of insoluble polymers includes ethyl cellulose, etc.

A device may be designed such that the rate of release of the active agent varies with time which may be used to achieve a chronotherapeutic effect not normally possible with some conventional art-known sustained release devices.

### Microparticle or Microcapsule Type Technology Section

In light of the foregoing an example of a delivery system suitable for use in the present invention is described by a microparticle or microcapsule technology system, which may be include, but is not limited to a capsule that contains two or more populations of pellets, coated to provide earlier and later release. The early-release pellets may be coated with a polymer that delays release by pH, hydration or other effect. The later-releasing pellets may be coated with a polymer that dissolves at higher pH than the polymer coating the units providing early release.

Such a delivery system is exemplified by U.S. Pat. No. 5,004,614 to Autant et al., which is hereby incorporated by reference in its entirety.

In particular, U.S. Pat. No. 6,022,562 to Autant et al. discloses microcapsules used for oral administration of medicinal or nutritional active principles or particles (AP) and a process for making such microcapsules, which are smaller than or equal to 1000 µm in size, where such microcapsules are particles coated with a coating material (i.e., formed from a mixture of a film-forming polymer derivative, a hydrophobic plasticizer, a functional agent and a nitrogen-containing polymer) and are characterized by an ability to remain in the small intestine for a long time period (at least 5 hours) and allows, during the residence, release and absorption of the active principle.

In light of the foregoing, the present invention relates to microparticulate systems for the delayed and controlled release or modified release of carvedilol free base, salt, anhydrous or solvate forms thereof, which are active principle particles ("AP") according to the present invention designed for oral administration.

In general, carvedilol free base, salt, anhydrous or solvate forms thereof, which are active principle particles ("AP") according to the present invention, are coated by spraying with such a combination forming the coating film, as a dispersion or a suspension in an organic solvent or a mixture of organic solvents.

In another aspect of the present invention, the coating process is within the scope of micro-encapsulation techniques, which are summarized or exemplified in an article by C. Duverney and J. P. Benoit in "L'actualite chimique" December 1986 and cf. book entitled "Novel drug delivery and its therapeutic application" L. F. Prescott & W. S. Nimmo, Ed. John Wiley & Sons, which is hereby incorporated by reference in its entirety).

The technique is characterized as microencapsulation by film formation, which results in the formation of "reservoir" systems versus matrix systems.

Essential parameters of the present invention relate to residence time and in vivo absorption of microcapsules containing carvedilol free base, salt, anhydrous or solvate forms thereof, which are active principle particles in the gastrointestinal tract, such in the small intestine. The residence time in the small intestine of microcapsules, administered orally, as well as the in vivo absorption may by determined by measurement of the plasma concentration of carvedilol free base, salt, anhydrous or solvate forms thereof, which are active principle particles according to the present invention, which has a short half-life in the body of a mammal, i.e., which is not absorbed in the gastrointestinal tract components, such as the colon.

One of the important parameters of the present invention relates to microcapsule particle size. Desired particle size is determined by screening, which may be, but is not limited to be microcapsules between 100 microns and 500 microns in size.

Another important component of the present invention relates to the coating film or composition associated or applied to the microparticles. This coating composition is formed from a non-arbitrary choice of four compounds with unique functionalities and characteristics that combine to achieve the desired effects associated with delivery systems of the present invention. For example, ethylcellulose and cellulose acetate may be combined to a film-forming polymer P1 and may be soluble in at least one organic solvent of boiling point between 35°C and 120°C. Polyvinylpyrrolidone and/or polyacrylamide representing P2 are polymers soluble in at least one solvent for P1.

Suitable plasticizer, surface-active and/or lubricating agents for use in the present invention may include, but are not limited to castor oil, and/or diethyl phthalate, and/or triethyl citrate and/or salicylic acid, and magnesium stearate, and/or sodium oleate and/or polyoxyethylenated sorbitan laurate and the like.

As example of a coating composition suitable for use in the present invention may comprise, but is not limited to: ethylcellulose (P1)/polyvinylpyrrolidone (P2)/castor oil (plasticizer)/magnesium stearate (lubricating agent), which may be present respectively in the following specific relative percentages: 60% - 80% / 5 % -10 % / 5% - 10% / 2%-8% (i.e., where such percentages are defined by weight % relative to the total components of each coating composition).

Moreover, it is possible to add other conventionally art known adjuvants to a coating composition of the present invention, which may include, but are not limited to pigments, fillers and the like.

In order to prevent the caking problems associated with coated particles that constitute microcapsule or coated compositions of the present invention, at least one anti-agglomerating agent may be included. Suitable anti-agglomerating agents, may include, but are not limited to talc, colloidal silica or of a mixture of the two and the like, which may be in amounts of from 0.5% by weight to 5% by weight, preferably from 1.5% by weight to 3% by weight.

A general process for making coated microparticle containing compositions of the present invention may include, but are not limited to the following steps: [a] selecting, or in case of need making, microparticles of carvedilol free base, salt, anhydrous or solvate forms thereof, which are active principle particles ("AP") according to the present invention, with a particle size of between 50 and 1000 microns, preferably of between 100 and 750 microns and, more preferably, of between 100 and 500 microns; [b] preparing the coating composition by mixing together a polymer P1, a polymer P2, the plasticizer and the surface-active and/or lubricating agent in a solvent system; [c] applying the coating composition/solvent system mixture to particles of carvedilol free base, salt, anhydrous or solvate forms thereof, which are active principle particles according to the present invention; [d] drying the microcapsules thus obtained; and [e] optionally, mixing these microcapsules with at least one antiagglomerating agent.

Suitable solvents suitable for use in the composition of such a solvent system may include, but are not limited to, ketones, esters, chlorinated solvents, alcohols, which are preferably aliphatic, alkanes or mixtures thereof and the like. Specific solvent examples, may include, but are not limited to C₁ - C₆ compound solvents, such as acetone, methyl ethyl ketone, methanol, ethanol, isopropanol, cyclohexane and methylene chloride and the like.

In order to go into greater detail into the coating methodology which may be used in accordance with the invention, it may be pointed out that the coating composition/solvent system mixture is applied by spraying onto the particles of carvedilol free base, salt, anhydrous or solvate forms thereof, which are active principle particles according to the present invention, set in motion, preferably by mechanical stirring or by fluidization.

In order to obtain microcapsules according to the invention, it is necessary to encapsulate particles of carvedilol free base, salt, anhydrous or solvate forms thereof, which are active principle particles ("AP") according to the present invention, of size between 50 microns and 1000 microns. For example, such particles of carvedilol free base, salt, anhydrous or solvate forms thereof, which are active principle particles according to the present invention are between 100 microns and 750 microns; or between 100 microns and 500 microns.

The active principle particles of carvedilol free base, salt, anhydrous or solvate forms thereof, of desired particle size and necessary for the production of microcapsules according to the invention, may be crystals of pure carvedilol free base, salt, anhydrous or solvate forms thereof, which have undergone a pretreatment by one of the conventional techniques of the art such as, for example, granulation in the presence of a small amount of at least one standard binder and/or of an agent modifying the intrinsic solubility feature of the carvedilol free base, salt, anhydrous or solvate forms thereof, which are active principle particles according to the present invention.

According to an embodiment of the present invention, the content of carvedilol free base, salt, anhydrous or solvate forms thereof, which are active principle particles according to the present invention, before coating is between 75% and 100% by weight, preferably between 95% and 100% by weight. The amount of coating agent in the microcapsules may represent from 5% to 40% of the weight of the coated microcapsules. The actual density of the microcapsules according to the invention is not critical, but may be between 1.0 grams per cubic centimeter and 1.35 grams per cubic centimeter.

In accordance with the present invention, a embodiment of the process for the micro-encapsulation of particles of carvedilol free base, salt, anhydrous or solvate forms thereof, which are active principle particles according to the present invention, may include, but are not limited to the following steps: [a₁] preparation of a mixture, which comprises from 70% by weight to 80% by weight of a film-forming polymer P1 and 5 % by weight to 10% by weight of a plasticizer for 5% by weight to 10% by weight of a nitrogen-containing polymer P2 in solution, either in an acetone/alkanol mixture such that the acetone/alkanol volume ratio is between 50/50 and 70/30, or in a solvent, which may be chosen from, but not limited to cyclohexane, toluene, carbon tetrachloride, chloroform and methylene chloride and the like; [a₂] placing in suspension a solution prepared in the step [a₁], of 2% by weight to 8% by weight of surface-active; [b] lubricating and/or spraying of the resulting mixture onto microparticles of active principle, in a fluidized bed; [c] drying of microcapsules after spraying in a fluidized bed and/or in the oven; and/or [d] mixing of obtained or formed microcapsules with 0.5 by weight to 3% by weight of anti-adhesion agent, on the basis of 100% of final product obtained after mixing.

As described herein the microcapsules obtained by a example process as outlined above, may be used for manufacture of novel pharmaceutical or nutritional preparations of carvedilol free base, salt, anhydrous or solvate forms thereof, which are active principle particles according to the present invention, having optimized therapeutic or nutritional performance, which may be provided specifically in the form of tablets that can advantageously be crumbled, or powders or gelatin capsules.

Thus, the present invention relates to new galenic systems, which may be defined by the following forms: tablets, powders, gelatin capsules, which contain microcapsules of the present invention.

The microcapsules of the present invention are well tolerated by the human body, in particular at the gastric level.

The present invention also relates to these novel microparticle containing pharmaceutical preparations or compositions, which may be administered orally, preferably by single daily doses.

It also may be advantageous to mix within the same gelatin capsule, the same tablet or the same powder materials, at least two types of microcapsules which have release kinetics that are different, but are within the characteristic scope of the invention.

In accordance with the present invention, such microcapsules also may be mixed with a certain amount of other active principle particles ("AP") immediately available to the body.

The present invention also relates to the use of microcapsules as vehicles for at least one medicinal and/or nutritional active principle particle (AP) capable of residing in the small intestine for a prolonged period, where such microcapsules: Δ being designed for oral administration (i.e., e.g., which may be able to reside in the small intestine for at least about 5 hours, preferably at least about 7 hours and, even more preferably, for a period of between 8 and 24 hours, to allow the release of the active principles ("AP") in the small intestine for at least part of their residence time), Δ and consisting of particles of active principles ("AP") each coated with at least one coating film of specific composition and having a particle size of between 50 µm and 1000 µm, preferably of between 100 and 750 µm and, even more preferably, of between 100 µm and 500 µm.

PCT International Application WO 03/030878 to Flamel Technologies, which is incorporated by reference in its entirety, also discloses a microparticulate system based upon oral administration for delayed and controlled release of active principles, where the in vivo absorption window is limited to parts of the gastrointestinal tract.

The WO 03/030878 Application is aimed at providing a system for reliably releasing active principles through a double time dependent and pH-dependent mechanism.

In light of the foregoing, the present invention also relates microparticulate systems for the delayed and controlled-release or modified release of carvedilol free base, salt, anhydrous or solvate forms thereof, which are active principle particles ("AP") according to the present invention, such as a multiple microcapsule galenic oral formulation designed for therapeutic efficacy, such that release of active principles is controlled by a double release triggering mechanism, involving time triggering and pH-triggering.

In particular, the present invention relates to a microparticulate galenic (i.e., tablets, powders, gelatin capsules, which contain microcapsules of the present invention) formulation with delayed and controlled-release for which the controlled-release phase is triggered in a specific way due to a double mechanism: "time-dependent" release triggered after a certain amount of time in the stomach, and "pH-dependent" release triggered by a change in pH when the particles enter into the small intestine and which starts without a latency period.

The microparticles of this invention are microcapsules containing at least one carvedilol free base, salt, anhydrous or solvate forms thereof, which are active principle particles according to the present invention, with granulometry such as between 100 microns and 1,200 microns individually covered with a film coating allowing the delayed and controlled-release of the active principle.

Modified-release or delayed and controlled-release systems containing active principles are useful when it is desirable, for chronobiological reasons, for such active principles to be "bioabsorbed" at a specific time of day so that it is in phase with the circadian cycle. This approach is appropriate for the treatment of cancer, hypertension, atherosclerosis, administration of antiinflammatory drugs or regulation of glycemia in the treatment of diabetes.

For example, it may be advantageous for an active carvedilol free base, salt, anhydrous or solvate forms thereof to be bioabsorbed very early in the morning in order to ensure therapeutic coverage when the patient awakens without requiring early awakening. To achieve this, the galenic system ingested by the patient in the evening after a meal, should allow the delayed-release of the active principle.

In the case of a modified, delayed or controlled release formulation, it is therefore crucial to have a complete guarantee of release and absorption of the active principle by a patient at a specific moment in order to achieve the therapeutic effect. However, it must be pointed out that the delayed-release forms cannot definitely ensure the release of the active principle in a prescribed amount of time. For example, achievement of accurate timed release of carvedilol free base, salt, anhydrous or solvate forms thereof, which are active principle particles according to the present invention, are important especially for patients undergoing cardiovascular disease or diabetes treatments.

In fact, traditionally, delayed-release forms are obtained by coating the active principle with a layer of enteric polymer. This type of enteric coating is known to present reduced permeability in the acid pH conditions in the stomach and dissolves when the pH increases to a value close to what exists in the small intestine, thus releasing the active principle. However, the intra and inter-individual variability of gastric pH conditions and of gastric emptying duration do not allow the definite release of the active principle after a specific amount of time.

The purely "time-dependent" delayed-release systems for which release of the active principle is triggered after it spends a specific amount of time in the gastro-intestinal tract, are not satisfactory either. In fact, due to the intra and inter-individual variability of gastric residence time, release of the active principle may occur after the latter has passed its absorption window, which is located for a majority of active principle's in the upper part of the gastro-intestinal tract. Bio-absorption may thus be very low, even non-existent.

In this context, it would be particularly advantageous to have a delayed and controlled-release formulation of the active principle, which ensures active principle release based upon the aforementioned double triggering release of such active principles: i.e., "time-dependent" release triggered after a controlled amount of time in the stomach, without pH change, and "pH-dependent" release triggered by an increase in the pH when the galenic formulation penetrates into the intestine. These two triggering factors for release of carvedilol free base, salt, anhydrous or solvate forms thereof, which are active principle particles according to the present invention, occurring in serial fashion would make formulations of the present invention very safe to use. The release of the active principle would be guaranteed, after a pre-regulated latency time, even if the pH variation did not intervene as a triggering factor (i.e., even if such a galenic formulation did not pass from the stomach into the intestine).

In order to minimize the inter-individual variability of the absorption of the principle particle of carvedilol free base, salt, anhydrous or solvate forms thereof, which are active principle particles according to the present invention, it is necessary to adjust the latency time preceding the release of the carvedilol free base, salt, anhydrous or solvate forms thereof active principle particle in the stomach by taking into consideration the physiological conditions of the gastrointestinal tract in humans. According to the well-known results of Davis et al., J. of Controlled-Release, 2, 27-38 (1985), the residence time in the stomach of a preparation varies greatly, on the order of 0.5 to 10 hours. It would therefore be advantageous to have a galenic formulation releasing the active principle in the stomach after a given constant latency time, within this 0.5 hours to 10 hours interval, so that the drug's action time is the same from one individual to another, or even from one day to the next for the same individual.

In addition, in order to optimize the bioavailability of the active principles whose absorption is limited mainly to the upper portions of the gastro-intestinal tract, it would be advantageous for the "pH-dependent" release in the intestine to occur without latency time because otherwise the active principle will not be released within its absorption window and the patient will consequently not be treated.

Another unique interesting factor of such a system would be to allow us to achieve, by mixing with an immediate-release galenic formulation of active principle carvedilol free base, salt, anhydrous or solvate forms thereof, or by mixing with another delayed- and controlled-release galenic formulation of active principle carvedilol free base, salt, anhydrous or solvate forms thereof, release profiles presenting several waves of active principle carvedilol free base, salt, anhydrous or solvate forms thereof release (i.e., which represents a single active principle or several identical or different active principles) or ensuring with appropriate adjustment of the various fractions a constant plasma concentration level of active principle.

Suitable delayed and controlled-release formulations of the present invention may be comprised of, but not limited to a large number of microcapsules with a diameter of less than 2000 microns. In fact, for such a formulation, the dose of active principles to be administered is distributed among a great number of microcapsules (typically 10,000 for a dose of 500 mg).

In accordance with the present invention, the following intrinsic advantages result from the use of such delayed and controlled-release formulations: [1] prolonged residence time of microcapsules in regions of the gastrointestinal tract, which ensures an increase in the amount of time the active principle carvedilol free base, salt, anhydrous or solvate forms thereof spends within absorption windows to maximize active principle bioavailability; [2] implementation of a mixture of microcapsules with various immediate and/or modified, delayed or controlled-release profiles allows release profiles to be achieved that present several waves of release to ensure a constant plasma concentration level of active principle carvedilol free base, salt, anhydrous or solvate forms thereof by adequate adjustment of various fractions; [3] sensitivity to variability of gastric emptying is less, because the emptying, which occurs here with a large number of particles, is statistically more reproducible; [4] contact of the tissues with a high dose of active principle carvedilol free base, salt, anhydrous or solvate forms thereof is avoided: "dose dumping", where each microcapsule contains only a very reduced dose of the active principle carvedilol free base, salt, anhydrous or solvate forms thereof and risk of deterioration of tissues by a local superconcentration of aggressive active principle carvedilol free base, salt, anhydrous or solvate forms thereof is eliminated.; [5] possibility to form "multi-microcapsular" systems, i.e., to combine several galenic forms (immediate-release and/or delayed-release and/or prolonged-release) containing one or several active principle forms of carvedilol free base, salt, anhydrous or solvate forms thereof; [6] possibility to present these microcapsules in a packet, capsule or tablet form (i.e., as in cases where active principle dose is high (500 mg or more), monolithic formulations are too large to be easily swallowed, such that it is important for a microparticulate formulation to ensure delayed and controlled-release of an active principle carvedilol free base, salt, anhydrous or solvate forms thereof that one skilled in the art can put into the form of splittable tablets or packets.; and [7] desirability for film coating around microcapsules to not be very thick.

In particular, it would be important to have an oral microparticulate galenic formulation with delayed and controlled-release of AP carvedilol free base, salt, anhydrous or solvate forms thereof, simultaneously having the following properties: where active principles release may be triggered, by time-dependent release when duration of particulates in the stomach exceeds 5 hours; and by pH variation-dependent release, also called "pH-dependent", which starts without latency time when the system penetrates into the intestine and the pH increases.

Importantly, these two triggering factors affect release of active principle carvedilol free base, salt, anhydrous or solvate forms thereof occurring in serial fashion to guarantee release of the active principle after a pre-regulated latency time, even if the pH variation is not involved as a triggering factor. It is composed of a large number of microcapsules of coated active principle carvedilol free base, salt, anhydrous or solvate forms thereof small in size; and/or mass fraction in excipients of coating is limited.

Further in accordance with the present invention is the use of suitable swelling agents in compositions or controlled-release formulations as described herein.

As used in the present invention, the term "swelling agent" may include, but is not limited to at least one pharmaceutically acceptable hydrophilic compound, having a swelling rate or swelling amount in water at about 25°C that is: greater than or equal to at least 10% by weight (wt/wt), greater than or equal to at least 15% by weight (wt/wt), or greater than or equal to at least 20% by weight (wt/wt).

According to an advantageous or remarkable characteristic of the present invention, a swelling agent is selected from among swelling agents with chemical, physical or pharmaceutically acceptable characteristics which make possible for microcapsules of the present invention to release at least 50% by weight (wt/wt) of the active principle carvedilol free base or carvedilol salt, anhydrous or solvate thereof, after at least 16 hours at about a pH of 1.4 and after a latency phase or lag time of less than or equal to about 7 hours, or after a latency phase or lag time of less than or equal to about 5 hours or after a latency period or lag time of between about 1 hour to 1.5 hours in an *in vitro* dissolution test performed according to guidelines as specified in the European Pharmacopoeia, 4th Edition, entitled: "Dissolution Test of Solid Oral Forms": a type II dissolutest performed in SINK conditions kept at 37°C and stirred or agitated at 100 rpm.

According to the present invention, it is possible to adjust the rate of release at about a pH of 1.4 of the active principle carvedilol free base or carvedilol salt, anhydrous or solvate thereof, from the microcapsules described herein, by carefully selecting the concentration (Cd) of the swelling agent particles in the microcapsules.

When a swelling agent is included into a microparticle form of the present invention, the mean diameter (Td) of a swelling agent particle is selected from particle sizes with ranges of at least between 5 micrometers (µm) to 200 micrometers (µm), or of at least between 10 micrometers (µm) to 50 micrometers (µm).

In light of the above and in an embodiment of the present invention, the concentration (Cd) of the swelling agent is selected from to include, but is not limited to be in the following ranges of percentage by weight (in wt%) relative to the total mass of the microcapsules of the present invention as follows:
- 3 < = Cd <= 40;
- 4 < =Cd <= 30; or
- 5<=Cd<=25.

In one embodiment swelling agents for use in the present invention may include, but are not limited to, crosslinked polyvinylpyrrolidones (for example, such as polyplasdone, crospovidone and the like), crosslinked carboxyalkylcelluloses, such as crosslinked carboxymethylcellulose (for example, such as crosslinked sodium croscarmellose and the like), hydrophilic polymers of high molar mass (for example, i.e., which may be, but not limited to being greater than or equal to 100000 Dalton) which may include, but are not limited to: polyvinylpyrrolidone, polyalkylene oxides (for example, such as polyethylene oxide or polypropylene oxide and the like), hydroxyalkylcelluloses (for example, such as hydroxypropylcellulose, hydroxypropylmethylcellulose and the like), carboxyalkylcellulose (for example, carboxymethylcellulose and the like), modified starch (for example, sodium glycolate and the like), starch (for example, such as corn , wheat, rice, potato and the like), cellulose (for example, which may be, but not limited to being in powder form or microcrystalline), sodium alginate, potassium polacriline , and corresponding blends thereof.

In another embodiment, a swelling agent for use in the present invention may be chosen from, but not limited to, the following sub-set of compounds: crosslinked polyvinylpyrrolidone (e;g; polyplasdone or crospovidone), crosslinked carboxyalkylcelluloses (such as the crosslinked carboxymethylcellulose (e;g; crosslinked sodium croscarmellose), and the like.

In another embodiment, a suitable swelling agent for use in the present invention may also a nitrogen containing polymer, which may include but is not limited to polyvinylpyrrolidone, crosslinked polyvinylpyrrolidone and the like.

Also in accordance with the present invention, to reduce the possibility of active principle carvedilol free base, salt, anhydrous or solvate forms thereof being insufficiently wetted by water and therefore tending to aggregate, it is proposed in an advantageous variant of the present invention is to make sure that a formulation or pharmaceutically acceptable composition of the present invention may include, but is not limited to including at least one wetting agent, selected from, but not limited to the following group of products: anionic surfactants (for example, such as those in the sub-group of the alkaline or alkaline-earth salts of fatty acids, stearic acid, oleic acid and the like) and/or non-ionic surfactants (for example, which may include, but are not limited to polyoxyethylenated oils (for example, such as polyoxyethylenated hydrogenated castor oil and the like), polyoxyethylene-polyoxypropylene copolymers, polyoxyethylenated sorbitan esters, polyoxyethylenated castor oil derivatives, stearates (for example, such as calcium, magnesium, aluminum, zinc stearate and the like), stearyl fumarates (for example, such as sodium stearyl fumarate and the like), glycerol behenate, or mixtures thereof.

In accordance with the present invention, mixtures of the following components in general form the basis of a controlled-release microparticle composition of the present invention:
i) Microparticle pellets or granules, which comprise an active carvedilol free base or a carvedilol salt, solvate or anhydrous form thereof formulated to release the drug relatively quickly, but not immediately, as there may be a time delay in release;
ii) pellets, granules or microparticles may be prepared by granulating the drug with a hydrophilic polymer such as cellulose ethers, which may include, but is not limited to methyl cellulose, hydroxypropyl methylcellulose, polyvinyl pyrrolidone and the like. Alternatively, the drug may be applied to a base granule in an aqueous slurry with hydrophilic polymers, followed by subsequent drying to provide an embedded drug/matrix pellet;
iii) pellets, granules or microparticles also may be coated with or containing the drug embedded in a polymer, which prevents release at the more acid pH values encountered in the gastrointestinal tract, but which effect ready release of drug, after a defined time has elapsed or when the pellet, granule or microparticle unit is in an environment of more neutral pH. Examples of suitable coating or release modifying materials, may include, but are not limited to methacrylic acid polymers, shellac or cellulose acetate phthalate or mixtures thereof and the like; or
iv) pellets, granules or microparticles coated with or containing embedded drug as described in [iii], but which release drug at higher pH, such as a pH of about 6.5 to a pH of about 7.5, prepared using identical or similar coating materials as in [iii] such that coating material ratios may be varied.

In light of the foregoing, the early releasing component would be formulated to start releasing drug shortly after dosing (i.e., when a pellet, granule or microcapsule unit enters the stomach) to provide a "pulse", peaking at about 1 hour to about 3 hours. At a later time, the more slowly releasing formulated components release drug in parts of the small intestine, where the associated polymer coat or matrix is soluble.

The overall dose of drug and ratios of the different pellets, granules or microparticles can be determined by studies in human volunteers to examine plasma levels for at least 24 hours after dosage.

A general representative process for forming a controlled release formulation of the present invention, may be, but is not limited to a process where:
[1] rapidly releasing microparticle units are formed by applying a aqueous or other suitable liquid dispersion, which contains a carvedilol free base, salt, solvate, or anhydrous form thereof and at least one nitrogen-containing polymer and, optionally other pharmaceutically acceptable agents, onto cellulose or similar pharmaceutically acceptable core microparticle, which are subsequently heated in environment where moisture is removed, leaving the aforementioned desired material solids embedded or layered on the core microparticles;
[2] delayed or controlled release microparticle core particles are formed by applying a aqueous or other suitable liquid dispersion, which contains a carvedilol free base, salt, solvate, or anhydrous form thereof and at least one nitrogen-containing polymer and, optionally other pharmaceutically acceptable agents, onto cellulose or similar pharmaceutically acceptable core microparticle, subsequently which are heated in environment where moisture is removed, leaving the aforementioned desired material solids embedded or layered on the core microparticles. A delaying or drug release-modifying coat is further applied to the each of the aforementioned heated core microparticles, where such a coat is comprised of at least one film-forming polymer, a plasticiser, a surface active agent and, optionally other pharmaceutically acceptable excipients know to those skilled in the art. The delaying or drug release-modifying coat components may be dissolved or dispersed in an aqueous or suitable non-aqueous solvent, the coat being applied in a warm environment such that the solvent is removed by evaporation;
[3] where each of the rapidly releasing microparticle units or delayed or controlled release microparticle core particles are held such that the coated particles are in a warm environment to ensure that each applied coat or coat layer is satisfactorily formed;
[4] where one or more rapidly releasing or delayed controlled release microparticle units are mixed together; or
   where one or more rapidly releasing or delayed controlled release populations respectively are formed from the each type of the above-identified microparticle units with a mixture formed from each one or more rapidly releasing population and each of the different controlled release populations.
[5] where each mixture type as identified in [4] are filled into a appropriate delivery form, such as a capsule.

In light of the foregoing technologies, it may be possible to develop stable pharmaceutical compositions or controlled release or modified dosage forms, containing such carvedilol free base or carvedilol salts, solvates, or anhydrous forms thereof of the present invention, which may be used in combination therapies with other for once-per-day dosage, delayed release or pulsatile release to optimize therapy by matching pharmacokinetic performance (i.e., which relates to the time-dependent changes of plasma drug concentration and the time dependent changes of the total amount of drug in a body following various routes of administration) with pharmacodynamic requirements (i.e., which relates to the biochemical and physiologic effects of drugs and their mechanisms of action).

As previously indicated herein, it would be expected that therapy would be more effective if peak plasma levels were provided times of greatest risk. In such a context a carvedilol based dosage form that is taken at night (at bedtime), that delivers drug in two phases to cover the midnight-3 am period, and the early morning surge ought provide optimum therapy, while maintaining a once-daily dosage regimen.

A controlled-release microparticle composition, dosage form or formulation of the present invention, may be comprised of, but is not limited to rapidly releasing microparticles or different types controlled release microparticles (such as first or second controlled release microparticles) or respective corresponding populations thereof, where each type of the aforementioned microparticles may include, but is not limited to a carvedilol free base or a carvedilol salt, solvate or anhydrous form thereof.

As described herein, each of the aforementioned microparticles also may contain, but are not limited to an active drug ingredient selected from the group consisting of salt forms of carvedilol mandelate, carvedilol lactate, carvedilol maleate, carvedilol sulfate, carvedilol glutarate, carvedilol mesylate, carvedilol phosphate, carvedilol citrate, carvedilol hydrogen bromide, carvedilol oxalate, carvedilol hydrogen chloride, carvedilol hydrogen bromide, carvedilol benzoate, and corresponding solvates thereof.

Further such specific microparticle types of the present invention may include, but are not limited to an active drug ingredient selected from carvedilol hydrogen phosphate, carvedilol dihydrogen phosphate, carvedilol dihydrogen phosphate hemihydrate, carvedilol dihydrogen phosphate dihydrate, carvedilol dihydrogen phosphate methanol solvate, carvedilol hydrobromide monohydrate, carvedilol hydrobromide dioxane solvate, carvedilol hydrobromide 1-pentanol solvate, carvedilol hydrobromide 2-methyl-1-propanol solvate, carvedilol hydrobromide trifluoroethanol solvate, carvedilol hydrobromide 2-propanol solvate, carvedilol hydrobromide n-propanol solvate #1, carvedilol hydrobromide n-propanol solvate #2, carvedilol hydrobromide anhydrous forms or anhydrous forms, carvedilol hydrobromide ethanol solvate, carvedilol hydrobromide dioxane solvate, carvedilol monocitrate monohydrate, carvedilol mandelate, carvedilol lactate, carvedilol hydrochloride, carvedilol maleate, carvedilol sulfate, carvedilol glutarate, or corresponding anhydrous forms, solvates thereof.

Also such specific microparticle types of the present invention may include, but are not limited to an active drug ingredient selected from carvedilol hydrogen phosphate, carvedilol dihydrogen phosphate, carvedilol dihydrogen phosphate hemihydrate, carvedilol dihydrogen phosphate dihydrate. In a specific embodiment, the active drug is selected from a carvedilol phosphate anhydrous form or carvedilol dihydrogen phosphate hemihydrate .

Therefore, in a specific embodiment of the present invention, such units, dosage forms, pharmaceutical compositions or controlled-release formulations of the present invention are formulated or prepared so that drug is released in "pulses", separated in time such that the first "peak" or Tₘₐₓ occurs within 1-4 hours of dosage, preferably the first "peak" or Tₘₐₓ occurs 1-2 hours of dosage or preferably the first "peak" or Tₘₐₓ occurs within 2-4 hours of dosage, with the second "peak" or Tₘₐₓ occurring 5-10 hours later or preferably the second "peak" or Tₘₐₓ occurring 5-8 hours later.

In accordance with the present invention, the pulses as described above may refer to a peak, plasma peak concentration level, "a first peak" or "a first peak plasma concentration level", a "second peak" or a "second peak plasma concentration level", etc.

In particular, such a pharmaceutical composition or controlled-release formulation of the present invention following oral dosage would be depicted by a unique substantially biphasic pharmacokinetic/ pharmacodynamic plasma profile, which exhibits a first Tₘₐₓ pulse and a plasma concentration peak level within 1-4 hours of ingestion and a second Tₘₐₓ pulse and a plasma concentration peak level within, 5-10 hours after ingestion.

In accordance with the present invention, a substantially biphasic plasma profile corresponding to a controlled release composition, dosage form or formulation of the present invention is represented by a graphical profile representation depicting a plasma profile curve, where a higher second plasma peak concentration level may or may not be substantially or significantly higher than a lower or first plasma peak concentration level as the substantially biphasic nature of the profile curve may be obscured as mean plasma level value variations may vary based upon intrinsic intersubject variation or variability.

In another specific embodiment, such a pharmaceutical composition or controlled-release formulation of the present invention following oral dosage would be depicted by a unique substantially biphasic pharmacokinetic/pharmacodynamic plasma profile, which exhibits a first Tₘₐₓ pulse and a plasma concentration peak level within 2-4 hours of ingestion and a second Tₘₐₓ pulse and a plasma concentration peak level within, 5-8 hours after ingestion.

In a specific embodiment, the aforementioned oral dosage or administration associated with a pharmaceutical composition or controlled-release formulation of the present invention, preferably occurs at night.

It is important that release from the first "pulse" or Tₘₐₓ occurs gradually, so that subsequent absorption is gradual, thereby avoiding a rapid fall in blood pressure. This would minimize the risk of orthostatic hypotension-related adverse events.

Such a profile can be obtained by formulating a drug suitable for use in the present invention with differential release, capitalizing on a combination of approaches to operate sequentially. It may be, for instance that a capsule may be formulated into pellets, capsules or microparticles which may be, but are not limited to coatings with different release-modifying components, such pellets being contained in capsule dosage forms such that release characteristics are affected or influenced by factors such as gastrointestinal pH, or time, to provide differentiated absorption profiles.

### Embodiments

In light of the foregoing discussion, the present invention relates to and is exemplified by, but not limited to the following embodiments present below, which include corresponding pharmaceutical compositions, different controlled release formulations, respectively comprised or formed from the following components, such as carvedilol free base, carvedilol salts, anhydrous forms or solvates thereof, and which also may include, but are not limited to the various components (i.e., such as conventionally known, adjuvants, carriers, diluents, excipients, agents, plasticizers, polymers, etc. as described herein) which may be in or formed into different dosage forms (i.e., which may include, but are not limited to, tablets, capsules and the like) as described herein.

In a general or first embodiment, the present invention relates to a controlled release formulation, which comprises:
a solubility enhanced carvedilol free base or a carvedilol salt, solvate or anhydrous forms thereof;
where the controlled release formulation following oral dosage exhibits a substantially biphasic plasma profile with a first plasma concentration peak level and a first Tₘₐₓ pulse within 1-4 hours of ingestion and a second plasma concentration peak level and a second Tₘₐₓ pulse within 5-10 hours after ingestion. In an embodiment of the present invention a first Tₘₐₓ pulse may occur within 2-4 hours of ingestion and the second Tₘₐₓ pulse may occur within 5-8 hours after ingestion.

In accordance with the present invention and the aforementioned general or other embodiments, a specific embodiment relates to a formulation in an oral dosage form. Such an oral dosage form may be in a capsule dosage form. A capsule dosage form of the present invention may be comprised of, but not limited to a mixture of two or more populations of coated pellets of different sizes with different associated immediate or controlled release characteristics.

In another or second embodiment, the present invention also relates to a controlled release formulation, comprising at least one of the following components:
[a] carvedilol free base; and [b] a solubility enhanced carvedilol salt, solvate or anhydrous forms; or
[a] carvedilol free base; or [b] a solubility enhanced carvedilol salt, solvate or anhydrous forms;
where the controlled release formulation following oral dosage exhibits a substantially biphasic plasma profile which exhibits a first plasma concentration peak level and a first Tₘₐₓ pulse within 1-4 hours of ingestion and a second plasma concentration peak level and a second Tₘₐₓ pulse within 5-8 hours after ingestion.

In yet another or third embodiment, the present invention also relates to a controlled release formulation, comprising at least one of these components:
[a] carvedilol free base; and [b] a solubility enhanced carvedilol salt, solvate or anhydrous forms; or
[a] carvedilol free base; or [b] a solubility enhanced carvedilol salt, solvate or anhydrous forms;
where the controlled release formulation is a capsule dosage form is comprised of a pellet or microparticle mixture of immediate release coated pellets or microparticles or controlled release coated pellets or microparticles of differing sizes; and
where the controlled release formulation following oral dosage at night exhibits a substantially biphasic plasma peak concentration profile which exhibits a first pharmacokinetic plasma concentration peak level and a first Tₘₐₓ pulse within 1-4 hours of ingestion and a second plasma concentration peak level and a second Tₘₐₓ pulse within 5-8 hours after ingestion.

In accordance with the present invention and the aforementioned third or other embodiments, a capsule dosage form of the present invention, may be, but is not limited to being a soft gelatin capsule or hard gelatin capsule. Such a pellet or microparticle mixture may consist of two or more different pellet or microparticle population types characterized by different pellet sizes and/or different immediate-release and/or modified-release characteristics. Moreover, different pellet or microparticle sizes and/or different immediate-release and/or modified-release characteristics are achieved by layering of active principle(s) and pharmaceutically acceptable components. The coated immediate-release pellets or microparticles, or modified-release pellets or microparticles, respectively, may be, but not limited to being coated with a polymer. The coated pellets consist of two or more different population types, with one population defined as immediate release pellets or microparticles and another population defined as modified release pellets or microparticles. More particularly, the capsule dosage form may be filled with differing ratios of immediate release pellets or microparticles and modified release pellets or microparticles blended together to a form a pharmaceutically acceptable amount or dosage. The capsule dosage form also may be filled with differing ratios of immediate release pellets or microparticles and modified release pellets or microparticles. In particular, immediate release pellets or microparticles and modified release pellets or microparticles may be in a ratio range from about 30% to about 70%, preferably the immediate release pellets and modified release pellets are in a ratio from about 40% to about 60%.

In yet another or fourth embodiment, the present invention also relates to a controlled release formulation, which comprises:
a solubility enhanced carvedilol free base or a carvedilol salt, solvate or anhydrous forms;
where the controlled release formulation is a capsule dosage form is comprised of a mixture of two or more populations of coated pellets or microparticles of differing sizes with differing release characteristics; and
where the controlled release formulation following oral dosage exhibits a substantially biphasic plasma peak concentration profile which exhibits a first pharmacokinetic plasma concentration peak level and first Tₘₐₓ pulse within 1-4 hours of ingestion and a second plasma concentration peak level and a second Tₘₐₓ pulse within 5-8 hours after ingestion.

In yet another or fifth embodiment, the present invention also relates to a controlled release formulation or a pharmaceutical form, which comprises:
several microcapsules for the modified or controlled release of carvedilol free base or a carvedilol salt, anhydrous or solvate form thereof,
   wherein at least one part of the microparticles are individually composed of a microparticle comprised of or loaded with the carvedilol free base or the carvedilol salt, anhydrous or solvate form thereof; or such microparticles are loaded with low or a poorly soluble carvedilol free base or a carvedilol salt, anhydrous or solvate form thereof; and
   coated by at least one coating layer ensuring the controlled or modified release of the carvedilol free base or a carvedilol salt, anhydrous or solvate form thereof,
   where these microcapsules are characterized by:
      (a) carvedilol free base or a carvedilol salt, anhydrous or solvate form thereof release governed by two different triggering mechanisms, one being based on a variation in pH and the other allowing the release of the carvedilol free base or a carvedilol salt, anhydrous or solvate form thereof after a predetermined residence time in the stomach,
      (b) the dissolution behavior in vitro (determined as indicated in the European Pharmacopeia, 4th edition, under the title: "Dissolution test for solid oral forms": type II dissolutest performed under SINK conditions, maintained at 37°C and agitated at 100 rpm) such that:
         - at a constant pH of 1.4, the dissolution profile includes a latency phase with a duration less than or equal to 7 hours, preferably less than or equal to 5 hours and more preferably between 1 and 5 hours; and
         - the change from about a pH of 1.4 to about a pH of 6.8, during the latency phase, results in a release phase that starts without a latency period; and
   (c) at least one part of these microcapsules contains at least one swelling agent.

In yet another or sixth embodiment, the present invention also relates to a controlled release formulation, comprising at least one of these components:
[a] carvedilol free base; and [b] a solubility enhanced carvedilol salt, solvate or anhydrous forms; or
[a] carvedilol free base; or [b] a solubility enhanced carvedilol salt, solvate or anhydrous forms;
where the controlled release formulation is a capsule dosage form is comprised of a mixture of two or more populations of coated pellets or microparticles of differing sizes and differing release characteristics; and
where the controlled release formulation following oral dosage at night exhibits a substantially biphasic plasma peak concentration profile which exhibits a first pharmacokinetic plasma concentration peak level and a first Tₘₐₓ pulse within 1-4 hours of ingestion and a second plasma concentration peak level and a second Tₘₐₓ pulse within 5-8 hours after ingestion.

In accordance with the present invention, and which may be relevant to the aforementioned fourth, fifth, sixth or other embodiments, each of the two or more populations of the mixture coated pellets or microparticles are characterized by different release-modifying components or characteristics based upon gastrointestinal environment, pH, or time. The mixture of two or more populations of coated pellets or microparticles is comprised of a first set of controlled release pellets or microparticles and a second set of controlled release pellets or microparticles, wherein the first set of controlled release pellets or microparticles provides earlier release of its chemical and physical properties than the second set of controlled release pellets. In particular, such a first set of controlled release pellets or microparticles may be, but is not limited to being coated with a pH sensitive polymer that delays active component release by pH or hydration effects. The second set of controlled release pellets or microparticles, may be, but is not limited to, being coated with a pH sensitive polymer that dissolves at a higher pH than the polymer which coats the first set of release pellets or microparticles.

In yet another or seventh embodiment, the present invention relates to a microparticle composition or formulation, which comprises:
a mixture of:
   [a] rapidly -releasing microparticles containing carvedilol free base, or a carvedilol salt, solvate, or anhydrous form thereof; and
   [b] at least two types of controlled-release microparticles containing carvedilol free base or a carvedilol salt, solvate, or anhydrous form thereof;
      where the rapidly releasing microparticles contain a first dosage amount of the carvedilol free base or a first dosage amount of the carvedilolsalt, solvate, or anhydrous form thereof different than each of the dosage amounts of the carvedilol free base or the carvedilol salt, solvate, or anhydrous form thereof contained in at least two different types of delayed or controlled-release microparticles; and
      where rapid release of the first dosage amount of the carvedilol free base or the carvedilol salt, solvate, or anhydrous form thereof contained in the rapidly releasing microparticles is followed by a serial or sequential time-triggered and pH-triggered release of each of the different dosage amounts of the carvedilol free base or the carvedilol salt, solvate, or anhydrous form thereof contained in each of the at least two different types of controlled release microparticles.

In accordance with the present invention and seventh or other embodiments, the serial or sequential pH triggered release of each of the different dosage amounts of the carvedilol free base or the carvedilol salt, solvate, or anhydrous form thereof contained in each of the at least two different types of controlled release microparticles occurs at a pH of about 5.5 to a pH of about a pH > 6.4. In such an embodiment, a first maximum plasma drug level following rapid release of the first dosage amount of the carvedilol free base, or the carvedilol salt, solvate, or anhydrous form thereof contained in the rapidly releasing microparticles occur at a time between about 1 hour to about 3 hour following dosage of the microparticle composition or formulation; and a second maximum plasma drug level following the serial or sequential time-triggered release of each of the different dosage amounts of the carvedilol free base or the carvedilol salt, solvate, or anhydrous form thereof contained in each of the at least two different types of controlled release microparticles occur at a time between about 5 hours to about 10 hours following dosage of the microparticle composition or formulation.

Also in accordance with a microparticle composition or formulation of the present invention, each of the first and second maximum plasma levels in such an embodiment following the time and pH triggered release of each of the different dosage amounts of the carvedilol free base or the carvedilol salt, solvate, or anhydrous form thereof contained in the rapidly releasing microparticles and each of the at least two different types of controlled release microparticles results in a controlled release of the total dosage amount of the carvedilol free base or the carvedilol salt, solvate, or anhydrous form thereof contained in the microparticle composition or formulation.

In addition, the first maximum plasma levels following the time and pH triggered release of each of the different dosage amounts of the the carvedilol free base or the carvedilol salt, solvate, or anhydrous form thereof contained in the rapidly releasing microparticles results in about 10% to about 15%, such as 12.5%_of the total dosage amount of carvedilolfree base or carvedilol salt, solvate, or anhydrous form thereof contained in the microparticle composition or formulation.

In addition, the second maximum plasma levels following the time and pH triggered release of each of the different dosage amounts of the carvedilol free base or the carvedilol salt, solvate, or anhydrous form thereof contained in each of the at least two different types of controlled release microparticles results in a controlled release of between about 85 % to about 90 %, such as 87.5% the total dosage amount of the carvedilol salt, solvate, or anhydrous form thereof contained in the microparticle composition or formulation he microparticle composition or formulation of claim 1, where a controlled release of the total dosage amount of the carvedilol free base or the carvedilol salt, solvate, or anhydrous form thereof contained in the microparticle composition or formulation occurs in a gastrointestinal tract system.

In yet another or eighth embodiment, a microparticle formulation for once-a-day therapy of the present invention relates to a mixture of:
[a] rapidly releasing microparticles containing carvedilol free base or a carvedilol salt, solvate, or anhydrous form thereof; and
[b] at least two types of controlled-release microparticles containing carvedilol free base or a carvedilol salt, solvate, or anhydrous form thereof;
   where the rapidly releasing microparticles contain a first dosage amount of the carvedilol free base or the carvedilol salt, solvate, or anhydrous form thereof different from each dosage amount contained in each of the at least two different types of delayed or controlled-release microparticles;
   where the rapidly releasing microparticles exhibit rapid release of the first dosage amount of the carvedilol free base or the carvedilol salt, solvate, or anhydrous form thereof and the at least two different types of controlled release microparticles exhibit a pH-dependent and time-dependent triggered serial or sequential sustained prolonged release or controlled release for each of the dosage amounts of the carvedilol free base or the carvedilol salt, solvate, or anhydrous form thereof contained in the at least two different types of controlled release microparticles.

In yet another or ninth embodiment a microparticle composition or formulation of the present invention relates to a :
[a] a rapidly releasing microparticle type containing carvedilol free base or a carvedilol salt, solvate, or anhydrous form thereof;
   where each rapidly releasing microparticle type is comprised of a rapidly releasing microparticle core unit formed from a mixture of the carvedilol free base or the carvedilol, salt, solvate, or anhydrous form thereof in combination with one or more nitrogen-containing pharmaceutical polymers and a plasticizer and optionallyother pharmaceutically acceptable excipients; and
[b] at least two different controlled-release microparticle types each containing carvedilol free base or a carvedilol salt, solvate, or anhydrous form thereof;
where each of the at least two different controlled-release microparticles is comprised of a drug-containing microparticle core unit as defined in [a] above and is further coated with a controlled release layer(s) formed from a film-forming polymer, a plasticizing agent and optionally other pharmaceutically acceptable excipients ;
where each of the rapidly releasing microparticle type contains carvedilol free or a carvedilol salt, solvate, or anhydrous dosage amount different from the carvedilol free base or the carvedilol salt, solvate, or anhydrous dosage amounts contained in each of the at least two different controlled release microparticle types;
where each of the rapidly releasing and at least two different controlled release microparticles are mixed together with materials selected from the group consisting of a surface active, a lubricating agent, an anti-agglomerating agent or other pharmaceutically acceptable excipients.

In accordance with the present invention, the ninth or other embodiments, a controlled release formulation, composition or dosage form of the present invention may include, but is not limited to containing a swelling agent as defined herein or other pharmaceutically acceptable adjuvants, carriers or excipients. In particular, such a suitable swelling agent, which may be selected from, but not limited to crosslinked polyvinylpyrrolidones (i.e., which may be selected from, but not limited to polyplasdone or crospovidone); or crosslinked carboxyalkylcelluloses (i.e., which may be selected from, but not limited to crosslinked carboxymethylcellulose or crosslinked sodium croscarmellose). In one embodiment of the present invention, a nitrogen-containing polymer in each first controlled release microparticle granule and second controlled release microparticle granule is a swelling agent as defined herein. In further embodiments of the present invention, such nitrogen containing polymers may be selected from, but not limited to polyvinyl pyrrolidone (also conventionally known as povidone or PVP), or cross-linked polyvinyl pyrrolidone (also conventionally known as cross-linked povidone). A nitrogen containing polymer in each first controlled release microparticle granule and in each second controlled release microparticle granule of the present invention, also may be, but is not limited to being a combination of polyvinyl pyrrolidone (povidone or PVP), or cross-linked polyvinyl pyrrolidone (cross-linked povidone).

In yet another or tenth embodiment, a microparticle composition or formulation of the present invention relates to:
a mixture of rapidly releasing or controlled-release microparticles containing carvedilol free base or a carvedilol salt, solvate or anhydrous form thereof;
where each rapidly releasing microparticle has a core unit formed by applying a mixture of the carvedilol free base or the carvedilol salt, solvate, or anhydrous form and materials selected from at least one nitrogen containing polymer, a plasticizer or pharmaceutically acceptable excipients onto a cellulose microparticle or similar pharmaceutically acceptable material.
where core each of the rapidly releasing or controlled-release microparticles further are layered or coated with an additional controlled release layer formed from pharmaceutically acceptable excipients.

In accordance with the present invention and ninth or other embodiments, a microparticle composition or formulation may include, but is not limited to a controlled release layer formed from a mixture of the pharmaceutically acceptable excipients selected from a film former, a plasticiser or other pharmaceutically acceptable excipients..

In yet another or eleventh embodiment, the present invention relates to acontrolled-release microparticle formulation, which comprises a microparticle ratio mixture formed from:
[1] a first rapidly releasingmicroparticle population,
[2] a first controlled release microparticle population; and
[3] a second controlled release microparticle population;

where each first rapidly releasing microparticle population is formed from a rapid releasing microparticle comprised of a combination of carvedilol free base or a carvedilol salt, solvate, or anhydrous form thereof, one or more nitrogen-containing polymers, a plasticizer and, other pharmaceutically acceptable excipients;
where each first controlled release microparticle population and each second controlled release microparticle population is comprised of a controlled-release microparticle unit comprised of an active drug loaded core comprised of a combination of carvedilol free base or a carvedilol salt, solvate, or anhydrous form thereof, one or more nitrogen-containing polymers, a plasticizer or pharmaceutically acceptable excipients;
   where the active drug loaded core of each first controlled release microcapsule and each second controlled release microcapsule is layered with a controlled release layer of film forming polymer(s), or mixtures of film forming polymers thereof, a and a plasticizer agent or pharmaceutically acceptable excipients;
where each first rapidly releasing microparticle unit, first controlled release microparticle unit and second controlled release microparticle unit each contain a different dosage amount of the carvedilol salt, solvate, or anhydrous form thereof.

In accordance with the present invention, a controlled-release microparticle formulation may have, but is not limited to a first rapidly releasing microparticle population contains at least 5% and no more than 20% of the total dosage of the carvedilol free base or the carvedilol salt, solvate or anhydrous form thereof. Further, the first rapidly releasing microparticle population may contain, but is not limited to at least 10% and no more than 15% of the total dosage of the carvedilol free base or the carvedilol salt, solvate or anhydrous form thereof. The first controlled-release microparticle population also may contain, but is not limited to at least 25% and no more than 50% of the total dosage of the carvedilol salt, solvate, or anhydrous form thereof. The controlled-release microparticle formulation also may have, but is not limited to a second controlled-release microparticle population, which contains at least 40% and no more than 60% of the total dosage of the carvedilol salt, solvate, or anhydrous form thereof.

In yet another or twelfth embodiment, the present invention relates to a controlled-release microparticle composition or formulation, which comprises:
a tri-component controlled-release microparticle composition or formulation product formed from:
   [1] a first rapidly releasing microparticle population comprised of rapidly releasing microparticlesrapidly releasing;
      where each rapidly releasing microparticle contains a layer comprised of carvedilol free base or a carvedilol, salt, solvate, or anhydrous form thereof, at least one nitrogen containing polymer and a plasticizer or other pharmaceutically acceptable excipients applied to a cellulose sphere or a cellulose core; and
   [2] a first controlled release microparticle population comprised of first controlled release microparticless;
      where each first controlled release microparticle contains a layer comprised of carvedilol free base or a carvedilol salt, solvate, or anhydrous form thereof, at least one nitrogen containing polymer and a plasticizer or other pharmaceutically acceptable excipients applied to a cellulose sphere or a cellulose core;
      where each first controlled release microparticle is coated with at least one release controlling coating layer(s) formed from a film forming polymer and a plasticizing agent or other pharmaceutically acceptable materials to form each first controlled release microparticle; and
      where each first controlled release granule triggers a release of the carvedilol free base, or the carvedilol salt, solvate, or anhydrous form thereof at a pH of about 5.5;
   [3] a second controlled release microparticle population comprised of second controlled release microparticle granules;
      where each second controlled release microparticle contains a layer comprised of carvedilol free base or a carvedilol salt, solvate, or anhydrous form thereof, at least one nitrogen containing polymer and a plasticizer and pharmaceutically acceptable excipients applied to a cellulose sphere or a cellulose core;
      where each second controlled release microparticle is coated with at least one release controlling coating layer(s) formed from a film forming polymer, or mixtures thereof and a plasticizing agent to form each second controlled release microparticle; and
      where each second controlled release granule triggers a release of the carvedilol free base or the carvedilol salt, solvate, or anhydrous form thereof at a pH of about pH > 6.4; and
      where each rapidly releasing population, first controlled release population and second controlled release population further are admixed with pharmaceutically acceptable adjuvants, carriers or excipients to form the tri-component controlled-release microparticle-containing dosage form.

In yet another or thirteenth embodiment of the present invention, a controlled-release microparticle composition or formulation, comprises:
a tri-component controlled-release microparticle composition, formulation or dosage product formed from:
   [1] a first rapidly releasing microparticle population comprised of microparticle rapidly releasing granules;
      where each rapidly releasing microcapsule population is comprised of microparticle rapidly releasing granules each of which contain a layer comprised of a carvedilol free base, salt, solvate, or anhydrous form thereof, at least one nitrogen containing polymer and a plasticizer or other pharmaceutically acceptable excipients applied to a cellulose sphere or core or other pharmaceutically acceptable core;and
   [2] a first controlled release microparticle population comprised of first controlled release microparticle granules;
      where each first controlled release population is comprised of first controlled release microparticle granules each of which contain a layer comprised of carvedilol free base, salt, solvate, or anhydrous form thereof, at least one nitrogen containing polymer and a plasticizer or other pharmaceutically acceptable excipients applied to a cellulose sphere or core or other pharmaceutically acceptable core;
      where each first controlled release microparticle is coated with at least one release controlling coating layer(s) formed from a film forming polymer and a plasticizing agent to form each first controlled release microparticle; and
      where each first controlled release granule triggers a release of the carvedilol free salt, solvate, or anhydrous form thereof at a pH of about 5.5.
   [3] a second controlled release microparticle population comprised of second controlled release microparticle granules;
      where each second controlled release population is comprised of second controlled release microparticle granules each of which contain a layer comprised of a carvedilol free base, salt, solvate, or anhydrous form thereof, at least one nitrogen containing polymer and a plasticizer or pharmaceutically acceptable excipients applied to a cellulose sphere or core or pharmaceutically acceptable core ;
      where each second controlled release microparticle is coated with at least one release controlling coating layer(s) formed from a film forming polymer and a plasticizing agent to form each second controlled release microparticle; and
      where each second controlled release granule triggers a release of the carvedilol free base, salt, solvate, or anhydrous form thereof at a pH of about pH > 6.4; and
      where each rapidly releasing population, first controlled release population and second controlled release population are admixed with pharmaceutically acceptable adjuvants, carriers or excipients to form the tri-component controlled-release microparticle composition or formulation product.

In yet another or fourteenth embodiment, the present invention relates to a controlled-release microparticle composition, formulation or dosage form, which comprises:
a tri-component controlled-release microparticle composition or formulation product formed from:
   [1] a first rapidly releasing microparticle population comprised of microparticle rapidly releasing granules;
      where each microparticle rapidly releasing granule contains a layer comprised of a carvedilol phosphate salt, solvate, or anhydrous form thereof, at least one nitrogen containing polymer and a plasticizer or other pharmaceutically acceptable excipients applied to a cellulose sphere or pharmaceutically acceptable core; and
   [2] a first controlled release microparticle population comprised of first controlled release microparticle granules;
      where each first controlled release microparticle granulecontains a layer comprised of a carvedilol phosphate salt, solvate, or anhydrous form thereof, at least one nitrogen containing polymer and a plasticizer or other pharmaceutically acceptable excipients applied to a cellulose sphere or pharmaceutically acceptable core;
      where each first controlled release microparticle is coated with at least one release controlling coating layer(s) formed from a film forming polymer and a plasticizing agent to form each first controlled release microparticle; and
      where each first controlled release granule triggers a release of the carvedilol phosphate, salt, solvate, or anhydrous form thereof at a pH of 5.5;
   [3] a second controlled release microparticle population comprised of second controlled release microparticle granules;
      where each second controlled release microparticle granule contains a layer comprised of a carvedilol phosphate salt, solvate, or anhydrous form thereof, at least one nitrogen containing polymer and a plasticizer or other pharmaceutically acceptable excipients applied to a cellulose sphere or pharmaceutically acceptable core;
      where each second controlled release microparticle is coated with at least one release controlling coating layer(s) formed from a film forming polymer and a plasticizing agent to form each second controlled release microparticle; and
      where each second controlled release granule triggers a release of the carvedilol phosphate salt, solvate, or anhydrous form thereof at a pH of pH > 6.4; and
where each rapidly releasing population, first controlled release population and second controlled release population are admixed with pharmaceutically acceptable adjuvants, carriers or excipients to form the tri-component controlled-release microparticle composition, formulation or dosage form.

In accordance with the present invention and which may be relevant, but not limited to the tenth, eleventh, twelfth, thirteenth and fourteenth embodiments, a controlled-release microparticle formulation of the present invention may be, but is not limited to microparticles having a particle size of between 50 and 1000 µm, preferably of between 100 µm and 750 µm and, even more preferably, of between 150 µm and 500 µm.

Also inaccordance with the present invention and which may be relevant, but not limited to the tenth, eleventh, twelfth, thirteenth and fourteenth embodiments, a controlled-release microparticle composition or formulation of the present invention may also include, but not be limited to:
where each microparticle rapidly releasing granule with a mean microparticle diameter size in a range from about 150 µm to 500 µm, such as 150 µm to 300 µm;
where each first controlled release microparticle granule with a mean microparticle diameter size in a range from about 200 µm to about 500 µm;
where each second controlled release microparticle granule may with a mean microparticle diameter size in a range from about 150 µm to about 400 µm, which may include mean microparticle diameter ranges from about 250 µm to about 360 µm or from about 270 µm to about 360 µm.

In accordance with the present invention and which may be relevant, but not limited to the tenth, eleventh, twelfth, thirteenth and fourteenth embodiments, a controlled-release microparticle composition or formulation may also include, but is not limited to:
where each microparticle rapidly releasing granule contains a carvedilol salt, solvate, or anhydrous form thereof contained in a dosage amount of 1.25 mg to 10 mg;
where each first controlled release microparticle granule containing a carvedilol salt, solvate, or anhydrous form thereof contained in a dosage amount of about 10 mg to about 80 mg, such as for example where, such a dosage amount may range from about 3.75 mg to about 30 mg; or
where each second controlled release microparticle granules contains a carvedilol salt, solvate, and/or anhydrous form thereof in a dosage amount range of about 5 mg to about 40 mg.

Also in accordance with the present invention and which may be relevant, but not limited to the tenth, eleventh, twelfth, thirteenth and fourteenth embodiments, a controlled-release microparticle composition or formulation of the present invention may include, but is not limited to a total dosage amount of the carvedilol free base or the carvedilol salt, solvate, or anhydrous form thereof dosage amount contained in a sum of each first rapidly releasing microparticle granule, a first controlled release microparticle granule and a second controlled release microparticle granule is the sum of the total dosage amount between about 10 mg to about 80 mg.

In accordance with the present invention and which may be relevant, but not limited to the tenth, eleventh, twelfth, thirteenth and fourteenth embodiments, a controlled-release microparticle composition or formulation of the present invention also may contain, but is not limited to at least one release controlling layer formed from at least one polymethylmethacrylate polymer(s) and a plasticizing agent, which may be, but is not limited to a ratio from about 60% (w/w) to about 40% (w/w).

In accordance with the present invention and which may be relevant, but not limited to the tenth, eleventh, twelfth, thirteenth and fourteenth embodiments, a controlled-release microparticle composition or formulation may include, but is not limited to containing a film forming polymer in at least one release controlling coating layer(s) of each first controlled release microparticle, which may be, but is not limited to a polymethylmethacrylate polymer selected from Eudragit L, Eudragit RL, Eudragit RS and other Eudragit NE polymers (i.e., such as commercially available polymers as supplied by the Rohm Pharma group), Acrycoat S100, Acrycoat L 100D and the like.

In accordance with the present invention and which may be relevant, but not limited to the tenth, eleventh, twelfth, thirteenth and fourteenth embodiments, a controlled-release microparticle composition or formulation of the present invention, may contain, but is not limited to containing a plasticizing agent in at least one release controlling coating layer(s) of each first controlled release microparticle, where such a plasticizing agent is selected, but is not limited to a hydrogenated vegetable oil, propan 2-ol or propylene glycol, diethyl phthalate or other pharmaceutically acceptable materials. Suitable plasticizers may include, but are not limited to hyrogenated vegetable oil, hydrogenated cottonseed oil, hydrogenated castor oil and the like.

In accordance with the present invention and which may be relevant, but not limited to the tenth, eleventh, twelfth, thirteenth and fourteenth embodiments, a controlled-release microparticle composition or formulation may contain, but is not limited to a different carvedilol free base salt, solvate or anhydrous form thereof dosage amounts contained in each rapidly releasing population, first controlled release population and second controlled release population are in a 1: 3: 4 active drug content ratio. A controlled-release microparticle composition or formulation of the present invention, may also exhibit, but is not limited to a where a serial or sequential release of each of the different dosage amounts of the carvedilol free base or the carvedilol salt, solvate or anhydrous form thereof contained in each immediate-release population, first controlled release population and second controlled release population.

In yet another or fifteenth embodiment, the present invention relates to a modified-release microparticle formulation, which comprises:
a microparticle mixture ratio formed from:
   a rapidly releasing microparticle population, a first delayed release microparticle population and a second delayed release microparticle population;
   wherein each microparticle population contains a different microparticle type with a different dosage amount of carvedilol free base, or a carvedilol salt, solvate, or anhydrous form thereof;
   wherein a rapid release or a controlled release of the different dosages contained in each of the microparticle populations achieves total plasma levels or total time concentrations (**A**rea **U**nder **C**urve) at C24 hours comparable to a conventional rapidly or immediately releasing carvedilol free base composition dosed twice daily at 12 hour intervals;
      wherein each of microparticle populations exhibits a different release profile where a first peak plasma concentration level and a first Tmax pulse is reached between about 1 hour to about 3 hours and a second peak plasma concentration level and a second Tmax pulse is reached between about 5 hours to about 10 hours after dosing the controlled-release microparticle formulation.

In yet another or sixteenth embodiment, the present invention relates to a modified-release microparticle formulation, which comprises:
a mixture of:
   [a] rapidly releasing microparticles containing a carvedilol free base or a carvedilol salt, solvate, or anhydrous form thereof; and
   [b] at least two types of controlled-release microparticles containing carvedilol free base or a carvedilol salt, solvate, or anhydrous form thereof;
where the rapidly releasing microparticles contain a first dosage amount of the carvedilol free base or the carvedilol salt, solvate, or anhydrous form thereof different than each of the dosage amounts contained in at least two different types of delayed or controlled-release microparticles; and
where the rapidly releasing microparticles exhibit different serial or sequential release for the first dosage amount of carvedilol free base or carvedilol salt, solvate, or anhydrous form thereof than the dosage amounts contained in the at least two different types of controlled release microparticles.

In accordance with the present invention, each different serial or sequential release of the rapidly releasing microparticles and at least two types of controlled release microparticles are defined by a mean plasma level representing a total carvedilol dosage amount as shown by a substantially biphasic profile; where the total carvedilol dosage amount is the sum of the first and second dosage amounts; where the rapidly releasing microparticles comprising release the carvedilol free base or the carvedilol salt, solvate or anhydrous form thereof to provide a peak plasma level at between 1 to 3 hours after dosing and each of the at least two types of delayed-controlled release microparticles release the carvedilol salt, solvate or anhydrous form thereof to provide a second peak plasma level between 5 to 10 hours after dosing.

Further, the serial or sequential release of the total carvedilol dosage amount from the rapidly releasing microparticles and at least two types of controlled release microparticles provide prolonged plasma levels as characterized by a substantially biphasic profile and residual drug plasma levels 24 hours after dosing comparable to a conventional carvedilol free base dosage dosed twice daily at 12 hour intervals for a total 24 hour period;
where the serial or sequential release of the rapidly releasing microparticles and at least two types of controlled release microparticles provide plasma levels of drug characterised by a substantially biphasic profile, with the rapidly releasing microparticles contributing a first peak plasma level of drug between 1 to 3 hours after dosing and the delayed-controlled release microparticles contributing a second peak plasma level of drug between 5 to 10 hours after dosing..

In yet another or seventeenth embodiment, the present invention relates to a controlled-release microparticle formulation for once-a-day administration, which comprises:
a microparticle mixture ratio formed from an rapidly releasing microparticle population, a first delayed release microparticle population; and a second delayed release microparticle population;
where each of the microparticles in each population contain carvedilol free base, salt, solvate, or anhydrous form thereof in a different dosage amount adapted to provide controlled release of the different dosages contained in each microparticle populations to achieve comparable total plasma or total time concentrations (Area Under Curve) and plasma levels at C24 hours comparable to a conventional(rapidly releasing) carvedilol composition dosed twice daily at 12 hour intervals.;
where each of the first, second, and third dosage form has a different release profile, said microparticle product reaching a first Plasma peak concentration level between 1 to 3 hours after dosing the controlled-release microparticle formulation and a second Cₘₐₓ between 5 to 10 hours after dosing the controlled-release microparticle formulation.

In accordance with the present invention, each release profile is a substantially biphasic profile shown by:first release microparticles exhibiting a release rate of the carvedilol free base or carvedilol salt, solvate, or anhydrous form providing a first plasma peak concentration level between 1 to 3 hours after dosing the microparticle composition or formulation; each of the at least two types of controlled release microparticles exhibiting a release rate of the carvedilol salt, solvate, or anhydrous form providing a second Plasma peak concentration level between 5 to 10 hours after dosing the microparticle composition or formulation exhibiting a combined higher plasma peak concentration level peak level than for the first release microparticles;
where the first peak plasma concentration level and the second Plasma peak concentration level are in a ratio of about 0.5 : 1.0;
where the peak plasma concentration levels contributed by either the first release microparticles or each of the two types of delayed controlled release microparticles are substantially comparable to peak plasma concentration level levels for an rapidly releasing carvedilol free base composition dosed twice daily at 12 hour intervals.

In accordance with the present invention, a substantially biphasic profile is shown by: an immediate-release microparticles exhibiting a release rate of the carvedilol free base, salt, solvate, or anhydrous form that provide a first peak plasma concentration between 1 to 3 hours after dosing the microparticle composition or formulation; and each of the at least two types of delayed-controlled release microparticles exhibiting a release rate of the carvedilol free base, salt, solvate, or anhydrous form that provide a second peak plasma concentration between 5 to 10 hours after dosing the microparticle composition or formulation.

In yet another or eighteenth embodiment, the present invention relates to a controlled release microparticle dosage product, which comprises:
a first immediate-release dosage form;
a second delayed release dosage form; and
a third delayed release dosage form,
where each of said first, second and third dosage forms comprise a carvedilol free base, salt, solvate or anhydrous form thereof and a pharmaceutically acceptable carrier(s), said three dosage forms having different release profiles, said microparticle dosage product reaching a first peak plasma concentration level from about one to about three hours and a second peak plasma concentration level from about five hours to about ten hours.

In accordance with the present invention, each rapidly releasing dosage form contains at least 10 % and no more than 15 % of the total dosage of carvedilol free base, salt, solvate or anhydrous form thereof; each second delayed release dosage form contains at least 30 % and no more than 50% of the total dosage of carvedilol free base, salt, solvate or anhydrous form thereof; and each third delayed release dosage form contains at least 40% and no more than 60% of the total dosage of carvedilol free base, salt, solvate or anhydrous form thereof. In addition, each first immediate-release dosage form; each second delayed release dosage form; and each third delayed release dosage form includes a total dosage of the carvedilol free base, salt, solvate or anhydrous form thereof that is effective for a twenty four hour period.

In yet another or ninteenth embodiment, the present invention relates to a microparticle composition or formulation, which comprises:
[a] a rapidly releasing microparticle population containing carvedilol free base or a carvedilol salt, solvate, or anhydrous form thereof;
   where each rapidly releasing microparticle population is comprised of a rapidly releasing microparticle core unit formed from a mixture of the carvedilol free base or the carvedilol, salt, solvate, or anhydrous form thereof in combination with one or more nitrogen-containing pharmaceutical polymers and a plasticizer or other pharmaceutically acceptable excipients;
[b] at least two different controlled-release microparticle populations each containing carvedilol free base or a carvedilol salt, solvate, or anhydrous form thereof;

where each of the at least two different controlled-release microparticle populations is comprised of a microparticle core unit formed from a mixture of the carvedilol free base or the carvedilol, salt, solvate, or anhydrous form thereof in combination with one or more nitrogen-containing pharmaceutical polymers and a plasticizer or other pharmaceutically acceptable excipients; and
where the microparticle core unit is further coated with a controlled release layer(s) formed from a film-forming polymer, a plasticizing agent and optionally other pharmaceutically acceptable excipients that controls aqueous drug medium release and exhibits a pH-dependent and time-dependent triggered serial or sequential release for each of the dosage amounts of carvedilol free base or carvedilol salt, solvate, or anhydrous form thereof contained in the at least two different types of controlled release microparticles;
where each of the rapidly releasing and at least two different controlled release microparticle populations are mixed together with additional materials selected from a surface active, a lubricating agent, an anti-agglomerating agent or other pharmaceutical acceptable excipients;
where each of the rapidly releasing microparticle core units contain a different dosage amount of carvedilol free base or a carvedilol salt, solvate, or anhydrous form thereof than the dosage amounts of carvedilol free base or carvedilol salt, solvate, or anhydrous dosage amounts contained in each of the at least two different controlled release microparticle units;
where each of the controlled release microparticle populations has a different release profile with a first peak plasma concentration level between about 1 to about 3 hours and a second peak plasma concentration level between about 5 to about 10 hours after dosing the controlled-release microparticle formulation to achieve total plasma levels or total time concentrations (Area Under Curve) at C24 hours comparable to a conventional rapidly or immediately releasing carvedilol free base composition dosed twice daily at 12 hour intervals.

### Methods of Treatment and Combination Theralaies

The compounds, pharmaceutical compositions, controlled release formulations or dosage forms prepared according to the present invention can be used to treat warm-blooded animals, such as mammals, which include humans.

Disclosed are methods of treating cardiovascular diseases, which may include, but is not limited to hypertension, congestive heart failure, atherosclerosis, or angina, which comprises administering to a subject in need thereof an effective amount of carvedilol free base or a carvedilol salt, anhydrous forms, or solvate thereof as defined herein, a pharmaceutical composition, or controlled release formulation as described herein.

For example, disclosed is a method of treating hypertension, congestive heart failure, atherosclerosis and angina, which comprises administering to a subject in need thereof an effective amount of a carvedilol phosphate salt (which may include, but are not limited to novel crystalline or other solid forms), anhydrous forms, or solvates thereof, a pharmaceutical composition or controlled release formulation (i.e., which contains such salts or solvates of carvedilol phosphate), etc.

Disclosed is a method of treating hypertension, which comprises administering to a subject in need thereof an effective amount of a carvedilol phosphate salt (which may include, but are not limited to novel crystalline or other solid forms), anhydrous forms, or solvates thereof, a pharmaceutical composition or controlled release formulation (i.e., which contains such salts or solvates of carvedilol phosphate), etc.

Disclosed is a method of treating atherosclerosis, which comprises administering to a subject in need thereof an effective amount of a carvedilol phosphate salt (which may include, but are not limited to novel crystalline or other solid forms), anhydrous forms, or solvates thereof, a pharmaceutical composition or controlled release formulation (i.e., which contains such salts or solvates of carvedilol phosphate), etc.

Disclosed is a method of delivering carvedilol to gastrointestinal tract of a subject in need thereof, which comprises administering an effective amount of a carvedilol salt, anhydrous forms, or solvate thereof, which may be in, but not limited to being in combination with carvedilol free base, corresponding pharmaceutical compositions or control-release formulations or dosage forms as described herein.

Specificly disclosed is a method of delivering carvedilol to the gastrointestinal tract, which comprises administering an effective amount of a carvedilol salt, anhydrous forms, or solvate thereof, which may be in, but not limited to being in combination with carvedilol free base, corresponding pharmaceutical compositions or control-release formulations or dosage forms as described herein.

Disclosed is a method of orally dosing a modified release composition, dosage form or formulation as described herein, which comprises progressive release of a drug amount of
carvedilol free base or a carvedilol salt, solvate or anhydrous form thereof from each microcapsule of the modified release composition, dosage form or formulation, which are absorped as the microparticles transit the GI tract to provide sustained and controlled release levels of the drug amount for maintenance of prolonged plasma levels.

The present invention also relates to a method of dosing a carvedilol dosage unit, which may include, but is not limited to a carvedilol free base or a carvedilol salt, solvate or anhydrous form thereof, to a patient in need thereof, which comprises administering to a subject in need thereof an effective amount of a controlled release composition, dosage form or formulation of the present invention, where release of the carvedilol dosage unit transits through a lower gastrointestinal tract.

In accordance with any of the methods of administration of the present invention, the term a "therapeutically effective amount", as used herein, generally includes within its meaning a non-toxic but sufficient amount of the particular drug to which it is referring to provide the desired therapeutic effect. The exact amount required will vary from subject to subject depending on factors such as the patient's general health, the patient's age, etc.

Also, the present invention relates to combination therapy methods for treatment of cardiovascular disorders to a subject in need thereof, which comprises a compound or controlled release composition, dosage form or formulation as described herein in a synergistic combination with other drug agents, which may, but not limited to a group selected from the group consisting of calcium channel blockers, beta blockers, diuretics, ACE inhibitors, Angiotensin II receptor antagonists, statin agents and or the like, or pharmaceutically acceptable adjuvant(s), carrier(s), diluent(s), or excipient(s).

In particular, compounds or controlled release composition, dosage forms or formulations of the present invention may be employed alone or in combination with each other or other suitable therapeutic agents useful in treatment of the aforementioned cardiovascular disorders, which may include, but are not limited to hypertension, congestive heart failure, atherosclerosis, angina and the like.

Examples of suitable calcium channel blocker agents (both L-type and T-type) for use in combination with compounds or a controlled release composition, dosage form or formulation of the present invention, may include, but are not limited to diltiazem, verapamil, nifedipine, amlodipine, mybefradil or any other calcium channel blocker and the like.

Suitable beta-blockers for use in combination with compounds or a controlled release composition, dosage form or formulation of the present invention, may include, but are not limited to atenolol, metoprolol, and the like .

Suitable statin agents, such as HMG-CoA reductase inhibitors, for use in combination with compounds or a controlled release composition, dosage form or formulation of the present invention, may include, but are not limited to lovastatin, simvastatin, pravastatin, fluvastatin, cerivastatin, atorvastatin or any other suitable statin agent and the like.

Suitable adrenoreceptor agents for use in combination with compounds or a controlled release composition, dosage form or formulation of the present invention, may include, but are not limited to may include metoprolol (toprol-XL), metoprol succinate, metoprol tartrate or any other suitable adrenoreceptor agents and the like,

Suitable ACE inhibitors for use in combination with compounds or a controlled release composition, dosage form or formulation of the present invention, may include, but are not limited to alacepril, benazepril, captopril, ceronapril, cilazepril, cilazopril, delapril, enalapril, enalaprilat, fosinopril, imidapril, libenzapril, lisinopril, moexipril, monopril, moveltipril, pentopril, perindopril, quinapril, ramipril, spirapril, temocapril, teprotide, trandolapril, zofenopril or any other suitable ACE inhibitor and the like.

Suitable diuretics for use in combination with compounds or a controlled release formulation of the present invention, may include, but are not limited to acetazolamide, flumethiazide, hydroflumethiazide, bendroflumethiazide, brinzolamide, dichlorphenamide, dorzolamide, methazolamide, azosemide, bumetanide, ethacrynic acid, etozolin, frusemide, piretanide, torasemide, isosorbide, mannitol, amiloride, canrenoate potassium, canrenone, spironolactone, triamterene, althiazide, bemetizide, bendrofluazide, benzthiazide, buthiazide, chlorothiazide, chlorthalidone, clopamide, cyclopenthiazide, cyclothiazide, epithiazide, hydrochlorothiazide, hydroflumethiazide, indapamide, mebutizide, mefruside, methylclothiazide, meticrane, metolazone, polythiazide, quinethazone, teclothiazide, trichlormethiazide, tripamide, xipamide, furosemide, musolimine, triamtrenene, amiloride, and spironolactone or other suitable diuretics and the like.

Suitable angiotensin II receptor antagonists for use in combination with compounds or a controlled release formulation of the present invention, may include, but are not limited to losartan, irbesartan, valsartan or any other angiotensin II receptor antagonist and the like.

Active drug or therapeutic agents or compounds, such as those described above may be prepared according to processes or methods taught by either the present disclosure or processes or methods known to those of skill in the art.

Active drug or therapeutic agents, when employed in combination with the compounds, controlled release compositions, dosage forms or formulations of the present invention, may be used or administered, for example, in dosage amounts indicated in the Physicians' Desk Reference (PDR) or as otherwise determined by one of ordinary skill in the art.

In the context of this specification, the term "simultaneously" when referring to simultaneous administration of the relevant drugs means at exactly the same time, as would be the case, for example in embodiments where the drugs are combined in a single preparation. In other embodiments, "simultaneously" can mean one drug taken a short duration after another, wherein "a short duration" means a duration which allows the drugs to have their intended synergistic effect.

In light of the foregoing, the present invention also relates to a combination therapy, which may be a comprised of a simultaneous or coadministration, or serial administration of a combination of compounds, controlled release compositions, dosage forms or formulations of the present invention with other active drug or therapeutic agents, such as described above, and where such administration also is determined by one of ordinary skill in the art. As previously indicated active drug compounds, controlled release compositions, dosage forms or formulations of the present invention, may include, but are not limited to a carvedilol free base or a carvedilol salt, solvate or anhydrous form thereof.

In addition, also disclosed is a combination therapy for the treatment or prevention of cardiovascular diseases as described herein, which is comprised of a composition, dosage form or formulation formed from a synergistic combination or mixture of compounds, controlled release compositions, dosage forms or formulations of the present invention and another active drug or therapeutic agent or agents as those described above and optionally which comprises pharmaceutically acceptable carrier, diluent or adjuvent. In such an aforementioned combination composition, dosage form or formulation of the present invention, each of the active drug components are contained in therapeutically effective and synergistic dosage amounts.

Disclosed is a combination therapy for the treatment of cardiovascular diseases, such as diseases described herein, which comprises administering a synergistic combination of:
[1] a therapeutically effective amount of a carvedilol free base or a carvedilol salt, solvate or anhydrous form thereof; or
   a corresponding controlled release composition, dosage form or formulation thereof which comprises a therapeutically effective amount of a carvedilol free base or a carvedilol salt, solvate or anhydrous form thereof; and
[2] a therapeutically effective amount of another active drug or therapeutic agent selected from the group consisting of at least one calcium channel blockers, beta blockers, diuretics, ACE inhibitors, Angiotensin II receptor antagonists, statin agents and or the like, any other drugs suitable for the treatment of cardiovascular diseases, or combinations thereof; and further comprises pharmaceutically acceptable carriers, diluents or adjuvants.

Conventional administration methods as described in examples and throughout this application above may be suitable for such use in methods of treatment or delivery methods of various forms of the present invention, including any combination therapy methods.

The Examples set forth below are illustrative of the present invention and are not intended to limit, in any way, the scope of the present invention.

### Examples

### Carvedilol Salt, Solvate, or Anhydrous forms Examples

### Carvedilol Phosphate Examples

### Example 1

### Form I Carvedilol Dihydrogen Phosphate Hemihydrate Preparation

A suitable reactor is charged with acetone. The acetone solution is sequentially charged with carvedilol and water. Upon addition of the water, the slurry dissolves quickly. To the solution is added aqueous H₃PO₄. The reaction mixture is stirred at room temperature and carvedilol dihydrogen phosphate seeds are added in one portion. The solid precipitate formed is stirred, then filtered and the collected cake is washed with aqueous acetone. The cake is dried under vacuum to a constant weight. The cake is weighed and stored in a polyethylene container.

### Example 2

### Form II Carvedilol Dihydrogen Phosphate Dihydrate Preparation

Form I is slurried in acetone/water mixture between 10 and 30°C for several days.

### Example 3

### Form III Carvedilol Dihydrogen phosphate Methanol Solvate Preparation

Form I is slurried in methanol between 10 and 30°C for several days.

### Example 4

### Form IV - Carvedilol Dihydrogen Phosphate Dihydrate Preparation

Carvedilol dihydrogen dihydrogen phosphate is dissolved in an acetone/water mixture. The acetone is removed by distillation. A solid crystallizes during acetone removal and is filtered and dried.

### Example 5

### Form V - Carvedilol Dihydrogen Phosphate Preparation

Carvedilol dihydrogen phosphate hemihydrate (Form I) was suspended in water, and the suspension was placed on a mechanical shaker at room temperature. After 48 hours of shaking, the solid was isolated from suspension by filtration, then dried in a desiccator under vacuum for a few days.

### Example 6

### Form VI - Carvedilol Hydrogen Phosphate Preparation

A suitable reactor is charged with acetone. The acetone solution is sequentially charged with SK&F 105517 and water. Upon addition of the water, the slurry dissolves quickly. To the solution is added aqueous H₃PO₄ (at 1/2 the molar quantity of Carvedilol). The reaction mixture is stirred and allowed to crystallize. The solid precipitate formed is stirred and cooled, then filtered and the collected cake is washed with aqueous acetone.

### Example 7

### ¹³C and ³¹P Solid State NMR Data Analysis of Carvedilol Dihydrogen Phosphate

A sample of carvedilol dihydrogen phosphate was analyzed by solid-state ¹³C NMR and ³¹P NMR (i.e., to probe solid compound form structure).

Carvedilol dihydrogen phosphate (Parent MW = 406.5; Salt MW = 504.5) has the following structure and numbering scheme:

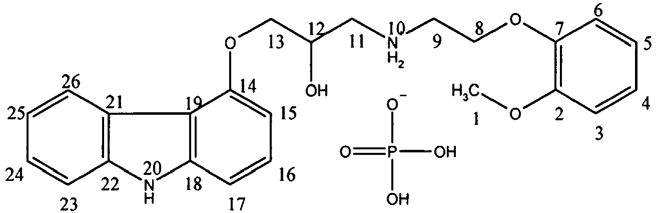

### Experimental Details and ¹³C and ³¹P Analysis

The solid state ¹³C NMR methods used to analyze compounds of the present invention produce a *qualitative* picture of the types of carbon sites within the solid material. Because of variable polarization transfer rates and the need for sideband suppression, the peak intensities are not quantitative (much like the case in solution-state ¹³C NMR).

However, the ³¹P spectra are inherently quantitative.

For the ¹³C analysis, approximately 100 mg of sample was packed into a 7-mm O.D. magic-angle spinning rotor and spun at 5 kHz. The ¹³C spectrum of the sample was recorded using a CP-TOSS pulse sequence (*c*ross-*p*olarization with *t*otal *s*uppression of *s*idebands). An edited spectrum containing only quaternary and methyl carbons was then obtained using an CP-TOSS sequence with NQS (non-quaternary suppression). The ¹³C spectra are referenced externally to tetramethylsilane via a sample of solid hexamethylbenzene.

For ³¹P Solid State NMR, approximately 40 mg of sample was packed into a 4-mm O.D. rotor and spun at 10 kHz. Both CP-MAS and single-pulse MAS ³¹P pulse sequences were used with ¹H decoupling. The ³¹P data are externally referenced to 85% phosphoric acid by a secondary solid-state reference (triphenylphosphine oxide). The Bruker AMX2-360 spectrometer used for this work operates at ¹³C, ³¹P and ¹H frequencies of 90.556, 145.782 and 360.097 MHz, respectively. All spectra were obtained at 298 K.

### Results and Discussion

The highly sensitive ¹³C and ³¹P Solid State NMR identification methods were used for the analysis and characterization of a polymorphic form of Carvedilol phosphate, which confirms its chemical structure in the solid-state.

The form of Carvedilol dihydrogen phosphate is defined by these spectra, where both ¹³C and ³¹P spectra show clear and distinct differences.

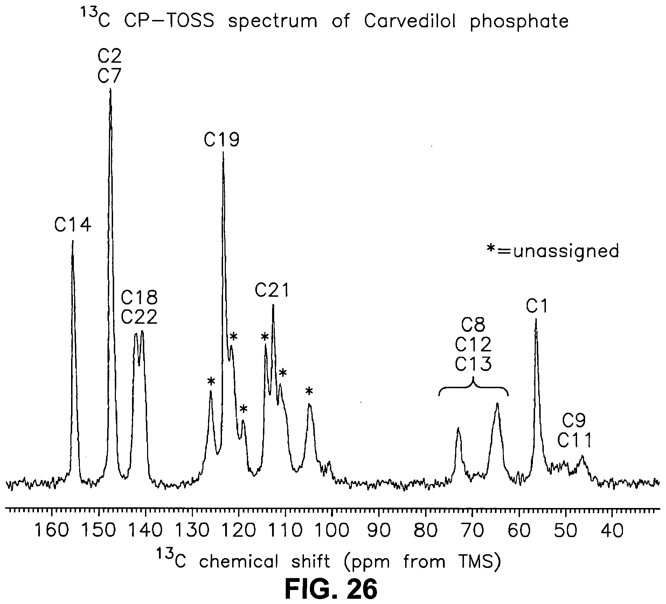

In particular, Figure 26 shows the ¹³C CP-TOSS spectrum of carevedilol dihydrogen phosphate. An assignment of the numerous ¹³C resonances in Figure 1 can be made by chemical shift assignment, the NQS spectrum and comparisons with solution-state ¹³C assignments. At least two non-equivalent molecules per unit cell are observed in this form of Carvedilol phosphate.

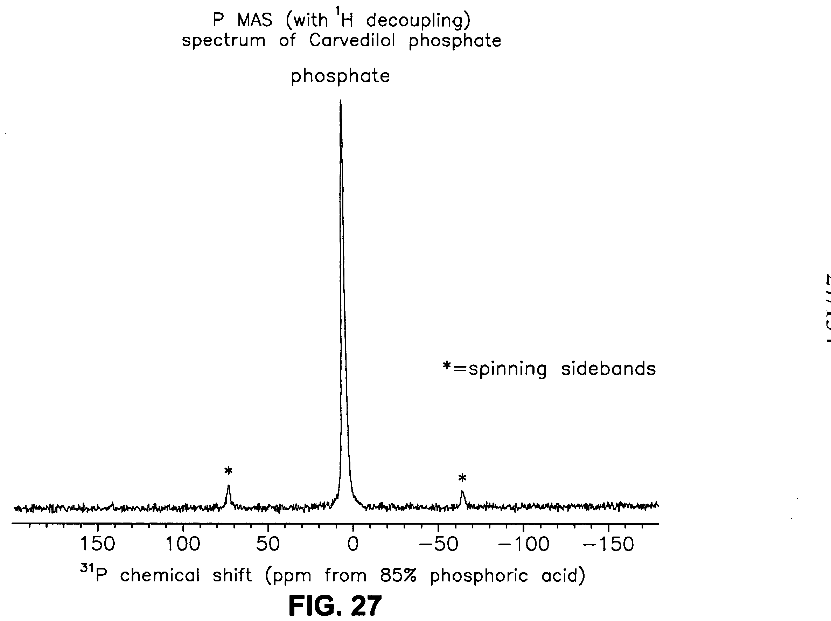

Figure 27 shows the ³¹P MAS spectrum of carvedilol dihydrogen phosphate. A single phosphorus signal is observed at 4.7 ppm, which is characteristic of phosphate salts.

### Carvedilol Hydrogen Bromide Examples

### Reference Example 8

### Form 1. Carvedilol HBr Monohydrate.

A suitable reactor is charged with acetone. The acetone solution is sequentially charged with carvedilol, water and 48% aqueous HBr. On addition of the water, the acetone slurry becomes a solution. The reaction mixture is stirred at room temperature. A solid precipitates during the course of the stir. The precipitate is filtered and the collected cake is washed with acetone. The cake is dried under vacuum to a constant weight. The cake is weighed and stored in a polyethylene container.

The single crystal x-ray data for carvedilol hydrobromide monohydrate is provided below.

**Table 1. Sample and Crystal Data for Carvedilol Hydrobromide Monohydrate.**

| | | |
|---|---|---|
| Crystallization solvents | Acetone, water | |
| Crystallization method | Slow cooling | |
| Empirical formula | C₂₄H₂₉BrN₂O₅ | |
| Formula weight | 505.40 | |
| Temperature | 150(2) K | |
| Wavelength | 0.71073 Å | |
| Crystal size | 0.18 x 0.14 x 0.08 mm | |
| Crystal habit | Clear colorless prism | |
| Crystal system | Monoclinic | |
| Space group | C2/c | |
| Unit cell dimensions | a = 18.0356(3) Å | α= 90° |
| | b = 20.8385(5) Å | β= 103.5680(10)° |
| | c = 12.9342(3) Å | γ= 90° |
| Volume | 4725.46(18) Å³ | |
| Z | 8 | |
| Density (calculated) | 1.421 Mg/m³ | |
| Absorption coefficient | 1.777 mm⁻¹ | |
| F(000) | 2096 | |

**Table 2. Data collection and structure refinement for Carvedilol Hydrobromide Monohydrate.**

| | |
|---|---|
| Diffractometer | KappaCCD |
| Radiation source | Fine-focus sealed tube, MoK_{α} |
| Data collection method | CCD; rotation images; thick slices |
| Theta range for data collection | 3.42 to 23.27° |
| Index ranges | 0 ≤ *h* ≤ 20, 0 ≤ *k* ≤ 23, -14 ≤ *l* ≤ 13 |
| Reflections collected | 30823 |
| Independent reflections | 3404 [R(int) = 0.042] |
| Coverage of independent reflections | 99.7 % |
| Variation in check reflections | N/A |
| Absorption correction | Symmetry-related measurements |
| Max. and min. transmission | 0.8709 and 0.7404 |
| Structure solution technique | Direct methods |
| Structure solution program | SHELXTL V5.10 UNIX (Bruker, 1997) |
| Refinement technique | Full-matrix least-squares on F² |
| Refinement program | SHELXTL V5.10 UNIX (Bruker, 1997) |
| Function minimized | ∑w(Fₒ²-F_{c}²)² |
| Data / restraints / parameters | 3404/11/336 |
| Goodness-of-fit on F² | 1.020 |
| Δ/σₘₐₓ | 0.000 |
| Final R indices | |
| 3071 data; I>2σ(I) | R1 = 0.0353, wR2 = 0.0797 |
| all data | R1 = 0.0405, wR2 = 0.0829 |
| Weighting scheme | w = 1/[σ²(Fₒ²)+[(0.0304P)²+14.1564P] |
| | where P=[MAX(Fₒ² ,0)+2F_{c}²]/3 |
| Largest diff. peak and hole | 0.786 and -0.914 e.Å⁻³ |
| Refinement summary: | |
| Ordered Non-H atoms, XYZ | Freely refined |
| Ordered Non-H atoms, U | Anisotropic |
| H atoms (on carbon), XYZ | Idealized positions riding on attached atom |
| H atoms (on carbon), U | Appropriate constant times Ueq of attached atom |
| H atoms (on heteroatoms), XYZ | Freely refined |
| H atoms (on heteroatoms), U | Refined Isotropically |
| Disordered atoms, OCC | See Table 10 |
| Disordered atoms, XYZ | Refined with distance restraints |
| Disordered atoms, U | Anisotropic |

**Table 3. Atomic Coordinates and Equivalent Isotropic Atomic Displacement Parameters (A²) for Carvedilol Hydrobromide Monohydrate.**

| U(eq) is defined as one third of the trace of the orthogonalized Uᵢⱼ tensor. | | | | |
|---|---|---|---|---|
| | x/a | y/b | z/c | U(eq) |
| Br1 | 0.5000 | 0.22079(2) | -0.2500 | 0.04329(15) |
| Br2 | 0.0000 | 0.40821(2) | -0.2500 | 0.04510(16) |
| O1 | 0.19543(10) | 0.37037(10) | -0.00168(15) | 0.0328(5) |
| O2 | 0.08660(19) | 0.48508(15) | 0.1085(2) | 0.0312(7) |
| O2' | 0.0825(3) | 0.4816(3) | -0.0328(4) | 0.0311(13) |
| O3 | -0.19428(10) | 0.39492(10) | -0.01310(15) | 0.0347(5) |
| O4 | -0.24723(12) | 0.46974(11) | 0.11008(16) | 0.0404(5) |
| O99A | -0.0880(5) | 0.4236(3) | 0.1967(7) | 0.0430(19) |
| O99B | -0.0833(5) | 0.4514(4) | 0.1784(7) | 0.0431(19) |
| N1 | 0.34092(16) | 0.25072(13) | -0.1793(2) | 0.0390(7) |
| N2 | -0.03151(14) | 0.39706(13) | -0.0026(2) | 0.0314(6) |
| C1 | 0.26859(15) | 0.35551(14) | -0.0070(2) | 0.0301(7) |
| C2 | 0.33380(16) | 0.38188(15) | 0.0568(2) | 0.0339(7) |
| C3 | 0.40553(17) | 0.36537(16) | 0.0409(3) | 0.0402(8) |
| C4 | 0.41433(17) | 0.32249(16) | -0.0364(3) | 0.0401(8) |
| C5 | 0.34850(16) | 0.29538(15) | -0.0986(2) | 0.0343(7) |
| C6 | 0.26499(17) | 0.23737(14) | -0.2202(2) | 0.0343(7) |
| C7 | 0.23145(19) | 0.19604(15) | -0.3022(2) | 0.0401(8) |
| C8 | 0.15313(19) | 0.19096(15) | -0.3275(2) | 0.0412(8) |
| C9 | 0.10866(18) | 0.22594(14) | -0.2721(2) | 0.0364(7) |
| C10 | 0.14185(17) | 0.26731(14) | -0.1910(2) | 0.0323(7) |
| C11 | 0.22085(16) | 0.27356(13) | -0.1639(2) | 0.0300(7) |
| C12 | 0.27490(16) | 0.31103(13) | -0.0855(2) | 0.0294(6) |
| C13 | 0.18523(16) | 0.41746(14) | 0.0740(2) | 0.0301(7) |
| C14 | 0.10181(16) | 0.43671(13) | 0.0452(2) | 0.0305(7) |
| C15 | 0.05016(15) | 0.37919(14) | 0.0363(2) | 0.0289(6) |
| C16 | -0.08143(16) | 0.33991(14) | -0.0272(2) | 0.0361(7) |
| C17 | -0.16200(16) | 0.35626(16) | -0.0833(2) | 0.0380(7) |
| C18 | -0.27156(15) | 0.40680(14) | -0.0445(2) | 0.0300(6) |
| C19 | -0.30049(16) | 0.44705(14) | 0.0236(2) | 0.0316(7) |
| C20 | -0.37754(18) | 0.46060(16) | 0.0007(3) | 0.0409(8) |
| C21 | -0.42545(18) | 0.43467(17) | -0.0895(3) | 0.0499(9) |
| C22 | -0.39733(18) | 0.39593(17) | -0.1567(3) | 0.0504(9) |
| C23 | -0.31949(17) | 0.38199(15) | -0.1342(3) | 0.0388(7) |
| C24 | -0.2743(2) | 0.50999(17) | 0.1833(3) | 0.0482(9) |

**Table 4. Selected Bond Lengths (A) for Carvedilol Hydrobromide Monohydrate.**

| | | | |
|---|---|---|---|
| O1-C1 | 1.373(3) | O1-C13 | 1.428(3) |
| O2-C14 | 1.366(4) | O2'-C14 | 1.360(6) |
| O3-C18 | 1.380(3) | O3-C17 | 1.435(3) |
| O4-C19 | 1.376(4) | O4-C24 | 1.433(4) |
| N1-C6 | 1.376(4) | N1-C5 | 1.381(4) |
| N2-C16 | 1.482(4) | N2-C15 | 1.488(4) |
| C1-C2 | 1.382(4) | C1-C12 | 1.399(4) |
| C2-C3 | 1.399(4) | C3-C4 | 1.378(5) |
| C4-C5 | 1.388(4) | C5-C12 | 1.415(4) |
| C6-C7 | 1.389(4) | C6-C11 | 1.416(4) |
| C7-C8 | 1.377(5) | C8-C9 | 1.399(4) |
| C9-C10 | 1.381(4) | C10-C11 | 1.391(4) |
| C11-C12 | 1.458(4) | C13-C14 | 1.517(4) |
| C14-C15 | 1.506(4) | C16-C17 | 1.503(4) |
| C18-C23 | 1.374(4) | C18-C19 | 1.403(4) |
| C19-C20 | 1.380(4) | C20-C21 | 1.388(5) |
| C21-C22 | 1.368(5) | C22-C23 | 1.396(4) |

**Table 5. Selected bond angles (°) for Carvedilol Hydrobromide Monohydrate.**

| | | | |
|---|---|---|---|
| C1-O1-C13 | 118.0(2) | C18-O3-C17 | 116.5(2) |
| C19-O4-C24 | 117.2(2) | C6-N1-C5 | 109.9(3) |
| C16-N2-C15 | 112.0(2) | O1-C1-C2 | 125.0(3) |
| O1-C1-C12 | 115.4(2) | C2-C1-C12 | 119.6(3) |
| C1-C2-C3 | 120.1(3) | C4-C3-C2 | 122.3(3) |
| C3-C4-C5 | 117.1(3) | N1-C5-C4 | 129.2(3) |
| N1-C5-C 12 | 108.5(3) | C4-C5-C12 | 122.4(3) |
| N1-C6-C7 | 129.4(3) | N1-C6-C11 | 108.9(3) |
| C7-C6-C11 | 121.7(3) | C8-C7-C6 | 117.9(3) |
| C7-C8-C9 | 121.1(3) | C10-C9-C8 | 121.0(3) |
| C9-C10-C11 | 119.1(3) | C10-C11-C6 | 119.1(3) |
| C10-C11-C12 | 134.7(3) | C6-C11-C12 | 106.2(3) |
| C1-C12-C5 | 118.6(3) | C1-C12-C11 | 134.8(3) |
| C5-C12-C11 | 106.6(3) | O1-C13-C14 | 107.0(2) |
| O2'-C14-O2 | 83.4(3) | O2'-C14-C15 | 116.4(3) |
| O2-C14-C15 | 115.2(3) | O2'-C14-C13 | 115.6(3) |
| O2-C14-C13 | 112.0(3) | C15-C14-C13 | 111.6(2) |
| N2-C15-C14 | 111.8(2) | N2-C16-C17 | 113.0(3) |
| O3-C17-C16 | 108.1(2) | C23-C18-O3 | 125.0(3) |
| C23-C18-C19 | 120.1(3) | O3-C18-C19 | 114.9(2) |
| O4-C19-C20 | 125.4(3) | O4-C19-C18 | 115.1(2) |
| C20-C19-C18 | 119.4(3) | C19-C20-C21 | 119.8(3) |
| C22-C21-C20 | 120.9(3) | C21-C22-C23 | 119.7(3) |
| C18-C23-C22 | 120.0(3) | | |

**Table 6. Hydrogen Bonds and Short C-H···X Contacts for Carvedilol Hydrobromide Monohydrate (A and °).**

| D-H···A | d(D-H) | d(H···A) | d(D···A) | <(DHA) |
|---|---|---|---|---|
| N1-H1N··· Br10.76(3) | 2.53(4) | | 3.269(3) | 166(3) |
| N2-H2NA···O99A | 0.83(4) | 2.29(4) | 3.037(10) | 149(3) |
| N2-H2NA···O99B | 0.83(4) | 2.13(4) | 2.943(10) | 165(4) |
| N2-H2NB···O2#1 | 0.89(5) | 2.17(4) | 2.873(4) | 135(4) |
| O2'-H2O'··· Br2 | 0.67(5) | 2.65(7) | 3.237(6) | 149(12) |
| O99A-H99A···Br1#2 | 0.94(3) | 2.49(4) | 3.395(8) | 163(6) |
| O99B-H99B···Br2#1 | 0.94(3) | 2.38(3) | 3.320(8) | 173(6) |
| | | | | |
| C15-H15A···O1 | 0.99 | 2.38 | 2.783(3) | 103.2 |
| C15-H15B···Br1#2 | 0.99 | 2.85 | 3.738(3) | 149.3 |
| C16-H16A···Br1#2 | 0.99 | 2.88 | 3.760(3) | 148.2 |

| | | | | |
|---|---|---|---|---|
| Symmetry transformations used to generate equivalent atoms: #1 -x,-y+1,-z #2 -x+1/2,-y+1/2,-z | | | | |

**Table 7. Selected torsion angles (°) for Carvedilol Hydrobromide Monohydrate.**

| | | | |
|---|---|---|---|
| C13-O1-C1-C2 | 1.2(4) | C13-O1-C1-C12 | -177.5(2) |
| O1-C1-C2-C3 | -177.0(3) | C12-C1-C2-C3 | 1.7(4) |
| C1-C2-C3-C4 | -0.8(5) | C2-C3-C4-C5 | -0.5(5) |
| C6-N1-C5-C4 | -179.7(3) | C6-N1-C5-C12 | 0.8(3) |
| C3-C4-C5-N1 | -178.6(3) | C3-C4-C5-C12 | 0.8(4) |
| C5-N1-C6-C7 | 179.4(3) | C5-N1-C6-C11 | -0.9(3) |
| N1-C6-C7-C8 | 179.5(3) | C11-C6-C7-C8 | -0.1(4) |
| C6-C7-C8-C9 | -0.4(5) | C7-C8-C9-C10 | 0.8(5) |
| C8-C9-C10-C11 | -0.6(4) | C9-C10-C11-C6 | 0.0(4) |
| C9-C10-C11-C12 | -179.9(3) | N1-C6-C11-C10 | -179.4(3) |
| C7-C6-C11-C10 | 0.3(4) | N1-C6-C11-C12 | 0.6(3) |
| C7-C6-C11-C12 | -179.7(3) | O1-C1-C12-C5 | 177.4(2) |
| C2-C1-C12-C5 | -1.4(4) | O1-C1-C12-C1 | -2.4(5) |
| C2-C1-C12-C11 | 178.8(3) | N1-C5-C12-C1 | 179.6(2) |
| C4-C5-C12-C1 | 0.1(4) | N1-C5-C12-C11 | -0.5(3) |
| C4-C5-C12-C11 | 180.0(3) | C10-C11-C12-C1 | -0.3(6) |
| C6-C11-C12-C1 | 179.8(3) | C10-C11-C12-C5 | 179.9(3) |
| C6-C11-C12-C5 | -0.1(3) | C1-O1-C13-C14 | 166.1(2) |
| O1-C13-C14-O2' | -82.6(4) | O1-C13-C14-O2 | -175.8(2) |
| O1-C13-C14-C15 | 53.4(3) | C16-N2-C15-C14 | 171.3(2) |
| O2'-C14-C15-N2 | -38.6(4) | O2-C14-C15-N2 | 56.6(3) |
| C13-C14-C15-N2 | -174.2(2) | C15-N2-C16-C17 | -170.5(2) |
| C18-O3-C17-C16 | -170.7(2) | N2-C16-C17-O3 | -63.3(3) |
| C17-O3-C18-C23 | 3.3(4) | C17-O3-C18-C19 | -177.3(3) |
| C24-O4-C19-C20 | 1.0(4) | C24-O4-C19-C18 | -178.7(3) |
| C23-C18-C19-O4 | -179.2(3) | O3-C18-C19-O4 | 1.4(4) |
| C23-C18-C19-C20 | 1.0(4) | O3-C18-C19-C20 | -178.3(3) |
| O4-C19-C20-C21 | 179.9(3) | C18-C19-C20-C21 | -0.4(5) |
| C19-C20-C21-C22 | -0.3(5) | C20-C21-C22-C23 | 0.3(6) |
| O3-C18-C23-C22 | 178.2(3) | C19-C18-C23-C22 | -1.1(5) |
| C21-C22-C23-C18 | 0.4(5) | | |

**Table 8. Anisotropic Atomic Displacement Parameters (Å²) for Carvedilol Hydrobromide Monohydrate.**

| The anisotropic atomic displacement factor exponent takes the form: | | | | | | |
|---|---|---|---|---|---|---|
| -2π²[h²a*²U₁₁ + ... + 2hka* b* U₁₂] | | | | | | |
| | U₁₁ | U₂₂ | U₃₃ | U₂₃ | U₁₃ | U₁₂ |
| Br1 | 0.0484(3) | 0.0447(3) | 0.0464(3) | 0.000 | 0.0306(2) | 0.000 |
| Br2 | 0.0707(3) | 0.0413(3) | 0.0234(2) | 0.000 | 0.0111(2) | 0.000 |
| O1 | 0.0272(11) | 0.0408(12) | 0.0323(11) | 0.0067(9) | 0.0108(9) | -0.0009(9) |
| O2 | 0.0416(18) | 0.0306(18) | 0.0215(17) | -0.0006(14) | 0.0077(15) | 0.0059(14) |
| O2' | 0.038(3) | 0.028(3) | 0.031(3) | 0.001(3) | 0.014(3) | 0.000(3) |
| O3 | 0.0254(11) | 0.0473(13) | 0.0308(11) | -0.0091(9) | 0.0058(9) | -0.0001(9) |
| O4 | 0.0400(12) | 0.0500(14) | 0.0323(11) | -0.0076(10) | 0.0108(10) | 0.0019(10) |
| O99A | 0.042(3) | 0.044(5) | 0.040(4) | -0.004(4) | 0.004(3) | 0.002(4) |
| O99B | 0.033(3) | 0.061(6) | 0.035(4) | -0.004(4) | 0.007(2) | -0.010(4) |
| N1 | 0.0384(17) | 0.0449(17) | 0.0393(16) | 0.0053(13) | 0.0203(14) | 0.0112(13) |
| N2 | 0.0270(13) | 0.0341(15) | 0.0332(15) | 0.0015(13) | 0.0075(12) | 0.0033(11) |
| C1 | 0.0283(16) | 0.0324(16) | 0.0321(16) | 0.0078(13) | 0.0124(13) | 0.0005(12) |
| C2 | 0.0321(17) | 0.0381(17) | 0.0327(16) | 0.0056(13) | 0.0100(13) | -0.0014(13) |
| C3 | 0.0301(17) | 0.048(2) | 0.0412(18) | 0.0104(16) | 0.0051(14) | -0.0044(14) |
| C4 | 0.0290(17) | 0.0471(19) | 0.0470(19) | 0.0133(16) | 0.0148(15) | 0.0064(14) |
| C5 | 0.0324(17) | 0.0390(17) | 0.0343(16) | 0.0113(14) | 0.0132(14) | 0.0065(14) |
| C6 | 0.0391(18) | 0.0334(17) | 0.0339(17) | 0.0099(14) | 0.0161(14) | 0.0088(14) |
| C7 | 0.056(2) | 0.0324(17) | 0.0362(18) | 0.0011(14) | 0.0204(16) | 0.0098(15) |
| C8 | 0.055(2) | 0.0337(18) | 0.0357(18) | -0.0020(14) | 0.0119(16) | 0.0003(15) |
| C9 | 0.0411(18) | 0.0344(17) | 0.0348(17) | 0.0030(14) | 0.0111(14) | -0.0009(14) |
| C10 | 0.0362(17) | 0.0321(16) | 0.0323(16) | 0.0038(13) | 0.0155(14) | 0.0022(13) |
| C11 | 0.0377(17) | 0.0275(15) | 0.0277(15) | 0.0079(12) | 0.0136(13) | 0.0040(13) |
| C12 | 0.0305(16) | 0.0309(16) | 0.0295(15) | 0.0085(13) | 0.0122(13) | 0.0017(12) |
| C13 | 0.0311(16) | 0.0331(16) | 0.0265(15) | -0.0019(12) | 0.0078(12) | -0.0021(12) |
| C14 | 0.0325(16) | 0.0307(16) | 0.0290(16) | 0.0010(13) | 0.0083(13) | 0.0015(13) |
| C15 | 0.0263(15) | 0.0327(16) | 0.0289(15) | 0.0031(12) | 0.0090(12) | 0.0043(12) |
| C16 | 0.0322(16) | 0.0347(17) | 0.0390(18) | -0.0078(14) | 0.0036(14) | 0.0016(13) |
| C17 | 0.0298(16) | 0.0477(19) | 0.0342(17) | -0.0106(15) | 0.0031(13) | 0.0023(14) |
| C18 | 0.0246(15) | 0.0317(16) | 0.0337(16) | 0.0031(13) | 0.0069(13) | -0.0014(12) |
| C19 | 0.0299(16) | 0.0352(17) | 0.0313(16) | 0.0063(13) | 0.0103(13) | -0.0031(13) |
| C20 | 0.0379(18) | 0.0382(18) | 0.051(2) | 0.0048(15) | 0.0194(16) | 0.0033(15) |
| C21 | 0.0245(17) | 0.050(2) | 0.073(3) | 0.0038(19) | 0.0059(17) | 0.0012(15) |
| C22 | 0.0326(18) | 0.053(2) | 0.057(2) | -0.0075(18) | -0.0052(16) | -0.0012(16) |
| C23 | 0.0317(17) | 0.0407(18) | 0.0407(18) | -0.0045(14) | 0.0021(14) | -0.0004(14) |
| C24 | 0.065(2) | 0.050(2) | 0.0325(18) | -0.0027(15) | 0.0176(17) | 0.0098(17) |

**Table 9. Hydrogen Atom Coordinates and Isotropic Atomic Displacement Parameters (Å²) for Carvedilol Hydrobromide Monohydrate.**

| | x/a | y/b | z/c | U |
|---|---|---|---|---|
| H2O | 0.086(3) | 0.471(3) | 0.155(4) | 0.047 |
| H2O' | 0.082(6) | 0.465(5) | -0.077(6) | 0.047 |
| H99A | -0.073(4) | 0.3802(19) | 0.201(6) | 0.064 |
| H99B | -0.060(4) | 0.490(2) | 0.205(6) | 0.065 |
| H99 | -0.1344(19) | 0.4409(13) | 0.157(3) | 0.065 |
| H1N | 0.373(2) | 0.2411(16) | -0.205(3) | 0.039(10) |
| H2NA | -0.043(2) | 0.4188(18) | 0.045(3) | 0.058(12) |
| H2NB | -0.036(2) | 0.422(2) | -0.060(4) | 0.077(14) |
| H2A | 0.3299 | 0.4112 | 0.1114 | 0.041 |
| H3A | 0.4497 | 0.3844 | 0.0850 | 0.048 |
| H4A | 0.4633 | 0.3119 | -0.0468 | 0.048 |
| H7A | 0.2616 | 0.1720 | -0.3395 | 0.048 |
| H8A | 0.1289 | 0.1632 | -0.3836 | 0.049 |
| H9A | 0.0548 | 0.2212 | -0.2906 | 0.044 |
| H10A | 0.1112 | 0.2912 | -0.1543 | 0.039 |
| H13A | 0.2180 | 0.4552 | 0.0713 | 0.036 |
| H13B | 0.1990 | 0.3994 | 0.1468 | 0.036 |
| H14 | 0.0925 | 0.4552 | -0.0281 | 0.037 |
| H14' | 0.0943 | 0.4596 | 0.1099 | 0.037 |
| H15A | 0.0642 | 0.3477 | -0.0132 | 0.035 |
| H15B | 0.0576 | 0.3585 | 0.1069 | 0.035 |
| H16A | -0.0819 | 0.3172 | 0.0400 | 0.043 |
| H16B | -0.0599 | 0.3103 | -0.0723 | 0.043 |
| H17A | -0.1625 | 0.3802 | -0.1496 | 0.046 |
| H17B | -0.1922 | 0.3165 | -0.1021 | 0.046 |
| H20A | -0.3977 | 0.4876 | 0.0466 | 0.049 |
| H21A | -0.4785 | 0.4439 | -0.1048 | 0.060 |
| H22A | -0.4306 | 0.3786 | -0.2183 | 0.060 |
| H23A | -0.2996 | 0.3553 | -0.1809 | 0.047 |
| H24A | -0.2310 | 0.5242 | 0.2397 | 0.072 |
| H24B | -0.3101 | 0.4858 | 0.2148 | 0.072 |
| H24C | -0.3002 | 0.5475 | 0.1455 | 0.072 |

**Table 10. Site Occupation Factors that Deviate from Unity for Carvedilol Hydrobromide Monohydrate.**

| Atom | sof | Atom | sof | Atom | sof |
|---|---|---|---|---|---|
| Br1 | 1 | Br2 | 1 | O1 | 1 |
| O2 | 0.65 | H2O | 0.65 | O2' | 0.35 |
| H2O' | 0.35 | O99A | 0.50 | H99A | 0.50 |
| O99B | 0.50 | H99B | 0.50 | H99 | 1 |
| H14 | 0.65 | H14' | 0.35 | | |

### Reference Example 9

### Form 2. Carvedilol HBr (dioxane solvate)

Form 1 is slurried in dioxane between 0 and 40°C for 2 days. The product is filtered and mildly dried.

### Reference Example 10

### Form 3. Carvedilol HBr (1-pentanol solvate)

Form 1 is slurried in 1-pentanol between 0°C and 40°C for 2 days. The product is filtered and mildly dried.

### Reference Example 11

### Form 4. Carvedilol HBr (2-Methyl-1-Propanol solvate)

Form 1 is slurried in 2-Methyl-1-Propanol between 0°C and 40°C for 2 days. The product is filtered and mildly dried.

### Reference Example 12

### Form 5. Carvedilol HBr (trifluoroethanol solvate)

Form 1 is slurried in trifluoroethanol between 0°C and 40°C for 2 days. The product is filtered and mildly dried.

### Reference Example 13

### Form 6. Carvedilol HBr (2-propanol solvate)

Form 1 is slurried in 2-propanol between 0°C and 40°C for 2 days. The product is filtered and mildly dried.

### Reference Example 14

### Form 7. Carvedilol HBr (n-propanol solvate #1)

Carvedilol free base is dissolved in n-propanol/water (95:5), and stoichiometric hydrobromic acid is added. The solution is cooled, and crystallization ensues. The product is filtered, washed with process solvent, and dried.

### Reference Example 15

### Form 8. Carvedilol HBr (n-propanol solvate #2)

Carvedilol HBr monohydrate (Form 1) is dissolved in n-propanol at ambient temperature. The n-propanol is slowly evaporated off, giving a white solid.

### Reference Example 16

### Form 9. Carvedilol HBr (anhydrous forms and solvent free)

Carvedilol free base is dissolved in a solvent (dichloromethane, isopropyl acetate, and acetonitrile have been used) and anhydrous forms HBr is added (HBr in acetic acid or gaseous HBr). The solution is cooled, and crystallization ensues. The product is filtered, washed with process solvent, and dried.

### Reference Example 17

### Form 10. Carvedilol HBr (ethanol solvate)

Carvedilol free base is dissolved in ethanol, and anhydrous forms HBr is added (HBr in acetic acid). The solution is cooled, and crystallization ensues. The product is filtered, washed with process solvent, and dried.

### Reference Example 18

### Carvedilol Monocitrate Monohydrate Preparation

In a 150 mL glass beaker, 100 gram of 20% w/w citric acid solution was prepared and 2.2 gram of carvedilol was added. The solution became slightly brownish after 15 minutes stirring, with only a little solid sticking on the bottom of the beaker. The beaker was then placed in a fume hood for evaporation. After staying in the hood overnight, large single crystals appeared in the beaker. The solid crystals were isolated and dried in a desiccator under vacuum. Similarly single crystals of citrate salt could be obtained by slow evaporation of carveditol/citric acid solutions (containing citric acid 5%, 10% or 20% w/w) in Petri dishes (150 mm diameter) placed in a desiccator connected to a house vacuum.

### Reference Example 19

### Carvedilol Monocitrate Monohydrate Preparation

A 250 mL three-necked flask equipped with stirrer bar, thermometer, and an addition funnel is charged with acetone (20 mL, 2.5 volumes). The solution is sequentially charged with carvedilol (8 g, 19.7 mmol), and 2 M citric acid solution (40 mL, 5 volumes). Upon addition of the citric acid solution, the slurry dissolves quickly. The solution is filtered through a Buchner funnel fitted with Whatman filter paper and the solution is returned to a 250 mL flask fitted with a stirrer. To the light brown solution is added water (20 mL, 2.5 volumes). No exotherm is noted. The reaction mixture becomes cloudy but disappears upon stirring (heating up to 40 °C maybe needed to remove cloudiness). The mixture is stirred at room temperature and when judged clear is charged with carvedilol monocitrate monohydrate seeds (80 mgs) in one portion. An immediate cloudiness is observed (solid starts to precipitate out over 12-24 hours). The precipitate formed is stirred for 24-48 hours and is filtered through a Buchner funnel fitted with Whatman filter paper and the collected cake is washed with water (2 x 16 mL). The cake is dried in the oven under house vacuum at 50 °C to a constant weight. The cake (7.95 g, 67 %) is weighed and stored in a polyethylene container.

### Reference Example 20

### Carvedilol Monocitrate Monohydrate Preparation

A suitable reactor is charged with acetone. The solution is sequentially charged with carvedilol, and aqueous citric acid solution. Upon addition of the citric acid solution, the slurry dissolves quickly. To the solution is added water. The mixture is stirred at room temperature and is charged with carvedilol seeds in one portion. The precipitate formed is stirred for a period of time, filtered and the collected cake is washed with water. The cake is dried under vacuum to a constant weight and stored in a polyethylene container.

### Reference Example 21

### Characterization of Carvedilol Monocitrate Monohydrate Preparation

The HPLC assay and ¹H-NMR revealed that the molar ratio of carvedilol and citric acid in carvedilol citrate salt prepared was approximately 1:1. The characterization by several other techniques are listed below:

### Scanning Electron Microscopy (SEM)

The SEM used for the study was a Hitachi S-3500N. SEM was performed using an acceleration voltage of 5 kV. The samples were gold sputtered.

The carvedilol monocitrate salt consists of crystals with plate-shape, and various sizes depending on the preparation method. Crystals as large as 1 mm width and length were observed.

### Differential Scanning Calorimetry (DSC)

DSC measurements were performed with a MDSC 2920 (TA Instruments, Inc.). Approximately 5 mg of the sample was placed in an open aluminum pan. The sample was scanned at 10 °C/min. An endothermic event was observed with an onset temperature near 82-83 °C. The heat of fusion was calculated as 63 kJ/mol.

### Fourier Transform Infrared Spectroscopy (FT-IR)

Approximately 2 mg of sample was diluted with 300 mg of dried potassium bromide (KBr). The mixture was ground with a mortar and pestle, then transferred to a die that is placed under high pressure for 3 minutes. The instrument was a PerkinElmer Spectrum GX FTIR instrument. Forty scans were collected at 4 cm⁻¹
resolution. The typical FT-IR spectrum of carvedilol monocitrate salt is shown in Figure 1. The characteristic peaks in the 1800 to 600 cm⁻¹ region are found at about 1727, 1709, 1636, 1625, 1604, 1586, 1508, 1475, 1454, 1443, 1396, 1346, 1332, 1305, 1255, 1221, 1129, 1096, 1077, 1054, 1021, 1008, 984, 939, 919, 902, 826, 787, 755, 749, 729, 676, 664, 611 cm⁻¹.

### X-Ray Powder Diffraction (XRPD)

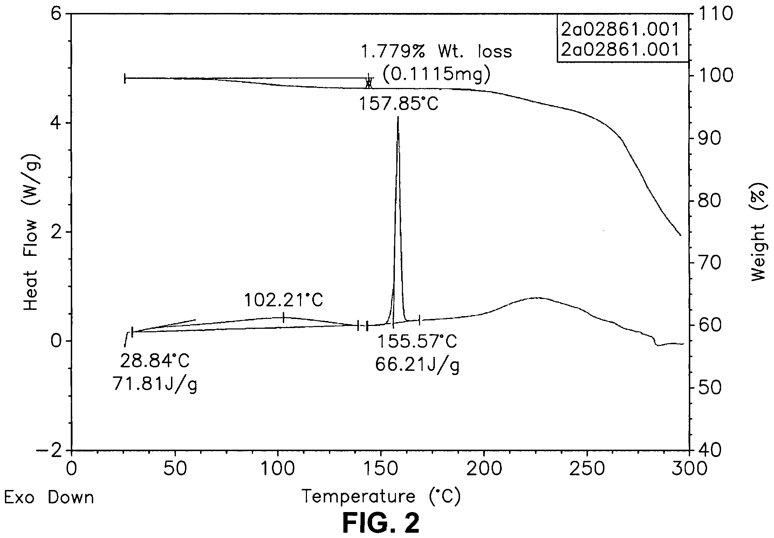

XRPD patterns were collected using a Philips X'Pert Pro Diffractometer. Approximately 30 mg of sample was gently flattened on a silicon sample holder and scanned from 2-35 degrees two-theta, at 0.02 degrees two-theta per step and a step time of 2.5 seconds. The sample was rotated at 25 rpm. The XRPD patterns of two different batches of Carvedilol monocitrate salt are shown in Figure 2.

### Solubility in Water

Glass vials containing water and excess amount of carvedilol salts were shaken by a mechanical shaker at ambient conditions. Aliquots were taken out at various time-point, filtered through 0.45 µm Acrodisc GHP filter. The pH of the filtered solutions was measured and suitable dilution was performed prior to UV-Vis analysis of carvedilol concentration.

The solubility of carvedilol monocitrate salt in water at room temperature was determined. The drug concentrations and pH values at different time-points are presented in Table 11. This crystalline form of carvedilol monocitrate salt exhibited high solubility in water (1.63 mg/mL at 1 hour and 1.02 mg/mL at 48 hour).

**Table 11: Aqueous Solubility (expressed as mg of carvedilol free base/mL of solution) over time at 25°C for Carvedilol Free Base and Its Monocitrate Salt.**

| **Time, hr** | **Carvedilol Free Base** | **Carvedilol Mono-Citrate Salt** |
|---|---|---|
| 1 | 0.0098 | 1.63 (pH=3.5) |
| 4 | | 1.47 (pH=3.4) |
| 24 | 0.0116 | 1.07 (pH=3.0) |
| 48 | | 1.02 (pH=3.0) |

Carvedilol monocitrate salt has two free carboxylic acid groups in one unit salt, which contributes the low pH value (near pH 3) observed for monocitrate salt when dissolved in water. This may potentially lead to improved formulations by providing a low pH microenvironment within the formulation as it traverses the GI tract. This may provide an environment at a molecular level that is more conductive to dissolution, particularly in the lower GI tract, where the pH of the environment is near neutral pH and the intrinsic solubility of the drug substance is limited. Such a microenvironmental pH should lead to greater dissolution rate because of higher solubility in the solid/liquid interface, leading to improved absorption of drug in the lower GI tract thereby sustaining overall absorption and, in consequence providing prolonged blood levels and allowing less frequent dosing. Therefore, a once-per-day carvedilol formulation may be possible by incorporating carvedilol monocitrate salt. Such a unit is more convenient for patients and result in higher patient compliance with the dosage regimen and hence a better therapeutic effect.

### Crystalline Structure of Carvedilol Monocitrate Salt

The crystalline structure of carvedilol citrate salt was determined by Single Crystal X-Ray Diffraction analysis on the large crystals formed by evaporation. The result indicated that the salt form was a carvedilol monocitrate, where the molar ratio of carvedilol and citric acid was 1:1. Surprisingly, the hydroxyl of carvedilol is disordered in the crystalline packing. In other words, the monocitrate salt has both R(+) and S(-) carvedilol enantiomers at 1:1 molar ratio, and the two enantiomers are randomly distributed, without any specific order.

This crystalline packing habit is very unusual for a salt formed between a chiral compound and a chiral counter-ion (monocitrate). Typically, chiral counter-ion tends to differentiate the two stereoisomers of the compound when forming crystals. However, in the case of the monocitrate salt, there seems to be enough space in the crystal packing to allow the carbonyl group of the terminal carboxylic acid group of citrate to form equivalent hydrogen bond with the hydroxyl from either the R(+) or the S(-) carvedilol stereoisomer.

This avoids generation of yet more optically active forms that could potentially complicate stability, dissolution rates and possibly in vivo absorption and pharmacologic effects.

The above data demonstrates that a novel crystalline form of carvedilol monocitrate monohydrate can be prepared with a unique crystalline packing habit, which exhibits high aqueous solubility and can provide a low pH microenvironment for enhanced dissolution.

### Reference Example 22

### Crystalline Carvedilol Benzoate Preparation

A suitable reactor is charged with acetone. The solution is sequentially charged with carvedilol (4.1 grams, 0.1 moles), and benzoic acid solution. Upon addition of the benzoic acid (1.4 grams, 0.011 moles) solution, all material dissolves into the solution. To the stirred solution is added tert-butyl methyl ether (60 ml). The precipitate formed is stirred for a period of time, filtered and the collected cake is washed with water. The cake is dried under vacuum to a constant weight and stored in a polyethylene container.

### Reference Example 23

### Crystalline Carvedilol Mandelate Preparation

A suitable reactor is charged with acetone (38 mL). The acetone solution is sequentially charged with carvedilol (11.08 grams) and water (8 mL) Upon addition of the water, the slurry dissolves completely with heating. To the solution, 1N Mandelic acid in methanol (1 Equiv. 27.3 mL.) is added. The resulting mixture is stirred at the range between 17°C and 35 °C, and the solid precipitate is formed over 10 hours to 24 hours. Later, the mixture filtered and the cake is washed with a mixture of acetone and water (10 to 1) at 3 volumes or 33 mL. The cake is then dried under vacuum to a constant weight. The final weight is 8.34 g, 54,5% yield.

### Reference Example 24

### Crystalline Carvedilol Lactate Preparation

A suitable reactor is charged with acetone (50 mL). The acetone solution is sequentially charged with carvedilol (15.0 grams) and water (7 mL). Upon addition of the water, the slurry dissolves completely with heating. To the solution is added 1N aqueous D, L-Lactic acid (1 equiv., 36.9 mL). The reaction mixture is stirred at between 17°C and 35 °C and seeded in one portion. The solid precipitate is formed over 10 hours to 24 hours. Later, the mixture is filtered and the cake is washed with a mixture of acetone and water (10 to 1) at 2 volume or 30 mL. The cake is dried under vacuum to a constant weight. The final weight is 9.16 grams.

### Reference Example 25

### Crystalline Carvedilol Sulfate Preparation

A suitable reactor is charged with acetone (38 mL). The acetone solution is sequentially charged with carvedilol (10.25 grams) and water (6 mL). Upon addition of the water, the slurry dissolves completely with heating. To the solution, 1N aqueous sulfuric acid (1 equiv., 25.2 mL) is added. The reaction mixture is stirred at between 17°C and 35 °C. and the solid precipitate is formed over 10 hours to 24 hours. Later, the mixture is filtered and the cake is washed with a mixture of acetone and water at 2 volumes or 20.5 mL. The cake is then added a mixture of acetone and water (10 to 1) for ripening between 20 °C - 35°C over 24 hours to 48 hours. The slurry is filtered and the cake is dried under vacuum to a constant weight. The final weight is 5.48 grams.

### Reference Example 26

### Crystalline Carvedilol Maleate Preparation

A suitable reactor is charged with acetone (56 mL). The acetone solution is sequentially charged with carvedilol (15.0 grams) and water (8 mL). Upon addition of the water, the slurry dissolves completely with heating. To the solution is added 1M of aqueous Maleic acid (1 Equiv. 36.9 mL.) The reaction mixture is stirred at between 17°C and 35 °C. The solid precipitate is formed over 10 hours to 24 hours. Later, the mixture is filtered and the cake is washed with a mixture of acetone and water (10 to 1) at 3 volume or 45.0 mL. The cake is dried under vacuum to a constant weight. The final weight is 14.08 grams.

### Reference Example 27

### Crystalline Carvedilol Glutarate Preparation

A suitable reactor is charged with 2 grams of carvedilol and a mixture of acetone and water (in a 7 to 1 ratio) at 8 mL. The contents were warmed to 35°C to 40 °C to a clear solution. 1N *D,L*-Glutaric acid in water (1 equivalent. 4.9 mL.) is added to the solution. The resulting mixture is stirred at the temperature between 17°C and 35°C until the solid precipitate is formed over 10 hours to 24 hours. Subsequently, the mixture filtered and the cake is washed with a mixture of acetone and water (in a 10 to 1) at about 5 mL. The cake is then dried under vacuum to a constant weight. The final weight is 1.35 grams.

### Example 28

### Solubility Enhancement in the GI tract

### Background:

Drug absorption following oral dosage requires that drug first dissolves in the gastro-intestinal milieu. In most cases such dissolution is primarily a function of drug solubility. If solubility is affected by pH it is likely that absorption will vary in different regions of the gastro intestinal tract, because pH varies from acidic in the stomach to more neutral values in the intestine.

Such pH-dependent solubility can complicate dosage form design when drug absorption needs to be prolonged, delayed or otherwise controlled, to evince a sustained or delayed action effect. Variations in solubility can lead to variable dissolution, absorption and subsequent therapeutic effect.

Carvedilol is a drug used to treat hypertension and congestive heart failure, being usually administered twice daily. For chronic diseases such as these a once-daily dosage regimen is desirable, to enhance patient compliance and reduce "pill burden". However, the dose response and time course of carvedilol in the body is such that a conventional dosage form, releasing all the drug immediately on ingestion does not provide once-a-day therapy. Release from the dosage form needs to be slowed down so that absorption and subsequent systemic residence is prolonged. This however requires that release and dissolution occurs along the GI tract, not just in the stomach.

The pH-dependent solubility of the currently used form of carvedilol (free base) is such that, while gastric solubility is adequate, solubility is much poorer at pH values encountered in the small intestine and beyond (see, Figure 126), which depicts a pH-solubility profile for carvedilol.

Consequently, while drug dissolution rate and extent from an immediate release dosage form is likely to be acceptable (such dissolution occurring in the stomach) it could be inadequate in regions beyond the stomach, with absorption compromised as a consequence.

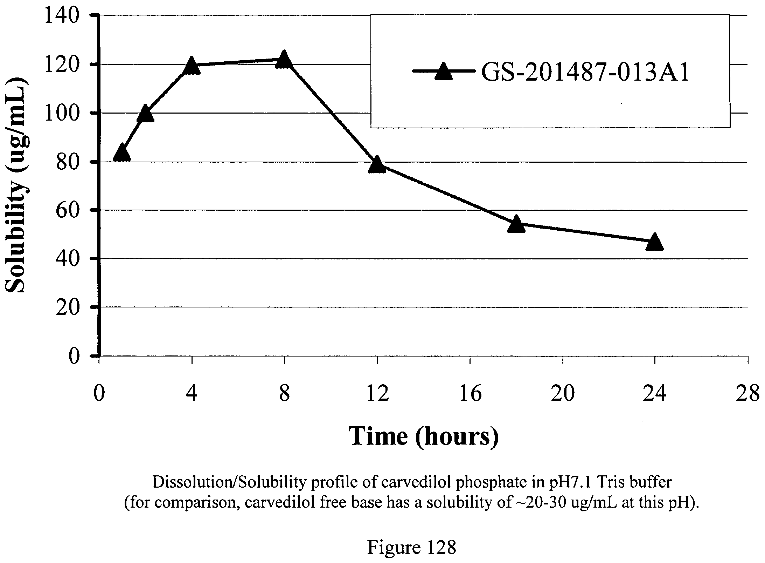

However, when drug is administered as a solution (in cyclodextrin in this example), directly to the colon it can be seen that absorption is significantly improved (Figure 128, which depicts mean plasma profiles in beagle dogs following intra-colonic administration of a carvedilol solution containing Captisol or carvedilol in aqueous suspension.). All this information suggests that absorption throughout the GI tract could be significant, provided that drug can be solubilized.

Moreover, solubilization may mean that drug stability is compromised.

The secondary amino group of carvedilol is prone to chemically react with excipients normally included in a dosage form to aid manufacture, maintain quality or enhance dissolution rate. For example, this type of amine groups can react with aldehydes or ester functional groups through nucleophilic reactions. Many excipients have ester functional groups. Furthermore, aldehydes and other such residues are common residues in excipients. This often results in marginal or unacceptable chemical stability of conventionally formulated carvedilol dosage forms, where drug is simply blended with excipients before being compressed to tablets. As drug-excipient interactions are likely to be even faster in the solvated state it follows that solubilization does not provide facile resolution of dissolution-limited absorption challenges. This is illustrated in Table 12. Solutions of carvedilol in oleic acid degraded rapidly. Other approaches to solubilization evince the same effect. Thus solubilization might enhance absorption but is not a practical approach because of the destabilizing effect.

**Table 12 Drug content (mg/g) in carvedilol/Oleic acid solution during storage.**

| | Initial | 1 month at 25°C | 3 months at 25°C |
|---|---|---|---|
| 7.788%w/w carvedilol solution in Oleic acid | 76.6 | 71.3 | 64.3 |

It has now been unexpectedly shown that salts of carvedilol afford significant improvement in absorption from the lower GI tract in dogs over that seen when carvedilol base is used. There is no reason to believe that this surprising effect does not also apply to humans and it may be feasible as a consequence to design dosage forms that enable drug to be absorbed as the unit traverses the gastrointestinal tract. This ought provide more gradual absorption and prolonged plasma profiles that facilitate once-a-day dosage.

The better absorption may be partially due to the better solubilities of salts of carvedilol. It can be seen from the data in Table 13 that citrate, hydrobromide and phosphate salts have much better aqueous solubility than the free base.

**Table 13 Aqueous Solubility (expressed as mg of Carvedilol free base/mL of solution) at 25°C for Carvedilol free base and three salts.**

| **Time** | **Free Base** | **Citrate salt** | **Phosphate salt** | **HBr salt** |
|---|---|---|---|---|
| 1 hr | -- | 1.64 (pH=3.3) | 2.35 (pH=3.0) | 0.62 (pH=6.1) |
| 4 hr | -- | 1.74 (pH=3.2) | 2.25 (pH=3.0) | 0.61 (pH=6.3) |
| 24 hr | 0.024 (pH=7.0) | 1.46 (pH=3.2) | 2.21 (pH=3.0) | 0.61 (pH=6.2) |

Ostensibly, it can be claimed that these acidic salts simply generate low pH when dissolved in water (Table 13), leading to solubility enhancement (because of the pH/solubility relationship shown in Figure 126). However, it is also possible that any pH-lowering effect contributed by the modest amounts of drug (that would be included in a dosage form to provide a therapeutic effect) would be readily swamped in the *in vivo* situation, with pH soon reverting to that of the general intestinal milieu. Consequently, any short term solubilization would be quickly negatived. However, it has been surprisingly shown that when pH is adjusted to neutral, the solubilities of salts remain higher than free base for a significant period, rather than equilibrating rapidly. Such prolonged solubility could be crucial in vivo, allowing dissolution and absorption to occur more readily at neutral pH than for free base (Figure 128, which depicts dissolution/solubility profile of carvedilol phosphate in pH = 7.1 Tris buffer (for comparison, carvedilol free base has a solubility of ∼20-30 ug/mL at this pH).

Furthermore, it has been shown that, if carvedilol salts are dissolved in solubilizing agents, stability is much better than when free base is used in the same system (Table 14). Thus, if solubilizing agents were to be required in the formulation, to provide even greater solubility enhancement, salts would be preferred to the base because of such better stability.

**Table 14 Chemical stability data of carvedilol/Vitamin E TPGS granulation containing carvedilol free base or carvedilol HBr salt.**

| Formulation | Assay/Impurity after 1 month's storage at 40°C/75%RH (open vials) | |
|---|---|---|
| | %of initial level * | Total Impurities (% peak area) |
| Carvedilol free base granulation containing Vitamin E TPGS (Lot 200412-144) | 81.5* | 7.77 |
| Carvedilol HBr salt granulation containing Vitamin E TPGS (Lot 200746-102) | 89.9* | 0.15 |

| | | |
|---|---|---|
| * Lower % of nominal due to additional moisture in the system. | | |

The foregoing facts and considerations suggest but do not provide conclusive proof that forms of carvedilol with superior solubility, whether effected by using a solvent to dissolve carvedilol base, or by using a carvedilol salt have better potential than conventionally formulated base for prolonged absorption along the GI tract. To provide stronger evidence that solubilization enhances absorption, formulations containing carvedilol base, formulated in a conventional manner, and also fully solvated by dissolving in n-methyl pyrrolidone were dosed to beagle dogs in units that were activated to make drug available after the dosage unit had passed the pyloric sphincter separating the stomach from the duodenum. Intestinal absorption efficiency was determined by monitoring plasma levels of carvedilol following such dosage. Results are provided in Table 5 and Figure 128 (which depicts mean plasma profiles in beagle dogs following oral administration of the formulations listed in Table 15).

**Table 15 Pharmacokinetic values following dosage of 10 mg carvedilol (base) to three fasted beagle dogs.**

| **Formulation** | **Solubility in pH 6.8 Phosphate Buffer Over 4-hour Period (ug/mL)** | **Cmax (ng/mL)** | **Tmax (min)** | **AUC (0-t) (ug.min/mL)** |
|---|---|---|---|---|
| Carvedilol Pharmasolve® Granulation | 86 - 120 | 31.32 ± 3.43 (n=3) | 15^{b.}, 30, 45^{a} (n=3) | 4.03 ± 1.34 (n=3) |
| Carvedilol Vitamin ETPGS Granulation | 108 - 94 | 16.26 ± 1.20 (n=3) | 30, 120, 45^{a} (n=3) | 2.75 ± 0.55 (n=3) |
| Carvedilol in conventional granules | 29 - 36 | 13.08, 12.74, 2.89^{a} (n=3) | 45, 30, 120^{a} (n=3) | 2.14, 1.19, 0.60^{a} (n=3) |

| | | | | |
|---|---|---|---|---|
| ^{a.} = values listed individually due to large variability; animals always listed in the same order. AUC(0-t) refers to the area from time 0 to the last quantifiable concentration. ^{b.} = Pharmasolve® capsule was leaking slightly before firing in-vivo. | | | | |

It can be seen that, when drug was fully dissolved absorption was rapid and high, contrasting with lower concentrations in dogs that were dosed intraduodenally with base in a conventional solid dosage unit. These findings indicated that bioavailability from carvedilol base in the small intestine is constrained by its low solubility at neutral pH. When units are introduced to the stomach the low gastric pH can be expected to facilitate dissolution and absorption but this will not be the case in the more neutral small intestine or beyond.

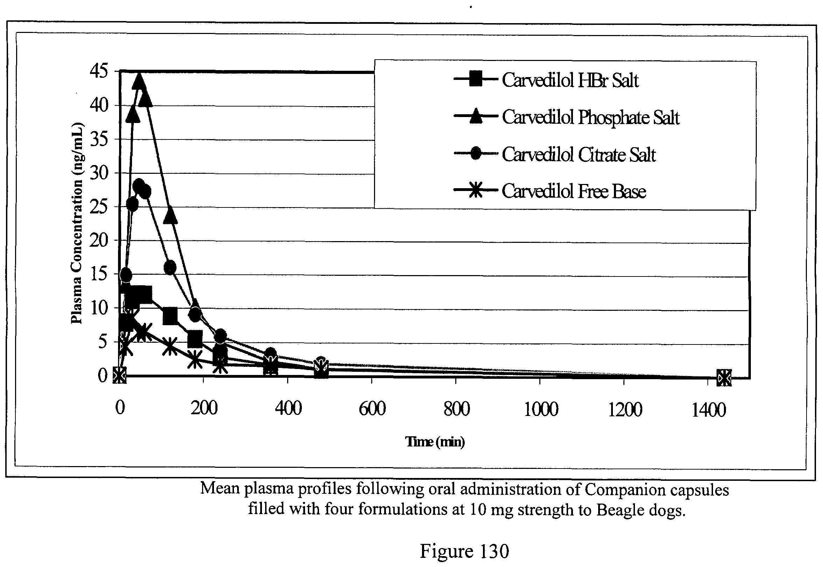

A further dog study utilized salts of carvedilol, formulated using conventional (non-solubilizing) excipients. The mode of dosage was the same as for the first dog study, the formulations being delivered such that drug did not become available until units were beyond the gastric milieu. Results are provided in Table 16 and Figure 130 (which depicts mean plasma profiles following oral administration of Companion capsules filled with four formulations at 10 mg strength to Beagle dogs).

**Table 16 Pharmacokinetic analysis of 10 mg dose formulations in three fasted beagle dogs from study.**

| **Formulation** | **Cₘₐₓ (ng/mL)** | **Tₘₐₓ (min)** | **AUC (0-t)^{a} (ug.min/mL)** | **AUC (0-inf) (ug.min/mL)** |
|---|---|---|---|---|
| Carvedilol HBr Salt granules | 12.9 ± 7.11 | 45 ± 15 | 2.22 ± 1.37 | 2.35 ± 1.46 |
| Carvedilol Phosphate Salt Granules^{b} | 61.8, 28.4 | 45, 60 | 6.69, 4.56 | 6.75, 4.90 |
| Carvedilol Citrate Salt Granules | 30.4 ± 16.9 | 45 ± 15 | 4.41 ± 2.43 | 4.66 ± 2.54 |
| Carvedilol Base Granulesx^{c} | 13.08, 12.74, 2.89 | 45, 30, 120 | 2.14, 1.19, 0.60 | --- |

| | | | | |
|---|---|---|---|---|
| ^{a.} AUC(0-t) refers to the area from time 0 to the last quantifiable concentration ^{b.} n=2 only, due to malfunction of one InteliSite® Companion capsule; animals always listed in the same order ^{c.} data from dog study DI01251; values listed individually due to large variability; animals always listed in the same order. | | | | |

The findings from the second dog study, illustrated graphically in Figure 130 conclusively showed that drug, administered in salt form was rapidly and more completely absorbed than the free base form.

### Example 29

The present invention relates to dosage forms of carvedilol to match drug delivery with pharmacodynamic requirements

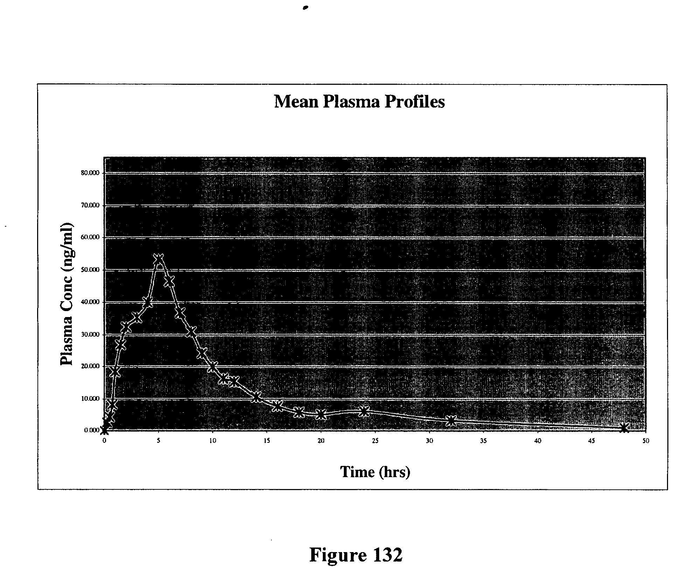

Accordingly the present invention provides a unit dose composition that comprises:
Example B. A second controlled release component, providing a peak plasma level about 5-8 hours post-dose. Figure 132 is a plasma profile from capsules formulated according to the Example].

Units, formulated as described in two examples described above have been evaluated for their biopharmaceutical profiles in human subjects and provide the requisite substantially biphasic pulsed profiles.

It can be seen that both types of dosage form provided distinctive substantially biphasic profiles, and timecourses aligned with those defined in earlier discussions..

### Example 30

### Dosage forms utilized in PK Studies

Dosage forms comprised capsules containing a mixture of beads (pellets) to provide rapid and delayed release components. The delayed release effect was provided by a pH or time-mediated mechanism. Rapid release was attained by having no release barrier in one population of pellets.

### Table 21: Capsule composition

### 1. Immediate Release pellets

Carvedilol Free base*
Cellulose microspheres (Cellsphere CP-303
Polyvinyl Pyrollidone (PVP)

### Modified Release Pellets

Carvedilol Phosphate
Cellulose microspheres (Cellsphere CP-303
Polyvinyl pyrollidone (PVP)
Cross Linked PVP (Plasdone)
Cremophor RH 40
Methacrylic acid polymer (Eudragit L100-55)
Hydrogenated vegetable oil.

### Manufacture

### Immediate-Release Pellets:

An aqueous suspension of drug and PVP was sprayed on to fluidized cellulose pellets in a stream of warm air.

### Modified Release Pellets:

A mixture of drug, PVP, Cremophor RH40 and cross linked PVP was sprayed on fluidized cellulose microspheres to provide a layer of drug on the pellets. The fluidized pellets were then coated, using either a suspension of Methacrylic acid polymer (Eudragit L100-55) and hydrogenated vegetable oil in isopropyl alcohol, providing an acid-insoluble coat, or a coat comprising ethylcellulose, dibutyl sebacate and PVP to retard release of drug from the pellet over time.

The level of coat applied was varied in different batches to determine the impact on in vivo absorption.

Pellets were filled in to capsules in differing ratios of immediate and delayed release forms to determine the impact on plasma profiles. Details are shown in Table 22.

**Table 22**

| **Formulation** | **Coat** | **Ratio IR to DR** | |
|---|---|---|---|
| | **Type** | **level on pellet** | **in capsule (%)** |
| B | ethylcellulose | 9% | 30/70 |
| D | " | 12% | " |
| E | Eudragit | 18% | " |
| F | " | 22% | " |
| G | " | 25% | 40/60 |

A Phase 1 volunteer study was performed to determine the impact of various formula variants on in vivo performance.

### Example 31

A controlled release composition, dosage form or formulation of the present invention is formed from mixtures of the following components:
[1] pellets, granules or microparticles, which comprise an active carvedilol free base or a carvedilol salt, solvate or anhydrous form thereof formulated to release the drug relatively quickly, but not immediately, as there may be a time delay in release;
[2] pellets, granules or microparticles may be prepared by granulating the drug with a hydrophilic polymer such as cellulose ethers, which may include, but is not limited to methyl cellulose, hydroxypropyl methylcellulose, polyvinyl pyrrolidone and the like. Alternatively, the drug may be applied to a base granule in an aqueous slurry with hydrophilic polymers, followed by subsequent drying to provide an embedded drug/matrix pellet;
[3] pellets, granules or microparticles also may be coated with or containing the drug embedded in a polymer, which prevents release at the more acid pH values encountered in the gastrointestinal tract, but which effect ready release of drug, after a defined time has elapsed or when the pellet, granule or microparticle unit is in an environment of more neutral pH. Examples of suitable coating or release modifying materials, may include, but are not limited to methacrylic acid polymers, shellac or cellulose acetate phthalate or mixtures thereof and the like; or
[4] pellets, granules or microparticles coated with or containing embedded drug as described in [3], but which release drug at higher pH, such as a pH of about 6.5 to a pH of about 7.5, prepared using identical or similar coating materials as in [3] such that coating material ratios may be varied.

In light of the foregoing, the early releasing component would be formulated to start releasing drug shortly after dosing (i.e., when a pellet, granule or microcapsule unit enters the stomach) to provide a "pulse", peaking at about 1 hour to about 3 hours. At a later time, the more slowly releasing formulated components release drug in parts of the small intestine, where the associated polymer coat or matrix is soluble.

The overall dose of drug and ratios of the different pellets, granules or microparticles can be determined by studies in human volunteers to examine plasma levels for at least 24 hours after dosage.

Examples of formulations and modes of preparation as described above are exemplified below:

### (i) Early Releasing Pellets

| | |
|---|---|
| carvedilol phosphate hemihydrate | 10mg* |
| polyvinyl pyrrolidone (PVP) | 2mg* |

| | |
|---|---|
| *quantities refer to levels contained in the final dosage form. The active ingredient content is expressed as the equivalent of anhydrous phosphate salt. The amounts of the non-active ingredients are approximate. | |

Early releasing pellets, granules or microparticles are prepared by spraying a liquid aqueous suspension of the drug and PVP onto cellulose pellets, fluidized in a stream of warmed air. The solvent is removed during fluidization, providing free flowing beads, which may contain, but is not limited to containing 15-30% of drug, although other drug loading levels also are acceptable.

### (ii) Delayed Release Pellets- Type I

| | |
|---|---|
| carvedilol phosphate hemihydrate | 30mg* |
| cross linked PVP (Polyplasdone XL10) | 30mg* |
| PVP | 20mg* |
| polyoxylated hydrogenated castor oil (Cremophor RH40) | 2mg* |

| | |
|---|---|
| *quantities refer to levels contained in the final dosage form. The active ingredient content is expressed as the equivalent of anhydrous phosphate salt. The amounts of the non-active ingredients are approximate. | |

Delayed release pellets, granules or microparticles - Type I are prepared by spraying a liquid suspension of above-identified components onto cellulose pellets, fluidized in a stream of warmed air. The solvent is removed during fluidization providing free flowing beads, which may contain, but is not limited to 10-50% of drug (although other drug inclusion levels are also acceptable). Such pellets, granules or microparticles are then coated, using conventional fluidization and spraying technology, with a suspension containing methacrylic acid co polymer (Eudragit L100 55) and hydrogenated cottonseed oil (Lubritab) in a suitable ratio that is determined by monitoring release rate in vitro.

### (iii) Delayed Release Pellets - Type II

| | |
|---|---|
| carvedilol phosphate hemihydrate | 40mg* |
| cross linked PVP | 12mg* |
| PVP | 12mg* |
| Polyoxylated hydrogenated castor oil (Cremophor RH40) | 3mg* |

| | |
|---|---|
| *quantities refer to levels contained in the final dosage form. The active ingredient content is expressed as the equivalent of anhydrous phosphate salt. The amounts of the non-active ingredients are approximate. | |

Delayed release pellets, granules or microparticles - Type II are prepared by spraying a liquid suspension of the above-identified components onto cellulose pellets, fluidized in a stream of warmed air. The solvent is removed during fluidization providing free flowing beads, which may contain, but is not limited to 10-20% of drug (although other drug inclusion levels are also acceptable). The pellets, granules or microparticles are then coated, using identical technology to above with a suspension, comprising a mixture of methacrylic acid copolymers *viz* Eudragit L 55 (25%) and Eudragit S 100 (35%) and Lubritab (40%)

### (iv) Capsule Formation

Mixtures of the three types of pellets, granules or microgranule types as identified above either are blended and filled into capsules or filled directly into capsules to provide the requisite overall dosage.

It will be obvious to those skilled in the art that differing levels of each type of pellet, granule or microparticle or corresponding populations may be employed to provide different doses and ratios of each of the differently releasing pellet, granule or microparticle units to provide the requisite drug-plasma-time profiles.

### Example 32

### Biopharmaceutical Performance of Units prepared according to Example 32

In accordance with the present invention, capsules were formulated according to descriptions as in Example 32, where each capsule contain a total dose of 80mg carvedilol phosphate (anhydrous equivalent) divided according to Table 23 below.

**Table 23**

| **Pellet type** | **Dose (Carvedilol pfb equivalent)** |
|---|---|
| early releasing pellets | 7.5 mg |
| delayed release pellets I | 22.5 mg |
| delayed release pellets II | 30.0 mg |

Capsules of the present invention were evaluated in a study in after administration to human volunteers to determine plasma profiles. Volunteers were administered one capsule, after food. Plasma samples were withdrawn at regular intervals over a several hour period (i.e., such as a 24 hour period), for determination of drug content, thereby enabling profiles to be constructed. To provide comparative data in the present study, one conventional, immediate release dosage form (commercial Coreg® Tablet) containing 25mg of drug, was dosed twice, at an interval of 12 hours (giving a total dose of 50mg).

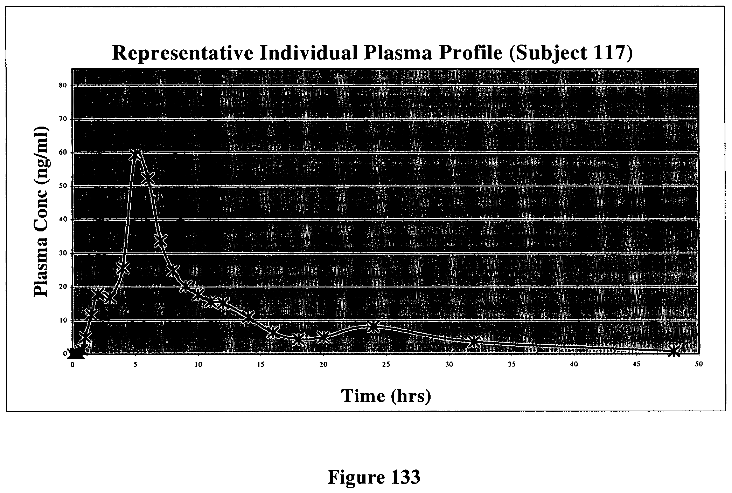

Figure 133 shows mean plasma profiles of subjects for a formulation of the present invention described in Table 23.

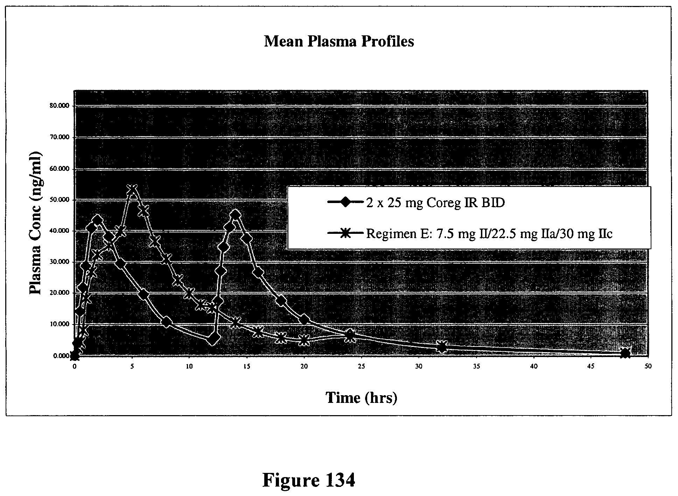

As mean values tend to obscure profiles to some extent (because of intrinsic intersubject variation) a representative profile from a single subject is shown in Figure 134 to illustrate more clearly the substantially biphasic nature of a plasma profile from the formulation described in Example 32/Table 23.

Figure 135 compares the profiles for the test product (mean values as in Figure 133) with those obtained form the conventional (immediate release) product dosed twice daily. It is noteworthy that the test product has comparable plasma levels to the conventional product at the 24-hour timepoint, indicating that efficacy will or should be maintained for a once-daily dosage interval.

In summary, mean and individual profiles of the present invention exemplify or indicate that a single dose of a test controlled release formulation delivered a plasma profile incorporate the following characteristics:
a more gradual release of drug at the early stages than the conventional or current commercially available COREG® product;
a first plasma concentration peak about 1 hour to about-3 hours after dosage of a formulation of the present invention and a second plasma concentration peak after about 5 hours to 10 hours after dosage of a formulation of the present invention; and
levels at 24 hours that were comparable to those obtained after twice daily dosage of the current commercial product COREG®.

Thus, data from dosage to humans show that the required plasma level profile is attainable with this dosage form.

It is to be understood that the invention is not limited to the embodiments illustrated hereinabove and the right is reserved to the illustrated embodiments and all modifications coming within the scope of the following claims.

## Claims

1. A controlled-release microparticle composition, formulation or dosage form, which comprises:
a tri-component controlled-release microparticle composition, formulation or dosage form formed from:
[1] a first rapidly releasing microparticle population comprised of microparticle rapidly releasing granules;
wherein each microparticle rapidly releasing granule contains a layer comprised of a carvedilol phosphate salt, solvate, or anhydrous form thereof, at least one nitrogen containing polymer and a plasticizer or other pharmaceutically acceptable excipients applied to a cellulose sphere or pharmaceutically acceptable core; and
[2] a first controlled release microparticle population comprised of first controlled release microparticle granules;
wherein each first controlled release microparticle granule contains a layer comprised of a carvedilol phosphate salt, solvate, or anhydrous form thereof, at least one nitrogen containing polymer and a plasticizer or other pharmaceutically acceptable excipients applied to a cellulose sphere or pharmaceutically acceptable core;
wherein each first controlled release microparticle granule is coated with at least one release controlling coating layer(s) formed from a film forming polymer and a plasticizing agent to form each first controlled release microparticle; and
wherein each first controlled release microparticle granule triggers a release of the carvedilol phosphate, salt, solvate, or anhydrous form thereof at a pH of 5.5;
[3] a second controlled release microparticle population comprised of second controlled release microparticle granules;
wherein each second controlled release microparticle granule contains a layer comprised of a carvedilol phosphate salt, solvate, or anhydrous form thereof, at least one nitrogen containing polymer and a plasticizer or other pharmaceutically acceptable excipients applied to a cellulose sphere or pharmaceutically acceptable core;
wherein each second controlled release microparticle granule is coated with at least one release controlling coating layer(s) formed from a film forming polymer and a plasticizing agent to form each second controlled release microparticle; and
wherein each second controlled release microparticle granule triggers a release of the carvedilol phosphate salt, solvate, or anhydrous form thereof at a pH of pH > 6.4; and
wherein each first rapidly releasing population, first controlled release microparticle population and second controlled release microparticle population are admixed with pharmaceutically acceptable adjuvants, carriers or excipients to form the tri-component controlled-release microparticle composition, formulation or dosage form.

2. The controlled-release microparticle composition, formulation or dosage form, according to claim 1, wherein each first rapidly releasing microparticle population, first controlled release microparticle population and second controlled release microparticle population are in a 1: 3: 4 active drug content ratio.

3. The controlled-release microparticle composition, formulation or dosage form according to claim 1, wherein the carvedilol phosphate salt, solvate or anhydrous form is selected from the group consisting of carvedilol hydrogen phosphate, carvedilol dihydrogen phosphate, carvedilol dihydrogen phosphate hemihydrate, carvedilol dihydrogen phosphate dihydrate, carvedilol dihydrogen phosphate methanol solvate.

4. The controlled-release microparticle composition, formulation or dosage form according to claim 2, wherein the carvedilol phosphate salt, solvate or anhydrous form is carvedilol dihydrogen phosphate hemihydrate.

5. The controlled-release microparticle composition, formulation or dosage form according to claim 1, wherein:
each first rapidly releasing microparticle granule contains a carvedilol phosphate salt, solvate, or anhydrous form thereof contained in a dosage amount of 1.25 mg to 10 mg; and
each first controlled release microparticle granule contains a carvedilol phosphate salt, solvate, or anhydrous form thereof contained in a dosage amount of 10 mg to 80 mg.

6. The controlled-release microparticle composition, formulation or dosage form according to claim 1, wherein a total dosage amount of the carvedilol phosphate salt, solvate, or anhydrous form thereof dosage amount contained in a sum of each first rapidly releasing microparticle granule, a first controlled release microparticle granule and a second controlled release microparticle granule is the sum of the total dosage amount between 10 mg to 80 mg.

7. The controlled-release microparticle composition, formulation or dosage form according to claim 1, wherein each first controlled release microparticle granule and each second controlled release microparticle granule further contain swelling agents selected from crosslinked polyvinylpyrrolidones, crosslinked carboxyalkylcelluloses, hydrophilic polymers of high molar mass, hydroxyalkylcelluloses, carboxyalkylcellulose, modified starch, starch, cellulose, sodium alginate, potassium polacriline, or corresponding blends thereof.

8. The controlled-release microparticle composition, formulation or dosage form according to claim 1, wherein the nitrogen-containing polymer in each first controlled release microparticle granule and second controlled release microparticle granule is a swelling agent.

9. The controlled-release microparticle composition, formulation or dosage form according to claim 1, wherein the nitrogen containing polymers are selected from polyvinyl pyrrolidone (povidone or PVP), or cross-linked polyvinyl pyrrolidone (cross-linked povidone).

10. The controlled-release microparticle composition, formulation or dosage form according to claim 1, wherein the nitrogen containing polymer in each first controlled release microparticle granule and in each second controlled release microparticle granule is a combination of polyvinyl pyrrolidone (povidone or PVP), or cross-linked polyvinyl pyrrolidone (cross-linked povidone).

11. The controlled-release microparticle composition, formulation or dosage form according to claim 7, wherein in each of the first microparticle rapidly releasing granules, the first controlled release microparticle granules and the second controlled release microparticle granules, the plasticizer or the other pharmaceutically acceptable excipients selected from surface-active or lubricating agents are selected from castor oil, diethyl phthalate, triethyl citrate, salicylic acid, magnesium stearate, sodium oleate or polyoxyethylenated sorbitan laurate.

12. The controlled-release microparticle composition, formulation or dosage form according to claim 1, wherein at least one release controlling layer is formed from at least one film forming polymer and a plasticizing agent in a ratio from about 60% (w/w) to about 40% (w/w).

13. The controlled-release microparticle composition, formulation or dosage form according to claim 1, wherein the film forming polymer in at least one release controlling coating layer(s) of each first controlled release microparticle granule is selected from a polymethylmethacrylate polymer.

14. The controlled-release microparticle composition, formulation or dosage form according to claim 1, wherein the plasticizing agent in at least one release controlling coating layer(s) of each first controlled release microparticle is selected from a hydrogenated vegetable oil, propan 2-ol or propylene glycol, diethyl phthalate or other pharmaceutically acceptable materials.

15. The use of a controlled-release microparticle composition, formulation or dosage form according to claim 1, in the manufacture of a medicament for treating cardiovascular diseases selected from the group consisting of hypertension, atherosclerosis, congestive heart failure and angina.

16. A controlled-release microparticle composition, formulation or dosage form according to claim 1 for use in the treatment of cardiovascular diseases selected from the group consisting of hypertension, atherosclerosis, congestive heart failure and angina.

## Patentansprüche

1. Mikropartikelzusammensetzung, -formulierung oder -dosierungsform mit kontrollierter Freisetzung, welche umfasst:
eine Dreikomponenten-Mikropartikelzusammensetzung, -formulierung oder -dosierungsform mit kontrollierter Freisetzung, gebildet aus:
[1] einer ersten rasch freisetzenden Mikropartikelpopulation, umfassend rasch freisetzende Mikropartikelkörnchen;
wobei jedes rasch freisetzende Mikropartikelkörnchen eine Schicht enthält, die ein Carvedilolphosphatsalz, Solvat oder wasserfreie Form davon, wenigstens ein stickstoffhaltiges Polymer und ein Plastifiziermittel oder andere pharmazeutisch annehmbare Hilfsstoffe, die auf einer Cellulosekugel oder einem pharmazeutisch annehmbaren Kern aufgebracht sind, umfasst;
[2] einer ersten Mikropartikelpopulation mit kontrollierter Freisetzung, umfassend erste Mikropartikelkörnchen mit kontrollierter Freisetzung;
wobei jedes erste Mikropartikelkörnchen mit kontrollierter Freisetzung eine Schicht enthält, die ein Carvedilolphosphatsalz, Solvat oder wasserfreie Form davon, wenigstens ein stickstoffhaltiges Polymer und ein Plastifiziermittel oder andere pharmazeutisch annehmbare Hilfsstoffe, die auf einer Cellulosekugel oder einem pharmazeutisch annehmbaren Kern aufgebracht sind, umfasst;
wobei jedes erste Mikropartikelkörnchen mit kontrollierter Freisetzung mit wenigstens einer freisetzungskontrollierenden Beschichtungsschicht(en), gebildet aus einem filmbildenden Polymer und einem Plastifizierungsmittel, beschichtet ist, so dass ein erstes Mikropartikel mit kontrollierter Freisetzung gebildet wird; und
wobei jedes erste Mikropartikelkörnchen mit kontrollierter Freisetzung eine Freisetzung des Carvedilolphosphats, Salzes, Solvats oder der wasserfreien Form davon bei einem pH-Wert von 5,5 auslöst;
[3] einer zweiten Mikropartikelpopulation mit kontrollierter Freisetzung, umfassend zweite Mikropartikelkörnchen mit kontrollierter Freisetzung;
wobei jedes zweite Mikropartikelkörnchen mit kontrollierter Freisetzung eine Schicht enthält, die ein Carvedilolphosphatsalz, Solvat oder wasserfreie Form davon, wenigstens ein stickstoffhaltiges Polymer und ein Plastifiziermittel oder andere pharmazeutisch annehmbare Hilfsstoffe, die auf einer Cellulosekugel oder einem pharmazeutisch annehmbaren Kern aufgebracht sind, umfasst;
wobei jedes zweite Mikropartikelkörnchen mit kontrollierter Freisetzung mit wenigstens einer freisetzungskontrollierenden Beschichtungsschicht(en), gebildet aus einem filmbildenden Polymer und einem Plastifizierungsmittel, beschichtet ist, so dass ein zweites Mikropartikel mit kontrollierter Freisetzung gebildet wird;
wobei jedes zweite Mikropartikelkörnchen mit kontrollierter Freisetzung eine Freisetzung des Carvedilolphosphatsalzes, des Solvats oder der wasserfreien Form davon bei einem pH-Wert von pH > 6,4 auslöst; und
wobei jede erste rasch freisetzende Population, erste Mikropartikelpopulation mit kontrollierter Freisetzung und zweite Mikropartikelpopulation mit kontrollierter Freisetzung jeweils mit pharmazeutisch annehmbaren Adjuvanzien, Trägern oder Hilfsstoffen vermischt sind, so dass die Dreikomponenten-Mikropartikelzusammensetzung, -formulierung oder -dosierungsform mit kontrollierter Freisetzung gebildet wird.

2. Mikropartikelzusammensetzung, -formulierung oder -dosierungsform mit kontrollierter Freisetzung gemäß Anspruch 1, wobei jede erste rasch freisetzende Mikropartikelpopulation, erste Mikropartikelpopulation mit kontrollierter Freisetzung und zweite Mikropartikelpopulation mit kontrollierter Freisetzung jeweils in einem Wirkstoffgehaltsverhältnis von 1:3:4 vorliegen.

3. Mikropartikelzusammensetzung, -formulierung oder -dosierungsform gemäß Anspruch 1, wobei das Carvedilolphosphatsalz, das Solvat oder die wasserfreie Form aus der Gruppe, bestehend aus Carvedilolhydrogenphosphat, Carvediloldihydrogenphosphat, Carvediloldihydrogenphosphat-Hemihydrat, Carvediloldihydrogenphosphat-Dihydrat, Carvediloldihydrogenphosphat-Methanolsolvat, ausgewählt ist.

4. Mikropartikelzusammensetzung, -formulierung oder -dosierungsform mit kontrollierter Freisetzung gemäß Anspruch 2, wobei das Carvedilolphosphatsalz, das Solvat oder die wasserfreie Form Carvediloldihydrogenphosphat-Hemihydrat ist.

5. Mikropartikelzusammensetzung, -formulierung oder -dosierungsform mit kontrollierter Freisetzung gemäß Anspruch 1, wobei
jedes erste rasch freisetzende Mikropartikelkörnchen ein Carvedilolphosphatsalz, Solvat oder wasserfreie Form davon in einer Dosierungsmenge von 1,25 mg bis 10 mg enthält; und
jedes erste Mikropartikelkörnchen mit kontrollierter Freisetzung ein Carvedilolphosphatsalz, Solvat oder wasserfreie Form davon in einer Dosierungsmenge von 10 mg bis 80 mg enthält.

6. Mikropartikelzusammensetzung, -formulierung oder -dosierungsform mit kontrollierter Freisetzung gemäß Anspruch 1, wobei eine Gesamtdosierungsmenge der Dosierungsmenge des Carvedilolphosphatsalzes, des Solvats oder der wasserfreien Form davon, die in einer Summe von jedem rasch freisetzenden Mikropartikelkörnchen, einem ersten Mikropartikelkörnchen mit kontrollierter Freisetzung und einem zweiten Mikropartikelkörnchen mit kontrollierter Freisetzung enthalten ist, die Summe der Gesamtdosierungsmenge zwischen 10 mg bis 80 mg ist.

7. Mikropartikelzusammensetzung, -formulierung oder -dosierungsform mit kontrollierter Freisetzung gemäß Anspruch 1, wobei jedes erste Mikropartikelkörnchen mit kontrollierter Freisetzung und jedes zweite Mikropartikelkörnchen mit kontrollierter Freisetzung ferner Quellmittel, ausgewählt aus vernetzten Polyvinylpyrrolidonen, vernetzten Carboxyalkylcellulosen, hydrophilen Polymeren mit hoher Molmasse, Hydroxyalkylcellulosen, Carboxyalkylcellulose, modifizierter Stärke, Stärke, Cellulose, Natriumalginat, Kaliumpolacrilin oder entsprechenden Mischungen davon, enthalten.

8. Mikropartikelzusammensetzung, -formulierung oder -dosierungsform mit kontrollierter Freisetzung gemäß Anspruch 1, wobei das stickstoffhaltige Polymer in jedem ersten Mikropartikelkörnchen mit kontrollierter Freisetzung und jedem zweitem Mikropartikelkörnchen mit kontrollierter Freisetzung jeweils ein Quellmittel ist.

9. Mikropartikelzusammensetzung, -formulierung oder -dosierungsform mit kontrollierter Freisetzung gemäß Anspruch 1, wobei die stickstoffhaltigen Polymere aus Polyvinylpyrrolidon (Povidon oder PVP) oder vernetztem Polyvinylpyrrolidon (vernetztem Povidon) ausgewählt sind.

10. Mikropartikelzusammensetzung, -formulierung oder -dosierungsform mit kontrollierter Freisetzung gemäß Anspruch 1, wobei das stickstoffhaltige Polymer in jedem ersten Mikropartikelkörnchen mit kontrollierter Freisetzung und in jedem zweiten Mikropartikelkörnchen mit kontrollierter Freisetzung jeweils eine Kombination von Polyvinylpyrrolidon (Povidon oder PVP) oder vernetztem Polyvinylpyrrolidon (vernetztem Povidon) ist.

11. Mikropartikelzusammensetzung, -formulierung oder -dosierungsform mit kontrollierter Freisetzung gemäß Anspruch 7, wobei das Plastifiziermittel oder die anderen pharmazeutisch annehmbaren Hilfsstoffe in den ersten rasch freisetzenden Mikropartikelkörnchen, den ersten Mikropartikelkörnchen mit kontrollierter Freisetzung und den zweiten Mikropartikelkörnchen mit kontrollierter Freisetzung jeweils ausgewählt sind aus oberflächenaktiven oder Gleitmitteln, die ausgewählt sind aus Rizinusöl, Diethylphthalat, Triethylcitrat, Salicylsäure, Magnesiumstearat, Natriumoleat oder polyoxyethyleniertem Sorbitanlaurat.

12. Mikropartikelzusammensetzung, -formulierung oder -dosierungsform mit kontrollierter Freisetzung gemäß Anspruch 1, wobei die wenigstens eine freisetzungskontrollierende Schicht gebildet ist aus wenigstens einem filmbildenden Polymer und einem plastifizierenden Mittel in einem Verhältnis von etwa 60% (G/G) bis etwa 40% (G/G).

13. Mikropartikelzusammensetzung, -formulierung oder -dosierungsform mit kontrollierter Freisetzung gemäß Anspruch 1, wobei das filmbildende Polymer in wenigstens einer freisetzungskontrollierenden Beschichtungsschicht(en) jedes ersten Mikropartikelkörnchens mit kontrollierter Freisetzung aus einem Polymethylmethacrylatpolymer ausgewählt ist.

14. Mikropartikelzusammensetzung, -formulierung oder -dosierungsform mit kontrollierter Freisetzung gemäß Anspruch 1, wobei das Plastifizierungsmittel in wenigstens einer freisetzungskontrollierenden Beschichtungsschicht(en) jedes ersten Mikropartikels mit kontrollierter Freisetzung aus einem hydrogenierten Pflanzenöl, Propan-2-ol oder Propylenglykol, Diethylphthalat oder anderen pharmazeutisch annehmbaren Materialien ausgewählt ist.

15. Verwendung einer Mikropartikelzusammensetzung, -formulierung oder -dosierungsform mit kontrollierter Freisetzung gemäß Anspruch 1 bei der Herstellung eines Arzneimittels zur Behandlung kardiovaskulärer Erkrankungen, die aus der Gruppe, bestehend aus Hypertension, Arteriosklerose, kongestivem Herzversagen und Angina pectoris, ausgewählt sind.

16. Mikropartikelzusammensetzung, -formulierung oder -dosierungsform mit kontrollierter Freisetzung gemäß Anspruch 1 zur Verwendung bei der Behandlung von kardiovaskulären Erkrankungen, die aus der Gruppe, bestehend aus Hypertension, Arteriosklerose, kongestivem Herzversagen und Angina pectoris, ausgewählt sind.

## Revendications

1. Composition, formulation ou forme posologique microparticulaire à libération contrôlée, qui comprend :
une composition, une formulation ou une forme posologique microparticulaire à trois composants à libération contrôlée formée à partir de :
[1] une première population microparticulaire à libération rapide composée de granules microparticulaires à libération rapide ;
dans laquelle chaque granule microparticulaire à libération rapide contient une couche composée d'un sel phosphate de carvédilol, de l'un de ses solvates ou de sa forme anhydre, au moins un polymère contenant de l'azote et un agent plastifiant ou d'autres excipients pharmaceutiquement acceptables appliqués sur une sphère de cellulose ou un noyau pharmaceutiquement acceptable ; et
[2] une première population microparticulaire à libération contrôlée composée de premiers granules microparticulaires à libération contrôlée ;
dans laquelle chaque premier granule microparticulaire à libération contrôlée contient une couche composée d'un sel phosphate de carvédilol, de l'un de ses solvates ou de sa forme anhydre, au moins un polymère contenant de l'azote et un agent plastifiant ou d'autres excipients pharmaceutiquement acceptables appliqués sur une sphère de cellulose ou un noyau pharmaceutiquement acceptable ;
dans laquelle chaque premier granule microparticulaire à libération contrôlée est enrobé avec au moins une couche d'enrobage contrôlant la libération formée à partir d'un polymère formant un film et d'un agent plastifiant pour former chaque première microparticule à libération contrôlée ; et
dans laquelle chaque premier granule microparticulaire à libération contrôlée déclenche une libération du sel phosphate de carvédilol, de son solvate, ou de sa forme anhydre à un pH de 5,5 ;
[3] une seconde population microparticulaire à libération contrôlée composée de seconds granules microparticulaires à libération contrôlée ;
dans laquelle chaque second granule microparticulaire à libération contrôlée contient une couche composée d'un sel phosphate de carvédilol, de l'un de ses solvates ou de sa forme anhydre, au moins un polymère contenant de l'azote et un agent plastifiant ou d'autres excipients pharmaceutiquement acceptables appliqués sur une sphère de cellulose ou un noyau pharmaceutiquement acceptable ;
dans laquelle chaque second granule microparticulaire à libération contrôlée est enrobé avec au moins une couche d'enrobage contrôlant la libération formée à partir d'un polymère formant un film et d'un agent plastifiant pour former chaque seconde microparticule à libération contrôlée ; et
dans laquelle chaque second granule microparticulaire à libération contrôlée déclenche une libération du sel phosphate de carvédilol, de son solvate, ou de sa forme anhydre à un pH > 6,4 ; et
dans laquelle chaque première population à libération rapide, première population microparticulaire à libération contrôlée et seconde population microparticulaire à libération contrôlée sont mélangées avec des adjuvants, des supports ou des excipients pharmaceutiquement acceptables pour former la composition, la formulation ou la forme posologique microparticulaire à trois composants à libération contrôlée.

2. Composition, formulation ou forme posologique microparticulaire à libération contrôlée, selon la revendication 1, dans laquelle chaque première population à libération rapide, première population microparticulaire à libération contrôlée et seconde population microparticulaire à libération contrôlée sont dans un rapport de 1/3/4 de teneur en médicament actif.

3. Composition, formulation ou forme posologique microparticulaire à libération contrôlée selon la revendication 1, dans laquelle le sel phosphate de carvédilol, le solvate ou la forme anhydre est choisi dans le groupe constitué d'hydrogénophosphate de carvédilol, dihydrogénophosphate de carvédilol, dihydrogénophosphate de carvédilol hémihydraté, dihydrogénophosphate de carvédilol dihydraté, solvate méthanolique de dihydrogénophosphate de carvédilol.

4. Composition, formulation ou forme posologique microparticulaire à libération contrôlée selon la revendication 2, dans laquelle le sel phosphate de carvédilol, le solvate ou la forme anhydre est le dihydrogénophosphate de carvédilol hémihydraté.

5. Composition, formulation ou forme posologique microparticulaire à libération contrôlée selon la revendication 1, dans laquelle :
chaque granule microparticulaire à libération rapide contient un sel phosphate de carvédilol, son solvate, ou sa forme anhydre contenu dans une quantité posologique de 1,25 mg à 10 mg ; et
chaque premier granule microparticulaire à libération contrôlée contient un sel phosphate de carvédilol, son solvate, ou sa forme anhydre contenu dans une quantité posologique de 10 mg à 80 mg.

6. Composition, formulation ou forme posologique microparticulaire à libération contrôlée selon la revendication 1, dans laquelle une quantité posologique totale de la quantité posologique du sel phosphate de carvédilol, de son solvate, ou de sa forme anhydre contenue dans une somme de chaque granule microparticulaire à libération rapide, premier granule microparticulaire à libération contrôlée et second granule microparticulaire à libération contrôlée est la somme de la quantité posologique totale située entre 10 mg et 80 mg.

7. Composition, formulation ou forme posologique microparticulaire à libération contrôlée selon la revendication 1, dans laquelle chaque premier granule microparticulaire à libération contrôlée et chaque second granule microparticulaire à libération contrôlée contiennent en outre des agents de gonflement choisis parmi des polyvinylpyrrolidones réticulées, des carboxyalkylcelluloses réticulées, des polymères hydrophiles de masse molaire élevée, des hydroxyalkylcelluloses, des carboxyalkylcelluloses, l'amidon modifié, l'amidon, la cellulose, l'alginate de sodium, la polacriline potassique, ou leurs mélanges correspondants.

8. Composition, formulation ou forme posologique microparticulaire à libération contrôlée selon la revendication 1, dans laquelle le polymère contenant de l'azote dans chaque premier granule microparticulaire à libération contrôlée et second granule microparticulaire à libération contrôlée est un agent de gonflement.

9. Composition, formulation ou forme posologique microparticulaire à libération contrôlée selon la revendication 1, dans laquelle les polymères contenant de l'azote sont choisis parmi la polyvinylpyrrolidone (povidone ou PVP), ou la polyvinylpyrrolidone réticulée (povidone réticulée).

10. Composition, formulation ou forme posologique microparticulaire à libération contrôlée selon la revendication 1, dans laquelle le polymère contenant de l'azote dans chaque premier granule microparticulaire à libération contrôlée et dans chaque second granule microparticulaire à libération contrôlée est une combinaison de polyvinylpyrrolidone (povidone ou PVP) ou de polyvinylpyrrolidone réticulée (povidone réticulée).

11. Composition, formulation ou forme posologique microparticulaire à libération contrôlée selon la revendication 7, dans laquelle chacun des premiers granules microparticulaires à libération rapide, des premiers granules microparticulaires à libération contrôlée et des seconds granules microparticulaires à libération contrôlée, de l'agent plastifiant ou des autres excipients pharmaceutiquement acceptables choisis parmi des agents tensioactifs ou lubrifiants sont choisis parmi l'huile de ricin, le phtalate de diéthyle, le citrate de triéthyle, l'acide salicylique, le stéarate de magnésium, l'oléate de sodium ou le laurate de sorbitane polyoxyéthyléné.

12. Composition, formulation ou forme posologique microparticulaire à libération contrôlée selon la revendication 1, dans laquelle au moins une couche contrôlant la libération est formée à partir d'au moins un polymère formant un film et d'un agent plastifiant dans un rapport d'environ 60 % (p/p) à environ 40 % (p/p).

13. Composition, formulation ou forme posologique microparticulaire à libération contrôlée selon la revendication 1, dans laquelle le polymère formant un film dans au moins une couche d'enrobage contrôlant la libération de chaque premier granule microparticulaire à libération contrôlée est choisi parmi des polymères de polyméthacrylate de méthyle.

14. Composition, formulation ou forme posologique microparticulaire à libération contrôlée selon la revendication 1, dans laquelle l'agent plastifiant dans au moins une couche d'enrobage contrôlant la libération de chaque première microparticule à libération contrôlée est choisi parmi une huile végétale hydrogénée, le propan-2-ol ou le propylène glycol, le phtalate de diéthyle ou d'autres matériaux pharmaceutiquement acceptables.

15. Utilisation d'une composition, d'une formulation ou d'une forme posologique microparticulaire à libération contrôlée selon la revendication 1, dans la fabrication d'un médicament destiné au traitement de maladies cardiovasculaires choisies dans le groupe constitué de l'hypertension, l'athérosclérose, l'insuffisance cardiaque congestive et l'angine de poitrine.

16. Composition, formulation ou forme posologique microparticulaire à libération contrôlée selon la revendication 1 pour une utilisation dans le traitement de maladies cardiovasculaires choisies dans le groupe constitué de l'hypertension, l'athérosclérose, l'insuffisance cardiaque congestive et l'angine de poitrine.
